(19) Europäisches Patentamt
European Patent Office
Office européen des brevets

(11) EP 1 403 283 A1

(12) **EUROPEAN PATENT APPLICATION**
published in accordance with Art. 158(3) EPC

(43) Date of publication:
**31.03.2004 Bulletin 2004/14**

(21) Application number: **02733472.1**

(22) Date of filing: **11.06.2002**

(51) Int Cl.[7]: **C07K 14/705**, A61K 38/08, C12N 15/12, A61K 38/17, A61P 35/00, C12P 21/02, C07K 16/30, G01N 33/50, G01N 33/15, C12Q 1/68

(86) International application number:
**PCT/JP2002/005799**

(87) International publication number:
**WO 2003/008450 (30.01.2003 Gazette 2003/05)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **12.06.2001 JP 2001177058**
**21.08.2001 JP 2001250728**

(71) Applicant: **Itoh, Kyogo**
**Miyaki-gun, Saga 841-0205 (JP)**

(72) Inventors:
- **ITOH, Kyogo**
  **Miyaki-gun, Saga 841-0205 (JP)**
- **SHICHIJO, Shigeki**
  **Kurume-shi, Fukuoka 830-0003 (JP)**

(74) Representative: **Krauss, Jan**
**Forrester & Boehmert,**
**Pettenkoferstrasse 20-22**
**80336 München (DE)**

(54) **TUMOR ANTIGEN**

(57) A gene encoding a tumor antigen that is capable of being recognized by and/or inducing cytotoxic T lymphocyte (CTL) in an HLA-A2 restricted manner was identified from cDNA library of human colon cancer cell line by using a gene expression cloning method, and further found was a peptide having an epitope of the tumor antigen, which is capable of being recognized by and/or inducing cytotoxic T lymphocyte (CTL) in an HLA-A2 restricted manner. In addition, a gene encoding a tumor antigen that is capable of being recognized by and/or inducing cytotoxic T lymphocyte (CTL) in an HLA-A26 restricted manner was identified from a cDNA library of human esophageal cancer cell line, and further found was a peptide having an epitope of the tumor antigen.

Further provided are a polypeptide and a peptide encoded by the above-described gene, a polynucleotide encoding any one of those or the complementary strand thereof, a recombinant vector, a transformant, an antibody against the polypeptide or the peptide, a compound that interacts with the polypeptide or the peptide and /or HLA-A2 or HLA-A26, a compound that interacts with the polynucleotide, a CTL inducer consisting of the peptide and/or polypeptide, a pharmaceutical composition comprising any one of those, a method for inducing CTL that comprises using the peptide and/or polypeptide, a method for producing the peptide and/or polypeptide, a method for screening for the compound, a method for measuring the same, and a reagent kit for use in the screening method or the measuring method.

EP 1 403 283 A1

SEQUENCE LISTING

<110> ITOH, Kyogo

<120> Tumor antigen

<130> GP02-1004PCT

<140>
<141>

<150> JP P2001-177058
<151> 2001-06-12

<150> JP P2001-250728
<151> 2001-08-21

<160> 408

<170> PatentIn Ver. 2.1

<210> 1
<211> 9
<212> PRT
<213> Artificial Sequence

<220>
<223> Description of Artificial Sequence:Designed peptide recognized by HLA-A2 restricted cytotoxic T lymphocytes

<400> 1
Lys Leu Thr Gly Met Ala Phe Arg Val
1 5

<210> 2
<211> 10
<212> PRT
<213> Artificial Sequence

<220>
<223> Description of Artificial Sequence:Designed peptide recognized by HLA-A2 restricted cytotoxic T lymphocytes

<400> 2
Ala Leu Asn Asp His Phe Val Lys Leu Ile

1 5 10

<210> 3
<211> 9
<212> PRT
<213> Artificial Sequence

<220>
<223> Description of Artificial Sequence:Designed peptide recognized by HLA-A2 restricted cytotoxic T lymphocytes

<400> 3
Ile Leu Gly Tyr Thr Glu His Gln Val
1 5

<210> 4
<211> 10
<212> PRT
<213> Artificial Sequence

<220>
<223> Description of Artificial Sequence:Designed peptide recognized by HLA-A2 restricted cytotoxic T lymphocytes

<400> 4
Gly Ile Val Glu Gly Leu Met Thr Thr Val
1 5 10

<210> 5
<211> 10
<212> PRT
<213> Artificial Sequence

<220>
<223> Description of Artificial Sequence:Designed peptide recognized by HLA-A2 restricted cytotoxic T lymphocytes

<400> 5
Thr Leu Leu Ala Gly Met Asn Lys Phe Leu
1 5 10

<210> 6
<211> 10
<212> PRT
<213> Artificial Sequence

<220>
<223> Description of Artificial Sequence:Designed peptide recognized by HLA-A2 restricted cytotoxic T lymphocytes

<400> 6
Thr Leu Ser Gly Gly Glu Leu Gln Arg Val
1 5 10

<210> 7
<211> 9
<212> PRT
<213> Artificial Sequence

<220>
<223> Description of Artificial Sequence:Designed peptide recognized by HLA-A2 restricted cytotoxic T lymphocytes

<400> 7
Ile Val Val Glu His Asp Leu Ser Val
1 5

<210> 8
<211> 10
<212> PRT
<213> Artificial Sequence

<220>
<223> Description of Artificial Sequence:Designed peptide recognized by HLA-A2 restricted cytotoxic T lymphocytes

<400> 8
Ile Leu Thr Tyr Phe Arg Gly Ser Glu Leu
1 5 10

<210> 9
<211> 10
<212> PRT

<213> Artificial Sequence

<220>
<223> Description of Artificial Sequence:Designed peptide recognized by HLA-A2 restricted cytotoxic T lymphocytes

<400> 9
Ile Leu Ala Gly Lys Gln Lys Pro Asn Leu
1 5 10

<210> 10
<211> 10
<212> PRT
<213> Artificial Sequence

<220>
<223> Description of Artificial Sequence:Designed peptide recognized by HLA-A2 restricted cytotoxic T lymphocytes

<400> 10
Lys Glu Gly Glu Tyr Ile Lys Leu Lys Val
1 5 10

<210> 11
<211> 10
<212> PRT
<213> Artificial Sequence

<220>
<223> Description of Artificial Sequence:Designed peptide recognized by HLA-A2 restricted cytotoxic T lymphocytes

<400> 11
Ala Leu Gly Glu Glu Trp Lys Gly Tyr Val
1 5 10

<210> 12
<211> 10
<212> PRT
<213> Artificial Sequence

<220>

<223> Description of Artificial Sequence:Designed peptide recognized by HLA-A2 restricted cytotoxic T lymphocytes

<400> 12
Asn Leu Ser Val Leu Asn Leu Val Ile Val
1 5 10

<210> 13
<211> 9
<212> PRT
<213> Artificial Sequence

<220>
<223> Description of Artificial Sequence:Designed peptide recognized by HLA-A2 restricted cytotoxic T lymphocytes

<400> 13
Val Leu Thr His Gly Arg Val Arg Leu
1 5

<210> 14
<211> 10
<212> PRT
<213> Artificial Sequence

<220>
<223> Description of Artificial Sequence:Designed peptide recognized by HLA-A2 restricted cytotoxic T lymphocytes

<400> 14
Lys Ile Gln Arg Leu Val Thr Pro Arg Val
1 5 10

<210> 15
<211> 9
<212> PRT
<213> Artificial Sequence

<220>
<223> Description of Artificial Sequence:Designed peptide recognized by HLA-A2 restricted cytotoxic T lymphocytes

<400> 15
Val Gln Phe Val Gln Gly Ile Phe Val
1 5

<210> 16
<211> 9
<212> PRT
<213> Artificial Sequence

<220>
<223> Description of Artificial Sequence:Designed peptide recognized by HLA-A2 restricted cytotoxic T lymphocytes

<400> 16
Lys Ser Ala Leu Thr Val Gln Phe Val
1 5

<210> 17
<211> 10
<212> PRT
<213> Artificial Sequence

<220>
<223> Description of Artificial Sequence:Designed peptide recognized by HLA-A2 restricted cytotoxic T lymphocytes

<400> 17
Ile Met Asn Gln Glu Lys Leu Ala Lys Leu
1 5 10

<210> 18
<211> 10
<212> PRT
<213> Artificial Sequence

<220>
<223> Description of Artificial Sequence:Designed peptide recognized by HLA-A2 restricted cytotoxic T lymphocytes

<400> 18
Leu Gln Phe Ser Leu Lys Lys Leu Gly Val

1 5 10

<210> 19
<211> 9
<212> PRT
<213> Artificial Sequence

<220>
<223> Description of Artificial Sequence:Designed peptide recognized by HLA-A2 restricted cytotoxic T lymphocytes

<400> 19
Asn Met Phe Thr Asn Gln Gly Thr Val
1 5

<210> 20
<211> 10
<212> PRT
<213> Artificial Sequence

<220>
<223> Description of Artificial Sequence:Designed peptide recognized by HLA-A2 restricted cytotoxic T lymphocytes

<400> 20
Arg Leu His Asn Asp Arg Val Tyr Tyr Val
1 5 10

<210> 21
<211> 10
<212> PRT
<213> Artificial Sequence

<220>
<223> Description of Artificial Sequence:Designed peptide recognized by HLA-A2 restricted cytotoxic T lymphocytes

<400> 21
Tyr Ile Gly Glu Asn Leu Gln Leu Leu Val
1 5 10

<210> 22
<211> 10
<212> PRT
<213> Artificial Sequence

<220>
<223> Description of Artificial Sequence:Designed peptide recognized by HLA-A2 restricted cytotoxic T lymphocytes

<400> 22
Ser Leu Gly Thr Cys Phe Gly Lys Phe Thr
1 5 10

<210> 23
<211> 10
<212> PRT
<213> Artificial Sequence

<220>
<223> Description of Artificial Sequence:Designed peptide recognized by HLA-A2 restricted cytotoxic T lymphocytes

<400> 23
Arg Leu His Val Thr Ala Leu Asp Tyr Leu
1 5 10

<210> 24
<211> 10
<212> PRT
<213> Artificial Sequence

<220>
<223> Description of Artificial Sequence:Designed peptide recognized by HLA-A2 restricted cytotoxic T lymphocytes

<400> 24
Leu Leu Gly Lys Thr Pro Val Thr Gln Val
1 5 10

<210> 25
<211> 10
<212> PRT

<213> Artificial Sequence

<220>
<223> Description of Artificial Sequence:Designed
peptide recognized by HLA-A2 restricted cytotoxic
T lymphocytes

<400> 25
Ala Leu Gln Lys Asp Val Glu Asp Phe Leu
1 5 10

<210> 26
<211> 10
<212> PRT
<213> Artificial Sequence

<220>
<223> Description of Artificial Sequence:Designed
peptide recognized by HLA-A2 restricted cytotoxic
T lymphocytes

<400> 26
Val Glu Ser Val Asp Glu Tyr Gln Phe Val
1 5 10

<210> 27
<211> 10
<212> PRT
<213> Artificial Sequence

<220>
<223> Description of Artificial Sequence:Designed
peptide recognized by HLA-A2 restricted cytotoxic
T lymphocytes

<400> 27
Ile Leu Gly Asp Lys Phe Pro Cys Thr Leu
1 5 10

<210> 28
<211> 10
<212> PRT
<213> Artificial Sequence

<220>

<223> Description of Artificial Sequence:Designed peptide recognized by HLA-A2 restricted cytotoxic T lymphocytes

<400> 28
Ala Leu Gly Gly Leu Pro Gly Pro Tyr Ile
1 5 10

<210> 29
<211> 9
<212> PRT
<213> Artificial Sequence

<220>
<223> Description of Artificial Sequence:Designed peptide recognized by HLA-A2 restricted cytotoxic T lymphocytes

<400> 29
Val Leu Val Glu Asp Thr Cys Leu Cys
1 5

<210> 30
<211> 9
<212> PRT
<213> Artificial Sequence

<220>
<223> Description of Artificial Sequence:Designed peptide recognized by HLA-A2 restricted cytotoxic T lymphocytes

<400> 30
Ala Leu Cys Thr Phe Ala Leu Ser Thr
1 5

<210> 31
<211> 10
<212> PRT
<213> Artificial Sequence

<220>
<223> Description of Artificial Sequence:Designed peptide recognized by HLA-A2 restricted cytotoxic T lymphocytes

<400> 31
Tyr Gln Gly Glu Pro Asp Glu Ile Ser Ile
1 5 10

<210> 32
<211> 9
<212> PRT
<213> Artificial Sequence

<220>
<223> Description of Artificial Sequence:Designed peptide recognized by HLA-A2 restricted cytotoxic T lymphocytes

<400> 32
Ile Leu Ala Leu Phe Met Pro Pro Thr
1 5

<210> 33
<211> 10
<212> PRT
<213> Artificial Sequence

<220>
<223> Description of Artificial Sequence:Designed peptide recognized by HLA-A2 restricted cytotoxic T lymphocytes

<400> 33
Val Leu Ile Glu Ile Leu Cys Thr Arg Thr
1 5 10

<210> 34
<211> 9
<212> PRT
<213> Artificial Sequence

<220>
<223> Description of Artificial Sequence:Designed peptide recognized by HLA-A2 restricted cytotoxic T lymphocytes

<400> 34
Arg Leu Tyr Tyr Ala Met Lys Gly Ala

1 5

<210> 35
<211> 10
<212> PRT
<213> Artificial Sequence

<220>
<223> Description of Artificial Sequence:Designed peptide recognized by HLA-A2 restricted cytotoxic T lymphocytes

<400> 35
Gly Val Pro Pro Gly Gln Gly Phe Gly Val
1 5 10

<210> 36
<211> 9
<212> PRT
<213> Artificial Sequence

<220>
<223> Description of Artificial Sequence:Designed peptide recognized by HLA-A2 restricted cytotoxic T lymphocytes

<400> 36
Tyr Gln Ser Glu Phe Gly Arg Asp Leu
1 5

<210> 37
<211> 9
<212> PRT
<213> Artificial Sequence

<220>
<223> Description of Artificial Sequence:Designed peptide recognized by HLA-A2 restricted cytotoxic T lymphocytes

<400> 37
Gly Val Ile Ser Pro Arg Phe Asp Val
1 5

<210> 38
<211> 9
<212> PRT
<213> Artificial Sequence

<220>
<223> Description of Artificial Sequence:Designed peptide recognized by HLA-A2 restricted cytotoxic T lymphocytes

<400> 38
Thr Leu Met Met Lys His Gly Tyr Ile
1 5

<210> 39
<211> 10
<212> PRT
<213> Artificial Sequence

<220>
<223> Description of Artificial Sequence:Designed peptide recognized by HLA-A2 restricted cytotoxic T lymphocytes

<400> 39
Leu Leu Pro Ser Arg Gln Phe Gly Phe Ile
1 5 10

<210> 40
<211> 10
<212> PRT
<213> Artificial Sequence

<220>
<223> Description of Artificial Sequence:Designed peptide recognized by HLA-A2 restricted cytotoxic T lymphocytes

<400> 40
Ser Val Tyr Ala His Phe Pro Ile Asn Val
1 5 10

<210> 41
<211> 9
<212> PRT

<213> Artificial Sequence

<220>
<223> Description of Artificial Sequence:Designed peptide recognized by HLA-A2 restricted cytotoxic T lymphocytes

<400> 41
Val Ile Gln Glu Asn Gly Ser Leu Val
1 5

<210> 42
<211> 9
<212> PRT
<213> Artificial Sequence

<220>
<223> Description of Artificial Sequence:Designed peptide recognized by HLA-A2 restricted cytotoxic T lymphocytes

<400> 42
Ile Leu Ser Asn Gln Thr Val Asp Ile
1 5

<210> 43
<211> 10
<212> PRT
<213> Artificial Sequence

<220>
<223> Description of Artificial Sequence:Designed peptide recognized by HLA-A2 restricted cytotoxic T lymphocytes

<400> 43
Lys Ile Phe Leu Ile Phe Phe Phe Phe Leu
1 5 10

<210> 44
<211> 9
<212> PRT
<213> Artificial Sequence

<220>

<223> Description of Artificial Sequence:Designed peptide recognized by HLA-A2 restricted cytotoxic T lymphocytes

<400> 44
Val Val Ile Phe Lys Ile Phe Leu Ile
1 5

<210> 45
<211> 10
<212> PRT
<213> Artificial Sequence

<220>
<223> Description of Artificial Sequence:Designed peptide recognized by HLA-A2 restricted cytotoxic T lymphocytes

<400> 45
Gly Met Ala Asp Ser Gln Asn Met Leu Val
1 5 10

<210> 46
<211> 10
<212> PRT
<213> Artificial Sequence

<220>
<223> Description of Artificial Sequence:Designed peptide recognized by HLA-A2 restricted cytotoxic T lymphocytes

<400> 46
Ile Ile Ser Glu Lys Tyr Gln Val Phe Ile
1 5 10

<210> 47
<211> 9
<212> PRT
<213> Artificial Sequence

<220>
<223> Description of Artificial Sequence:Designed peptide recognized by HLA-A2 restricted cytotoxic T lymphocytes

<400> 47
Lys Leu Ile Glu Glu Lys Gly Ala Val
1 5

<210> 48
<211> 9
<212> PRT
<213> Artificial Sequence

<220>
<223> Description of Artificial Sequence:Designed peptide recognized by HLA-A2 restricted cytotoxic T lymphocytes

<400> 48
Phe Leu Phe Pro Ile Gln Ala Lys Thr
1 5

<210> 49
<211> 10
<212> PRT
<213> Artificial Sequence

<220>
<223> Description of Artificial Sequence:Designed peptide recognized by HLA-A2 restricted cytotoxic T lymphocytes

<400> 49
Ser Leu Ile Asn Ser Asn Val Gly Phe Val
1 5 10

<210> 50
<211> 10
<212> PRT
<213> Artificial Sequence

<220>
<223> Description of Artificial Sequence:Designed peptide recognized by HLA-A2 restricted cytotoxic T lymphocytes

<400> 50
Lys Leu Gly Val Cys Phe Asp Val Pro Thr

1 5 10

<210> 51
<211> 10
<212> PRT
<213> Artificial Sequence

<220>
<223> Description of Artificial Sequence:Designed peptide recognized by HLA-A2 restricted cytotoxic T lymphocytes

<400> 51
Tyr Gln His Lys Glu Glu Tyr Gln Leu Val
1 5 10

<210> 52
<211> 10
<212> PRT
<213> Artificial Sequence

<220>
<223> Description of Artificial Sequence:Designed peptide recognized by HLA-A2 restricted cytotoxic T lymphocytes

<400> 52
Met Val Phe Leu Lys Gly Lys Leu Gly Val
1 5 10

<210> 53
<211> 9
<212> PRT
<213> Artificial Sequence

<220>
<223> Description of Artificial Sequence:Designed peptide recognized by HLA-A2 restricted cytotoxic T lymphocytes

<400> 53
Ala Leu Ala Ala Ala Leu Ala His Ile
1 5

<210> 54
<211> 9
<212> PRT
<213> Artificial Sequence

<220>
<223> Description of Artificial Sequence:Designed peptide recognized by HLA-A2 restricted cytotoxic T lymphocytes

<400> 54
Ile Gln Ala Lys Thr Phe His His Val
1 5

<210> 55
<211> 10
<212> PRT
<213> Artificial Sequence

<220>
<223> Description of Artificial Sequence:Designed peptide recognized by HLA-A2 restricted cytotoxic T lymphocytes

<400> 55
Lys Val Val Ser Ser Lys Thr Lys Lys Val
1 5 10

<210> 56
<211> 10
<212> PRT
<213> Artificial Sequence

<220>
<223> Description of Artificial Sequence:Designed peptide recognized by HLA-A2 restricted cytotoxic T lymphocytes

<400> 56
Ala Thr Phe Lys Ser Phe Glu Asp Arg Val
1 5 10

<210> 57
<211> 10
<212> PRT

<213> Artificial Sequence

<220>
<223> Description of Artificial Sequence:Designed peptide recognized by HLA-A2 restricted cytotoxic T lymphocytes

<400> 57
Lys Ile Gly Pro Arg Arg Ile His Thr Val
1 5 10

<210> 58
<211> 10
<212> PRT
<213> Artificial Sequence

<220>
<223> Description of Artificial Sequence:Designed peptide recognized by HLA-A2 restricted cytotoxic T lymphocytes

<400> 58
Thr Leu Val Lys Asn Cys Ile Val Leu Ile
1 5 10

<210> 59
<211> 9
<212> PRT
<213> Artificial Sequence

<220>
<223> Description of Artificial Sequence:Designed peptide recognized by HLA-A2 restricted cytotoxic T lymphocytes

<400> 59
Arg Gln Trp Tyr Glu Ser His Tyr Ala
1 5

<210> 60
<211> 9
<212> PRT
<213> Artificial Sequence

<220>

<223> Description of Artificial Sequence:Designed peptide recognized by HLA-A2 restricted cytotoxic T lymphocytes

<400> 60
Lys Leu Thr Pro Glu Glu Glu Glu Ile
1 5

<210> 61
<211> 10
<212> PRT
<213> Artificial Sequence

<220>
<223> Description of Artificial Sequence:Designed peptide recognized by HLA-A2 restricted cytotoxic T lymphocytes

<400> 61
Ile Leu Asn Lys Lys Arg Ser Lys Lys Ile
1 5 10

<210> 62
<211> 9
<212> PRT
<213> Artificial Sequence

<220>
<223> Description of Artificial Sequence:Designed peptide recognized by HLA-A2 restricted cytotoxic T lymphocytes

<400> 62
Tyr Leu Asn Glu Gln Val Lys Ala Ile
1 5

<210> 63
<211> 10
<212> PRT
<213> Artificial Sequence

<220>
<223> Description of Artificial Sequence:Designed peptide recognized by HLA-A2 restricted cytotoxic T lymphocytes

<400> 63
Ser Leu Leu Glu Leu His Lys Leu Ala Thr
1 5 10

<210> 64
<211> 10
<212> PRT
<213> Artificial Sequence

<220>
<223> Description of Artificial Sequence:Designed peptide recognized by HLA-A2 restricted cytotoxic T lymphocytes

<400> 64
Phe Leu Gln Asp Ile Lys Lys Pro Asp Cys
1 5 10

<210> 65
<211> 10
<212> PRT
<213> Artificial Sequence

<220>
<223> Description of Artificial Sequence:Designed peptide recognized by HLA-A2 restricted cytotoxic T lymphocytes

<400> 65
Ile Asn Leu Glu Leu Tyr Ala Ser Tyr Val
1 5 10

<210> 66
<211> 10
<212> PRT
<213> Artificial Sequence

<220>
<223> Description of Artificial Sequence:Designed peptide recognized by HLA-A2 restricted cytotoxic T lymphocytes

<400> 66
Lys Ile Tyr Lys Ile Gly Gln Gly Tyr Leu

1 5 10

<210> 67
<211> 9
<212> PRT
<213> Artificial Sequence

<220>
<223> Description of Artificial Sequence:Designed peptide recognized by HLA-A2 restricted cytotoxic T lymphocytes

<400> 67
Lys Leu Ile Lys Asn Asn Ala Ser Thr
1 5

<210> 68
<211> 9
<212> PRT
<213> Artificial Sequence

<220>
<223> Description of Artificial Sequence:Designed peptide recognized by HLA-A2 restricted cytotoxic T lymphocytes

<400> 68
Gly Met Thr His Ile Val Arg Glu Val
1 5

<210> 69
<211> 10
<212> PRT
<213> Artificial Sequence

<220>
<223> Description of Artificial Sequence:Designed peptide recognized by HLA-A2 restricted cytotoxic T lymphocytes

<400> 69
Arg Leu Leu Pro Leu Arg Gln Lys Lys Ala
1 5 10

<210> 70
<211> 10
<212> PRT
<213> Artificial Sequence

<220>
<223> Description of Artificial Sequence:Designed peptide recognized by HLA-A2 restricted cytotoxic T lymphocytes

<400> 70
Phe Leu Ile Phe Glu Asp Arg Lys Phe Ala
1 5 10

<210> 71
<211> 9
<212> PRT
<213> Artificial Sequence

<220>
<223> Description of Artificial Sequence:Designed peptide recognized by HLA-A2 restricted cytotoxic T lymphocytes

<400> 71
Ala Leu Gly Pro Ser Ile Cys Met Leu
1 5

<210> 72
<211> 10
<212> PRT
<213> Artificial Sequence

<220>
<223> Description of Artificial Sequence:Designed peptide recognized by HLA-A2 restricted cytotoxic T lymphocytes

<400> 72
Gln Leu Ala Asp Ala Leu Gly Pro Ser Ile
1 5 10

<210> 73
<211> 10
<212> PRT

<213> Artificial Sequence

<220>
<223> Description of Artificial Sequence:Designed peptide recognized by HLA-A2 restricted cytotoxic T lymphocytes

<400> 73
Gly Leu Pro Leu His Arg Gly Cys Leu Leu
1 5 10

<210> 74
<211> 9
<212> PRT
<213> Artificial Sequence

<220>
<223> Description of Artificial Sequence:Designed peptide recognized by HLA-A2 restricted cytotoxic T lymphocytes

<400> 74
Tyr Leu Tyr Leu Ile Ser Ser Cys Ile
1 5

<210> 75
<211> 10
<212> PRT
<213> Artificial Sequence

<220>
<223> Description of Artificial Sequence:Designed peptide recognized by HLA-A2 restricted cytotoxic T lymphocytes

<400> 75
Tyr Leu Ile Ser Ser Cys Ile Lys Pro Ile
1 5 10

<210> 76
<211> 9
<212> PRT
<213> Artificial Sequence

<220>

<223> Description of Artificial Sequence:Designed peptide recognized by HLA-A2 restricted cytotoxic T lymphocytes

<400> 76
Val Ile Ser Cys Tyr Ile Cys Lys Val
1 5

<210> 77
<211> 10
<212> PRT
<213> Artificial Sequence

<220>
<223> Description of Artificial Sequence:Designed peptide recognized by HLA-A2 restricted cytotoxic T lymphocytes

<400> 77
Trp Leu Ser Asp Gln Leu Gln Asn Asn Cys
1 5 10

<210> 78
<211> 9
<212> PRT
<213> Artificial Sequence

<220>
<223> Description of Artificial Sequence:Designed peptide recognized by HLA-A2 restricted cytotoxic T lymphocytes

<400> 78
Met Leu Cys Gly Asn Ile Tyr Pro Ile
1 5

<210> 79
<211> 10
<212> PRT
<213> Artificial Sequence

<220>
<223> Description of Artificial Sequence:Designed peptide recognized by HLA-A2 restricted cytotoxic T lymphocytes

<400> 79
Tyr Leu Pro Ser Gly Ser Ser Ala His Leu
1 5 10

<210> 80
<211> 9
<212> PRT
<213> Artificial Sequence

<220>
<223> Description of Artificial Sequence:Designed peptide recognized by HLA-A2 restricted cytotoxic T lymphocytes

<400> 80
Ser Met Gln Asp Asp Ala Phe Pro Ala
1 5

<210> 81
<211> 9
<212> PRT
<213> Artificial Sequence

<220>
<223> Description of Artificial Sequence:Designed peptide recognized by HLA-A2 restricted cytotoxic T lymphocytes

<400> 81
Thr Leu Ile Pro Thr Phe Asp Ser Val
1 5

<210> 82
<211> 10
<212> PRT
<213> Artificial Sequence

<220>
<223> Description of Artificial Sequence:Designed peptide recognized by HLA-A2 restricted cytotoxic T lymphocytes

<400> 82
Phe Gln Arg Val Arg Ala Leu Cys Tyr Val

1 5 10

<210> 83
<211> 10
<212> PRT
<213> Artificial Sequence

<220>
<223> Description of Artificial Sequence:Designed peptide recognized by HLA-A2 restricted cytotoxic T lymphocytes

<400> 83
Val Leu Gly Ser Asn Gly Met Val Ser Met
1 5 10

<210> 84
<211> 10
<212> PRT
<213> Artificial Sequence

<220>
<223> Description of Artificial Sequence:Designed peptide recognized by HLA-A2 restricted cytotoxic T lymphocytes

<400> 84
Phe Leu Thr Lys Ile Phe His Pro Asn Val
1 5 10

<210> 85
<211> 10
<212> PRT
<213> Artificial Sequence

<220>
<223> Description of Artificial Sequence:Designed peptide recognized by HLA-A2 restricted cytotoxic T lymphocytes

<400> 85
Leu Leu Leu Glu Asn Tyr Glu Glu Tyr Ala
1 5 10

<210> 86
<211> 9
<212> PRT
<213> Artificial Sequence

<220>
<223> Description of Artificial Sequence:Designed peptide recognized by HLA-A2 restricted cytotoxic T lymphocytes

<400> 86
Val Leu Leu Thr Ile Lys Cys Leu Leu
1 5

<210> 87
<211> 9
<212> PRT
<213> Artificial Sequence

<220>
<223> Description of Artificial Sequence:Designed peptide recognized by HLA-A2 restricted cytotoxic T lymphocytes

<400> 87
Gly Leu Phe Arg Met Lys Leu Leu Leu
1 5

<210> 88
<211> 9
<212> PRT
<213> Artificial Sequence

<220>
<223> Description of Artificial Sequence:Designed peptide recognized by HLA-A2 restricted cytotoxic T lymphocytes

<400> 88
Asn Leu Pro Pro His Ile Ile Arg Leu
1 5

<210> 89
<211> 9
<212> PRT

<213> Artificial Sequence

<220>
<223> Description of Artificial Sequence:Designed peptide recognized by HLA-A2 restricted cytotoxic T lymphocytes

<400> 89
Lys Leu Thr Asn Thr Tyr Cys Leu Val
1 5

<210> 90
<211> 9
<212> PRT
<213> Artificial Sequence

<220>
<223> Description of Artificial Sequence:Designed peptide recognized by HLA-A2 restricted cytotoxic T lymphocytes

<400> 90
Gly Leu Leu Val Pro Asn Asn Thr Thr
1 5

<210> 91
<211> 9
<212> PRT
<213> Artificial Sequence

<220>
<223> Description of Artificial Sequence:Designed peptide recognized by HLA-A2 restricted cytotoxic T lymphocytes

<400> 91
Gly Met Val Val Asn Asp Trp Cys Ala
1 5

<210> 92
<211> 10
<212> PRT
<213> Artificial Sequence

<220>

<223> Description of Artificial Sequence:Designed peptide recognized by HLA-A2 restricted cytotoxic T lymphocytes

<400> 92
Val Leu Leu Arg Gln Gly Val Leu Gly Ile
1 5 10

<210> 93
<211> 10
<212> PRT
<213> Artificial Sequence

<220>
<223> Description of Artificial Sequence:Designed peptide recognized by HLA-A2 restricted cytotoxic T lymphocytes

<400> 93
Gly Leu Met Ile His Ser Gly Asp Pro Val
1 5 10

<210> 94
<211> 9
<212> PRT
<213> Artificial Sequence

<220>
<223> Description of Artificial Sequence:Designed peptide recognized by HLA-A2 restricted cytotoxic T lymphocytes

<400> 94
Ile Leu Ala Thr Arg Thr Gln Asn Val
1 5

<210> 95
<211> 9
<212> PRT
<213> Artificial Sequence

<220>
<223> Description of Artificial Sequence:Designed peptide recognized by HLA-A2 restricted cytotoxic T lymphocytes

<400> 95
Phe Val Ala Asp Gly Ile Phe Lys Ala
1 5

<210> 96
<211> 10
<212> PRT
<213> Artificial Sequence

<220>
<223> Description of Artificial Sequence:Designed peptide recognized by HLA-A2 restricted cytotoxic T lymphocytes

<400> 96
Phe Ile Met Glu Ser Gly Ala Lys Gly Cys
1 5 10

<210> 97
<211> 10
<212> PRT
<213> Artificial Sequence

<220>
<223> Description of Artificial Sequence:Designed peptide recognized by HLA-A2 restricted cytotoxic T lymphocytes

<400> 97
Trp Ile Pro Asn Asn Val Lys Thr Ala Val
1 5 10

<210> 98
<211> 9
<212> PRT
<213> Artificial Sequence

<220>
<223> Description of Artificial Sequence:Designed peptide recognized by HLA-A2 restricted cytotoxic T lymphocytes

<400> 98
Arg Ile Met Asn Thr Phe Ser Val Val

1 5

<210> 99
<211> 9
<212> PRT
<213> Artificial Sequence

<220>
<223> Description of Artificial Sequence:Designed peptide recognized by HLA-A2 restricted cytotoxic T lymphocytes

<400> 99
Leu Val Ser Ala Thr Met Ser Gly Val
1 5

<210> 100
<211> 9
<212> PRT
<213> Artificial Sequence

<220>
<223> Description of Artificial Sequence:Designed peptide recognized by HLA-A2 restricted cytotoxic T lymphocytes

<400> 100
Ser Leu Asn Arg Arg Ile Gln Leu Val
1 5

<210> 101
<211> 9
<212> PRT
<213> Artificial Sequence

<220>
<223> Description of Artificial Sequence:Designed peptide recognized by HLA-A2 restricted cytotoxic T lymphocytes

<400> 101
Arg Leu Ala Thr Ala Leu Gln Lys Leu
1 5

<210> 102
<211> 10
<212> PRT
<213> Artificial Sequence

<220>
<223> Description of Artificial Sequence:Designed peptide recognized by HLA-A2 restricted cytotoxic T lymphocytes

<400> 102
Gln Leu Val Glu Glu Glu Leu Asp Arg Ala
1 5 10

<210> 103
<211> 10
<212> PRT
<213> Artificial Sequence

<220>
<223> Description of Artificial Sequence:Designed peptide recognized by HLA-A2 restricted cytotoxic T lymphocytes

<400> 103
Gly Ile Ser Leu Ala Asn Gln Gln Tyr Val
1 5 10

<210> 104
<211> 9
<212> PRT
<213> Artificial Sequence

<220>
<223> Description of Artificial Sequence:Designed peptide recognized by HLA-A2 restricted cytotoxic T lymphocytes

<400> 104
Phe Leu His Ser Gly His Leu His Ala
1 5

<210> 105
<211> 9
<212> PRT

<213> Artificial Sequence

<220>
<223> Description of Artificial Sequence:Designed peptide recognized by HLA-A2 restricted cytotoxic T lymphocytes

<400> 105
Glu Leu Val Arg Phe Arg Gln Lys Val
1 5

<210> 106
<211> 9
<212> PRT
<213> Artificial Sequence

<220>
<223> Description of Artificial Sequence:Designed peptide recognized by HLA-A2 restricted cytotoxic T lymphocytes

<400> 106
Lys Leu Ser Glu Ala Ala Gly Arg Val
1 5

<210> 107
<211> 9
<212> PRT
<213> Artificial Sequence

<220>
<223> Description of Artificial Sequence:Designed peptide recognized by HLA-A2 restricted cytotoxic T lymphocytes

<400> 107
Met Val Leu Asp Leu Met Gln Gln Leu
1 5

<210> 108
<211> 10
<212> PRT
<213> Artificial Sequence

<220>

<223> Description of Artificial Sequence:Designed peptide recognized by HLA-A2 restricted cytotoxic T lymphocytes

<400> 108
Ile Met Gln Asn Leu Leu Ser Lys Asp Val
1 5 10

<210> 109
<211> 10
<212> PRT
<213> Artificial Sequence

<220>
<223> Description of Artificial Sequence:Designed peptide recognized by HLA-A2 restricted cytotoxic T lymphocytes

<400> 109
Glu Leu Ala Glu Glu Glu Pro His Leu Val
1 5 10

<210> 110
<211> 9
<212> PRT
<213> Artificial Sequence

<220>
<223> Description of Artificial Sequence:Designed peptide recognized by HLA-A2 restricted cytotoxic T lymphocytes

<400> 110
Gly Leu Ala Asp Ser Gly Trp Phe Leu
1 5

<210> 111
<211> 9
<212> PRT
<213> Artificial Sequence

<220>
<223> Description of Artificial Sequence:Designed peptide recognized by HLA-A2 restricted cytotoxic T lymphocytes

<400> 111
Lys Gln Tyr Arg His Thr Asp Cys Val
1 5

<210> 112
<211> 10
<212> PRT
<213> Artificial Sequence

<220>
<223> Description of Artificial Sequence:Designed peptide recognized by HLA-A2 restricted cytotoxic T lymphocytes

<400> 112
Val Gln Trp Leu Phe Asp Glu Ala Gln Leu
1 5 10

<210> 113
<211> 10
<212> PRT
<213> Artificial Sequence

<220>
<223> Description of Artificial Sequence:Designed peptide recognized by HLA-A2 restricted cytotoxic T lymphocytes

<400> 113
Ile Ile Ile Arg Ser His Trp Thr Asp Val
1 5 10

<210> 114
<211> 10
<212> PRT
<213> Artificial Sequence

<220>
<223> Description of Artificial Sequence:Designed peptide recognized by HLA-A2 restricted cytotoxic T lymphocytes

<400> 114
Asn Leu Gly Arg Glu Leu Arg His Thr Leu

1 5 10

<210> 115
<211> 9
<212> PRT
<213> Artificial Sequence

<220>
<223> Description of Artificial Sequence:Designed peptide recognized by HLA-A2 restricted cytotoxic T lymphocytes

<400> 115
Leu Leu Gly Arg Gly Leu Ser Gly Ala
1 5

<210> 116
<211> 9
<212> PRT
<213> Artificial Sequence

<220>
<223> Description of Artificial Sequence:Designed peptide recognized by HLA-A2 restricted cytotoxic T lymphocytes

<400> 116
Val Leu Tyr Leu Phe Tyr Glu Asp Met
1 5

<210> 117
<211> 9
<212> PRT
<213> Artificial Sequence

<220>
<223> Description of Artificial Sequence:Designed peptide recognized by HLA-A2 restricted cytotoxic T lymphocytes

<400> 117
Tyr Val Ala Arg Asn Ala Lys Asp Val
1 5

<210> 118
<211> 10
<212> PRT
<213> Artificial Sequence

<220>
<223> Description of Artificial Sequence:Designed peptide recognized by HLA-A2 restricted cytotoxic T lymphocytes

<400> 118
Leu Ile Gln Asp Thr Ser Arg Pro Pro Leu
1 5 10

<210> 119
<211> 10
<212> PRT
<213> Artificial Sequence

<220>
<223> Description of Artificial Sequence:Designed peptide recognized by HLA-A2 restricted cytotoxic T lymphocytes

<400> 119
Gly Leu Phe Ile Phe Ser Ile Val Phe Leu
1 5 10

<210> 120
<211> 10
<212> PRT
<213> Artificial Sequence

<220>
<223> Description of Artificial Sequence:Designed peptide recognized by HLA-A2 restricted cytotoxic T lymphocytes

<400> 120
Trp Leu Leu Leu Pro Leu Leu Gly Ala Val
1 5 10

<210> 121
<211> 9
<212> PRT

<213> Artificial Sequence

<220>
<223> Description of Artificial Sequence:Designed peptide recognized by HLA-A2 restricted cytotoxic T lymphocytes

<400> 121
Ile Leu Phe Arg Gly Val Gly Met Val
1 5

<210> 122
<211> 10
<212> PRT
<213> Artificial Sequence

<220>
<223> Description of Artificial Sequence:Designed peptide recognized by HLA-A2 restricted cytotoxic T lymphocytes

<400> 122
Gly Leu Gln Ala Arg Asn Asn Ala Arg Val
1 5 10

<210> 123
<211> 10
<212> PRT
<213> Artificial Sequence

<220>
<223> Description of Artificial Sequence:Designed peptide recognized by HLA-A2 restricted cytotoxic T lymphocytes

<400> 123
Asp Val Tyr Gly Val Phe Gln Phe Lys Val
1 5 10

<210> 124
<211> 10
<212> PRT
<213> Artificial Sequence

<220>

<223> Description of Artificial Sequence:Designed peptide recognized by HLA-A2 restricted cytotoxic T lymphocytes

<400> 124
Ser Leu Asn Pro Ile Leu Phe Arg Gly Val
1 5 10

<210> 125
<211> 9
<212> PRT
<213> Artificial Sequence

<220>
<223> Description of Artificial Sequence:Designed peptide recognized by HLA-A2 restricted cytotoxic T lymphocytes

<400> 125
Thr Leu His Thr Trp Gly Ser Lys Val
1 5

<210> 126
<211> 9
<212> PRT
<213> Artificial Sequence

<220>
<223> Description of Artificial Sequence:Designed peptide recognized by HLA-A2 restricted cytotoxic T lymphocytes

<400> 126
Cys Leu Pro Ser Gly Phe Pro Gly Leu
1 5

<210> 127
<211> 9
<212> PRT
<213> Artificial Sequence

<220>
<223> Description of Artificial Sequence:Designed peptide recognized by HLA-A2 restricted cytotoxic T lymphocytes

<400> 127
Asn Leu Val Lys Cys Ile Lys Arg Leu
1 5

<210> 128
<211> 9
<212> PRT
<213> Artificial Sequence

<220>
<223> Description of Artificial Sequence:Designed peptide recognized by HLA-A2 restricted cytotoxic T lymphocytes

<400> 128
Thr Val Phe Leu Glu Gly Asn Leu Val
1 5

<210> 129
<211> 9
<212> PRT .
<213> Artificial Sequence

<220>
<223> Description of Artificial Sequence:Designed peptide recognized by HLA-A2 restricted cytotoxic T lymphocytes

<400> 129
Phe Leu Leu Leu Leu Leu Phe Glu Thr
1 5

<210> 130
<211> 9
<212> PRT
<213> Artificial Sequence

<220>
<223> Description of Artificial Sequence:Designed peptide recognized by HLA-A2 restricted cytotoxic T lymphocytes

<400> 130
Tyr Ile Phe Phe Cys Val Leu Phe Leu

1 5

<210> 131
<211> 9
<212> PRT
<213> Artificial Sequence

<220>
<223> Description of Artificial Sequence:Designed peptide recognized by HLA-A2 restricted cytotoxic T lymphocytes

<400> 131
Phe Leu Leu Leu Phe Gly Phe Trp Lys
1 5

<210> 132
<211> 9
<212> PRT
<213> Artificial Sequence

<220>
<223> Description of Artificial Sequence:Designed peptide recognized by HLA-A2 restricted cytotoxic T lymphocytes

<400> 132
Ser Val His Pro Arg Leu Phe Leu Leu
1 5

<210> 133
<211> 9
<212> PRT
<213> Artificial Sequence

<220>
<223> Description of Artificial Sequence:Designed peptide recognized by HLA-A2 restricted cytotoxic T lymphocytes

<400> 133
Ile Leu Phe Pro Arg Lys Pro Ser Ala
1 5

<210> 134
<211> 10
<212> PRT
<213> Artificial Sequence

<220>
<223> Description of Artificial Sequence:Designed peptide recognized by HLA-A2 restricted cytotoxic T lymphocytes

<400> 134
Lys Val Ala Arg Thr Ile Gly Ile Ser Val
1 5 10

<210> 135
<211> 10
<212> PRT
<213> Artificial Sequence

<220>
<223> Description of Artificial Sequence:Designed peptide recognized by HLA-A2 restricted cytotoxic T lymphocytes

<400> 135
Phe Leu Ala Ile Leu Gly Gly Ala Lys Val
1 5 10

<210> 136
<211> 9
<212> PRT
<213> Artificial Sequence

<220>
<223> Description of Artificial Sequence:Designed peptide recognized by HLA-A2 restricted cytotoxic T lymphocytes

<400> 136
Val Val Met Arg Val Asp Phe Asn Val
1 5

<210> 137
<211> 9
<212> PRT

<213> Artificial Sequence

<220>
<223> Description of Artificial Sequence:Designed peptide recognized by HLA-A2 restricted cytotoxic T lymphocytes

<400> 137
Lys Ile Thr Leu Pro Val Asp Phe Val
1 5

<210> 138
<211> 10
<212> PRT
<213> Artificial Sequence

<220>
<223> Description of Artificial Sequence:Designed peptide recognized by HLA-A2 restricted cytotoxic T lymphocytes

<400> 138
Ser Leu Phe Asp Glu Glu Gly Ala Lys Ile
1 5 10

<210> 139
<211> 10
<212> PRT
<213> Artificial Sequence

<220>
<223> Description of Artificial Sequence:Designed peptide recognized by HLA-A2 restricted cytotoxic T lymphocytes

<400> 139
Gln Leu Ile Asn Asn Met Leu Asp Lys Val
1 5 10

<210> 140
<211> 10
<212> PRT
<213> Artificial Sequence

<220>

<223> Description of Artificial Sequence:Designed peptide recognized by HLA-A2 restricted cytotoxic T lymphocytes

<400> 140
Phe Cys Leu Asp Asn Gly Ala Lys Ser Val
1 5 10

<210> 141
<211> 9
<212> PRT
<213> Artificial Sequence

<220>
<223> Description of Artificial Sequence:Designed peptide recognized by HLA-A2 restricted cytotoxic T lymphocytes

<400> 141
Ile Ile Gly Gly Gly Met Ala Phe Thr
1 5

<210> 142
<211> 10
<212> PRT
<213> Artificial Sequence

<220>
<223> Description of Artificial Sequence:Designed peptide recognized by HLA-A2 restricted cytotoxic T lymphocytes

<400> 142
Ala Leu Phe Val Ser Phe Ile Ile Asn Val
1 5 10

<210> 143
<211> 10
<212> PRT
<213> Artificial Sequence

<220>
<223> Description of Artificial Sequence:Designed peptide recognized by HLA-A2 restricted cytotoxic T lymphocytes

<400> 143
Val Leu Ile Thr Ile Ala Asp Thr Phe Val
1 5 10

<210> 144
<211> 9
<212> PRT
<213> Artificial Sequence

<220>
<223> Description of Artificial Sequence:Designed peptide recognized by HLA-A2 restricted cytotoxic T lymphocytes

<400> 144
Phe Leu Phe Leu Asp Lys Tyr Gly Leu
1 5

<210> 145
<211> 9
<212> PRT
<213> Artificial Sequence

<220>
<223> Description of Artificial Sequence:Designed peptide recognized by HLA-A2 restricted cytotoxic T lymphocytes

<400> 145
Ala Leu Thr Phe Gly Tyr Glu Tyr Val
1 5

<210> 146
<211> 10
<212> PRT
<213> Artificial Sequence

<220>
<223> Description of Artificial Sequence:Designed peptide recognized by HLA-A2 restricted cytotoxic T lymphocytes

<400> 146
Tyr Leu Gly Trp Gln Cys Leu Ile Ala Leu

1 5 10

<210> 147
<211> 10
<212> PRT
<213> Artificial Sequence

<220>
<223> Description of Artificial Sequence:Designed peptide recognized by HLA-A2 restricted cytotoxic T lymphocytes

<400> 147
Lys Leu Leu Trp Ile Leu Leu Leu Ala Thr
1 5 10

<210> 148
<211> 9
<212> PRT
<213> Artificial Sequence

<220>
<223> Description of Artificial Sequence:Designed peptide recognized by HLA-A2 restricted cytotoxic T lymphocytes

<400> 148
Met Leu Phe Ile His Ala Glu Val Ile
1 5

<210> 149
<211> 9
<212> PRT
<213> Artificial Sequence

<220>
<223> Description of Artificial Sequence:Designed peptide recognized by HLA-A2 restricted cytotoxic T lymphocytes

<400> 149
Lys Leu Ile Lys Arg Ser Gly Tyr Ile
1 5

<210> 150
<211> 10
<212> PRT
<213> Artificial Sequence

<220>
<223> Description of Artificial Sequence:Designed peptide recognized by HLA-A2 restricted cytotoxic T lymphocytes

<400> 150
Ser Leu Pro Val Cys Ser Leu Lys Leu Ile
1 5 10

<210> 151
<211> 10
<212> PRT
<213> Artificial Sequence

<220>
<223> Description of Artificial Sequence:Designed peptide recognized by HLA-A2 restricted cytotoxic T lymphocytes

<400> 151
Phe Val Ile Ser Leu Pro Val Cys Ser Leu
1 5 10

<210> 152
<211> 10
<212> PRT
<213> Artificial Sequence

<220>
<223> Description of Artificial Sequence:Designed peptide recognized by HLA-A2 restricted cytotoxic T lymphocytes

<400> 152
Lys Gln Phe Asp Glu Asn Thr Asn Trp Leu
1 5 10

<210> 153
<211> 9
<212> PRT

<213> Artificial Sequence

<220>
<223> Description of Artificial Sequence:Designed peptide recognized by HLA-A2 restricted cytotoxic T lymphocytes

<400> 153
Phe Leu Asn Gly Tyr Asn Cys Thr Val
1 5

<210> 154
<211> 10
<212> PRT
<213> Artificial Sequence

<220>
<223> Description of Artificial Sequence:Designed peptide recognized by HLA-A2 restricted cytotoxic T lymphocytes

<400> 154
Ala Met Leu Lys Thr Arg Arg Ser Tyr Leu
1 5 10

<210> 155
<211> 10
<212> PRT
<213> Artificial Sequence

<220>
<223> Description of Artificial Sequence:Designed peptide recognized by HLA-A2 restricted cytotoxic T lymphocytes

<400> 155
Thr Leu Met Lys Pro Ser Ser Phe Thr Thr
1 5 10

<210> 156
<211> 10
<212> PRT
<213> Artificial Sequence

<220>

<223> Description of Artificial Sequence:Designed peptide recognized by HLA-A2 restricted cytotoxic T lymphocytes

<400> 156
Leu Leu Val Asn Ser Gly Pro Leu Ala Val
1 5 10

<210> 157
<211> 10
<212> PRT
<213> Artificial Sequence

<220>
<223> Description of Artificial Sequence:Designed peptide recognized by HLA-A2 restricted cytotoxic T lymphocytes

<400> 157
Met Leu Gly Ser Ala Asp Glu Pro Gly Val
1 5 10

<210> 158
<211> 10
<212> PRT
<213> Artificial Sequence

<220>
<223> Description of Artificial Sequence:Designed peptide recognized by HLA-A2 restricted cytotoxic T lymphocytes

<400> 158
Lys Gln Asn Asp Leu Pro Gly Ile Ser Val
1 5 10

<210> 159
<211> 10
<212> PRT
<213> Artificial Sequence

<220>
<223> Description of Artificial Sequence:Designed peptide recognized by HLA-A2 restricted cytotoxic T lymphocytes

<400> 159
Tyr Leu Thr Met Leu His Leu Tyr Lys Cys
1 5 10

<210> 160
<211> 10
<212> PRT
<213> Artificial Sequence

<220>
<223> Description of Artificial Sequence:Designed peptide recognized by HLA-A2 restricted cytotoxic T lymphocytes

<400> 160
Ile Thr Gly Glu Ala Phe Val Gln Phe Ala
1 5 10

<210> 161
<211> 10
<212> PRT
<213> Artificial Sequence

<220>
<223> Description of Artificial Sequence:Designed peptide recognized by HLA-A2 restricted cytotoxic T lymphocytes

<400> 161
Val Val Ala Cys Asn Leu Tyr Pro Phe Val
1 5 10

<210> 162
<211> 9
<212> PRT
<213> Artificial Sequence

<220>
<223> Description of Artificial Sequence:Designed peptide recognized by HLA-A2 restricted cytotoxic T lymphocytes

<400> 162
Met Leu Gly Gly Arg Val Lys Thr Leu

1 5

<210> 163
<211> 9
<212> PRT
<213> Artificial Sequence

<220>
<223> Description of Artificial Sequence:Designed peptide recognized by HLA-A2 restricted cytotoxic T lymphocytes

<400> 163
Gln Leu Tyr Thr Leu Gln Pro Lys Leu
1 5

<210> 164
<211> 9
<212> PRT
<213> Artificial Sequence

<220>
<223> Description of Artificial Sequence:Designed peptide recognized by HLA-A2 restricted cytotoxic T lymphocytes

<400> 164
Gly Leu Val Glu Phe Ala Arg Asn Leu
1 5

<210> 165
<211> 10
<212> PRT
<213> Artificial Sequence

<220>
<223> Description of Artificial Sequence:Designed peptide recognized by HLA-A2 restricted cytotoxic T lymphocytes

<400> 165
Phe Val Ala Leu Ser Asp Val Cys Asp Val
1 5 10

<210> 166
<211> 9
<212> PRT
<213> Artificial Sequence

<220>
<223> Description of Artificial Sequence:Designed peptide recognized by HLA-A2 restricted cytotoxic T lymphocytes

<400> 166
Arg Leu Asp Phe Asn Leu Ile Arg Val
1 5

<210> 167
<211> 9
<212> PRT
<213> Artificial Sequence

<220>
<223> Description of Artificial Sequence:Designed peptide recognized by HLA-A2 restricted cytotoxic T lymphocytes

<400> 167
Ile Leu Ala His Thr Asn Leu Arg Leu
1 5

<210> 168
<211> 10
<212> PRT
<213> Artificial Sequence

<220>
<223> Description of Artificial Sequence:Designed peptide recognized by HLA-A2 restricted cytotoxic T lymphocytes

<400> 168
Cys Met Val Tyr Asp Leu Tyr Lys Thr Leu
1 5 10

<210> 169
<211> 10
<212> PRT

<213> Artificial Sequence

<220>
<223> Description of Artificial Sequence:Designed peptide recognized by HLA-A2 restricted cytotoxic T lymphocytes

<400> 169
Trp Gln Leu Val Lys Glu Leu Lys Glu Ala
1 5 10

<210> 170
<211> 9
<212> PRT
<213> Artificial Sequence

<220>
<223> Description of Artificial Sequence:Designed peptide recognized by HLA-A2 restricted cytotoxic T lymphocytes

<400> 170
Leu Leu Leu Thr Ala Pro Asn Leu Leu
1 5

<210> 171
<211> 10
<212> PRT
<213> Artificial Sequence

<220>
<223> Description of Artificial Sequence:Designed peptide recognized by HLA-A2 restricted cytotoxic T lymphocytes

<400> 171
Ala Leu Phe Pro Gly Leu Ala Pro Glu Thr
1 5 10

<210> 172
<211> 9
<212> PRT
<213> Artificial Sequence

<220>

<223> Description of Artificial Sequence:Designed peptide recognized by HLA-A2 restricted cytotoxic T lymphocytes

<400> 172
Trp Leu Leu Gly Gly His Val Glu Leu
1 5

<210> 173
<211> 10
<212> PRT
<213> Artificial Sequence

<220>
<223> Description of Artificial Sequence:Designed peptide recognized by HLA-A2 restricted cytotoxic T lymphocytes

<400> 173
Phe Leu His Leu Leu Gln Ala Asp Asn Val
1 5 10

<210> 174
<211> 10
<212> PRT
<213> Artificial Sequence

<220>
<223> Description of Artificial Sequence:Designed peptide recognized by HLA-A2 restricted cytotoxic T lymphocytes

<400> 174
Leu Gln Ser Asp His Phe Leu His Leu Leu
1 5 10

<210> 175
<211> 10
<212> PRT
<213> Artificial Sequence

<220>
<223> Description of Artificial Sequence:Designed peptide recognized by HLA-A2 restricted cytotoxic T lymphocytes

<400> 175
Met Met Met Leu Gln Asn Ile Leu Gln Ile
1 5 10

<210> 176
<211> 10
<212> PRT
<213> Artificial Sequence

<220>
<223> Description of Artificial Sequence:Designed peptide recognized by HLA-A2 restricted cytotoxic T lymphocytes

<400> 176
Gln Leu Val Gly Leu Leu Ser Pro Met Val
1 5 10

<210> 177
<211> 10
<212> PRT
<213> Artificial Sequence

<220>
<223> Description of Artificial Sequence:Designed peptide recognized by HLA-A2 restricted cytotoxic T lymphocytes

<400> 177
Leu Leu Met Ala Glu Ser His Gln Glu Ile
1 5 10

<210> 178
<211> 9
<212> PRT
<213> Artificial Sequence

<220>
<223> Description of Artificial Sequence:Designed peptide recognized by HLA-A2 restricted cytotoxic T lymphocytes

<400> 178
Lys Leu His Gln Ala Ala Cys Leu Ile

1 5

<210> 179
<211> 9
<212> PRT
<213> Artificial Sequence

<220>
<223> Description of Artificial Sequence:Designed peptide recognized by HLA-A2 restricted cytotoxic T lymphocytes

<400> 179
Ile Leu Ser His Cys Cys Val Gly Leu
1 5

<210> 180
<211> 10
<212> PRT
<213> Artificial Sequence

<220>
<223> Description of Artificial Sequence:Designed peptide recognized by HLA-A2 restricted cytotoxic T lymphocytes

<400> 180
Ser Leu Phe Trp Leu Leu Gly Gly His Val
1 5 10

<210> 181
<211> 9
<212> PRT
<213> Artificial Sequence

<220>
<223> Description of Artificial Sequence:Designed peptide recognized by HLA-A2 restricted cytotoxic T lymphocytes

<400> 181
Lys Leu Phe Ala Pro Trp Arg Gly Leu
1 5

<210> 182
<211> 10
<212> PRT
<213> Artificial Sequence

<220>
<223> Description of Artificial Sequence:Designed peptide recognized by HLA-A2 restricted cytotoxic T lymphocytes

<400> 182
Lys Leu Gly Glu Glu Ser Gly Asp Glu Ile
1 5 10

<210> 183
<211> 10
<212> PRT
<213> Artificial Sequence

<220>
<223> Description of Artificial Sequence:Designed peptide recognized by HLA-A2 restricted cytotoxic T lymphocytes

<400> 183
Tyr Asp Tyr Asp Gly Tyr Arg Leu Arg Val
1 5 10

<210> 184
<211> 9
<212> PRT
<213> Artificial Sequence

<220>
<223> Description of Artificial Sequence:Designed peptide recognized by HLA-A2 restricted cytotoxic T lymphocytes

<400> 184
Arg Gly Gly Pro Pro Phe Ala Phe Val
1 5

<210> 185
<211> 9
<212> PRT

<213> Artificial Sequence

<220>
<223> Description of Artificial Sequence:Designed peptide recognized by HLA-A2 restricted cytotoxic T lymphocytes

<400> 185
Thr Leu Gly Asp Ala His Ile Tyr Leu
1 5

<210> 186
<211> 9
<212> PRT
<213> Artificial Sequence

<220>
<223> Description of Artificial Sequence:Designed peptide recognized by HLA-A2 restricted cytotoxic T lymphocytes

<400> 186
Tyr Met Ile Ala His Ile Thr Gly Leu
1 5

<210> 187
<211> 10
<212> PRT
<213> Artificial Sequence

<220>
<223> Description of Artificial Sequence:Designed peptide recognized by HLA-A2 restricted cytotoxic T lymphocytes

<400> 187
Tyr Leu Asn His Ile Glu Pro Leu Lys Ile
1 5 10

<210> 188
<211> 10
<212> PRT
<213> Artificial Sequence

<220>

<223> Description of Artificial Sequence:Designed peptide recognized by HLA-A2 restricted cytotoxic T lymphocytes

<400> 188
Leu Met Ala Leu Pro Pro Cys His Ala Leu
1 5 10

<210> 189
<211> 9
<212> PRT
<213> Artificial Sequence

<220>
<223> Description of Artificial Sequence:Designed peptide recognized by HLA-A2 restricted cytotoxic T lymphocytes

<400> 189
Lys Leu Leu Trp Thr Thr Ser Arg Val
1 5

<210> 190
<211> 10
<212> PRT
<213> Artificial Sequence

<220>
<223> Description of Artificial Sequence:Designed peptide recognized by HLA-A2 restricted cytotoxic T lymphocytes

<400> 190
Arg Leu Val Gln Asn Cys Leu Trp Thr Leu
1 5 10

<210> 191
<211> 9
<212> PRT
<213> Artificial Sequence

<220>
<223> Description of Artificial Sequence:Designed peptide recognized by HLA-A2 restricted cytotoxic T lymphocytes

<400> 191
Val Leu Phe Tyr Ala Ile Thr Thr Leu
1 5

<210> 192
<211> 9
<212> PRT
<213> Artificial Sequence

<220>
<223> Description of Artificial Sequence:Designed peptide recognized by HLA-A2 restricted cytotoxic T lymphocytes

<400> 192
Ile Met Phe Asp Val Thr Ser Arg Val
1 5

<210> 193
<211> 10
<212> PRT
<213> Artificial Sequence

<220>
<223> Description of Artificial Sequence:Designed peptide recognized by HLA-A2 restricted cytotoxic T lymphocytes

<400> 193
Leu Thr Gly Glu Phe Glu Lys Lys Tyr Val
1 5 10

<210> 194
<211> 10
<212> PRT
<213> Artificial Sequence

<220>
<223> Description of Artificial Sequence:Designed peptide recognized by HLA-A2 restricted cytotoxic T lymphocytes

<400> 194
Ala Leu Tyr Glu Lys Asp Asn Thr Tyr Leu

1 5 10

<210> 195
<211> 10
<212> PRT
<213> Artificial Sequence

<220>
<223> Description of Artificial Sequence:Designed peptide recognized by HLA-A2 restricted cytotoxic T lymphocytes

<400> 195
Phe Met Ile Leu Ala Ser Pro Arg Tyr Val
1 5 10

<210> 196
<211> 10
<212> PRT
<213> Artificial Sequence

<220>
<223> Description of Artificial Sequence:Designed peptide recognized by HLA-A2 restricted cytotoxic T lymphocytes

<400> 196
Lys Leu Thr Ser Leu Gln Leu Gln His Leu
1 5 10

<210> 197
<211> 10
<212> PRT
<213> Artificial Sequence

<220>
<223> Description of Artificial Sequence:Designed peptide recognized by HLA-A2 restricted cytotoxic T lymphocytes

<400> 197
Ser Leu Gln Leu Gln His Leu Phe Met Ile
1 5 10

<210> 198
<211> 9
<212> PRT
<213> Artificial Sequence

<220>
<223> Description of Artificial Sequence:Designed peptide recognized by HLA-A2 restricted cytotoxic T lymphocytes

<400> 198
Gln Val Leu Pro Met Leu Arg Phe Val
1 5

<210> 199
<211> 9
<212> PRT
<213> Artificial Sequence

<220>
<223> Description of Artificial Sequence:Designed peptide recognized by HLA-A2 restricted cytotoxic T lymphocytes

<400> 199
Lys Met Val Thr Met Val Ser Val Leu
1 5

<210> 200
<211> 9
<212> PRT
<213> Artificial Sequence

<220>
<223> Description of Artificial Sequence:Designed peptide recognized by HLA-A2 restricted cytotoxic T lymphocytes

<400> 200
Ala Leu Phe Lys Cys Tyr Met Phe Leu
1 5

<210> 201
<211> 9
<212> PRT

<213> Artificial Sequence

<220>
<223> Description of Artificial Sequence:Designed peptide recognized by HLA-A2 restricted cytotoxic T lymphocytes

<400> 201
Phe Leu Ala Leu Pro Leu Glu Asp Val
1 5

<210> 202
<211> 10
<212> PRT
<213> Artificial Sequence

<220>
<223> Description of Artificial Sequence:Designed peptide recognized by HLA-A2 restricted cytotoxic T lymphocytes

<400> 202
Arg Leu Pro Leu Cys Arg Pro Gln Phe Leu
1 5 10

<210> 203
<211> 9
<212> PRT
<213> Artificial Sequence

<220>
<223> Description of Artificial Sequence:Designed peptide recognized by HLA-A2 restricted cytotoxic T lymphocytes

<400> 203
Leu Met Pro Glu Arg Arg Pro His Leu
1 5

<210> 204
<211> 10
<212> PRT
<213> Artificial Sequence

<220>

<223> Description of Artificial Sequence:Designed peptide recognized by HLA-A2 restricted cytotoxic T lymphocytes

<400> 204
Phe Leu Gln Leu Gln Ser Ile Lys Asp Ala
1 5 10

<210> 205
<211> 9
<212> PRT
<213> Artificial Sequence

<220>
<223> Description of Artificial Sequence:Designed peptide recognized by HLA-A2 restricted cytotoxic T lymphocytes

<400> 205
Lys Ile Leu Phe Lys Thr Trp His Leu
1 5

<210> 206
<211> 9
<212> PRT
<213> Artificial Sequence

<220>
<223> Description of Artificial Sequence:Designed peptide recognized by HLA-A2 restricted cytotoxic T lymphocytes

<400> 206
Ile Leu Phe Lys Thr Trp His Leu Ile
1 5

<210> 207
<211> 9
<212> PRT
<213> Artificial Sequence

<220>
<223> Description of Artificial Sequence:Designed peptide recognized by HLA-A2 restricted cytotoxic T lymphocytes

<400> 207
Phe Leu Pro Pro Phe Ser Leu Ser Leu
1 5

<210> 208
<211> 10
<212> PRT
<213> Artificial Sequence

<220>
<223> Description of Artificial Sequence:Designed peptide recognized by HLA-A2 restricted cytotoxic T lymphocytes

<400> 208
Ser Leu Pro Leu Phe Leu Pro Pro Phe Leu
1 5 10

<210> 209
<211> 10
<212> PRT
<213> Artificial Sequence

<220>
<223> Description of Artificial Sequence:Designed peptide recognized by HLA-A2 restricted cytotoxic T lymphocytes

<400> 209
Gly Leu Tyr Phe Leu Tyr Ser Met Pro Val
1 5 10

<210> 210
<211> 10
<212> PRT
<213> Artificial Sequence

<220>
<223> Description of Artificial Sequence:Designed peptide recognized by HLA-A2 restricted cytotoxic T lymphocytes

<400> 210
Phe Val Gly Gly His Val Gly Trp Pro Thr

1 5 10

<210> 211
<211> 10
<212> PRT
<213> Artificial Sequence

<220>
<223> Description of Artificial Sequence:Designed peptide recognized by HLA-A2 restricted cytotoxic T lymphocytes

<400> 211
Arg Leu His Asn Asp Arg Val Tyr Tyr Val
1 5 10

<210> 212
<211> 10
<212> PRT
<213> Artificial Sequence

<220>
<223> Description of Artificial Sequence:Designed peptide recognized by HLA-A2 restricted cytotoxic T lymphocytes

<400> 212
Tyr Ile Gly Glu Asn Leu Gln Leu Leu Val
1 5 10

<210> 213
<211> 9
<212> PRT
<213> Artificial Sequence

<220>
<223> Description of Artificial Sequence:Designed peptide recognized by HLA-A2 restricted cytotoxic T lymphocytes

<400> 213
Tyr Val Ser Glu Lys Ile Met Lys Leu
1 5

<210> 214
<211> 335
<212> PRT
<213> Homo sapiens

<400> 214
Met Gly Lys Val Lys Val Gly Val Asn Gly Phe Gly Arg Ile Gly Arg
1 5 10 15

Leu Val Thr Arg Ala Ala Phe Asn Ser Gly Lys Val Asp Ile Val Ala
20 25 30

Ile Asn Asp Pro Phe Ile Asp Leu Asn Tyr Met Val Tyr Met Phe Gln
35 40 45

Tyr Asp Ser Thr His Gly Lys Phe His Gly Thr Val Lys Ala Glu Asn
50 55 60

Gly Lys Leu Val Ile Asn Gly Asn Pro Ile Thr Ile Phe Gln Glu Arg
65 70 75 80

Asp Pro Ser Lys Ile Lys Trp Gly Asp Ala Gly Ala Glu Tyr Val Val
85 90 95

Glu Ser Thr Gly Val Phe Thr Thr Met Glu Lys Ala Gly Ala His Leu
100 105 110

Gln Gly Gly Ala Lys Arg Val Ile Ile Ser Ala Pro Ser Ala Asp Ala
115 120 125

Pro Met Phe Val Met Gly Val Asn His Glu Lys Tyr Asp Asn Ser Leu
130 135 140

Lys Ile Ile Ser Asn Ala Ser Cys Thr Thr Asn Cys Leu Ala Pro Leu
145 150 155 160

Ala Lys Val Ile His Asp Asn Phe Gly Ile Val Glu Gly Leu Met Thr
165 170 175

Thr Val His Ala Ile Thr Ala Thr Gln Lys Thr Val Asp Gly Pro Ser
180 185 190

Gly Lys Leu Trp Arg Asp Gly Arg Gly Ala Leu Gln Asn Ile Ile Pro
195 200 205

Ala Ser Thr Gly Ala Ala Lys Ala Val Gly Lys Val Ile Pro Glu Leu
210 215 220

Asn Gly Lys Leu Thr Gly Met Ala Phe Arg Val Pro Thr Ala Asn Val
225 230 235 240

Ser Val Val Asp Leu Thr Cys Arg Leu Glu Lys Pro Ala Lys Tyr Asp
245 250 255

Asp Ile Lys Lys Val Val Lys Gln Ala Ser Glu Gly Pro Leu Lys Gly
260 265 270

Ile Leu Gly Tyr Thr Glu His Gln Val Val Ser Ser Asp Phe Asn Ser
275 280 285

Asp Thr His Ser Ser Thr Phe Asp Ala Gly Ala Gly Ile Ala Leu Asn
290 295 300

Asp His Phe Val Lys Leu Ile Ser Trp Tyr Asp Asn Glu Phe Gly Tyr
305 310 315 320

Ser Asn Arg Val Val Asp Leu Met Ala His Met Ala Ser Lys Glu
325 330 335

<210> 215
<211> 599
<212> PRT
<213> Homo sapiens

<400> 215
Met Ala Asp Lys Leu Thr Arg Ile Ala Ile Val Asn His Asp Lys Cys
1 5 10 15

Lys Pro Lys Lys Cys Arg Gln Glu Cys Lys Lys Ser Cys Pro Val Val
20 25 30

Arg Met Gly Lys Leu Cys Ile Glu Val Thr Pro Gln Ser Lys Ile Ala
35 40 45

Trp Ile Ser Glu Thr Leu Cys Ile Gly Cys Gly Ile Cys Ile Lys Lys
50 55 60

Cys Pro Phe Gly Ala Leu Ser Ile Val Asn Leu Pro Ser Asn Leu Glu
65 70 75 80

Lys Glu Thr Thr His Arg Tyr Cys Ala Asn Ala Phe Lys Leu His Arg
85 90 95

Leu Pro Ile Pro Arg Pro Gly Glu Val Leu Gly Leu Val Gly Thr Asn
100 105 110

Gly Ile Gly Lys Ser Thr Ala Leu Lys Ile Leu Ala Gly Lys Gln Lys
115 120 125

Pro Asn Leu Gly Lys Tyr Asp Asp Pro Pro Asp Trp Gln Glu Ile Leu
130 135 140

Thr Tyr Phe Arg Gly Ser Glu Leu Gln Asn Tyr Phe Thr Lys Ile Leu
145 150 155 160

Glu Asp Asp Leu Lys Ala Ile Ile Lys Pro Gln Tyr Val Asp Gln Ile
165 170 175

Pro Lys Ala Ala Lys Gly Thr Val Gly Ser Ile Leu Asp Arg Lys Asp
180 185 190

Glu Thr Lys Thr Gln Ala Ile Val Cys Gln Gln Leu Asp Leu Thr His
195 200 205

Leu Lys Glu Arg Asn Val Glu Asp Leu Ser Gly Gly Glu Leu Gln Arg
210 215 220

Phe Ala Cys Ala Val Val Cys Ile Gln Lys Ala Asp Ile Phe Met Phe
225 230 235 240

Asp Glu Pro Ser Ser Tyr Leu Asp Val Lys Gln Arg Leu Lys Ala Ala
245 250 . 255

Ile Thr Ile Arg Ser Leu Ile Asn Pro Asp Arg Tyr Ile Ile Val Val
260 265 270

Glu His Asp Leu Ser Val Leu Asp Tyr Leu Ser Asp Phe Ile Cys Cys
275 280 285

Leu Tyr Gly Val Pro Ser Ala Tyr Gly Val Val Thr Met Pro Phe Ser
290 295 300

Val Arg Glu Gly Ile Asn Ile Phe Leu Asp Gly Tyr Val Pro Thr Glu
305 310 315 320

Asn Leu Arg Phe Arg Asp Ala Ser Leu Val Phe Lys Val Ala Glu Thr
325 330 335

Ala Asn Glu Glu Glu Val Lys Lys Met Cys Met Tyr Lys Tyr Pro Gly
340 345 350

Met Lys Lys Lys Met Gly Glu Phe Glu Leu Ala Ile Val Ala Gly Glu
355 360 365

Phe Thr Asp Ser Glu Ile Met Val Met Leu Gly Glu Asn Gly Thr Gly
370 375 380

Lys Thr Thr Phe Ile Arg Met Leu Ala Gly Arg Leu Lys Pro Asp Glu
385 390 395 400

Gly Gly Glu Val Pro Val Leu Asn Val Ser Tyr Lys Pro Gln Lys Ile
405 410 415

Ser Pro Lys Ser Thr Gly Ser Val Arg Gln Leu Leu His Glu Lys Ile
420 425 430

Arg Asp Ala Tyr Thr His Pro Gln Phe Val Thr Asp Val Met Lys Pro
435 440 445

Leu Gln Ile Glu Asn Ile Ile Asp Gln Glu Val Gln Thr Leu Ser Gly
450 455 460

Gly Glu Leu Gln Arg Val Ala Leu Ala Leu Cys Leu Gly Lys Pro Ala
465 470 475 480

Asp Val Tyr Leu Ile Asp Glu Pro Ser Ala Tyr Leu Asp Ser Glu Gln
485 490 495

Arg Leu Met Ala Ala Arg Val Val Lys Arg Phe Ile Leu His Ala Lys
500 505 510

Lys Thr Ala Phe Val Val Glu His Asp Phe Ile Met Ala Thr Tyr Leu
515 520 525

Ala Asp Arg Val Ile Val Phe Asp Gly Val Pro Ser Lys Asn Thr Val
530 535 540

Ala Asn Ser Pro Gln Thr Leu Leu Ala Gly Met Asn Lys Phe Leu Ser
545 550 555 560

Gln Leu Glu Ile Thr Phe Arg Arg Asp Pro Asn Asn Tyr Arg Pro Arg
565 570 575

Ile Asn Lys Leu Asn Ser Ile Lys Asp Val Glu Gln Lys Lys Ser Gly
580 585 590

Asn Tyr Phe Phe Leu Asp Asp
595

<210> 216

<211> 101
<212> PRT
<213> Homo sapiens

<400> 216
Met Ser Asp Gln Glu Ala Lys Pro Ser Thr Glu Asp Leu Gly Asp Lys
1               5                   10                  15

Lys Glu Gly Glu Tyr Ile Lys Leu Lys Val Ile Gly Gln Asp Ser Ser
            20                  25                  30

Glu Ile His Phe Lys Val Lys Met Thr Thr His Leu Lys Lys Leu Lys
        35                  40                  45

Glu Ser Tyr Cys Gln Arg Gln Gly Val Pro Met Asn Ser Leu Arg Phe
    50                  55                  60

Leu Phe Glu Gly Gln Arg Ile Ala Asp Asn His Thr Pro Lys Glu Leu
65                  70                  75                  80

Gly Met Glu Glu Glu Asp Val Ile Glu Val Tyr Gln Glu Gln Thr Gly
                85                  90                  95

Gly His Ser Thr Val
            100

<210> 217
<211> 249
<212> PRT
<213> Homo sapiens

<400> 217
Met Lys Leu Asn Ile Ser Phe Pro Ala Thr Gly Cys Gln Lys Leu Ile
1               5                   10                  15

Glu Val Asp Asp Glu Arg Lys Leu Arg Thr Phe Tyr Glu Lys Arg Met
            20                  25                  30

Ala Thr Glu Val Ala Ala Asp Ala Leu Gly Glu Glu Trp Lys Gly Tyr
        35                  40                  45

Val Val Arg Ile Ser Gly Gly Asn Asp Lys Gln Gly Phe Pro Met Lys
    50                  55                  60

Gln Gly Val Leu Thr His Gly Arg Val Arg Leu Leu Leu Ser Lys Gly
65                  70                  75                  80

His Ser Cys Tyr Arg Pro Arg Arg Thr Gly Glu Arg Lys Arg Lys Ser
85 90 95

Val Arg Gly Cys Ile Val Asp Ala Asn Leu Ser Val Leu Asn Leu Val
100 105 110

Ile Val Lys Lys Gly Glu Lys Asp Ile Pro Gly Leu Thr Asp Thr Thr
115 120 125

Val Pro Arg Arg Leu Gly Pro Lys Arg Ala Ser Arg Ile Arg Lys Leu
130 135 140

Phe Asn Leu Ser Lys Glu Asp Asp Val Arg Gln Tyr Val Val Arg Lys
145 150 155 160

Pro Leu Asn Lys Glu Gly Lys Lys Pro Arg Thr Lys Ala Pro Lys Ile
165 170 175

Gln Arg Leu Val Thr Pro Arg Val Leu Gln His Lys Arg Arg Arg Ile
180 185 190

Ala Leu Lys Lys Gln Arg Thr Lys Lys Asn Lys Glu Glu Ala Ala Glu
195 200 205

Tyr Ala Lys Leu Leu Ala Lys Arg Met Lys Glu Ala Lys Glu Lys Arg
210 215 220

Gln Glu Gln Ile Ala Lys Arg Arg Arg Leu Ser Ser Leu Arg Ala Ser
225 230 235 240

Thr Ser Lys Ser Glu Ser Ser Gln Lys
245

<210> 218
<211> 184
<212> PRT
<213> Homo sapiens

<400> 218
Met Arg Glu Tyr Lys Leu Val Val Leu Gly Ser Gly Gly Val Gly Lys
1 5 10 15

Ser Ala Leu Thr Val Gln Phe Val Gln Gly Ile Phe Val Glu Lys Tyr
20 25 30

Asp Pro Thr Ile Glu Asp Ser Tyr Arg Lys Gln Val Glu Val Asp Ala
35 40 45

Gln Gln Cys Met Leu Glu Ile Leu Asp Thr Ala Gly Thr Glu Gln Phe
50 55 60

Thr Ala Met Arg Asp Leu Tyr Met Lys Asn Gly Gln Gly Phe Ala Leu
65 70 75 80

Val Tyr Ser Ile Thr Ala Gln Ser Thr Phe Asn Asp Leu Gln Asp Leu
85 90 95

Arg Glu Gln Ile Leu Arg Val Lys Asp Thr Asp Asp Val Pro Met Ile
100 105 110

Leu Val Gly Asn Lys Cys Asp Leu Glu Asp Glu Arg Val Val Gly Lys
115 120 125

Glu Gln Gly Gln Asn Leu Ala Arg Gln Trp Asn Asn Cys Ala Phe Leu
130 135 140

Glu Ser Ser Ala Lys Ser Lys Ile Asn Val Asn Glu Ile Phe Tyr Asp
145 150 155 160

Leu Val Arg Gln Ile Asn Arg Lys Thr Pro Val Pro Gly Lys Ala Arg
165 170 175

Lys Lys Ser Ser Cys Gln Leu Leu
180

<210> 219
<211> 162
<212> PRT
<213> Homo sapiens

<400> 219
Met Lys Glu Thr Ile Met Asn Gln Glu Lys Leu Ala Lys Leu Gln Ala
1 5 10 15

Gln Val Arg Ile Gly Gly Lys Gly Thr Ala Arg Arg Lys Lys Lys Val
20 25 30

Val His Arg Thr Ala Thr Ala Asp Asp Lys Lys Leu Gln Phe Ser Leu
35 40 45

Lys Lys Leu Gly Val Asn Asn Ile Ser Gly Ile Glu Glu Val Asn Met
50 55 60

Phe Thr Asn Gln Gly Thr Val Ile His Phe Asn Asn Pro Lys Val Gln

65 70 75 80

Ala Ser Leu Ala Ala Asn Thr Phe Thr Ile Thr Gly His Ala Glu Thr
85 90 95

Lys Gln Leu Thr Glu Met Leu Pro Ser Ile Leu Asn Gln Leu Gly Ala
100 105 110

Asp Ser Leu Thr Ser Leu Arg Arg Leu Ala Glu Ala Leu Pro Lys Gln
115 120 125

Ser Val Asp Gly Lys Ala Pro Leu Ala Thr Gly Glu Asp Asp Asp Asp
130 135 140

Glu Val Pro Asp Leu Val Glu Asn Phe Asp Glu Ala Ser Lys Asn Glu
145 150 155 160

Ala Asn

<210> 220
<211> 180
<212> PRT
<213> Homo sapiens

<400> 220
Met Arg Pro Leu Thr Glu Glu Glu Thr Arg Val Met Phe Glu Lys Ile
1 5 10 15

Ala Lys Tyr Ile Gly Glu Asn Leu Gln Leu Leu Val Asp Arg Pro Asp
20 25 30

Gly Thr Tyr Cys Phe Arg Leu His Asn Asp Arg Val Tyr Tyr Val Ser
35 40 45

Glu Lys Ile Met Lys Leu Ala Ala Asn Ile Ser Gly Asp Lys Leu Val
50 55 60

Ser Leu Gly Thr Cys Phe Gly Lys Phe Thr Lys Thr His Lys Phe Arg
65 70 75 80

Leu His Val Thr Ala Leu Asp Tyr Leu Ala Pro Tyr Ala Lys Tyr Lys
85 90 95

Val Trp Ile Lys Pro Gly Ala Glu Gln Ser Phe Leu Tyr Gly Asn His
100 105 110

Val Leu Lys Ser Gly Leu Gly Arg Ile Thr Glu Asn Thr Ser Gln Tyr
115 120 125

Gln Gly Val Val Val Tyr Ser Met Ala Asp Ile Pro Leu Gly Phe Gly
130 135 140

Val Ala Ala Lys Ser Thr Gln Asp Cys Arg Lys Val Asp Pro Met Ala
145 150 155 160

Ile Val Val Phe His Gln Ala Asp Ile Gly Glu Tyr Val Arg His Glu
165 170 175

Glu Thr Leu Thr
180

<210> 221
<211> 166
<212> PRT
<213> Homo sapiens

<400> 221
Met Ala Ala Thr Met Phe Arg Ala Thr Leu Arg Gly Trp Arg Thr Gly
1 5 10 15

Val Gln Arg Gly Cys Gly Leu Arg Leu Leu Ser Gln Thr Gln Gly Pro
20 25 30

Pro Asp Tyr Pro Arg Phe Val Glu Ser Val Asp Glu Tyr Gln Phe Val
35 40 45

Glu Arg Leu Leu Pro Ala Thr Arg Ile Pro Asp Pro Pro Lys His Glu
50 55 60

His Tyr Pro Thr Pro Ser Gly Trp Gln Pro Pro Arg Asp Pro Pro Pro
65 70 75 80

Asn Leu Pro Tyr Phe Val Arg Arg Ser Arg Met His Asn Ile Pro Val
85 90 95

Tyr Lys Asp Ile Thr His Gly Asn Arg Gln Met Thr Val Ile Arg Lys
100 105 110

Val Glu Gly Asp Ile Trp Ala Leu Gln Lys Asp Val Glu Asp Phe Leu
115 120 125

Ser Pro Leu Leu Gly Lys Thr Pro Val Thr Gln Val Asn Glu Val Thr
130 135 140

Gly Thr Leu Arg Ile Lys Gly Tyr Phe Asp Gln Glu Leu Lys Ala Trp
145 150 155 160

Leu Leu Glu Lys Gly Phe
165

<210> 222
<211> 194
<212> PRT
<213> Homo sapiens

<400> 222
Met Ala Ala Ser Leu Val Gly Lys Lys Ile Val Phe Val Thr Gly Asn
1 5 10 15

Ala Lys Lys Leu Glu Glu Val Val Gln Ile Leu Gly Asp Lys Phe Pro
20 25 30

Cys Thr Leu Val Ala Gln Lys Ile Asp Leu Pro Glu Tyr Gln Gly Glu
35 40 45

Pro Asp Glu Ile Ser Ile Gln Lys Cys Gln Glu Ala Val Arg Gln Val
50 55 60

Gln Gly Pro Val Leu Val Glu Asp Thr Cys Leu Cys Phe Asn Ala Leu
65 70 75 80

Gly Gly Leu Pro Gly Pro Tyr Ile Lys Trp Phe Leu Glu Lys Leu Lys
85 90 95

Pro Glu Gly Leu His Gln Leu Leu Ala Gly Phe Glu Asp Lys Ser Ala
100 105 110

Tyr Ala Leu Cys Thr Phe Ala Leu Ser Thr Gly Asp Pro Ser Gln Pro
115 120 125

Val Arg Leu Phe Arg Gly Arg Thr Ser Gly Arg Ile Val Ala Pro Arg
130 135 140

Gly Cys Gln Asp Phe Gly Trp Asp Pro Cys Phe Gln Pro Asp Gly Tyr
145 150 155 160

Glu Gln Thr Tyr Ala Glu Met Pro Lys Ala Glu Lys Asn Ala Val Ser
165 170 175

His Arg Phe Arg Ala Leu Leu Glu Leu Gln Glu Tyr Phe Gly Ser Leu

180 185 190

Ala Ala

<210> 223
<211> 466
<212> PRT
<213> Homo sapiens

<400> 223
Met Ser Tyr Pro Gly Tyr Pro Pro Thr Gly Tyr Pro Pro Phe Pro Gly
1 5 10 15

Tyr Pro Pro Ala Gly Gln Glu Ser Ser Phe Pro Pro Ser Gly Gln Tyr
20 25 30

Pro Tyr Pro Ser Gly Phe Pro Pro Met Gly Gly Gly Ala Tyr Pro Gln
35 40 45

Val Pro Ser Ser Gly Tyr Pro Gly Ala Gly Gly Tyr Pro Ala Pro Gly
50 55 60

Gly Tyr Pro Ala Pro Gly Gly Tyr Pro Gly Ala Pro Gln Pro Gly Gly
65 70 75 80

Ala Pro Ser Tyr Pro Gly Val Pro Pro Gly Gln Gly Phe Gly Val Pro
85 90 95

Pro Gly Gly Ala Gly Phe Ser Gly Tyr Pro Gln Pro Pro Ser Gln Ser
100 105 110

Tyr Gly Gly Gly Pro Ala Gln Val Pro Leu Pro Gly Gly Phe Pro Gly
115 120 125

Gly Gln Met Pro Ser Gln Tyr Pro Gly Gly Gln Pro Thr Tyr Pro Ser
130 135 140

Gln Pro Ala Thr Val Thr Gln Val Thr Gln Gly Thr Ile Arg Pro Ala
145 150 155 160

Ala Asn Phe Asp Ala Ile Arg Asp Ala Glu Ile Leu Arg Lys Ala Met
165 170 175

Lys Gly Phe Gly Thr Asp Glu Gln Ala Ile Val Asp Val Val Ala Asn
180 185 190

Arg Ser Asn Asp Gln Arg Gln Lys Ile Lys Ala Ala Phe Lys Thr Ser
195 200 205

Tyr Gly Lys Asp Leu Ile Lys Asp Leu Lys Ser Glu Leu Ser Gly Asn
210 215 220

Met Glu Glu Leu Ile Leu Ala Leu Phe Met Pro Pro Thr Tyr Tyr Asp
225 230 235 240

Ala Trp Ser Leu Arg Lys Ala Met Gln Gly Ala Gly Thr Gln Glu Arg
245 250 255

Val Leu Ile Glu Ile Leu Cys Thr Arg Thr Asn Gln Glu Ile Arg Glu
260 265 270

Ile Val Arg Cys Tyr Gln Ser Glu Phe Gly Arg Asp Leu Glu Lys Asp
275 280 285

Ile Arg Ser Asp Thr Ser Gly His Phe Glu Arg Leu Leu Val Ser Met
290 295 300

Cys Gln Gly Asn Arg Asp Glu Asn Gln Ser Ile Asn His Gln Met Ala
305 310 315 320

Gln Glu Asp Ala Gln Arg Leu Tyr Gln Ala Gly Glu Gly Arg Leu Gly
325 330 335

Thr Asp Glu Ser Cys Phe Asn Met Ile Leu Ala Thr Arg Ser Phe Pro
340 345 350

Gln Leu Arg Ala Thr Met Glu Ala Tyr Ser Arg Met Ala Asn Arg Asp
355 360 365

Leu Leu Ser Ser Val Ser Arg Glu Phe Ser Gly Tyr Val Glu Ser Gly
370 375 380

Leu Lys Thr Ile Leu Gln Cys Ala Leu Asn Arg Pro Ala Phe Phe Ala
385 390 395 400

Glu Arg Leu Tyr Tyr Ala Met Lys Gly Ala Gly Thr Asp Asp Ser Thr
405 410 415

Leu Val Arg Ile Val Val Thr Arg Ser Glu Ile Asp Leu Val Gln Ile
420 425 430

Lys Gln Met Phe Ala Gln Met Tyr Gln Lys Thr Leu Gly Thr Met Ile
435 440 445

Ala Gly Asp Thr Ser Gly Asp Tyr Arg Arg Leu Leu Leu Ala Ile Val
450 455 460

Gly Gln
465

<210> 224
<211> 130
<212> PRT
<213> Homo sapiens

<400> 224
Met Val Arg Met Asn Val Leu Ala Asp Ala Leu Lys Ser Ile Asn Asn
1 5 10 15

Ala Glu Lys Arg Gly Lys Arg Gln Val Leu Ile Arg Pro Cys Ser Lys
20 25 30

Val Ile Val Arg Phe Leu Thr Val Met Met Lys His Gly Tyr Ile Gly
35 40 45

Glu Phe Glu Ile Ile Asp Asp His Arg Ala Gly Lys Ile Val Val Asn
50 55 60

Leu Thr Gly Arg Leu Asn Lys Cys Gly Val Ile Ser Pro Arg Phe Asp
65 70 75 80

Val Gln Leu Lys Asp Leu Glu Lys Trp Gln Asn Asn Leu Leu Pro Ser
85 90 95

Arg Gln Phe Gly Phe Ile Val Leu Thr Thr Ser Ala Gly Ile Met Asp
100 105 110

His Glu Glu Ala Arg Arg Lys His Thr Gly Gly Lys Ile Leu Gly Phe
115 120 125

Phe Phe
130

<210> 225
<211> 192
<212> PRT
<213> Homo sapiens

<400> 225
Met Lys Thr Ile Leu Ser Asn Gln Thr Val Asp Ile Pro Glu Asn Val

1 5 10 15

Asp Ile Thr Leu Lys Gly Arg Thr Val Ile Val Lys Gly Pro Arg Gly
20 25 30

Thr Leu Arg Arg Asp Phe Asn His Ile Asn Val Glu Leu Ser Leu Leu
35 40 45

Gly Lys Lys Lys Lys Arg Leu Arg Val Asp Lys Trp Trp Gly Asn Arg
50 55 60

Lys Glu Leu Ala Thr Val Arg Thr Ile Cys Ser His Val Gln Asn Met
65 70 75 80

Ile Lys Gly Val Thr Leu Gly Phe Arg Tyr Lys Met Arg Ser Val Tyr
85 90 95

Ala His Phe Pro Ile Asn Val Val Ile Gln Glu Asn Gly Ser Leu Val
100 105 110

Glu Ile Arg Asn Phe Leu Gly Glu Lys Tyr Ile Arg Arg Val Arg Met
115 120 125

Arg Pro Gly Val Ala Cys Ser Val Ser Gln Ala Gln Lys Asp Glu Leu
130 135 140

Ile Leu Glu Gly Asn Asp Ile Glu Leu Val Ser Asn Ser Ala Ala Leu
145 150 155 160

Ile Gln Gln Ala Thr Thr Val Lys Asn Lys Asp Ile Arg Lys Phe Leu
165 170 175

Asp Gly Ile Tyr Val Ser Glu Lys Gly Thr Val Gln Gln Ala Asp Glu
180 185 190

<210> 226
<211> 67
<212> PRT
<213> Homo sapiens

<400> 226
Met Leu Leu Tyr Ile Asn Arg Ala Arg Pro Glu Gly Gly Arg Gly Ala
1 5 10 15

Gly Ala Glu Gly Arg Ser Asn Gln Ile Ser Asn Phe Leu Leu Ile Ile
20 25 30

Asn Pro Leu Phe Thr Ala Val Ser Val Val Ile Phe Lys Ile Phe Leu
35 40 45

Ile Phe Phe Phe Phe Leu Leu Leu Leu Phe Thr Ser Cys Val Tyr Val
50 55 60

Gly Asn Leu
65

<210> 227
<211> 66
<212> PRT
<213> Homo sapiens

<400> 227
Met His Phe His Asn Ile Cys Leu Leu Glu Arg Ser Ile Ile Ser Glu
1 5 10 15

Lys Tyr Gln Val Phe Ile Lys Phe Leu Gly Met Ala Asp Ser Gln Asn
20 25 30

Met Leu Val Ser Leu Gln Tyr Ser Ser Arg Arg Ala Asn Gln Gly Arg
35 40 45

Ala Gly Met Arg Ser Asp Ile Cys Val Thr Lys Ser Ile Phe Leu Ile
50 55 60

Ser Leu
65

<210> 228
<211> 145
<212> PRT
<213> Homo sapiens

<400> 228
Met Ile Leu Gln Cys Ser Ile Glu Met Pro Asn Ile Ser Tyr Ala Trp
1 5 10 15

Lys Glu Leu Lys Glu Gln Leu Gly Glu Glu Ile Asp Ser Lys Val Lys
20 25 30

Gly Met Val Phe Leu Lys Gly Lys Leu Gly Val Cys Phe Asp Val Pro

35 40 45

Thr Ala Ser Val Thr Glu Ile Gln Glu Lys Trp His Asp Ser Arg Arg
50 55 60

Trp Gln Leu Ser Val Ala Thr Glu Gln Pro Glu Leu Glu Gly Pro Arg
65 70 75 80

Glu Gly Tyr Gly Gly Phe Arg Gly Gln Arg Glu Gly Ser Arg Gly Phe
85 90 95

Arg Gly Gln Arg Asp Gly Asn Arg Arg Phe Arg Gly Gln Arg Glu Gly
100 105 110

Ser Arg Gly Pro Arg Gly Gln Arg Ser Gly Gly Gly Asn Lys Ser Asn
115 120 125

Arg Ser Gln Asn Lys Gly Gln Lys Arg Ser Phe Ser Lys Ala Phe Gly
130 135 140

Gln
145

<210> 229
<211> 49
<212> PRT
<213> Homo sapiens

<400> 229
Met Arg Asn Ser Ala Thr Phe Lys Ser Phe Glu Asp Arg Val Gly Thr
1 5 10 15

Ile Lys Ser Lys Val Val Gly Asp Arg Glu Asn Gly Ser Asp Asn Leu
20 25 30

Pro Ser Ser Ala Gly Ser Gly Asp Lys Pro Leu Ser Asp Pro Ala Pro
35 40 45

Phe

<210> 230
<211> 208
<212> PRT
<213> Homo sapiens

<400> 230

Met Gly Ile Ser Arg Asp Asn Trp His Lys Arg Arg Lys Thr Gly Gly
1               5                   10                  15

Lys Arg Lys Pro Tyr His Lys Lys Arg Lys Tyr Glu Leu Gly Arg Pro
            20                  25                  30

Ala Ala Asn Thr Lys Ile Gly Pro Arg Arg Ile His Thr Val Arg Val
        35                  40                  45

Arg Gly Gly Asn Lys Lys Tyr Arg Ala Leu Arg Leu Asp Val Gly Asn
    50                  55                  60

Phe Ser Trp Gly Ser Glu Cys Cys Thr Arg Lys Thr Arg Ile Ile Asp
65                  70                  75                  80

Val Val Tyr Asn Ala Ser Asn Asn Glu Leu Val Arg Thr Lys Thr Leu
                85                  90                  95

Val Lys Asn Cys Ile Val Leu Ile Asp Ser Thr Pro Tyr Arg Gln Trp
            100                 105                 110

Tyr Glu Ser His Tyr Ala Leu Pro Leu Gly Arg Lys Lys Gly Ala Lys
        115                 120                 125

Leu Thr Pro Glu Glu Glu Glu Ile Leu Asn Lys Lys Arg Ser Lys Lys
    130                 135                 140

Ile Gln Lys Lys Tyr Asp Glu Arg Lys Lys Asn Ala Lys Ile Ser Ser
145                 150                 155                 160

Leu Leu Glu Glu Gln Phe Gln Gln Gly Lys Leu Leu Ala Cys Ile Ala
                165                 170                 175

Ser Arg Pro Gly Gln Cys Gly Arg Ala Asp Gly Tyr Val Leu Glu Gly
            180                 185                 190

Lys Glu Leu Glu Phe Tyr Leu Arg Lys Ile Lys Ala Arg Lys Gly Lys
        195                 200                 205

<210> 231
<211> 183
<212> PRT
<213> Homo sapiens

<400> 231
Met Thr Thr Ala Ser Thr Ser Gln Val Arg Gln Asn Tyr His Gln Asp
1 5 10 15

Ser Glu Ala Ala Ile Asn Arg Gln Ile Asn Leu Glu Leu Tyr Ala Ser
20 25 30

Tyr Val Tyr Leu Ser Met Ser Tyr Tyr Phe Asp Arg Asp Asp Val Ala
35 40 45

Leu Lys Asn Phe Ala Lys Tyr Phe Leu His Gln Ser His Glu Glu Arg
50 55 60

Glu His Ala Glu Lys Leu Met Lys Leu Gln Asn Gln Arg Gly Gly Arg
65 70 75 80

Ile Phe Leu Gln Asp Ile Lys Lys Pro Asp Cys Asp Asp Trp Glu Ser
85 90 95

Gly Leu Asn Ala Met Glu Cys Ala Leu His Leu Glu Lys Asn Val Asn
100 105 110

Gln Ser Leu Leu Glu Leu His Lys Leu Ala Thr Asp Lys Asn Asp Pro
115 120 125

His Leu Cys Asp Phe Ile Glu Thr His Tyr Leu Asn Glu Gln Val Lys
130 135 140

Ala Ile Lys Glu Leu Gly Asp His Val Thr Asn Leu Arg Lys Met Gly
145 150 155 160

Ala Pro Glu Ser Gly Leu Ala Glu Tyr Leu Phe Asp Lys His Thr Leu
165 170 175

Gly Asp Ser Asp Asn Glu Ser
180

<210> 232
<211> 403
<212> PRT
<213> Homo sapiens

<400> 232
Met Ser His Arg Lys Phe Ser Ala Pro Arg His Gly Ser Leu Gly Phe
1 5 10 15

Leu Pro Arg Lys Arg Ser Ser Arg His Arg Gly Lys Val Lys Ser Phe
20 25 30

Pro Lys Asp Asp Pro Ser Lys Pro Val His Leu Thr Ala Phe Leu Gly
35 40 45

Tyr Lys Ala Gly Met Thr His Ile Val Arg Glu Val Asp Arg Pro Gly
50 55 60

Ser Lys Val Asn Lys Lys Glu Val Val Glu Ala Val Thr Ile Val Glu
65 70 75 80

Thr Pro Pro Met Val Val Val Gly Ile Val Gly Tyr Val Glu Thr Pro
85 90 95

Arg Gly Leu Arg Thr Phe Lys Thr Val Phe Ala Glu His Ile Ser Asp
100 105 110

Glu Cys Lys Arg Arg Phe Tyr Lys Asn Trp His Lys Ser Lys Lys Lys
115 120 125

Ala Phe Thr Lys Tyr Cys Lys Lys Trp Gln Asp Glu Asp Gly Lys Lys
130 135 140

Gln Leu Glu Lys Asp Phe Ser Ser Met Lys Lys Tyr Cys Gln Val Ile
145 150 155 160

Arg Val Ile Ala His Thr Gln Met Arg Leu Leu Pro Leu Arg Gln Lys
165 170 175

Lys Ala His Leu Met Glu Ile Gln Val Asn Gly Gly Thr Val Ala Glu
180 185 190

Lys Leu Asp Trp Ala Arg Glu Arg Leu Glu Gln Gln Val Pro Val Asn
195 200 205

Gln Val Phe Gly Gln Asp Glu Met Ile Asp Val Ile Gly Val Thr Lys
210 215 220

Gly Lys Gly Tyr Lys Gly Val Thr Ser Arg Trp His Thr Lys Lys Leu
225 230 235 240

Pro Arg Lys Thr His Arg Gly Leu Arg Lys Val Ala Cys Ile Gly Ala
245 250 255

Trp His Pro Ala Arg Val Ala Phe Ser Val Ala Arg Ala Gly Gln Lys
260 265 270

Gly Tyr His His Arg Thr Glu Ile Asn Lys Lys Ile Tyr Lys Ile Gly
275 280 285

Gln Gly Tyr Leu Ile Lys Asp Gly Lys Leu Ile Lys Asn Asn Ala Ser
290 295 300

Thr Asp Tyr Asp Leu Ser Asp Lys Ser Ile Asn Pro Leu Gly Gly Phe
305 310 315 320

Val His Tyr Gly Glu Val Thr Asn Asp Phe Val Met Leu Lys Gly Cys
325 330 335

Val Val Gly Thr Lys Lys Arg Val Leu Thr Leu Arg Lys Ser Leu Leu
340 345 350

Val Gln Thr Lys Arg Arg Ala Leu Glu Lys Ile Asp Leu Lys Phe Ile
355 360 365

Asp Thr Thr Ser Lys Phe Gly His Gly Arg Phe Gln Thr Met Glu Glu
370 375 380

Lys Lys Ala Phe Met Gly Pro Leu Lys Lys Asp Arg Ile Ala Lys Glu
385 390 395 400

Glu Gly Ala

<210> 233
<211> 480
<212> PRT
<213> Homo sapiens

<400> 233
Met Ala Val Ala Arg Ala Ala Leu Gly Pro Leu Val Thr Gly Leu Tyr
1 5 10 15

Asp Val Gln Ala Phe Lys Phe Gly Asp Phe Val Leu Lys Ser Gly Leu
20 25 30

Ser Ser Pro Ile Tyr Ile Asp Leu Arg Gly Ile Val Ser Arg Pro Arg
35 40 45

Leu Leu Ser Gln Val Ala Asp Ile Leu Phe Gln Thr Ala Gln Asn Ala
50 55 60

Gly Ile Ser Phe Asp Thr Val Cys Gly Val Pro Tyr Thr Ala Leu Pro
65 70 75 80

Leu Ala Thr Val Ile Cys Ser Thr Asn Gln Ile Pro Met Leu Ile Arg
85 90 95

Arg Lys Glu Thr Lys Asp Tyr Gly Thr Lys Arg Leu Val Glu Gly Thr
100 105 110

Ile Asn Pro Gly Glu Thr Cys Leu Ile Ile Glu Asp Val Val Thr Ser
115 120 125

Gly Ser Ser Val Leu Glu Thr Val Glu Val Leu Gln Lys Glu Gly Leu
130 135 140

Lys Val Thr Asp Ala Ile Val Leu Leu Asp Arg Glu Gln Gly Gly Lys
145 150 155 160

Asp Lys Leu Gln Ala His Gly Ile Arg Leu His Ser Val Cys Thr Leu
165 170 175

Ser Lys Met Leu Glu Ile Leu Glu Gln Gln Lys Lys Val Asp Ala Glu
180 185 190

Thr Val Gly Arg Val Lys Arg Phe Ile Gln Glu Asn Val Phe Val Ala
195 200 205

Ala Asn His Asn Gly Ser Pro Leu Ser Ile Lys Glu Ala Pro Lys Glu
210 215 220

Leu Ser Phe Gly Ala Arg Ala Glu Leu Pro Arg Ile His Pro Val Ala
225 230 235 240

Ser Lys Leu Leu Arg Leu Met Gln Lys Lys Glu Thr Asn Leu Cys Leu
245 250 255

Ser Ala Asp Val Ser Leu Ala Arg Glu Leu Leu Gln Leu Ala Asp Ala
260 265 270

Leu Gly Pro Ser Ile Cys Met Leu Lys Thr His Val Asp Ile Leu Asn
275 280 285

Asp Phe Thr Leu Asp Val Met Lys Glu Leu Ile Thr Leu Ala Lys Cys
290 295 300

His Glu Phe Leu Ile Phe Glu Asp Arg Lys Phe Ala Asp Ile Gly Asn
305 310 315 320

Thr Val Lys Lys Gln Tyr Glu Gly Gly Ile Phe Lys Ile Ala Ser Trp
325 330 335

Ala Asp Leu Val Asn Ala His Val Val Pro Gly Ser Gly Val Val Lys
340 345 350

Gly Leu Gln Glu Val Gly Leu Pro Leu His Arg Gly Cys Leu Leu Ile
355 360 365

Ala Glu Met Ser Ser Thr Gly Ser Leu Ala Thr Gly Asp Tyr Thr Arg
370 375 380

Ala Ala Val Arg Met Ala Glu Glu His Ser Glu Phe Val Val Gly Phe
385 390 395 400

Ile Ser Gly Ser Arg Val Ser Met Lys Pro Glu Phe Leu His Leu Thr
405 410 415

Pro Gly Val Gln Leu Glu Ala Gly Gly Asp Asn Leu Gly Gln Gln Tyr
420 425 430

Asn Ser Pro Gln Glu Val Ile Gly Lys Arg Gly Ser Asp Ile Ile Ile
435 440 445

Val Gly Arg Gly Ile Ile Ser Ala Ala Asp Arg Leu Glu Ala Ala Glu
450 455 460

Met Tyr Arg Lys Ala Ala Trp Glu Ala Tyr Leu Ser Arg Leu Gly Val
465 470 475 480

<210> 234
<211> 86
<212> PRT
<213> Homo sapiens

<400> 234
Met Tyr Leu Tyr Leu Ile Ser Ser Cys Ile Lys Pro Ile Asn Leu Cys
1 5 10 15

Tyr Cys Ser Ser Asn Leu Met His Thr Val Ile Ser Cys Tyr Ile Cys
20 25 30

Lys Val Gly Asn Cys Phe Leu Ser Tyr Arg Ser Phe Lys Leu His Phe
35 40 45

Cys Ala Val Glu Thr Lys Val Gly Tyr Ser Leu Cys His Val Asp Val

50 55 60

Gln Phe Leu Lys Leu Phe Tyr Lys Thr Leu Ile Ile Lys Pro Leu Asn
65 70 75 80

Leu Lys Lys Lys Lys Lys
85

<210> 235
<211> 54
<212> PRT
<213> Homo sapiens

<400> 235
Met Leu Cys Gly Asn Ile Tyr Pro Ile Asp His Pro Ile Leu Met Cys
1 5 10 15

Leu Trp Leu Ser Asp Gln Leu Gln Asn Asn Cys Val Val Ile Leu Cys
20 25 30

Pro Lys Leu Leu Ile Asn Phe Tyr Leu Gln Ile Glu Lys Glu Gly Pro
35 40 45

Cys Lys Glu Asn Gly Lys
50

<210> 236
<211> 672
<212> PRT
<213> Homo sapiens

<400> 236
Met Gly Val Gly Arg Leu Asp Met Tyr Val Leu His Pro Pro Ser Ala
1 5 10 15

Gly Ala Glu Arg Thr Leu Ala Ser Val Cys Ala Leu Leu Val Trp His
20 25 30

Pro Ala Gly Pro Gly Glu Lys Val Val Arg Val Leu Phe Pro Gly Cys
35 40 45

Thr Pro Pro Ala Cys Leu Leu Asp Gly Leu Val Arg Leu Gln His Leu
50 55 60

Arg Phe Leu Arg Glu Pro Val Val Thr Pro Gln Asp Leu Glu Gly Pro
65 70 75 80

Gly Arg Ala Glu Ser Lys Glu Ser Val Gly Ser Arg Asp Ser Ser Lys
85 90 95

Arg Glu Gly Leu Leu Ala Thr His Pro Arg Pro Gly Gln Glu Arg Pro
100 105 110

Gly Val Ala Arg Lys Glu Pro Ala Arg Ala Glu Ala Pro Arg Lys Thr
115 120 125

Glu Lys Glu Ala Lys Ala Pro Arg Glu Leu Lys Lys Asp Pro Lys Pro
130 135 140

Ser Val Ser Arg Thr Gln Pro Arg Glu Val Arg Arg Ala Ala Ser Ser
145 150 155 160

Val Pro Asn Leu Lys Lys Thr Asn Ala Gln Ala Ala Pro Lys Pro Arg
165 170 175

Lys Ala Pro Ser Thr Ser His Ser Gly Phe Pro Pro Val Ala Asn Gly
180 185 190

Pro Arg Ser Pro Pro Ser Leu Arg Cys Gly Glu Ala Ser Pro Pro Ser
195 200 205

Ala Ala Cys Gly Ser Pro Ala Ser Gln Leu Val Ala Thr Pro Ser Leu
210 215 220

Glu Leu Gly Pro Ile Pro Ala Gly Glu Glu Lys Ala Leu Glu Leu Pro
225 230 235 240

Leu Ala Ala Ser Ser Ile Pro Arg Pro Arg Thr Pro Ser Pro Glu Ser
245 250 255

His Arg Ser Pro Ala Glu Gly Ser Glu Arg Leu Ser Leu Ser Pro Leu
260 265 270

Arg Gly Gly Glu Ala Gly Pro Asp Ala Ser Pro Thr Val Thr Thr Pro
275 280 285

Thr Val Thr Thr Pro Ser Leu Pro Ala Glu Val Gly Ser Pro His Ser
290 295 300

Thr Glu Val Asp Glu Ser Leu Ser Val Ser Phe Glu Gln Val Leu Pro
305 310 315 320

Pro Ser Ala Pro Thr Ser Glu Ala Gly Leu Ser Leu Pro Leu Arg Gly
325 330 335

Pro Arg Ala Arg Arg Ser Ala Ser Pro His Asp Val Asp Leu Cys Leu
340 345 350

Val Ser Pro Cys Glu Phe Glu His Arg Lys Ala Val Pro Met Ala Pro
355 360 365

Ala Pro Ala Ser Pro Gly Ser Ser Asn Asp Ser Ser Ala Arg Ser Gln
370 375 380

Glu Arg Ala Gly Gly Leu Gly Ala Glu Glu Thr Pro Pro Thr Ser Val
385 390 395 400

Ser Glu Ser Leu Pro Thr Leu Ser Asp Ser Asp Pro Val Pro Leu Ala
405 410 415

Pro Gly Ala Ala Asp Ser Asp Glu Asp Thr Glu Gly Phe Gly Val Pro
420 425 430

Arg His Asp Pro Leu Pro Asp Pro Leu Lys Val Pro Pro Pro Leu Pro
435 440 445

Asp Pro Ser Ser Ile Cys Met Val Asp Pro Glu Met Leu Pro Pro Lys
450 455 460

Thr Ala Arg Gln Thr Glu Asn Val Ser Arg Thr Arg Lys Pro Leu Ala
465 470 475 480

Arg Pro Asn Ser Arg Ala Ala Ala Pro Lys Ala Thr Pro Val Ala Ala
485 490 495

Ala Lys Thr Lys Gly Leu Ala Gly Gly Asp Arg Ala Ser Arg Pro Leu
500 505 510

Ser Ala Arg Ser Glu Pro Ser Glu Lys Gly Gly Arg Ala Pro Leu Ser
515 520 525

Arg Lys Ser Ser Thr Pro Lys Thr Ala Thr Arg Gly Pro Ser Gly Ser
530 535 540

Ala Ser Ser Arg Pro Gly Val Ser Ala Thr Pro Pro Lys Ser Pro Val
545 550 555 560

Tyr Leu Asp Leu Ala Tyr Leu Pro Ser Gly Ser Ser Ala His Leu Val
565 570 575

Asp Glu Glu Phe Phe Gln Arg Val Arg Ala Leu Cys Tyr Val Ile Ser
580 585 590

Gly Gln Asp Gln Arg Lys Glu Glu Gly Met Arg Ala Val Leu Asp Ala
595 600 605

Leu Leu Ala Ser Lys Gln His Trp Asp Arg Asp Leu Gln Val Thr Leu
610 615 620

Ile Pro Thr Phe Asp Ser Val Ala Met His Thr Trp Tyr Ala Glu Thr
625 630 635 640

His Ala Arg His Gln Ala Leu Gly Ile Thr Val Leu Gly Ser Asn Ser
645 650 655

Met Val Ser Met Gln Asp Asp Ala Phe Pro Ala Cys Lys Val Glu Phe
660 665 670

<210> 237
<211> 222
<212> PRT
<213> Homo sapiens

<400> 237
Met Asn Ser Asn Val Glu Asn Leu Pro Pro His Ile Ile Arg Leu Val
1 5 10 15

Tyr Lys Glu Val Thr Thr Leu Thr Ala Asp Pro Pro Asp Gly Ile Lys
20 25 30

Val Phe Pro Asn Glu Glu Asp Leu Thr Asp Leu Gln Val Thr Ile Glu
35 40 45

Gly Pro Glu Gly Thr Pro Tyr Ala Gly Gly Leu Phe Arg Met Lys Leu
50 55 60

Leu Leu Gly Lys Asp Phe Pro Ala Ser Pro Pro Lys Gly Tyr Phe Leu
65 70 75 80

Thr Lys Ile Phe His Pro Asn Val Gly Ala Asn Gly Glu Ile Cys Val
85 90 95

Asn Val Leu Lys Arg Asp Trp Thr Ala Glu Leu Gly Ile Arg His Val
100 105 110

Leu Leu Thr Ile Lys Cys Leu Leu Ile His Pro Asn Pro Glu Ser Ala

115 120 125

Leu Asn Glu Glu Ala Gly Arg Leu Leu Leu Glu Asn Tyr Glu Glu Tyr
130 135 140

Ala Ala Arg Ala Arg Leu Leu Thr Glu Ile His Gly Gly Ala Gly Gly
145 150 155 160

Pro Ser Gly Arg Ala Glu Ala Gly Arg Ala Leu Ala Ser Gly Thr Glu
165 170 175

Ala Ser Ser Thr Asp Pro Gly Ala Pro Gly Gly Pro Gly Gly Ala Glu
180 185 190

Gly Thr Met Ala Lys Lys His Ala Gly Glu Arg Asp Lys Lys Leu Ala
195 200 205

Ala Lys Lys Lys Thr Asp Lys Lys Arg Ala Leu Arg Arg Leu
210 215 220

<210> 238
<211> 245
<212> PRT
<213> Homo sapiens

<400> 238
Met Ala Val Arg Ala Ser Phe Glu Asn Asn Cys Glu Ile Gly Cys Phe
1 5 10 15

Ala Lys Leu Thr Asn Thr Tyr Cys Leu Val Ala Ile Gly Gly Ser Glu
20 25 30

Asn Phe Tyr Ser Val Phe Glu Gly Glu Leu Ser Asp Thr Ile Pro Val
35 40 45

Val His Ala Ser Ile Ala Gly Cys Arg Ile Ile Gly Arg Met Cys Val
50 55 60

Gly Asn Arg His Gly Leu Leu Val Pro Asn Asn Thr Thr Asp Gln Glu
65 70 75 80

Leu Gln His Ile Arg Asn Ser Leu Pro Asp Thr Val Gln Ile Arg Arg
85 90 95

Val Glu Glu Arg Leu Ser Ala Leu Gly Asn Val Thr Thr Cys Asn Asp
100 105 110

Tyr Val Ala Leu Val His Pro Asp Leu Asp Arg Glu Thr Glu Glu Ile
115 120 125

Leu Ala Asp Val Leu Lys Val Glu Val Phe Arg Gln Thr Val Ala Asp
130 135 140

Gln Val Leu Val Gly Ser Tyr Cys Val Phe Ser Asn Gln Gly Gly Leu
145 150 155 160

Val His Pro Lys Thr Ser Ile Glu Asp Gln Asp Glu Leu Ser Ser Leu
165 170 175

Leu Gln Val Pro Leu Val Ala Gly Thr Val Asn Arg Gly Ser Glu Val
180 185 190

Ile Ala Ala Gly Met Val Val Asn Asp Trp Cys Ala Phe Cys Gly Leu
195 200 205

Asp Thr Thr Ser Thr Glu Leu Ser Val Val Glu Ser Val Phe Lys Leu
210 215 220

Asn Glu Ala Gln Pro Ser Thr Ile Ala Thr Ser Met Arg Asp Ser Leu
225 230 235 240

Ile Asp Ser Leu Thr
245

<210> 239
<211> 117
<212> PRT
<213> Homo sapiens

<400> 239
Met Glu Ser Gly Ala Lys Gly Cys Glu Val Val Val Ser Gly Lys Leu
1 5 10 15

Arg Gly Gln Arg Ala Lys Ser Met Lys Phe Val Asp Gly Leu Met Ile
20 25 30

His Ser Gly Asp Pro Val Asn Tyr Tyr Val Asp Thr Ala Val Arg His
35 40 45

Val Leu Leu Arg Gln Gly Val Leu Gly Ile Lys Val Lys Ile Met Leu
50 55 60

Pro Trp Asp Pro Thr Gly Lys Ile Gly Pro Lys Lys Pro Leu Pro Asp
65 70 75 80

His Val Ser Ile Val Glu Pro Lys Asp Glu Ile Leu Pro Thr Thr Pro
85 90 95

Ile Ser Glu Gln Lys Gly Gly Lys Pro Glu Pro Pro Ala Met Pro Gln
100 105 110

Pro Val Pro Thr Ala
115

<210> 240
<211> 444
<212> PRT
<213> Homo sapiens

<400> 240
Met Arg Glu Ile Val His Ile Gln Ala Gly Gln Cys Gly Asn Gln Ile
1 5 10 15

Gly Ala Lys Phe Trp Glu Val Ile Ser Asp Glu His Gly Ile Asp Pro
20 25 30

Thr Gly Thr Tyr His Gly Asp Ser Asp Leu Gln Leu Asp Arg Ile Ser
35 40 45

Val Tyr Tyr Asn Glu Ala Thr Gly Gly Lys Tyr Val Pro Arg Ala Ile
50 55 60

Leu Val Asp Leu Glu Pro Gly Thr Met Asp Ser Val Arg Ser Gly Pro
65 70 75 80

Phe Gly Gln Ile Phe Arg Pro Asp Asn Phe Val Phe Gly Gln Ser Gly
85 90 95

Ala Gly Asn Asn Trp Ala Lys Gly His Tyr Thr Glu Gly Ala Glu Leu
100 105 110

Val Asp Ser Val Leu Asp Val Val Arg Lys Glu Ala Glu Ser Cys Asp
115 120 125

Cys Leu Gln Gly Phe Gln Leu Thr His Ser Leu Gly Gly Gly Thr Gly
130 135 140

Ser Gly Met Gly Thr Leu Leu Ile Ser Lys Ile Arg Glu Glu Tyr Pro
145 150 155 160

Asp Arg Ile Met Asn Thr Phe Ser Val Val Pro Ser Pro Lys Val Ser

165 170 175

Asp Thr Val Val Glu Pro Tyr Asn Ala Thr Leu Ser Val His Gln Leu
180 185 190

Val Glu Asn Thr Asp Glu Thr Tyr Cys Ile Asp Asn Glu Ala Leu Tyr
195 200 205

Asp Ile Cys Phe Arg Thr Leu Lys Leu Thr Thr Pro Thr Tyr Gly Asp
210 215 220

Leu Asn His Leu Val Ser Ala Thr Met Ser Gly Val Thr Thr Cys Leu
225 230 235 240

Arg Phe Pro Gly Gln Leu Asn Ala Asp Leu Arg Lys Leu Ala Val Asn
245 250 255

Met Val Pro Phe Pro Arg Leu His Phe Phe Met Pro Gly Phe Ala Pro
260 265 270

Leu Thr Ser Arg Gly Ser Gln Gln Tyr Arg Ala Leu Thr Val Pro Glu
275 280 285

Leu Thr Gln Gln Val Phe Asp Ala Lys Asn Met Met Ala Ala Cys Asp
290 295 300

Pro Arg His Gly Arg Tyr Leu Thr Val Ala Ala Val Phe Arg Gly Arg
305 310 315 320

Met Ser Met Lys Glu Val Asp Glu Gln Met Leu Asn Val Gln Asn Lys
325 330 335

Asn Ser Ser Tyr Phe Val Glu Trp Ile Pro Asn Asn Val Lys Thr Ala
340 345 350

Val Cys Asp Ile Pro Pro Arg Gly Leu Lys Met Ala Val Thr Phe Ile
355 360 365

Gly Asn Ser Thr Ala Ile Gln Glu Leu Phe Lys Arg Ile Ser Glu Gln
370 375 380

Phe Thr Ala Met Phe Arg Arg Lys Ala Phe Leu His Trp Tyr Thr Gly
385 390 395 400

Glu Gly Met Asp Glu Met Glu Phe Thr Glu Ala Glu Ser Asn Met Asn
405 410 415

Asp Leu Val Ser Glu Tyr Gln Gln Tyr Gln Asp Ala Thr Ala Glu Glu

420 425 430

Glu Glu Asp Phe Gly Glu Glu Ala Glu Glu Glu Ala
435 440

<210> 241
<211> 92
<212> PRT
<213> Homo sapiens

<400> 241
Met Asp Glu Gln Ile Arg Leu Met Asp Gln Asn Leu Lys Cys Leu Ser
1 5 10 15

Ala Ala Glu Glu Lys Tyr Ser Gln Lys Glu Asp Lys Tyr Glu Glu Glu
20 25 30

Ile Lys Ile Leu Thr Asp Lys Leu Lys Glu Ala Glu Thr Arg Ala Glu
35 40 45

Phe Ala Glu Arg Ser Val Ala Lys Leu Glu Lys Thr Ile Asp Asp Leu
50 55 60

Glu Asp Lys Leu Lys Cys Thr Lys Glu Glu His Leu Cys Thr Gln Arg
65 70 75 80

Met Leu Asp Gln Thr Leu Leu Asp Leu Asn Glu Met
85 90

<210> 242
<211> 453
<212> PRT
<213> Homo sapiens

<400> 242
Met Val Met Gly Ile Thr Asp Val Asp Asp Lys Ile Ile Lys Arg Ala
1 5 10 15

Asn Glu Met Asn Ile Ser Pro Ala Ser Leu Ala Ser Leu Tyr Glu Glu
20 25 30

Asp Phe Lys Gln Asp Met Ala Ala Leu Lys Val Leu Pro Pro Thr Val
35 40 45

Tyr Leu Arg Val Thr Glu Asn Ile Pro Gln Ile Ile Ser Phe Ile Glu
50 55 60

Gly Ile Ile Ala Ser Trp Glu Arg Leu Phe Asn Gly Lys Arg Gln Cys
65 70 75 80

Leu Leu Arg Ser Glu Ser Leu Glu Glu Thr Lys Tyr Gly Lys Ile Gly
85 90 95

Arg Arg Gly Pro Trp Ser Ser Pro Glu Thr Ser Gly Leu Leu Thr Ser
100 105 110

Arg His Ala Asn Asp Phe Ala Leu Trp Lys Ala Ala Lys Pro Gln Glu
115 120 125

Val Phe Trp Ala Ser Pro Trp Gly Pro Gly Arg Pro Gly Trp His Ile
130 135 140

Glu Cys Ser Ala Ile Ala Ser Met Val Phe Gly Ser Gln Leu Asp Ile
145 150 155 160

His Ser Gly Gly Ile Asp Leu Ala Phe Pro His His Glu Asn Glu Ile
165 170 175

Ala Gln Cys Glu Val Phe His Gln Cys Glu Gln Trp Gly Asn Tyr Phe
180 185 190

Leu His Ser Gly His Leu His Ala Lys Gly Lys Glu Glu Lys Met Ser
195 200 205

Lys Ser Leu Lys Asn Tyr Ile Thr Ile Lys Asp Phe Leu Lys Thr Phe
210 215 220

Ser Pro Asp Val Phe Arg Phe Phe Cys Leu Arg Ser Ser Tyr Arg Ser
225 230 235 240

Ala Ile Asp Tyr Ser Asp Ser Ala Met Leu Gln Ala Gln Gln Leu Leu
245 250 255

Leu Gly Leu Gly Ser Phe Leu Glu Asp Ala Arg Ala Tyr Met Lys Gly
260 265 270

Gln Leu Ala Cys Gly Ser Val Arg Glu Ala Met Leu Trp Glu Arg Leu
275 280 285

Ser Ser Thr Lys Arg Ala Val Lys Ala Ala Leu Ala Asp Asp Phe Asp
290 295 300

Thr Pro Arg Val Val Asp Ala Ile Leu Gly Leu Ala His His Gly Asn
305 310 315 320

Gly Gln Leu Arg Ala Ser Leu Lys Glu Pro Glu Gly Pro Arg Ser Pro
325 330 335

Ala Val Phe Gly Ala Ile Ile Ser Tyr Phe Glu Gln Phe Phe Glu Thr
340 345 350

Val Gly Ile Ser Leu Ala Asn Gln Gln Tyr Val Ser Gly Asp Gly Ser
355 360 365

Glu Ala Thr Leu His Gly Val Val Asp Glu Leu Val Arg Phe Arg Gln
370 375 380

Lys Val Arg Gln Phe Ala Leu Ala Met Pro Glu Ala Thr Gly Asp Ala
385 390 395 400

Arg Arg Gln Gln Leu Leu Glu Arg Gln Pro Leu Leu Glu Ala Cys Asp
405 410 415

Thr Leu Arg Arg Gly Leu Thr Ala His Gly Ile Asn Ile Lys Asp Arg
420 425 430

Ser Ser Thr Thr Ser Thr Trp Glu Leu Leu Asp Gln Arg Thr Lys Asp
435 440 445

Gln Lys Ser Ala Gly
450

<210> 243
<211> 209
<212> PRT
<213> Homo sapiens

<400> 243
Met Lys Glu Leu Ala Glu Glu Glu Pro His Leu Val Glu Gln Phe Gln
1 5 10 15

Lys Leu Ser Glu Ala Ala Gly Arg Val Gly Ser Asp Met Thr Ser Gln
20 25 30

Gln Glu Phe Thr Ser Cys Leu Lys Glu Thr Leu Ser Gly Leu Ala Lys
35 40 45

Asn Ala Thr Asp Leu Gln Asn Ser Ser Met Ser Glu Glu Glu Leu Thr
50 55 60

Lys Ala Met Glu Gly Leu Gly Met Asp Glu Gly Asp Gly Glu Gly Asn

65 70 75 80

Ile Leu Pro Ile Met Gln Ser Ile Met Gln Asn Leu Leu Ser Lys Asp
85 90 95

Val Leu Tyr Pro Ser Leu Lys Glu Ile Thr Glu Lys Tyr Pro Glu Trp
100 105 110

Leu Gln Ser His Arg Glu Ser Leu Pro Pro Glu Gln Phe Glu Lys Tyr
115 120 125

Gln Glu Gln His Ser Val Met Cys Lys Ile Cys Glu Gln Phe Glu Ala
130 135 140

Glu Thr Pro Thr Asp Ser Glu Thr Thr Gln Lys Ala Arg Phe Glu Met
145 150 155 160

Val Leu Asp Leu Met Gln Gln Leu Gln Asp Leu Gly His Pro Pro Lys
165 170 175

Glu Leu Ala Gly Glu Met Pro Pro Gly Leu Asn Phe Asp Leu Asp Ala
180 185 190

Leu Asn Leu Ser Gly Pro Pro Gly Ala Ser Gly Glu Gln Cys Leu Ile
195 200 205

Met

<210> 244
<211> 354
<212> PRT
<213> Homo sapiens

<400> 244
Met Arg Arg Leu Met Ser Ser Arg Asp Trp Pro Arg Thr Arg Thr Gly
1 5 10 15

Thr Gly Ile Leu Ser Ser Gln Pro Glu Glu Asn Pro Tyr Trp Trp Asn
20 25 30

Ala Asn Met Val Phe Ile Pro Tyr Cys Ser Ser Asp Val Trp Ser Gly
35 40 45

Ala Ser Ser Lys Ser Glu Lys Asn Glu Tyr Ala Phe Met Gly Ala Leu
50 55 60

Ile Ile Gln Glu Val Val Arg Glu Leu Leu Gly Arg Gly Leu Ser Gly
65 70 75 80

Ala Lys Val Leu Leu Leu Ala Gly Ser Ser Ala Gly Gly Thr Gly Val
85 90 95

Leu Leu Asn Val Asp Arg Val Ala Glu Gln Leu Glu Lys Leu Gly Tyr
100 105 110

Pro Ala Ile Gln Val Arg Gly Leu Ala Asp Ser Gly Trp Phe Leu Asp
115 120 125

Asn Lys Gln Tyr Arg His Thr Asp Cys Val Asp Thr Ile Thr Cys Ala
130 135 140

Pro Thr Glu Ala Ile Arg Arg Gly Ile Arg Tyr Trp Asn Gly Val Val
145 150 155 160

Pro Glu Arg Cys Arg Arg Gln Phe Gln Glu Gly Glu Glu Trp Asn Cys
165 170 175

Phe Phe Gly Tyr Lys Val Tyr Pro Thr Leu Arg Cys Pro Val Phe Val
180 185 190

Val Gln Trp Leu Phe Asp Glu Ala Gln Leu Thr Val Asp Asn Val His
195 200 205

Leu Thr Gly Gln Pro Val Gln Glu Gly Leu Arg Leu Tyr Ile Gln Asn
210 215 220

Leu Gly Arg Glu Leu Arg His Thr Leu Lys Asp Val Pro Ala Ser Phe
225 230 235 240

Ala Pro Ala Cys Leu Ser His Glu Ile Ile Ile Arg Ser His Trp Thr
245 250 255

Asp Val Gln Val Lys Gly Thr Ser Leu Pro Arg Ala Leu His Cys Trp
260 265 270

Asp Arg Ser Leu His Asp Ser His Lys Ala Ser Lys Thr Pro Leu Lys
275 280 285

Gly Cys Pro Val His Leu Val Asp Ser Cys Pro Trp Pro His Cys Asn
290 295 300

Pro Ser Cys Pro Thr Val Arg Asp Gln Phe Thr Gly Gln Glu Met Asn
305 310 315 320

Val Ala Gln Phe Leu Met His Met Gly Phe Asp Met Gln Thr Val Ala
325 330 335

Gln Pro Gln Gly Leu Glu Pro Ser Glu Leu Leu Gly Met Leu Ser Asn
340 345 350

Gly Ser

<210> 245
<211> 295
<212> PRT
<213> Homo sapiens

<400> 245
Met Glu Leu Ile Gln Asp Thr Ser Arg Pro Pro Leu Glu Tyr Val Lys
1 5 10 15

Gly Val Pro Leu Ile Lys Tyr Phe Ala Glu Ala Leu Gly Pro Leu Gln
20 25 30

Ser Phe Gln Ala Arg Pro Asp Asp Leu Leu Ile Ser Thr Tyr Pro Lys
35 40 45

Ser Gly Thr Thr Trp Val Ser Gln Ile Leu Asp Met Ile Tyr Gln Gly
50 55 60

Gly Asp Leu Glu Lys Cys His Arg Ala Pro Ile Phe Met Arg Val Pro
65 70 75 80

Phe Leu Glu Phe Lys Ala Pro Gly Ile Pro Ser Gly Met Glu Thr Leu
85 90 95

Lys Asp Thr Pro Ala Pro Arg Leu Leu Lys Thr His Leu Pro Leu Ala
100 105 110

Leu Leu Pro Gln Thr Leu Leu Asp Gln Lys Val Lys Val Val Tyr Val
115 120 125

Ala Arg Asn Ala Lys Asp Val Ala Val Ser Tyr Tyr His Phe Tyr His
130 135 140

Met Ala Lys Val His Pro Glu Pro Gly Thr Trp Asp Ser Phe Leu Glu
145 150 155 160

Lys Phe Met Val Gly Glu Val Ser Tyr Gly Ser Trp Tyr Gln His Val
165 170 175

Gln Glu Trp Trp Glu Leu Ser Arg Thr His Pro Val Leu Tyr Leu Phe
180 185 190

Tyr Glu Asp Met Lys Glu Asn Pro Lys Arg Glu Ile Gln Lys Ile Leu
195 200 205

Glu Phe Val Gly His Ser Leu Pro Glu Glu Thr Val Asp Phe Met Val
210 215 220

Gln His Thr Ser Phe Lys Glu Met Lys Lys Asn Pro Met Thr Asn Tyr
225 230 235 240

Thr Thr Val Pro Gln Glu Phe Met Asp His Ser Ile Ser Pro Phe Met
245 250 255

Arg Lys Gly Met Ala Gly Asp Trp Lys Thr Thr Phe Thr Val Ala Gln
260 265 270

Asn Glu Arg Phe Asp Ala Asp Tyr Ala Glu Lys Met Ala Gly Cys Ser
275 280 285

Leu Ser Phe Arg Ser Glu Leu
290 295

<210> 246
<211> 439
<212> PRT
<213> Homo sapiens

<400> 246
Met Glu Pro Ser Thr Ala Ala Arg Ala Trp Ala Leu Phe Trp Leu Leu
1 5 10 15

Leu Pro Leu Leu Gly Ala Val Cys Ala Ser Gly Pro Arg Thr Leu Val
20 25 30

Leu Leu Asp Asn Leu Asn Val Arg Glu Thr His Ser Leu Phe Phe Arg
35 40 45

Ser Leu Lys Asp Arg Gly Phe Glu Leu Thr Phe Lys Thr Ala Asp Asp
50 55 60

Pro Ser Leu Ser Leu Ile Lys Tyr Gly Glu Phe Leu Tyr Asp Asn Leu
65 70 75 80

Ile Ile Phe Ser Pro Ser Val Glu Asp Phe Gly Gly Asn Ile Asn Val

85 90 95

Glu Thr Ile Ser Ala Phe Ile Asp Gly Gly Gly Ser Val Leu Val Ala
100 105 110

Ala Ser Ser Asp Ile Gly Asp Pro Leu Arg Glu Leu Gly Ser Glu Cys
115 120 125

Gly Ile Glu Phe Asp Glu Glu Lys Thr Ala Val Ile Asp His His Asn
130 135 140

Tyr Asp Ile Ser Asp Leu Gly Gln His Thr Leu Ile Val Ala Asp Thr
145 150 155 160

Glu Asn Leu Leu Lys Ala Pro Thr Ile Val Gly Lys Ser Ser Leu Asn
165 170 175

Pro Ile Leu Phe Arg Gly Val Gly Met Val Ala Asp Pro Asp Asn Pro
180 185 190

Leu Val Leu Asp Ile Leu Thr Gly Ser Ser Thr Ser Tyr Ser Phe Phe
195 200 205

Pro Asp Lys Pro Ile Thr Gln Tyr Pro His Ala Val Gly Lys Asn Thr
210 215 220

Leu Leu Ile Ala Gly Leu Gln Ala Arg Asn Asn Ala Arg Val Ile Phe
225 230 235 240

Ser Gly Ser Leu Asp Phe Phe Ser Asp Ser Phe Phe Asn Ser Ala Val
245 250 255

Gln Lys Ala Ala Pro Gly Ser Gln Arg Tyr Ser Gln Thr Gly Asn Tyr
260 265 270

Glu Leu Ala Val Ala Leu Ser Arg Trp Val Phe Lys Glu Glu Gly Val
275 280 285

Leu Arg Val Gly Pro Val Ser His His Arg Val Gly Glu Thr Ala Pro
290 295 300

Pro Asn Ala Tyr Thr Val Thr Asp Leu Val Glu Tyr Ser Ile Val Ile
305 310 315 320

Gln Gln Leu Ser Asn Gly Lys Trp Val Pro Phe Asp Gly Asp Asp Ile
325 330 335

Gln Leu Glu Phe Val Arg Ile Asp Pro Phe Val Arg Thr Phe Leu Lys

340 345 350

Lys Lys Gly Gly Lys Tyr Ser Val Gln Phe Lys Leu Pro Asp Val Tyr
355 360 365

Gly Val Phe Gln Phe Lys Val Asp Tyr Asn Arg Leu Gly Tyr Thr His
370 375 380

Leu Tyr Ser Ser Thr Gln Val Ser Val Arg Pro Leu Gln His Thr Gln
385 390 395 400

Tyr Glu Arg Phe Ile Pro Ser Ala Tyr Pro Tyr Tyr Ala Ser Ala Phe
405 410 415

Ser Met Met Leu Gly Leu Phe Ile Phe Ser Ile Val Phe Leu His Met
420 425 430

Lys Glu Lys Glu Lys Ser Asp
435

<210> 247
<211> 56
<212> PRT
<213> Homo sapiens

<400> 247
Met Glu Thr Leu His Thr Trp Gly Ser Lys Val Leu Gly Tyr Ser Trp
1 5 10 15

Ile Phe Arg Thr Ser Ala Tyr Pro Gln Val Ser Gln Ala Ser Gly Gly
20 25 30

Glu Ala Ser Asp Pro Trp Pro Thr Cys Tyr Pro Pro Gln Gly Leu Asp
35 40 45

Leu Ser Ser Arg Glu Gly Thr Glu
50 55

<210> 248
<211> 46
<212> PRT
<213> Homo sapiens

<400> 248
Met Gly Phe Lys Gly Pro Gly Val Phe Leu Asp Leu Gln Asp Ile Cys
1 5 10 15

Leu Pro Ser Gly Phe Pro Gly Leu Gly Trp Gly Gly Ile Arg Ser Leu
20 25 30

Ala Asn Leu Leu Ser Thr Pro Gly Phe Arg Pro Leu Phe Pro
35 40 45

<210> 249
<211> 61
<212> PRT
<213> Homo sapiens

<400> 249
Ile Gly Thr Val Phe Leu Glu Gly Asn Leu Val Lys Cys Ile Lys Arg
1 5 10 15

Leu Lys Asn Thr Asp Val Leu Cys Ala Gly Asn Ser Thr Ser Ser Asn
20 25 30

Phe Ser Leu Lys Pro Tyr Gln Arg Cys Ile Gln Arg Ile Ile Tyr Lys
35 40 45

Glu Gly Cys Leu Ile Met Ile Val Ile Ile Ile Asn Asn
50 55 60

<210> 250
<211> 73
<212> PRT
<213> Homo sapiens

<400> 250
Met Phe Asp Ser Pro Phe Tyr Glu Leu Asn Tyr Phe Ile Arg Val Gly
1 5 10 15

Asn Phe Cys Phe Leu Ile Lys Trp Lys Leu Ala Phe Leu Thr Leu Phe
20 25 30

Leu Leu Leu Phe Tyr Arg Asn Ala Phe Cys Trp Pro Gly Thr Val Ala
35 40 45

His Pro Cys Asn Pro Ser Thr Val Gly Gly Arg Asp Gly Trp Ile Thr
50 55 60

Arg Ser Gly Asp Arg Asp His Pro Gly
65 70

<210> 251
<211> 43
<212> PRT
<213> Homo sapiens

<400> 251
Met Leu Phe Val Gly Arg Ala Gln Leu Leu Ile His Val Ile Pro Ala
1               5                   10                  15

Leu Trp Glu Ala Glu Thr Gly Gly Ser Gln Gly Gln Glu Ile Glu Thr
            20                  25                  30

Ile Leu Ala Asn Ala Leu Lys Leu Arg Leu Cys
        35                  40

<210> 252
<211> 30
<212> PRT
<213> Homo sapiens

<400> 252
Met Tyr Ile Phe Phe Cys Val Leu Phe Leu Leu Leu Leu Leu Phe Glu
1               5                   10                  15

Thr Gly Ser Cys Ser Val Ala Gln Ala Gly Val Gln Trp His
            20                  25                  30

<210> 253
<211> 87
<212> PRT
<213> Homo sapiens

<400> 253
Met Asn Cys Asn Thr Gln Ser Gln Thr Arg Ala Leu Pro Arg Pro Leu
1               5                   10                  15

Gly Gly Cys Thr Pro Ser Ser Ser Ala Arg Leu Arg Ser Leu Arg Pro
            20                  25                  30

Arg Leu Lys Glu Gly Val Ala Gly Asn Pro Gly Asn Leu Ser Glu Val
        35                  40                  45

Thr Pro His Pro Tyr Thr Pro Ser Val His Pro Arg Leu Phe Leu Leu
    50                  55                  60

Leu Phe Gly Phe Trp Lys Gly Ile His Leu Gln Ala Ala His Pro Gly
65 70 75 80

Gly Ala Cys Phe Leu Lys Pro
85

<210> 254
<211> 211
<212> PRT
<213> Homo sapiens

<400> 254
Met Ala Pro Ser Arg Asn Gly Met Val Leu Lys Pro His Phe His Lys
1 5 10 15

Asp Trp Gln Arg Arg Val Ala Thr Trp Phe Asn Gln Pro Ala Arg Lys
20 25 30

Ile Arg Arg Arg Lys Ala Arg Gln Ala Lys Ala Arg Arg Ile Ala Pro
35 40 45

Arg Pro Ala Ser Gly Pro Ile Arg Pro Ile Val Arg Cys Pro Thr Val
50 55 60

Arg Tyr His Thr Lys Val Arg Ala Gly Arg Gly Phe Ser Leu Glu Glu
65 70 75 80

Leu Arg Val Ala Gly Ile His Lys Lys Val Ala Arg Thr Ile Gly Ile
85 90 95

Ser Val Asp Pro Arg Arg Arg Asn Lys Ser Thr Glu Ser Leu Gln Ala
100 105 110

Asn Val Gln Arg Leu Lys Glu Tyr Arg Ser Lys Leu Ile Leu Phe Pro
115 120 125

Arg Lys Pro Ser Ala Pro Lys Lys Gly Asp Ser Ser Ala Glu Glu Leu
130 135 140

Lys Leu Ala Thr Gln Leu Thr Gly Pro Val Met Pro Val Arg Asn Val
145 150 155 160

Tyr Lys Lys Glu Lys Ala Arg Val Ile Thr Glu Glu Glu Lys Asn Phe
165 170 175

Lys Ala Phe Ala Ser Leu Arg Met Ala Arg Ala Asn Ala Arg Leu Phe
180 185 190

Gly Ile Arg Ala Lys Arg Ala Lys Glu Ala Ala Glu Gln Asp Val Glu
195 200 205

Lys Lys Lys
210

<210> 255
<211> 417
<212> PRT
<213> Homo sapiens

<400> 255
Met Ser Leu Ser Asn Lys Leu Thr Leu Asp Lys Leu Asp Val Lys Gly
1 5 10 15

Lys Arg Val Val Met Arg Val Asp Phe Asn Val Pro Met Lys Asn Asn
20 25 30

Gln Ile Thr Asn Asn Gln Arg Ile Lys Ala Ala Val Pro Ser Ile Lys
35 40 45

Phe Cys Leu Asp Asn Gly Ala Lys Ser Val Val Leu Met Ser His Leu
50 55 60

Gly Arg Pro Asp Gly Val Pro Met Pro Asp Lys Tyr Ser Leu Glu Pro
65 70 75 80

Val Ala Val Glu Leu Lys Ser Leu Leu Gly Lys Asp Val Leu Phe Leu
85 90 95

Lys Asp Cys Val Gly Pro Glu Val Glu Lys Ala Cys Ala Asn Pro Ala
100 105 110

Ala Gly Ser Val Ile Leu Leu Glu Asn Leu Arg Phe His Val Glu Glu
115 120 125

Glu Gly Lys Gly Lys Asp Ala Ser Gly Asn Lys Val Lys Ala Glu Pro
130 135 140

Ala Lys Ile Glu Ala Phe Arg Ala Ser Leu Ser Lys Leu Gly Asp Val
145 150 155 160

Tyr Val Asn Asp Ala Phe Gly Thr Ala His Arg Ala His Ser Ser Met
165 170 175

Val Gly Val Asn Leu Pro Gln Lys Ala Gly Gly Phe Leu Met Lys Lys

180 185 190

Glu Leu Asn Tyr Phe Ala Lys Ala Leu Glu Ser Pro Glu Arg Pro Phe
195 200 205

Leu Ala Ile Leu Gly Gly Ala Lys Val Ala Asp Lys Ile Gln Leu Ile
210 215 220

Asn Asn Met Leu Asp Lys Val Asn Glu Met Ile Ile Gly Gly Gly Met
225 230 235 240

Ala Phe Thr Phe Leu Lys Val Leu Asn Asn Met Glu Ile Gly Thr Ser
245 250 255

Leu Phe Asp Glu Glu Gly Ala Lys Ile Val Lys Asp Leu Met Ser Lys
260 265 270

Ala Glu Lys Asn Gly Val Lys Ile Thr Leu Pro Val Asp Phe Val Thr
275 280 285

Ala Asp Lys Phe Asp Glu Asn Ala Lys Thr Gly Gln Ala Thr Val Ala
290 295 300

Ser Gly Ile Pro Ala Gly Trp Met Gly Leu Asp Cys Gly Pro Glu Ser
305 310 315 320

Ser Lys Lys Tyr Ala Glu Ala Val Thr Arg Ala Lys Gln Ile Val Trp
325 330 335

Asn Gly Pro Val Gly Val Phe Glu Trp Glu Ala Phe Ala Arg Gly Thr
340 345 350

Lys Ala Leu Met Asp Glu Val Val Lys Ala Thr Ser Arg Gly Cys Ile
355 360 365

Thr Ile Ile Gly Gly Gly Asp Thr Ala Thr Cys Cys Ala Lys Trp Asn
370 375 380

Thr Glu Asp Lys Val Ser His Val Ser Thr Gly Gly Gly Ala Ser Leu
385 390 395 400

Glu Leu Leu Glu Gly Lys Val Leu Pro Gly Val Asp Ala Leu Ser Asn
405 410 415

Ile

<210> 256
<211> 568
<212> PRT
<213> Homo sapiens

<400> 256
Met Val Leu Gly Pro Glu Gln Lys Met Ser Asp Asp Ser Val Ser Gly
1               5                   10                  15

Asp His Gly Glu Ser Ala Ser Leu Gly Asn Ile Asn Pro Ala Tyr Ser
            20                  25                  30

Asn Pro Ser Leu Ser Gln Ser Pro Gly Asp Ser Glu Glu Tyr Phe Ala
        35                  40                  45

Thr Tyr Phe Asn Glu Lys Ile Ser Ile Pro Glu Glu Glu Tyr Ser Cys
    50                  55                  60

Phe Ser Phe Arg Lys Leu Trp Ala Phe Thr Gly Pro Gly Phe Leu Met
65                  70                  75                  80

Ser Ile Ala Tyr Leu Asp Pro Gly Asn Ile Glu Ser Asp Leu Gln Ser
                85                  90                  95

Gly Ala Val Ala Gly Phe Lys Leu Leu Trp Ile Leu Leu Leu Ala Thr
            100                 105                 110

Leu Val Gly Leu Leu Leu Gln Arg Leu Ala Ala Arg Leu Gly Val Val
        115                 120                 125

Thr Gly Leu His Leu Ala Glu Val Cys His Arg Gln Tyr Pro Lys Val
    130                 135                 140

Pro Arg Val Ile Leu Trp Leu Met Val Glu Leu Ala Ile Ile Gly Ser
145                 150                 155                 160

Asp Met Gln Glu Val Ile Gly Ser Ala Ile Ala Ile Asn Leu Leu Ser
                165                 170                 175

Val Gly Arg Ile Pro Leu Trp Gly Gly Val Leu Ile Thr Ile Ala Asp
            180                 185                 190

Thr Phe Val Phe Leu Phe Leu Asp Lys Tyr Gly Leu Arg Lys Leu Glu
        195                 200                 205

Ala Phe Phe Gly Phe Leu Ile Thr Ile Met Ala Leu Thr Phe Gly Tyr
    210                 215                 220

Glu Tyr Val Thr Val Lys Pro Ser Gln Ser Gln Val Leu Lys Gly Met
225 230 235 240

Phe Val Pro Ser Cys Ser Gly Cys Arg Thr Pro Gln Ile Glu Gln Ala
245 250 255

Val Gly Ile Val Gly Ala Val Ile Met Pro His Asn Met Tyr Leu His
260 265 270

Ser Ala Leu Val Lys Ser Arg Gln Val Asn Arg Asn Asn Lys Gln Glu
275 280 285

Val Arg Glu Ala Asn Lys Tyr Phe Phe Ile Glu Ser Cys Ile Ala Leu
290 295 300

Phe Val Ser Phe Ile Ile Asn Val Phe Val Val Ser Val Phe Ala Glu
305 310 315 320

Ala Phe Phe Gly Lys Thr Asn Glu Gln Val Val Glu Val Cys Thr Asn
325 330 335

Thr Ser Ser Pro His Ala Gly Leu Phe Pro Lys Asp Asn Ser Thr Leu
340 345 350

Ala Val Asp Ile Tyr Lys Gly Gly Val Val Leu Gly Cys Tyr Phe Gly
355 360 365

Pro Ala Ala Leu Tyr Ile Trp Ala Val Gly Ile Leu Ala Ala Gly Gln
370 375 380

Ser Ser Thr Met Thr Gly Thr Tyr Ser Gly Gln Phe Val Met Glu Gly
385 390 395 400

Phe Leu Asn Leu Lys Trp Ser Arg Phe Ala Arg Val Val Leu Thr Arg
405 410 415

Ser Ile Ala Ile Ile Pro Thr Leu Leu Val Ala Val Phe Gln Asp Val
420 425 430

Glu His Leu Thr Gly Met Asn Asp Phe Leu Asn Val Leu Gln Ser Leu
435 440 445

Gln Leu Pro Phe Ala Leu Ile Pro Ile Leu Thr Phe Thr Ser Leu Arg
450 455 460

Pro Val Met Ser Asp Phe Ala Asn Gly Leu Gly Trp Arg Ile Ala Gly
465 470 475 480

Gly Ile Leu Val Leu Ile Ile Cys Ser Ile Asn Met Tyr Phe Val Val
485 490 495

Val Tyr Val Arg Asp Leu Gly His Val Ala Leu Tyr Val Val Ala Ala
500 505 510

Val Val Ser Val Ala Tyr Leu Gly Phe Val Phe Tyr Leu Gly Trp Gln
515 520 525

Cys Leu Ile Ala Leu Gly Met Ser Phe Leu Asp Cys Gly His Thr Cys
530 535 540

His Leu Gly Leu Thr Ala Gln Pro Glu Leu Tyr Leu Leu Asn Thr Met
545 550 555 560

Asp Ala Asp Ser Leu Val Ser Arg
565

<210> 257
<211> 46
<212> PRT
<213> Homo sapiens

<400> 257
Met Leu Phe Ile His Ala Glu Val Ile Gln Phe Pro Pro Ser Tyr Arg
1 5 10 15

Ser Ile Leu Ile His Pro Thr Leu Glu Met Gln His Leu Cys Gly Arg
20 25 30

Leu Phe His Lys Pro Pro Arg Leu Leu Arg Leu Gly Arg Tyr
35 40 45

<210> 258
<211> 36
<212> PRT
<213> Homo sapiens

<400> 258
Met Ala Ser Leu Gln Phe Val Ile Ser Leu Pro Val Cys Ser Leu Lys
1 5 10 15

Leu Ile Lys Arg Ser Gly Tyr Ile Glu Leu Leu Tyr Arg Cys Glu Gly
20 25 30

Met Asp Lys Ser

35

<210> 259
<211> 898
<212> PRT
<213> Homo sapiens

<400> 259
Met Ser Val Thr Glu Glu Asp Leu Cys His His Met Lys Val Val Val
1 5 10 15

Arg Val Arg Pro Glu Asn Thr Lys Glu Lys Ala Ala Gly Phe His Lys
20 25 30

Val Val His Val Val Asp Lys His Ile Leu Val Phe Asp Pro Lys Gln
35 40 45

Glu Glu Val Ser Phe Phe His Gly Lys Lys Thr Thr Asn Gln Asn Val
50 55 60

Ile Lys Lys Gln Asn Lys Asp Leu Lys Phe Val Phe Asp Ala Val Phe
65 70 75 80

Asp Glu Thr Ser Thr Gln Ser Glu Val Phe Glu His Thr Thr Lys Pro
85 90 95

Ile Leu Arg Ser Phe Leu Asn Gly Tyr Asn Cys Thr Val Leu Ala Tyr
100 105 110

Gly Ala Thr Gly Ala Gly Lys Thr His Thr Met Leu Gly Ser Ala Asp
115 120 125

Glu Pro Gly Val Met Tyr Leu Thr Met Leu His Leu Tyr Lys Cys Met
130 135 140

Asp Glu Ile Lys Glu Glu Lys Ile Cys Ser Thr Ala Val Ser Tyr Leu
145 150 155 160

Glu Val Tyr Asn Glu Gln Ile Arg Asp Leu Leu Val Asn Ser Gly Pro
165 170 175

Leu Ala Val Arg Glu Asp Thr Gln Lys Gly Val Val Val His Gly Leu
180 185 190

Thr Leu His Gln Pro Lys Ser Ser Glu Glu Ile Leu His Leu Leu Asp
195 200 205

Asn Gly Asn Lys Asn Arg Thr Gln His Pro Thr Asp Met Asn Ala Thr
210 215 220

Ser Ser Arg Ser His Ala Val Phe Gln Ile Tyr Leu Arg Gln Gln Asp
225 230 235 240

Lys Thr Ala Ser Ile Asn Gln Asn Val Arg Ile Ala Lys Met Ser Leu
245 250 255

Ile Asp Leu Ala Gly Ser Glu Arg Ala Ser Thr Ser Gly Ala Lys Gly
260 265 270

Thr Arg Phe Val Glu Gly Thr Asn Ile Asn Arg Ser Leu Leu Ala Leu
275 280 285

Gly Asn Val Ile Asn Ala Leu Ala Asp Ser Lys Arg Lys Asn Gln His
290 295 300

Ile Pro Tyr Arg Asn Ser Lys Leu Thr Arg Leu Leu Lys Asp Ser Leu
305 310 315 320

Gly Gly Asn Cys Gln Thr Ile Met Ile Ala Ala Val Ser Pro Ser Ser
325 330 335

Val Phe Tyr Asp Asp Thr Tyr Asn Thr Leu Lys Tyr Ala Asn Arg Ala
340 345 350

Lys Asp Ile Lys Ser Ser Leu Lys Ser Asn Val Leu Asn Val Asn Asn
355 360 365

His Ile Thr Gln Tyr Val Lys Ile Cys Asn Glu Gln Lys Ala Glu Ile
370 375 380

Leu Leu Leu Lys Glu Lys Leu Lys Ala Tyr Glu Glu Gln Lys Ala Phe
385 390 395 400

Thr Asn Glu Asn Asp Gln Ala Lys Leu Met Ile Ser Asn Pro Gln Glu
405 410 415

Lys Glu Ile Glu Arg Phe Gln Glu Ile Leu Asn Cys Leu Phe Gln Asn
420 425 430

Arg Glu Glu Ile Arg Gln Glu Tyr Leu Lys Leu Glu Met Leu Leu Lys
435 440 445

Glu Asn Glu Leu Lys Ser Phe Tyr Gln Gln Gln Cys His Lys Gln Ile
450 455 460

Glu Met Met Cys Ser Glu Asp Lys Val Glu Lys Ala Thr Gly Lys Arg
465 470 475 480

Asp His Arg Leu Ala Met Leu Lys Thr Arg Arg Ser Tyr Leu Glu Lys
485 490 495

Arg Arg Glu Glu Glu Leu Lys Gln Phe Asp Glu Asn Thr Asn Trp Leu
500 505 510

His Arg Val Glu Lys Glu Met Gly Leu Leu Ser Gln Asn Gly His Ile
515 520 525

Pro Lys Glu Leu Lys Lys Asp Leu His Cys His His Leu His Leu Gln
530 535 540

Asn Lys Asp Leu Lys Ala Gln Ile Arg His Met Met Asp Leu Ala Cys
545 550 555 560

Leu Gln Glu Gln Gln His Arg Gln Thr Glu Ala Val Leu Asn Ala Leu
565 570 575

Leu Pro Thr Leu Arg Lys Gln Tyr Cys Thr Leu Lys Glu Ala Gly Leu
580 585 590

Ser Asn Ala Ala Phe Glu Ser Asp Phe Lys Glu Ile Glu His Leu Val
595 600 605

Glu Arg Lys Lys Val Val Val Trp Ala Asp Gln Thr Gly Glu Gln Pro
610 615 620

Lys Gln Asn Asp Leu Pro Gly Ile Ser Val Leu Met Thr Phe Ser Gln
625 630 635 640

Leu Gly Pro Val Gln Pro Ile Pro Cys Cys Ser Ser Ser Gly Gly Thr
645 650 655

Asn Leu Val Lys Ile Pro Thr Glu Lys Arg Thr Arg Arg Lys Leu Met
660 665 670

Pro Ser Pro Leu Lys Gly Gln His Thr Leu Lys Ser Pro Pro Ser Gln
675 680 685

Ser Val Gln Leu Asn Asp Ser Leu Ser Lys Glu Leu Gln Pro Ile Val
690 695 700

Tyr Thr Pro Glu Asp Cys Arg Lys Ala Phe Gln Asn Pro Ser Thr Val
705 710 715 720

Thr Leu Met Lys Pro Ser Ser Phe Thr Thr Ser Phe Gln Ala Ile Ser
725 730 735

Ser Asn Ile Asn Ser Asp Asn Cys Leu Lys Met Leu Cys Glu Val Ala
740 745 750

Ile Pro His Asn Arg Arg Lys Glu Cys Gly Gln Glu Asp Leu Asp Ser
755 760 765

Thr Phe Thr Ile Cys Glu Asp Ile Lys Ser Ser Lys Cys Lys Leu Pro
770 775 780

Glu Gln Glu Ser Leu Pro Asn Asp Asn Lys Asp Ile Leu Gln Arg Leu
785 790 795 800

Asp Pro Ser Ser Phe Ser Thr Lys His Ser Met Pro Val Pro Ser Met
805 810 815

Val Pro Ser Tyr Met Ala Met Thr Thr Ala Ala Lys Arg Lys Arg Lys
820 825 830

Leu Thr Ser Ser Thr Ser Asn Ser Ser Leu Thr Ala Asp Val Asn Ser
835 840 845

Gly Phe Ala Lys Arg Val Arg Gln Asp Asn Ser Ser Glu Lys His Leu
850 855 860

Gln Glu Asn Lys Pro Thr Met Glu His Lys Arg Asn Ile Cys Lys Ile
865 870 875 880

Asn Pro Ser Met Val Arg Lys Phe Gly Arg Asn Ile Ser Lys Gly Asn
885 890 895

Leu Arg

<210> 260
<211> 71
<212> PRT
<213> Homo sapiens

<400> 260
Met Ser Lys Asp Arg Ala Asn Met Gln His Arg Tyr Ile Glu Leu Phe
1 5 10 15

Leu Asn Ser Thr Thr Gly Ala Ser Asn Gly Ala Tyr Ser Ser Gln Val
20 25 30

Met Gln Gly Met Gly Val Ser Ala Ala Gln Ala Thr Tyr Ser Gly Leu
35 40 45

Glu Ser Gln Ser Val Ser Gly Cys Tyr Gly Ala Gly Tyr Ser Gly Gln
50 55 60

Asn Ser Met Gly Gly Tyr Asp
65 70

<210> 261
<211> 592
<212> PRT
<213> Homo sapiens

<400> 261
Met Ala Pro Gly Gln Leu Ala Leu Phe Ser Val Ser Asp Lys Thr Gly
1 5 10 15

Leu Val Glu Phe Ala Arg Asn Leu Thr Ala Leu Gly Leu Asn Leu Val
20 25 30

Ala Ser Gly Gly Thr Ala Lys Ala Leu Arg Asp Ala Gly Leu Ala Val
35 40 45

Arg Asp Val Ser Glu Leu Thr Gly Phe Pro Glu Met Leu Gly Gly Arg
50 55 60

Val Lys Thr Leu His Pro Ala Val His Ala Gly Ile Leu Ala Arg Asn
65 70 75 80

Ile Pro Glu Asp Asn Ala Asp Met Ala Arg Leu Asp Phe Asn Leu Ile
85 90 95

Arg Val Val Ala Cys Asn Leu Tyr Pro Phe Val Lys Thr Val Ala Ser
100 105 110

Pro Gly Val Thr Val Glu Glu Ala Val Glu Gln Ile Asp Ile Gly Gly
115 120 125

Val Thr Leu Leu Arg Ala Ala Ala Lys Asn His Ala Arg Val Thr Val
130 135 140

Val Cys Glu Pro Glu Asp Tyr Val Val Val Ser Thr Glu Met Gln Ser
145 150 155 160

Ser Glu Ser Lys Asp Thr Ser Leu Glu Thr Arg Arg Gln Leu Ala Leu

165 170 175

Lys Ala Phe Thr His Thr Ala Gln Tyr Asp Glu Ala Ile Ser Asp Tyr
180 185 190

Phe Arg Lys Gln Tyr Ser Lys Gly Val Ser Gln Met Pro Leu Arg Tyr
195 200 205

Gly Met Asn Pro His Gln Thr Pro Ala Gln Leu Tyr Thr Leu Gln Pro
210 215 220

Lys Leu Pro Ile Thr Val Leu Asn Gly Ala Pro Gly Phe Ile Asn Leu
225 230 235 240

Cys Asp Ala Leu Asn Ala Trp Gln Leu Val Lys Glu Leu Lys Glu Ala
245 250 255

Leu Gly Ile Pro Ala Ala Ala Ser Phe Lys His Val Ser Pro Ala Gly
260 265 270

Ala Ala Val Gly Ile Pro Leu Ser Glu Asp Glu Ala Lys Val Cys Met
275 280 285

Val Tyr Asp Leu Tyr Lys Thr Leu Thr Pro Ile Ser Ala Ala Tyr Ala
290 295 300

Arg Ala Arg Gly Ala Asp Arg Met Ser Ser Phe Gly Asp Phe Val Ala
305 310 315 320

Leu Ser Asp Val Cys Asp Val Pro Thr Ala Lys Ile Ile Ser Arg Glu
325 330 335

Val Ser Asp Gly Ile Ile Ala Pro Gly Tyr Glu Glu Glu Ala Leu Thr
340 345 350

Ile Leu Ser Lys Lys Lys Asn Gly Asn Tyr Cys Val Leu Gln Met Asp
355 360 365

Gln Ser Tyr Lys Pro Asp Glu Asn Glu Val Arg Thr Leu Phe Gly Leu
370 375 380

His Leu Ser Gln Lys Arg Asn Asn Gly Val Val Asp Lys Ser Leu Phe
385 390 395 400

Ser Asn Val Val Thr Lys Asn Lys Asp Leu Pro Glu Ser Ala Leu Arg
405 410 415

Asp Leu Ile Val Ala Thr Ile Ala Val Lys Tyr Thr Gln Ser Asn Ser

420 425 430

Val Cys Tyr Ala Lys Asn Gly Gln Val Ile Gly Ile Gly Ala Gly Gln
435 440 445

Gln Ser Arg Ile His Cys Thr Arg Leu Ala Gly Asp Lys Ala Asn Tyr
450 455 460

Trp Trp Leu Arg His His Pro Gln Val Leu Ser Met Lys Phe Lys Thr
465 470 475 480

Gly Val Lys Arg Ala Glu Ile Ser Asn Ala Ile Asp Gln Tyr Val Thr
485 490 495

Gly Thr Ile Gly Glu Asp Glu Asp Leu Ile Lys Trp Lys Ala Leu Phe
500 505 510

Glu Glu Val Pro Glu Leu Leu Thr Glu Ala Glu Lys Lys Glu Trp Val
515 520 525

Glu Lys Leu Thr Glu Val Ser Ile Ser Ser Asp Ala Phe Phe Pro Phe
530 535 540

Arg Asp Asn Val Asp Arg Ala Lys Arg Ser Gly Val Ala Tyr Ile Ala
545 550 555 560

Ala Pro Ser Gly Ser Ala Ala Asp Lys Val Val Ile Glu Ala Cys Asp
565 570 575

Glu Leu Gly Ile Ile Leu Ala His Thr Asn Leu Arg Leu Phe His His
580 585 590

<210> 262
<211> 62
<212> PRT
<213> Homo sapiens

<400> 262
Met Phe Glu Leu Leu Pro Asn Cys Met Leu Phe Ile Leu Asn Ser Pro
1 5 10 15

Ser Asp Arg Ile Pro Arg Pro Arg Glu Val Lys Lys Thr Ser Pro Arg
20 25 30

Ser Ile Thr Leu Leu Leu Thr Ala Pro Asn Leu Leu Asp Ser Lys Ser
35 40 45

Asn Gly Phe Pro Gly Thr Met Met Leu Val Asp Leu Lys Lys
50 55 60

<210> 263
<211> 43
<212> PRT
<213> Homo sapiens

<400> 263
Met Thr Ala Leu Phe Pro Gly Leu Ala Pro Glu Thr Glu Gln Pro Asp
1 5 10 15

Ile His Thr Pro Arg Arg Gln Leu Glu Val Pro Pro Gly Asn Gln Asn
20 25 30

His Pro Gln Arg Arg Pro Pro Asp Thr Asp Ile
35 40

<210> 264
<211> 303
<212> PRT
<213> Homo sapiens

<400> 264
Met Lys Pro Thr Gly Thr Asp Pro Arg Ile Leu Ser'Ile Ala Ala Glu
1 5 10 15

Val Ala Lys Ser Pro Glu Gln Asn Val Pro Val Ile Leu Leu Lys Leu
20 25 30

Lys Glu Ile Ile Asn Ile Thr Pro Leu Gly Ser Ser Glu Leu Lys Lys
35 40 45

Ile Lys Gln Asp Ile Tyr Cys Tyr Asp Leu Ile Gln Tyr Cys Leu Leu
50 55 60

Val Leu Ser Gln Asp Tyr Ser Arg Ile Gln Gly Gly Trp Thr Thr Ile
65 70 75 80

Ser Gln Leu Thr Gln Ile Leu Ser His Cys Cys Val Gly Leu Glu Pro
85 90 95

Gly Glu Asp Ala Glu Glu Phe Tyr Asn Glu Leu Leu Pro Ser Ala Ala

100 105 110

Glu Asn Phe Leu Val Leu Gly Arg Gln Leu Gln Thr Cys Phe Ile Asn
115 120 125

Ala Ala Lys Ala Glu Glu Lys Asp Glu Leu Leu His Phe Phe Gln Ile
130 135 140

Val Thr Asp Ser Leu Phe Trp Leu Leu Gly Gly His Val Glu Leu Ile
145 150 155 160

Gln Asn Val Leu Gln Ser Asp His Phe Leu His Leu Leu Gln Ala Asp
165 170 175

Asn Val Gln Ile Gly Ser Ala Val Met Met Met Leu Gln Asn Ile Leu
180 185 190

Gln Ile Asn Ser Gly Asp Leu Leu Arg Ile Gly Arg Lys Ala Leu Tyr
195 200 205

Ser Ile Leu Asp Glu Val Ile Phe Lys Leu Phe Ser Thr Pro Ser Pro
210 215 220

Val Ile Arg Ser Thr Ala Thr Lys Leu Leu Leu Leu Met Ala Glu Ser
225 230 235 240

His Gln Glu Ile Leu Ile Leu Leu Arg Gln Ser Thr Cys Tyr Lys Gly
245 250 255

Leu Arg Arg Leu Leu Ser Lys Gln Glu Thr Gly Thr Glu Phe Ser Gln
260 265 270

Glu Leu Arg Gln Leu Val Gly Leu Leu Ser Pro Met Val Tyr Gln Glu
275 280 285

Val Glu Glu Gln Ile Gln Thr Ile Lys Asp Val Ala Gly Asp Lys
290 295 300

<210> 265
<211> 264
<212> PRT
<213> Homo sapiens

<400> 265
Met Leu Leu Glu Ile Asn Arg Gln Lys Glu Glu Glu Asp Leu Lys Leu
1 5 10 15

Gln Leu Gln Leu Gln Arg Gln Arg Ala Met Arg Leu Ser Arg Glu Leu
20 25 30

Gln Leu Ser Met Leu Glu Ile Val His Pro Gly Gln Val Glu Lys His
35 40 45

Tyr Arg Glu Met Glu Glu Lys Ser Ala Leu Ile Ile Gln Lys His Trp
50 55 60

Arg Gly Tyr Arg Glu Arg Lys Asn Phe His Gln Gln Arg Gln Ser Leu
65 70 75 80

Ile Glu Tyr Lys Ala Ala Val Thr Leu Gln Arg Ala Ala Leu Lys Phe
85 90 95

Leu Ala Lys Tyr Arg Lys Lys Lys Lys Leu Phe Ala Pro Trp Arg Gly
100 105 110

Leu Gln Glu Leu Thr Asp Ala Arg Arg Val Glu Leu Lys Lys Arg Val
115 120 125

Asp Asp Tyr Val Arg Arg His Leu Gly Ser Pro Met Ser Asp Val Val
130 135 140

Ser Arg Glu Leu His Ala Gln Ala Gln Glu Arg Leu Gln His Tyr Phe
145 150 155 160

Met Gly Arg Ala Leu Glu Glu Arg Ala Gln Gln His Arg Glu Ala Leu
165 170 175

Ile Ala Gln Ile Ser Thr Asn Val Glu Gln Leu Met Lys Ala Pro Ser
180 185 190

Leu Lys Glu Ala Glu Gly Lys Glu Pro Glu Leu Phe Leu Ser Arg Ser
195 200 205

Arg Pro Val Ala Ala Lys Ala Lys Gln Ala His Leu Thr Thr Leu Lys
210 215 220

His Ile Gln Ala Pro Trp Trp Lys Lys Leu Gly Glu Glu Ser Gly Asp
225 230 235 240

Glu Ile Asp Val Pro Lys Asp Glu Leu Ser Ile Glu Leu Glu Asn Leu
245 250 255

Phe Ile Gly Gly Thr Lys Pro Pro
260

<210> 266
<211> 248
<212> PRT
<213> Homo sapiens

<400> 266
Met Ser Gly Gly Gly Val Ile Arg Gly Pro Ala Gly Asn Asn Asp Cys
1 5 10 15

Arg Ile Tyr Val Gly Asn Leu Pro Pro Asp Ile Arg Thr Lys Asp Ile
20 25 30

Glu Asp Val Phe Tyr Lys Tyr Gly Ala Ile Arg Asp Ile Asp Leu Lys
35 40 45

Asn Arg Arg Gly Gly Pro Pro Phe Ala Phe Val Glu Phe Glu Asp Pro
50 55 60

Arg Asp Ala Glu Asp Ala Val Tyr Gly Arg Asp Gly Tyr Asp Tyr Asp
65 70 75 80

Gly Tyr Arg Leu Arg Val Glu Phe Pro Arg Ser Gly Arg Gly Thr Gly
85 90 95

Arg Gly Gly Gly Gly Gly Gly Gly Gly Gly Ala Pro Arg Gly Arg Tyr
100 105 110

Gly Pro Pro Ser Arg Arg Ser Glu Asn Arg Val Val Val Ser Gly Leu
115 120 125

Pro Pro Ser Gly Ser Trp Gln Asp Leu Lys Asp His Met Arg Glu Ala
130 135 140

Gly Asp Val Cys Tyr Ala Asp Val Tyr Arg Asp Gly Thr Gly Val Val
145 150 155 160

Glu Phe Val Arg Lys Glu Asp Met Thr Tyr Ala Val Arg Lys Leu Asp
165 170 175

Asn Thr Lys Phe Arg Ser His Glu Gly Glu Thr Ala Tyr Ile Arg Val
180 185 190

Lys Val Asp Gly Pro Arg Ser Pro Ser Tyr Gly Arg Ser Arg Ser Arg
195 200 205

Ser Arg Ser Arg Ser Arg Ser Arg Ser Arg Ser Asn Ser Arg Ser Arg
210 215 220

Ser Tyr Ser Pro Arg Arg Ser Arg Gly Ser Pro Arg Tyr Ser Pro Arg
225 230 235 240

His Ser Arg Ser Arg Ser Arg Thr
245

<210> 267
<211> 313
<212> PRT
<213> Homo sapiens

<400> 267
Met Pro Val Ala Gly Ser Glu Leu Pro Arg Arg Pro Leu Pro Pro Ala
1 5 10 15

Ala Gln Glu Arg Asp Ala Glu Pro Arg Pro Pro His Gly Glu Leu Gln
20 25 30

Tyr Leu Gly Gln Ile Gln His Ile Leu Arg Cys Gly Val Arg Lys Asp
35 40 45

Asp Arg Thr Gly Thr Gly Thr Leu Ser Val Phe Gly Met Gln Ala Arg
50 55 60

Tyr Ser Leu Arg Asp Glu Phe Pro Leu Leu Thr Thr Lys Arg Val Phe
65 70 75 80

Trp Lys Gly Val Leu Glu Glu Leu Leu Trp Phe Ile Lys Gly Ser Thr
85 90 95

Asn Ala Lys Glu Leu Ser Ser Lys Gly Val Lys Ile Trp Asp Ala Asn
100 105 110

Gly Ser Arg Asp Phe Leu Asp Ser Leu Gly Phe Ser Thr Arg Glu Glu
115 120 125

Gly Asp Leu Gly Pro Val Tyr Gly Phe Gln Trp Arg His Phe Gly Ala
130 135 140

Glu Tyr Arg Asp Met Glu Ser Asp Tyr Ser Gly Gln Gly Val Asp Gln
145 150 155 160

Leu Gln Arg Val Ile Asp Thr Ile Lys Thr Asn Pro Asp Asp Arg Arg
165 170 175

Ile Ile Met Cys Ala Trp Asn Pro Arg Asp Leu Pro Leu Met Ala Leu

180 185 190

Pro Pro Cys His Ala Leu Cys Gln Phe Tyr Val Val Asn Ser Glu Leu
195 200 205

Ser Cys Gln Leu Tyr Gln Arg Ser Gly Asp Met Gly Leu Gly Val Pro
210 215 220

Phe Asn Ile Ala Ser Tyr Ala Leu Leu Thr Tyr Met Ile Ala His Ile
225 230 235 240

Thr Gly Leu Lys Pro Gly Asp Phe Ile His Thr Leu Gly Asp Ala His
245 250 255

Ile Tyr Leu Asn His Ile Glu Pro Leu Lys Ile Gln Leu Gln Arg Glu
260 265 270

Pro Arg Pro Phe Pro Lys Leu Arg Ile Leu Arg Lys Val Glu Lys Ile
275 280 285

Asp Asp Phe Lys Ala Glu Asp Phe Gln Ile Glu Gly Tyr Asn Pro His
290 295 300

Pro Thr Ile Lys Met Glu Met Ala Val
305 310

<210> 268
<211> 511
<212> PRT
<213> Homo sapiens

<400> 268
Met Ala Val Arg Leu Ala Gly Gly Leu Gln Lys Met Val Ala Leu Leu
1 5 10 15

Asn Lys Thr Asn Val Lys Phe Leu Ala Ile Thr Thr Asp Cys Leu Gln
20 25 30

Ile Leu Ala Tyr Gly Asn Gln Glu Ser Lys Leu Ile Ile Leu Ala Ser
35 40 45

Gly Gly Pro Gln Ala Leu Val Asn Ile Met Arg Thr Tyr Thr Tyr Glu
50 55 60

Lys Leu Leu Trp Thr Thr Ser Arg Val Leu Lys Val Leu Ser Val Cys
65 70 75 80

Ser Ser Asn Lys Pro Ala Ile Val Glu Ala Gly Gly Met Gln Ala Leu
85 90 95

Gly Leu His Leu Thr Asp Pro Ser Gln Arg Leu Val Gln Asn Cys Leu
100 105 110

Trp Thr Leu Arg Asn Leu Ser Asp Ala Ala Thr Lys Gln Glu Gly Met
115 120 125

Glu Gly Leu Leu Gly Thr Leu Val Gln Leu Leu Gly Ser Asp Asp Ile
130 135 140

Asn Val Val Thr Cys Ala Ala Gly Ile Leu Ser Asn Leu Thr Cys Asn
145 150 155 160

Asn Tyr Lys Asn Lys Met Met Val Cys Gln Val Gly Gly Ile Glu Ala
165 170 175

Leu Val Arg Thr Val Leu Arg Ala Gly Asp Arg Glu Asp Ile Thr Glu
180 185 190

Pro Ala Ile Cys Ala Leu Arg His Leu Thr Ser Arg His Gln Glu Ala
195 200 205

Glu Met Ala Gln Asn Ala Val Arg Leu His Tyr Gly Leu Pro Val Val
210 215 220

Val Lys Leu Leu His Pro Pro Ser His Trp Pro Leu Ile Lys Ala Thr
225 230 235 240

Val Gly Leu Ile Arg Asn Leu Ala Leu Cys Pro Ala Asn His Ala Pro
245 250 255

Leu Arg Glu Gln Gly Ala Ile Pro Arg Leu Val Gln Leu Leu Val Arg
260 265 270

Ala His Gln Asp Thr Gln Arg Arg Thr Ser Met Gly Gly Thr Gln Gln
275 280 285

Gln Phe Val Glu Gly Val Arg Met Glu Glu Ile Val Glu Gly Cys Thr
290 295 300

Gly Ala Leu His Ile Leu Ala Arg Asp Val His Asn Arg Ile Val Ile
305 310 315 320

Arg Gly Leu Asn Thr Ile Pro Leu Phe Val Gln Leu Leu Tyr Ser Pro
325 330 335

Ile Glu Asn Ile Gln Arg Val Ala Ala Gly Val Leu Cys Glu Leu Ala
340 345 350

Gln Asp Lys Glu Ala Ala Glu Ala Ile Glu Ala Glu Gly Ala Thr Ala
355 360 365

Pro Leu Thr Glu Leu Leu His Ser Arg Asn Glu Gly Val Ala Thr Tyr
370 375 380

Ala Ala Ala Val Leu Phe Arg Met Ser Glu Asp Lys Pro Gln Asp Tyr
385 390 395 400

Lys Lys Arg Leu Ser Val Glu Leu Thr Ser Ser Leu Phe Arg Thr Glu
405 410 415

Pro Met Ala Trp Asn Glu Thr Ala Asp Leu Gly Leu Asp Ile Gly Ala
420 425 430

Gln Gly Glu Pro Leu Gly Tyr Arg Gln Asp Asp Pro Ser Tyr Arg Ser
435 440 445

Phe His Ser Gly Gly Tyr Gly Gln Asp Ala Leu Gly Met Asp Pro Met
450 455 460

Met Glu His Glu Met Gly Gly His His Pro Gly Ala Asp Tyr Pro Val
465 470 475 480

Asp Gly Leu Pro Asp Leu Gly His Ala Gln Asp Leu Met Asp Gly Leu
485 490 495

Pro Pro Gly Asp Ser Asn Gln Leu Ala Trp Phe Asp Thr Asp Leu
500 505 510

<210> 269
<211> 128
<212> PRT
<213> Homo sapiens

<400> 269
Met Phe Asp Val Thr Ser Arg Val Thr Tyr Lys Asn Val Pro Asn Trp
1 5 10 15

His Arg Asp Leu Val Arg Val Cys Glu Asn Ile Pro Ile Val Leu Cys
20 25 30

Gly Asn Lys Val Asp Ile Lys Asp Arg Lys Val Lys Ala Lys Ser Ile
35 40 45

Val Phe His Arg Lys Lys Asn Leu Gln Tyr Tyr Asp Ile Ser Ala Lys
50 55 60

Ser Asn Tyr Asn Phe Glu Lys Pro Phe Leu Trp Leu Ala Arg Lys Leu
65 70 75 80

Ile Gly Asp Pro Asn Leu Glu Phe Val Ala Met Pro Ala Leu Ala Pro
85 90 95

Pro Glu Val Val Met Asp Pro Ala Leu Ala Ala Gln Tyr Glu His Asp
100 105 110

Leu Glu Val Ala Gln Thr Thr Ala Leu Pro Asp Glu Asp Asp Asp Leu
115 120 125

<210> 270
<211> 506
<212> PRT
<213> Homo sapiens

<400> 270
Met Glu Asp His Gln His Val Pro Ile Asp Ile Gln Thr Ser Lys Leu
1 5 10 15

Leu Asp Trp Leu Val Asp Arg Arg His Cys Ser Leu Lys Trp Gln Ser
20 25 30

Leu Val Leu Thr Ile Arg Glu Lys Ile Asn Ala Ala Ile Gln Asp Met
35 40 45

Pro Glu Ser Glu Glu Ile Ala Gln Leu Leu Ser Gly Ser Tyr Ile His
50 55 60

Tyr Phe His Cys Leu Arg Ile Leu Asp Leu Leu Lys Gly Thr Glu Ala
65 70 75 80

Ser Thr Lys Asn Ile Phe Gly Arg Tyr Ser Ser Gln Arg Met Lys Asp
85 90 95

Trp Gln Glu Ile Ile Ala Leu Tyr Glu Lys Asp Asn Thr Tyr Leu Val
100 105 110

Glu Leu Ser Ser Leu Leu Val Arg Asn Val Asn Tyr Glu Ile Pro Ser

115 120 125

Leu Lys Lys Gln Ile Ala Lys Cys Gln Gln Leu Gln Gln Glu Tyr Ser
130 135 140

Arg Lys Glu Glu Glu Cys Gln Ala Gly Ala Ala Glu Met Arg Glu Gln
145 150 155 160

Phe Tyr His Ser Cys Lys Gln Tyr Gly Ile Thr Gly Glu Asn Val Arg
165 170 175

Gly Glu Leu Leu Ala Leu Val Lys Asp Leu Pro Ser Gln Leu Ala Glu
180 185 190

Ile Gly Ala Ala Ala Gln Gln Ser Leu Gly Glu Ala Ile Asp Val Tyr
195 200 205

Gln Ala Ser Val Gly Phe Val Cys Glu Ser Pro Thr Glu Gln Val Leu
210 215 220

Pro Met Leu Arg Phe Val Gln Lys Arg Gly Asn Ser Thr Val Tyr Glu
225 230 235 240

Trp Arg Thr Gly Thr Glu Pro Ser Val Val Glu Arg Pro His Leu Glu
245 250 255

Glu Leu Pro Glu Gln Val Ala Glu Asp Ala Ile Asp Trp Gly Asp Phe
260 265 270

Gly Val Glu Ala Val Ser Glu Gly Thr Asp Ser Gly Ile Ser Ala Glu
275 280 285

Ala Ala Gly Ile Asp Trp Gly Ile Phe Pro Glu Ser Asp Ser Lys Asp
290 295 300

Pro Gly Gly Asp Gly Ile Asp Trp Gly Asp Asp Ala Val Ala Leu Gln
305 310 315 320

Ile Thr Val Leu Glu Ala Gly Thr Gln Ala Pro Glu Gly Val Ala Arg
325 330 335

Gly Pro Asp Ala Leu Thr Leu Leu Glu Tyr Thr Glu Thr Arg Asn Gln
340 345 350

Phe Leu Asp Glu Leu Met Glu Leu Glu Ile Phe Leu Ala Gln Arg Ala
355 360 365

Val Glu Leu Ser Glu Glu Ala Asp Val Leu Ser Val Ser Gln Phe Gln

370 375 380

Leu Ala Pro Ala Ile Leu Gln Gly Gln Thr Lys Glu Lys Met Val Thr
385 390 395 400

Met Val Ser Val Leu Glu Asp Leu Ile Gly Lys Leu Thr Ser Leu Gln
405 410 415

Leu Gln His Leu Phe Met Ile Leu Ala Ser Pro Arg Tyr Val Asp Arg
420 425 430

Val Thr Glu Phe Leu Gln Gln Lys Leu Lys Gln Ser Gln Leu Leu Ala
435 440 445

Leu Lys Lys Glu Leu Met Val Gln Lys Gln Gln Glu Ala Leu Glu Glu
450 455 460

Gln Ala Ala Leu Glu Pro Lys Leu Asp Leu Leu Leu Glu Lys Thr Lys
465 470 475 480

Glu Leu Gln Lys Leu Ile Glu Ala Asp Ile Ser Lys Arg Tyr Ser Gly
485 490 495

Arg Pro Val Asn Leu Met Gly Thr Ser Leu
500 505

<210> 271
<211> 136
<212> PRT
<213> Homo sapiens

<400> 271
Met Thr Ser Leu Cys Met Ala Met Thr Glu Glu Gln His Lys Ser Val
1 5 10 15

Val Ile Asp Cys Ser Ser Ser Gln Pro Gln Phe Cys Asn Ala Gly Ser
20 25 30

Asn Arg Phe Cys Glu Asp Trp Met Gln Ala Phe Leu Asn Gly Ala Lys
35 40 45

Gly Gly Asn Pro Phe Leu Phe Arg Gln Val Leu Glu Asn Phe Lys Leu
50 55 60

Lys Ala Ile Gln Asp Thr Asn Asn Leu Lys Arg Phe Ile Arg Gln Ala
65 70 75 80

Glu Met Asn His Tyr Ala Leu Phe Lys Cys Tyr Met Phe Leu Lys Asn
85 90 95

Cys Gly Ser Gly Asp Ile Leu Leu Lys Ile Val Lys Val Glu His Glu
100 105 110

Glu Met Pro Glu Ala Lys Asn Val Ile Ala Val Leu Glu Glu Phe Met
115 120 125

Lys Glu Ala Leu Asp Gln Ser Phe
130 135

<210> 272
<211> 509
<212> PRT
<213> Homo sapiens

<400> 272
Met Phe Thr Asn Asp Met Met Glu Cys Lys Gln Asp Glu Ile Val Met
1 5 10 15

Gln Gly Met Asp Pro Ser Ala Leu Glu Ala Leu Ile Asn Phe Ala Tyr
20 25 30

Asn Gly Asn Leu Ala Ile Asp Gln Gln Asn Val Gln Ser Leu Leu Met
35 40 45

Gly Ala Ser Phe Leu Gln Leu Gln Ser Ile Lys Asp Ala Cys Cys Thr
50 55 60

Phe Leu Arg Glu Arg Leu His Pro Lys Asn Cys Leu Gly Val Arg Gln
65 70 75 80

Phe Ala Glu Thr Met Met Cys Ala Val Leu Tyr Asp Ala Ala Asn Ser
85 90 95

Phe Ile His Gln His Phe Val Glu Val Ser Met Ser Glu Glu Phe Leu
100 105 110

Ala Leu Pro Leu Glu Asp Val Leu Glu Leu Val Ser Arg Asp Glu Leu
115 120 125

Asn Val Lys Ser Glu Glu Gln Val Phe Glu Ala Ala Leu Ala Trp Val
130 135 140

Arg Tyr Asp Arg Glu Gln Arg Gly Pro Tyr Leu Pro Glu Leu Leu Ser
145 150 155 160

Asn Ile Arg Leu Pro Leu Cys Arg Pro Gln Phe Leu Ser Asp Arg Val
165 170 175

Gln Gln Asp Asp Leu Val Arg Cys Cys His Lys Cys Arg Asp Leu Val
180 185 190

Asp Glu Ala Lys Asp Tyr His Leu Met Pro Glu Arg Arg Pro His Leu
195 200 205

Pro Ala Phe Arg Thr Arg Pro Arg Cys Cys Thr Ser Ile Ala Gly Leu
210 215 220

Ile Tyr Ala Val Gly Gly Leu Asn Ser Ala Gly Asp Ser Leu Asn Val
225 230 235 240

Val Glu Val Phe Asp Pro Ile Ala Asn Cys Trp Glu Arg Cys Arg Pro
245 250 255

Met Thr Thr Ala Arg Ser Arg Val Gly Val Ala Val Val Asn Gly Leu
260 265 270

Leu Tyr Ala Ile Gly Gly Tyr Asp Gly Gln Leu Arg Leu Ser Thr Val
275 280 285

Glu Ala Tyr Asn Pro Glu Thr Asp Thr Trp Thr Arg Val Gly Ser Met
290 295 300

Asn Ser Lys Arg Ser Ala Met Gly Thr Val Val Leu Asp Gly Gln Ile
305 310 315 320

Tyr Val Cys Gly Gly Tyr Asp Gly Asn Ser Ser Leu Ser Ser Val Glu
325 330 335

Thr Tyr Ser Pro Glu Thr Asp Lys Trp Thr Val Val Thr Ser Met Ser
340 345 350

Ser Asn Arg Ser Ala Ala Gly Val Thr Val Phe Glu Gly Arg Ile Tyr
355 360 365

Val Ser Gly Gly His Asp Gly Leu Gln Ile Phe Ser Ser Val Glu His
370 375 380

Tyr Asn His His Thr Ala Thr Trp His Pro Ala Ala Gly Met Leu Asn
385 390 395 400

Lys Arg Cys Arg His Gly Ala Ala Ser Leu Gly Ser Lys Met Phe Val
405 410 415

Cys Gly Gly Tyr Asp Gly Ser Gly Phe Leu Ser Ile Ala Glu Met Tyr
420 425 430

Ser Ser Val Ala Asp Gln Trp Cys Leu Ile Val Pro Met His Thr Arg
435 440 445

Arg Ser Arg Val Ser Leu Val Ala Ser Cys Gly Arg Leu Tyr Ala Val
450 455 460

Gly Gly Tyr Asp Gly Gln Ser Asn Leu Ser Ser Val Glu Met Tyr Asp
465 470 475 480

Pro Glu Thr Asp Cys Trp Thr Phe Met Ala Pro Met Ala Cys His Glu
485 490 495

Gly Gly Val Gly Val Gly Cys Ile Pro Leu Leu Thr Ile
500 505

<210> 273
<211> 49
<212> PRT
<213> Homo sapiens

<400> 273
Met Ser Phe Ser Ala Ile Leu Ser Pro Phe Ser Ser Leu Ser Val Asn
1 5 10 15

Val Arg Asn Leu Arg Gln Arg Gly Lys Gly Arg Gln Asn Ser Arg Ile
20 25 30

Leu Thr Leu Ile Val Lys Ile Leu Phe Lys Thr Trp His Leu Ile Phe
35 40 45

Leu

<210> 274
<211> 109
<212> PRT
<213> Homo sapiens

<400> 274
Met Glu Ser His Ser Val Thr Gln Ala Gly Val Gln Trp His Asp Leu
1 5 10 15

Gly Ser Leu His Ser Pro Leu Leu Gly Ser Ser Asp Ser Pro Thr Ser
20 25 30

Ala Ser Arg Val Ala Gly Ile Thr Gly Met Gln His His Thr Gln Leu
35 40 45

Ile Phe Leu Phe Leu Val Glu Met Gly Phe His His Val Gly Gln Ala
50 55 60

Gly Leu Lys Leu Leu Thr Ser Gly Asp Pro Pro Ala Ser Ala Ser Gln
65 70 75 80

Ser Ala Gly Ile Thr Gly Val Gly His His Thr Trp Pro Ile Met Glu
85 90 95

Asp Phe Leu Met Val Met Phe Glu Leu Gly Phe Gly Glu
100 105

<210> 275
<211> 54
<212> PRT
<213> Homo sapiens

<400> 275
Met Glu Ser Asn Ile Ile Tyr Thr Pro Ser Leu Pro Leu Phe Leu Pro
1 5 10 15

Pro Phe Leu Pro Pro Ser Leu Pro Pro Phe Leu Pro Pro Phe Ser Leu
20 25 30

Ser Leu Ser Leu Pro Ala Ser Leu Pro Phe Phe Leu Leu Cys Leu Leu
35 40 45

Pro Cys Asp Trp Gly Lys
50

<210> 276
<211> 66
<212> PRT
<213> Homo sapiens

<400> 276
Met Leu Leu Tyr Arg Leu Ala Gln Leu Gly Leu Tyr Phe Leu Tyr Ser
1 5 10 15

Met Pro Val Glu His Gln Met Leu Asn Thr Ser Thr Cys Cys Asp Phe

20 25 30

Ala Ile Pro Ala His Ile Thr His Leu Ile Ser Phe Val Gly Gly His
35 40 45

Val Gly Trp Pro Thr His Trp Gln Val Asn Ser Leu Ile Trp Thr Met
50 55 60

Ser His
65

<210> 277
<211> 180
<212> PRT
<213> Homo sapiens

<400> 277
Met Arg Pro Leu Thr Glu Glu Glu Thr Arg Val Met Phe Glu Lys Ile
1 5 10 15

Ala Lys Tyr Ile Gly Glu Asn Leu Gln Leu Leu Val Asp Arg Pro Asp
20 25 30

Gly Thr Tyr Cys Phe Arg Leu His Asn Asp Arg Val Tyr Tyr Val Ser
35 40 45

Glu Lys Ile Met Lys Leu Ala Ala Asn Ile Ser Gly Asp Lys Leu Val
50 55 60

Ser Leu Gly Thr Cys Phe Gly Lys Phe Thr Lys Thr His Lys Phe Arg
65 70 75 80

Leu His Val Thr Ala Leu Asp Tyr Leu Ala Pro Tyr Ala Lys Tyr Lys
85 90 95

Val Trp Ile Lys Pro Gly Ala Glu Gln Ser Phe Leu Tyr Gly Asn His
100 105 110

Val Leu Lys Ser Gly Leu Gly Arg Ile Thr Glu Asn Thr Ser Gln Tyr
115 120 125

Gln Gly Val Val Val Tyr Ser Met Ala Asp Ile Pro Leu Gly Phe Gly
130 135 140

Val Ala Ala Lys Ser Thr Gln Asp Cys Arg Lys Val Asp Pro Met Ala
145 150 155 160

Ile Val Val Phe His Gln Ala Asp Ile Gly Glu Tyr Val Arg His Glu
165 170 175

Glu Thr Leu Thr
180

<210> 278
<211> 34
<212> PRT
<213> Homo sapiens

<400> 278
Met Gly Leu Glu Arg Gly Phe Asp Pro Arg Ser Leu Cys Ala Phe Ala
1 5 10 15

Ala Glu Pro His Asn Leu Ser Phe Gln Lys His Phe Gln Asn Ala Asn
20 25 30

Ile Phe

<210> 279
<211> 168
<212> PRT
<213> Homo sapiens

<400> 279
Met Leu Arg Val Asn Phe Phe Phe Phe Phe Phe Phe Phe Phe Ser Phe
1 5 10 15

Ser Leu Arg Leu Gly Leu Ala Leu Leu Pro Arg Leu Glu Trp Ser Gly
20 25 30

Val Ile Leu Ala Tyr Cys Ser Leu Cys Leu Pro Gly Ser Ser Ser Pro
35 40 45

Ala Ser Ala Ser Gly Val Ala Gly Thr Thr Gly Ser Cys His His Gly
50 55 60

Gln Pro Thr Phe Ala Cys Phe Val Lys Met Gly Ser His Ser Val Ala
65 70 75 80

Gln Ala Gly Leu Lys Leu Leu Gly Ser Gly Asp Pro Pro Val Ser Ala
85 90 95

Ser Gln Ser Ala Gly Ile Thr Ile Val Ser His His Val Gln Leu Glu

100 105 110

Gly Ser Thr Ser Phe Thr Phe Cys Lys His Ile Cys Ile Phe Thr Pro
115 120 125

Pro Phe Pro Ser Phe Ser Leu Phe Ile Ser His Phe Tyr Ile Asp Leu
130 135 140

Leu Phe Tyr Asn Lys Thr Leu Leu Pro Lys Lys Lys Lys Lys Lys Lys
145 150 155 160

Lys Lys Lys Lys Lys Lys Lys Lys
165

<210> 280
<211> 158
<212> PRT
<213> Homo sapiens

<400> 280
Met Met Ile Trp Ile His Gln Asp Leu Phe Tyr Ala Gln Gly Gln Phe
1 5 10 15

Leu Phe Phe Phe Phe Phe Phe Phe Phe Phe Phe Phe Glu Thr Gly Ser
20 25 30

Arg Phe Val Ala Gln Ala Gly Val Glu Trp Arg Asp Leu Gly Leu Leu
35 40 45

Gln Pro Leu Pro Pro Arg Leu Glu Gln Ser Cys Leu Ser Leu Arg Ser
50 55 60

Ser Trp Asp His Arg Phe Met Pro Pro Trp Pro Ala Asn Phe Cys Met
65 70 75 80

Phe Cys Lys Asp Gly Val Ser Gln Cys Cys Pro Gly Trp Ser Gln Thr
85 90 95

Pro Gly Leu Arg Arg Ser Thr Cys Leu Ser Leu Pro Glu Cys Trp Asp
100 105 110

Tyr Asn Cys Glu Pro Pro Arg Pro Ala Gly Arg Val Asn Ile Phe Tyr
115 120 125

Ile Leu Gln Ala His Leu His Phe His Pro Thr Leu Pro Leu Leu Leu
130 135 140

Pro Phe Tyr Ile Pro Phe Leu Tyr Arg Ser Leu Ile Leu Gln
145 150 155

<210> 281
<211> 43
<212> PRT
<213> Homo sapiens

<400> 281
Met Pro Leu Gly Pro Val Gln Val Tyr Leu Ser Leu Ile Ser Glu Ser
1 5 10 15

Cys Ser Ser Cys Leu Thr Leu Pro His Gly Ser Ser Val His Leu Ser
20 25 30

Ile Thr Val Leu Asn Pro Phe Ser Ile Ser Val
35 40

<210> 282
<211> 61
<212> PRT
<213> Homo sapiens

<400> 282
Met Lys Lys Leu Thr Leu Pro Met Gly Leu Pro Pro Phe Leu Pro Leu
1 5 10 15

Phe Ser Leu Trp Tyr Pro Ser Arg Val Phe Pro Ser Pro Leu Gln Ser
20 25 30

Pro Ile Ser His Leu Phe Phe Phe Ser Pro Ser Ser Phe Ser Tyr Cys
35 40 45

Val Leu Pro Ala Thr Ser His Arg Leu Val Val Tyr Lys
50 55 60

<210> 283
<211> 207
<212> PRT
<213> Homo sapiens

<400> 283
Met Gln Lys Met Leu Pro Glu Ile Asp Gln Asn Lys Asp Arg Met Leu
1 5 10 15

Glu Ile Leu Glu Gly Lys Gly Leu Ser Phe Leu Phe Pro Leu Leu Lys
20 25 30

Leu Glu Lys Glu Leu Leu Lys Gln Ile Lys Leu Asp Pro Ser Pro Gln
35 40 45

Thr Ile Tyr Lys Trp Ile Lys Asp Asn Ile Ser Pro Lys Leu His Val
50 55 60

Asp Lys Gly Phe Val Asn Ile Leu Met Thr Ser Phe Leu Gln Tyr Ile
65 70 75 80

Ser Ser Glu Val Asn Pro Pro Ser Asp Glu Thr Asp Ser Ser Ser Ala
85 90 95

Pro Ser Lys Glu Gln Leu Glu Gln Glu Lys Gln Leu Leu Leu Ser Phe
100 105 110

Lys Pro Val Met Gln Lys Phe Leu His Asp His Val Asp Leu Gln Val
115 120 125

Ser Ala Leu Tyr Ala Leu Gln Val His Cys Tyr Asn Ser Asn Phe Pro
130 135 140

Lys Gly Met Leu Leu Arg Phe Phe Val His Phe Tyr Asp Met Glu Ile
145 150 155 160

Ile Glu Glu Glu Ala Phe Leu Ala Trp Lys Glu Asp Ile Thr Gln Glu
165 170 175

Phe Pro Gly Lys Gly Lys Ala Leu Phe Gln Val Asn Gln Trp Leu Thr
180 185 190

Trp Leu Glu Thr Ala Glu Glu Glu Glu Ser Glu Glu Glu Ala Asp
195 200 205

<210> 284
<211> 105
<212> PRT
<213> Homo sapiens

<220>
<221> UNSURE
<222> (80)
<223> "Xaa" may be "Asp" or "Glu".

<400> 284

Phe Ser Cys Leu Ser Phe Leu Ser Ser Trp Asp Tyr Arg His Ala Pro
1 5 10 15

Pro Cys Leu Ala Asn Phe Ala Phe Leu Val Glu Thr Gly Phe His His
20 25 30

Val Gly Gln Ala Gly Leu Lys Leu Pro Thr Ser Gly Asp Leu Pro Thr
35 40 45

Ser Ala Ser Gln Ser Ala Gly Ile Thr Gly Met Ser Tyr Arg Ala Trp
50 55 60

Pro Val Tyr Phe Trp Arg Gln Ser Leu Ala Leu Leu Pro Arg Leu Xaa
65 70 75 80

Gly Ser Gly Ala Thr Leu Asn Ser Ala Ser Arg Val Gln Ala Ile Leu
85 90 95

Val Arg His Leu Pro Ser Ser Trp Gly
100 105

<210> 285
<211> 91
<212> PRT
<213> Homo sapiens

<400> 285
Leu Thr Ala Val Phe Phe Ser Phe Ile His Phe Ala Phe Phe Leu Tyr
1 5 10 15

Phe Arg Phe Asn Ser Thr Phe Lys Lys Ser Tyr Leu Tyr Ile Cys Ile
20 25 30

Phe Ile Phe Ile Phe Gln Asp Leu Ile Cys Leu Phe Phe Ile Met Gly
35 40 45

Tyr Tyr Cys Ser Met Val Gln Asn Leu Leu Phe Phe Pro Lys Leu Leu
50 55 60

Val Ile Phe Lys Ile Phe Val Asn Phe Leu Pro Leu Ala Ser Ser Gln
65 70 75 80

Val Pro Ala Phe Ser Gln Ser Ala Gly Phe Pro
85 90

<210> 286

<211> 75
<212> PRT
<213> Homo sapiens

<400> 286
Pro Lys Ser Leu Pro Gly His Pro Leu Ala Tyr Ser Leu Thr Gly His
1 5 10 15

Ala Pro Ala Val His Thr Gly Ser Tyr Gln Ser Ser Ser Trp Ala Pro
20 25 30

Phe Gln Thr Ser Glu Glu Ser Phe Gln His Glu Glu Gly Val Gln Asn
35 40 45

Lys Gln Arg Glu Arg Glu Arg Glu Arg Glu Arg Glu Arg Glu Arg Glu
50 55 60

Lys Arg Asn Ile Asn Asn Ala Gly Ser Lys Arg
65 70 75

<210> 287
<211> 83
<212> PRT
<213> Homo sapiens

<400> 287
Met Tyr Cys Val Phe Asn Arg Asn Glu Asp Ala Cys Arg Tyr Gly Ser
1 5 10 15

Ala Ile Gly Val Leu Ala Ser Leu Ala Tyr Gln Arg Tyr Lys Ala Gly
20 25 30

Val Asp Asp Phe Ile Gln Asn Tyr Val Asp Pro Thr Pro Asp Pro Asn
35 40 45

Thr Ala Tyr Ala Ser Tyr Pro Gly Ala Ser Val Asp Asn Tyr Gln Gln
50 55 60

Pro Pro Phe Thr Gln Asn Ala Glu Thr Thr Glu Gly Tyr Gln Pro Pro
65 70 75 80

Pro Val Tyr

<210> 288
<211> 117

<212> PRT
<213> Homo sapiens

<400> 288
Met Val Arg Ala Thr Ala Met Pro Thr Ser Leu Ser Arg Cys Thr Ala
1               5                   10                  15

Cys Ser Thr Ala Thr Arg Met Pro Ala Ala Met Ala Val Pro Ser Gly
            20                  25                  30

Cys Trp Pro Pro Trp Pro Thr Ser Ala Thr Arg Leu Ala Trp Thr Thr
        35                  40                  45

Ser Ser Arg Ile Thr Leu Thr Pro Leu Arg Thr Pro Thr Leu Pro Thr
    50                  55                  60

Pro Pro Thr Gln Val His Leu Trp Thr Thr Thr Asn Ser His Pro Ser
65                  70                  75                  80

Pro Arg Thr Arg Arg Pro Pro Arg Ala Thr Ser Arg Pro Leu Cys Thr
                85                  90                  95

Glu Arg Arg Leu Ala Trp Glu Gly Gly Gln Arg Gly Pro Ser Pro Leu
            100                 105                 110

Pro Trp Thr Phe Pro
        115

<210> 289
<211> 1280
<212> DNA
<213> Homo sapiens

<400> 289
gtcagccgca tcttcttttg cgtcgccagc cgagccacat cgctcagaca ccatggggaa 60
ggtgaaggtc ggagtcaacg gatttggtcg tattgggcgc ctggtcacca gggctgcttt 120
taactctggt aaagtggata ttgttgccat caatgacccc ttcattgacc tcaactacat 180
ggtttacatg ttccaatatg attccaccca tggcaaattc catggcaccg tcaaggctga 240
gaacgggaag cttgtcatca atggaaatcc catcaccatc ttccaggagc gagatccctc 300
caaaatcaag tggggcgatg ctggcgctga gtacgtcgtg gagtccactg gcgtcttcac 360
caccatggag aaggctgggg ctcatttgca gggggagcc aaaagggtca tcatctctgc 420
cccctctgct gatgccccca tgttcgtcat gggtgtgaac catgagaagt atgacaacag 480
cctcaagatc atcagcaatg cctcctgcac caccaactgc ttagcacccc tggccaaggt 540
catccatgac aactttggta tcgtggaagg actcatgacc acagtccatg ccatcactgc 600
cacccagaag actgtggatg gcccctccgg gaaactgtgg cgtgatggcc gcggggctct 660
ccagaacatc atccctgcct ctactggcgc tgccaaggct gtgggcaagg tcatccctga 720
gctgaacggg aagctcactg gcatggcctt ccgtgtcccc actgccaacg tgtcagtggt 780

ggacctgacc tgccgtctag aaaaacctgc caaatatgat gacatcaaga aggtggtgaa 840
gcaggcgtcg gagggccccc tcaagggcat cctgggctac actgagcacc aggtggtctc 900
ctctgacttc aacagcgaca cccactcctc cacctttgac gctggggctg gcattgccct 960
caacgaccac tttgtcaagc tcatttcctg gtatgacaac gaatttggct acagcaacag 1020
ggtggtggac ctcatggccc acatggcctc caaggagtaa gacccctgga ccaccagccc 1080
cagcaagagc acaagaggaa gagagagacc ctcactgctg gggagtccct gccacactca 1140
gtcccccacc acactgaatc tcccctcctc acagttgcca tgtagacccc ttgaagaggg 1200
gaggggccta gggagccgca ccttgtcatg taccatcaat aaagtaccct gtgctcaacc 1260
aaaaaaaaaa aaaaaaaaaa 1280

<210> 290
<211> 2978
<212> DNA
<213> Homo sapiens

<400> 290
gccgtgagaa cacgctgtgt ggctgaaaag tgaaggcaag agctgatttg gcctctgtgc 60
tcccctccgc aaggggatcg ttttctccag aagagctgga tattctttcg cccagttatg 120
gcagacaagt taacgagaat tgctattgtc aaccatgaca aatgtaaacc taagaaatgt 180
cgacaggaat gcaaaaagag ttgtcctgta gttcgaatgg gaaaattatg catagaggtt 240
acaccccaga gcaaaatagc atggatttcc gaaactcttt gtattggttg tggtatctgt 300
attaagaaat gcccctttgg cgccttatca attgtcaatc taccaagcaa cttggaaaaa 360
gaaaccacac atcgatattg tgccaatgcc ttcaaacttc acaggttgcc tatccctcgt 420
ccaggtgaag ttttgggatt agttggaact aatggtattg gaaagtcaac tgctttaaaa 480
atttagcag gaaaacaaaa gccaaacctt ggaaagtacg atgatcctcc tgactggcag 540
gagattttga cttatttccg tggatctgaa ttacaaaatt actttacaaa gattctagaa 600
gatgacctaa aagccatcat caaacctcaa tatgtagacc agattcctaa ggctgcaaag 660
gggacagtgg gatctatttt ggaccgaaaa gatgaaacaa agacacaggc aattgtatgt 720
cagcagcttg atttaaccca cctaaaagaa cgaaatgttg aagatctttc aggaggagag 780
ttgcagagat ttgcttgtgc tgtcgtttgc atacagaaag ctgatatttt catgtttgat 840
gagccttcta gttacctaga tgtcaagcag cgtttaaagg ctgctattac tatacgatct 900
ctaataaatc cagatagata tatcattgtg gtggaacatg atctaagtgt attagactat 960
ctctccgact tcatctgctg tttatatggt gtaccaagcg cctatggagt tgtcactatg 1020
ccttttagtg taagagaagg cataaacatt tttttggatg gctatgttcc aacagaaaac 1080
ttgagattca gagatgcatc acttgttttt aaagtggctg agacagcaaa tgaagaagaa 1140
gttaaaaaga tgtgtatgta taaatatcca ggaatgaaga aaaaaatggg agaatttgag 1200
ctagcaattg tagctggaga gtttacagat tctgaaatta tggtgatgct gggggaaaat 1260
ggaacgggta aaacgacatt tatcagaatg cttgctggaa gacttaaacc tgatgaagga 1320
ggagaagtac cagttctaaa tgtcagttat aagccacaga aaattagtcc caaatcaact 1380
ggaagtgttc gccagttact acatgaaaag ataagagatg cttatactca cccacaattt 1440
gtgaccgatg taatgaagcc tctgcaaatt gaaaacatca ttgatcaaga ggtgcagaca 1500
ttatctggtg gtgaactaca gcgagtagct ttagcccttt gcttgggcaa acctgctgat 1560
gtctatttaa ttgatgaacc atctgcatat ttggattctg agcaaagact gatggcagct 1620
cgagttgtca aacgtttcat actccatgca aaaaagacag cctttgttgt ggaacatgac 1680
ttcatcatgg ccacctatct agcggatcgc gtcatcgttt ttgatggtgt tccatctaag 1740
aacacagttg caaacagtcc tcaaaccctt ttggctggca tgaataaatt tttgtctcag 1800
cttgaaatta cattcagaag agatccaaac aactataggc cacgaataaa caaacttaat 1860

tcaattaagg atgtagaaca aaagaagagt ggaaactact ttttcttgga tgattagact 1920
gactctgaga atattgataa gccatttatt aaaaggagta tttactagaa ttttttgtca 1980
tataaaactt gaatcaggat tttatgcccc acatactctg gaacttgaag tataatatac 2040
ttaatataac ataaaaagcc agttgggttc taaattgtag ttgaaacaca gaaaatgcca 2100
cttttctgtt cctgaagagg ctcttttgtg cataatattc taaaatgaag acatttcaag 2160
ctatacaaat tacttccaag ttttcatgat gtatgggaag attttcagta ggtgtattat 2220
attcacggta ccaaatgctg accagtgttg ctccattttt taaatcttga aaagggtttc 2280
tgtacttacc tggtttgcca agtatgccag tgtaatgaaa ctgcccttat tttaaaagcc 2340
agtcaaagat tccactgatt gacatttgat aaataaacat caggattatg tttattgttt 2400
gttttcagtc tttgcactat attaccagta tatggtttcc gaggaagatt atctactgca 2460
aaacaccact gttggaaaaa taggtatttt taaattgttt ttaatctttt ttggtgcttt 2520
taaacatgtt tagcaaaaac caattcagtt ccattccccg caaaaaaccc ctaactttac 2580
tctgaacttt ttttgttttt gcattccatg aggttctgta ttcagtcatt ctctaggtaa 2640
tgtcattttt gtacacatat atttatataa tcactgattg agatttagga aaaagcattt 2700
ctaaagaata tttgcttccc ttagaactac agactcgaaa tctttaaaga tggtgcctaa 2760
gcatctatgt atttttttta agttccacag atttttctgt tgggcagcca aggattataa 2820
accacttccc taaaggcaac attaatgcaa aagcccccac cccatggctt ccatcttttg 2880
catcaccacc actcctgaac ccccatttct gatttgtcag aatttttttt taacaaaact 2940
aaaaatgaaa aaaaaaaaaa aaaaaaaaaa aaaaaaa 2978

<210> 291
<211> 1218
<212> DNA
<213> Homo sapiens

<400> 291
gaagttactg cagccgcggt gttgtgctgt ggggaaggga gaaggatttg taaaccccgg 60
agcgaggttc tgcttacccg aggccgctgc tgtgcggaga cccccgggtg aagccaccgt 120
catcatgtct gaccaggagg caaaaccttc aactgaggac ttgggggata agaaggaagg 180
tgaatatatt aaactcaaag tcattggaca ggatagcagt gagattcact tcaaagtgaa 240
aatgacaaca catctcaaga aactcaaaga atcatactgt caaagacagg gtgttccaat 300
gaattcactc aggtttctct ttgagggtca gagaattgct gataatcata ctccaaaaga 360
actgggaatg gaggaagaag atgtgattga agtttatcag gaacaaacgg ggggtcattc 420
aacagtttag atattctttt tatttttttt cttttccctc aatccttttt tatttttaaa 480
aatagttctt ttgtaatgtg gtgttcaaaa cggaattgaa aactggcacc ccatctcttt 540
gaaacatctg gtaatttgaa ttctagtgct cattattcat tattgtttgt tttcattgtg 600
ctgatttttg gtgatcaagc ctcagtcccc ttcatattac cctctccttt ttaaaaatta 660
cgtgtgcaca gagaggtcac ctttttcagg acattgcatt ttcaggcttg tggtgataaa 720
taagatcgac caatgcaagt gttcataatg actttccaat tggccctgat gttctagcat 780
gtgattactt cactcctgga ctgtgacttt cagtgggaga tggaagtttt tcagagaact 840
gaactgtgga aaaatgacct ttccttaact tgaagctact tttaaaattt gagggtctgg 900
accaaaagaa gaggaatatc aggttgaagt caagatgaca gataaggtga gagtaatgac 960
taactccaaa gatggcttca ctgaagaaaa ggcattttaa gattttttaa aaatcttgtc 1020
agaagatccc agaaaagttc taattttcat tagcaattaa taaagctata catgcagaaa 1080
tgaatacaac agaacactgc tctttttgat tttatttgta ctttttggcc tgggatatgg 1140
gttttaaatg gacattgtct gtaccagctt cattaaaata aacaatattt gtaaaatca 1200
aaaaaaaaaa aaaaaaa 1218

<210> 292
<211> 825
<212> DNA
<213> Homo sapiens

<400> 292
cgcctcggag gcgttcagct gcttcaagat gaagctgaac atctccttcc cagccactgg 60
ctgccagaaa ctcattgaag tggacgatga acgcaaactt cgtactttct atgagaagcg 120
tatggccaca gaagttgctg ctgacgctct gggtgaagaa tggaagggtt atgtggtccg 180
aatcagtggt gggaacgaca aacaaggttt ccccatgaag cagggtgtct tgacccatgg 240
ccgtgtccgc ctgctactga gtaaggggca ttcctgttac agaccaagga gaactggaga 300
aagaaagaga aaatcagttc gtggttgcat tgtggatgca aatctgagcg ttctcaactt 360
ggttattgta aaaaaaggag agaaggatat tcctggactg actgatacta cagtgcctcg 420
ccgcctgggc cccaaaagag ctagcagaat ccgcaaactt ttcaatctct ctaaagaaga 480
tgatgtccgc cagtatgttg taagaaagcc cttaaataaa gaaggtaaga aacctaggac 540
caaagcaccc aagattcagc gtcttgttac tccacgtgtc ctgcagcaca aacggcggcg 600
tattgctctg aagaagcagc gtaccaagaa aaataaagaa gaggctgcag aatatgctaa 660
acttttggcc aagagaatga aggaggctaa ggagaagcgc caggaacaaa ttgcgaagag 720
acgcagactt tcctctctgc gagcttctac ttctaagtct gaatccagtc agaaataaga 780
ttttttgagt aacaaataaa taagatcaga ctctgaaaaa aaaaa 825

<210> 293
<211> 1978
<212> DNA
<213> Homo sapiens

<400> 293
ggccggagcg gcgcggcagc ggcaggaccg ccgtggcgcc tagagtagcg acccgggggg 60
agcgcggggc gacgctggct gcagggaccc ggtgacagcg tgagaggtac taggttttga 120
caagcttgca tcatgcgtga gtataagcta gtcgttcttg gctcaggagg cgttggaaag 180
tctgctttga ctgtacaatt tgttcaagga atttttgtag aaaaatacga tcctacgata 240
gaagattctt atagaaagca agttgaagta gatgcacaac agtgtatgct tgaaatcttg 300
gatactgcag gaacggagca atttacagca atgagggatt tatacatgaa aaatggacaa 360
ggatttgcat tagtttattc catcacagca cagtccacat ttaacgattt acaagacctg 420
agagaacaga ttcttcgagt taaagacact gatgatgttc caatgattct tgttggtaat 480
aagtgtgact tggaagatga aagagttgta gggaaggaac aaggtcaaaa tctagcaaga 540
caatggaaca actgtgcatt cttagaatct tctgcaaaat caaaaataaa tgttaatgag 600
atcttttatg acctagtgcg gcaaattaac agaaaaactc cagtgcctgg gaaggctcgc 660
aaaaagtcat catgtcagct gctttaatat actaaatgca ttgtagctct gagccaggtc 720
tgaagaactg ttgcccaatt caacagtgcc agcattccaa ctttgttaaa cctaccaaca 780
tcttaaatgg actttcctgt ggtggtaccc tttaagaggc ggatgaaagc tactatatca 840
gtttgcacat tctaatcact ttccagtatc acaagagaga tttttactta tataatagtc 900
ctagagtttg cagctggtaa aaccagaggc tacatccagt attactgcta agagacattc 960
ttcatccacc aatgttgtac atgtatgaaa atggtgtact gtatacttta acatgcccca 1020
tactttgtat tggagagtac aataatgtaa atcctaaaag caccactatt ttagcataat 1080

aaaagaaagt ccaaagagct cctatataga ctactccaga taacttcgct tctttgatac 1140
ttgtagctta ttgtaatttt ttttaagaaa ttcaaggtca ttattattgt acaaaataag 1200
cgctttgatt aacacagcta tatagttttt ttaattttta aaaaacctgt ggagacggtg 1260
atcttgtctt taaaacatga tagtcctttc agtataatgt cttagattaa agacgttgcc 1320
tttaatatct gttgggaagg aaatgtccag acttttcaaa tctcttatta tatgtttcct 1380
tttttgttt acatagggaa caatgtttat agtcgtgtgt acagtggggg tctacaacaa 1440
gaagtgtata ttttcaaaca attttttaat gatttaacaa tttttgtaaa tcattttcag 1500
gcttctgcag ctgtagattc tcactgtgaa tcccttgctt gctcatgcat aagtgtattt 1560
gcaataccaa atatacaggt ttagtatttt tgcctgttag tgattgtttc acatgtgtaa 1620
cgttttggtt gagatgttaa atggtggacg agtactgtgg atgtgaatgt gggaagtaat 1680
tttaatcata tgtaattggt cacaaggcct aatttgcagt aactattgct gttttattta 1740
acaatgcctt gttgctttgt atgcattaat gtttggatgt aaagattgtg tgtctatcca 1800
acagggagcc acagtattta aattgaccaa cctaatgtta caactacttt gaggtggcca 1860
aatgtaaact aaaagcctta attaaagtgg tgcaattttg tataacttag catcagtagt 1920
tcaataaatt tggattgcca tgcaagggct tgccttataa aaaaaaaaaa aaaaaaa 1978

<210> 294
<211> 895
<212> DNA
<213> Homo sapiens

<400> 294
gccatcttgc gtccccgcgt gtgtgcgcct aatctcaggt ggtccacccg agaccccttg 60
agcaccaacc ctagtccccc gcgcggcccc ttattcgctc cgacaagatg aaagaaacaa 120
tcatgaacca ggaaaaactc gccaaactgc aggcacaagt gcgcattggt gggaaaggaa 180
ctgctcgcag aaagaagaag gtggttcata gaacagccac agcagatgac aaaaaacttc 240
agttctcctt aaagaagtta ggggtaaaca atatctctgg tattgaagag gtgaatatgt 300
ttacaaacca aggaacagtg atccacttta acaaccctaa agttcaggca tctctggcag 360
cgaacacttt caccattaca ggccatgctg agacaaagca gctgacagaa atgctaccca 420
gcatcttaaa ccagcttggt gcggatagtc tgactagttt aaggagactg gccgaagctc 480
tgcccaaaca atctgtggat ggaaaagcac cacttgctac tggagaggat gatgatgatg 540
aagttccaga tcttgtggag aattttgatg aggcttccaa gaatgaggca aactgaattg 600
agtcaacttc tgaagataaa acctgaagaa gttactggga gctgctattt tatattatga 660
ctgcttttta agaaattttt gtttatggat ctgataaaat ctagatctct aatattttta 720
agcccaagcc ccttggacac tgcagctctt ttcagttttt gcttatacac aattcattct 780
ttgcagctaa ttaagccgaa gaagcctggg aatcaagttt gaaacaaaga ttaataaagt 840
tctttgccta gtaaaaaaaa aaaaaaaaaa aaaaataaaa aaaaaaaaaa aaaaa 895

<210> 295
<211> 1358
<212> DNA
<213> Homo sapiens

<400> 295
gttccaaccc agggggaaaa atgcggcctt tgactgaaga ggagacccgt gtcatgtttg 60
agaagatagc gaaatacatt ggggagaatc ttcaactgct ggtggaccgg cccgatggca 120

cctactgttt ccgtctgcac aacgaccggg tgtactatgt gagtgagaag attatgaagc 180
tggccgccaa tatttccggg gacaagctgg tgtcgctggg gacctgcttt ggaaaattca 240
ctaaaaccca caagtttcgg ttgcacgtca cagctctgga ttaccttgca ccttatgcca 300
agtataaagt ttggataaag cctggtgcag agcagtcctt cctgtatggg aaccatgtgt 360
tgaaatctgg tctgggtcga atcactgaaa atacttctca gtaccagggc gtggtggtgt 420
actccatggc agacatccct ttgggttttg gggtggcagc caaatctaca caagactgca 480
gaaaagtaga ccccatggcg attgtggtat ttcatcaagc agacattggg gaatatgtgc 540
ggcatgaaga gacgttgact taaaacgaag ccattccaag gacagacggc tgtatggaaa 600
ggccgagctt tgtttcctgt gtttgtgtgg actccaccat catgttgaat tttgtcaaca 660
ctctggcctc ttcagggact tcttatttac tgtactctct atcactgaca aatgcaggct 720
ggattcttat tatatacaga gatggctcaa aaatggggtt tcagatcttt gtgacgaaat 780
agaatactgt ttcatatttg aatcagaggg cttcttgttc tgagaaatag gttcaaaatc 840
attggaacca ggaacaagaa tagcttattg ttatctgtga taacactgtt ttctaaacac 900
aaggattttc ttttttatta atatgcaaca tagacattgc cataacagaa taataaacca 960
catgtggggt tttaaaaatg aaatttggct aataggagca attcagctat ttttctatac 1020
agtaattggt gtgtggtata gaagaaaaac gggttcaaac ccccacttctg ccacctacca 1080
gctatatggc cttgaatgag tcattcagct ttaataaggt tcattttctt ctgtttaaaa 1140
agacacaaaa cttgaaaatc agctttggcc atctacctga gaattagaaa gtctgatttt 1200
tggaattaga aatcatgatt gtaggctggg cacagtggct cgcgcctgta atcccagcac 1260
tttgggaggc caaggcggac ggatcacttg aggttaggag tttgagacca gcctggccaa 1320
catggtgaaa ccccatctct actaaaaaaa aaaaaaaa 1358

<210> 296
<211> 2033
<212> DNA
<213> Homo sapiens

<400> 296
ggtaaagatg gcagctacca tgttccgggc tacgctgcgg ggatggagaa ccggtgtcca 60
gcggggctgc gggctacggc tgttgagcca gacccagggc cctccagatt accccaggtt 120
tgtggagtct gtggatgaat atcagtttgt ggagcgcctg ttaccggcta ccaggatccc 180
agatccccca aagcatgaac attatcctac ccctagtggc tggcagcctc ccagagaccc 240
cccacccaac ctgccttact ttgtacgacg ctctcggatg cacaacatcc ccgtctacaa 300
ggacatcacg catggcaacc ggcagatgac tgtgatccgg aaagtggaag ggacatctg 360
ggccctgcag aaagacgtgg aagattttct gagcccgctg ctggggaaga cacctgtcac 420
ccaggtcaat gaggtgacag gtaccctacg gatcaagggc tactttgacc aggagcttaa 480
agcctggctc ttggagaaag gcttctgagg cccagcccga gcagcctgct tgtcagcatg 540
ccctgtggat caagtctagg gggcctcagg aggagggagg tgggtgttgg agcccctgag 600
acaggggata cagaaactag ggctaaagga ctttggggtc aggccttgct tgcataaagg 660
agaaaacaac tctatgtaca tgctggggga gagtgcctaa tgtgggagac caaataggga 720
tcaccaggct aatggggggc gtcagcagct ttctctccct cctatcttgg cctgttcttt 780
tttgtttttt gagacggagt ctcactctgt tgcccagggt ggagtgcagt ggcatgatct 840
tggctcactg caacttccac ctctgggatc aagggattct cctgcctcag cctcttgagt 900
agctgggatt acaggcgccc accaccacag cctgctaatt tttgtatttt tagtagagat 960
ggggtttcac catgttggcc aggctggtct caaactcctg actcgaagtg atccgcccac 1020
cttggcctcc caaagcgttg ggattatagg catgagccat gtgcctggtc caccttggcc 1080
tgttttgttt ttctttcctt gggctcagca attcaaattc tagttgttat ttggtggaag 1140

cagtagccca accccagttt aggggaaggt agcacagggc agagccactg ggcactttgt 1200
ttccttggcc ctccgaagct cactgttgca aataccccca agcctttgct ctaggccaga 1260
tcttgtttgg tgcaggtgat ggagaacaca gatgactcgg gcatgggtct tggagatctt 1320
ctgttcaaag tacagtgctg gcactggggc acagagtgcc cacgttagcc ccgggctctg 1380
atagagaggt aggaggcacg ttcttggtca ctgttccatt gcagaccaga cttgctggcc 1440
tgaccacaag ggagtggctg ggaactcaca gccagcatag ggacatcccc ctgcagcctt 1500
ctgacctgca atcaaggctg gggaggggtt tgcaggcagg aatatgctga cctttcaccc 1560
tgccatccca tcccaacccc agctcactag ccttcatata tgccttatac ttggagtcac 1620
aggggccaaa ggcctgagac cccaccctgc ccccaaactg gctaagacag ctttcagttc 1680
ctgactcccc aacttggtct ctgccctgaa gcagggcact gaactctggg ctgcttctct 1740
gtgtgtaaaa tgggcacatc ttcctaatct gttaatggtc agtggtgtcc ccaaggatag 1800
tgctggcttc catggaaacc ctcactcctg gagattccat tccattttca agtgtacagc 1860
cacagcaagg agcccgacac tgatttgatc gattctgtga cacaaacccc accaattgtt 1920
aatgcaagtt tttatttggc tgtatataca atttaagcta ttaaaatttg tacaatattt 1980
acaaattaaa taatcatctg aaactgtcaa aaaaaaaaaa aaaaaaaaaa aaa 2033

<210> 297
<211> 1059
<212> DNA
<213> Homo sapiens

<400> 297
gttttctgtc actggacgcc aaggagtttt cggtggctca gctgggtaac cggggatcac 60
catggcggcc tcattggtgg ggaagaagat cgtgtttgta acggggaacg ccaagaagct 120
ggaggaggtc gttcagattc taggagataa gtttccatgc actttggtgg cacagaaaat 180
tgacctgccg gagtaccaag gggagccgga tgagatttcc atacagaaat gtcaggaggc 240
agttcgccag gtacaggggc ccgtgctggt tgaggacact tgtctgtgct tcaatgccct 300
tggagggctc cccggcccct acataaagtg gtttctggag aagttaaagc ctgaaggtct 360
ccaccagctc ctggccgggt tcgaggacaa gtcagcctat gcgctctgca cgtttgcact 420
cagcaccggg gacccaagcc agcccgtgcg cctgttcagg ggccggacct cgggccggat 480
cgtggcaccc agaggctgcc aggactttgg ctgggacccc tgctttcagc ctgatggata 540
tgagcagacg tacgcagaga tgcctaaggc ggagaagaac gctgtctccc atcgcttccg 600
ggccctgctg gagctgcagg aatactttgg cagtttggca gcttgacttc tgcagctgga 660
ggaggcccct caggccgggg atctggggag ggctagccca aaacctcccg catcgggcag 720
gcacccccctg aagtacttcc ttcagggttt cccctttgtg agggtgtcga gtagcctcac 780
cggcctgtct ggaggagcag ctggctctgc tctgagaaac tctggcaagt ggacgccatt 840
ctcttgccct taggattcac tgctctctcc tacagccgcc aggcctgggg tcctgaaagg 900
accttgggtg gtaaagctgt acttggtggg agtgagggcg tggggaggaa ccatgcaaat 960
cgccttccat ggtttttaaa tgcagtaaat aacatttctg gatgagactt gtttccaaaa 1020
taaaccagct atatctgttt tgaaaaaaaa aaaaaaaaa 1059

<210> 298
<211> 1769
<212> DNA
<213> Homo sapiens

<400> 298
gggttgggct gtgacgctgc tgctggggtc agaatgtcat acccaggcta tcccccaaca 60
ggctacccac ctttccctgg atatcctcct gcaggtcagg agtcatcttt tccccccttct 120
ggtcagtatc cttatcctag tggctttcct ccaatgggag gaggtgccta cccacaagtg 180
ccaagtagtg gctacccagg agctggaggc taccctgcgc ctggaggtta tccagcccct 240
ggaggctatc ctggtgcccc acagccaggg ggagctccat cctatcccgg agttcctcca 300
ggccaaggat ttggagtccc accaggtgga gcaggctttt ctgggtatcc acagccacct 360
tcacagtctt atggaggtgg tccagcacag gttccactac ctggtggctt tcctggagga 420
cagatgcctt ctcagtatcc tggaggacaa cctacttacc ctagtcagcc tgccacagtg 480
actcaggtca ctcaaggaac tatccgacca gctgccaact tcgatgctat aagagatgca 540
gaaattcttc gtaaggcaat gaagggtttt gggacagatg agcaggcaat tgtggatgtg 600
gtggccaacc gttccaatga tcagaggcaa aaaattaaag cagcatttaa gacctcctat 660
ggcaaggatt taatcaaaga tctcaaatca gagttaagtg gaaatatgga agaactgatc 720
ctggccctct tcatgcctcc tacgtattac gatgcctgga gcttacggaa agcaatgcag 780
ggagcaggaa ctcaggaacg tgtattgatt gagattttgt gcacaagaac aaatcaggaa 840
atccgagaaa ttgtcagatg ttatcagtca gaatttggac gagaccttga aaaggacatt 900
aggtcagata catcaggaca ttttgaacgt ttacttgtgt ccatgtgcca gggaaatcgt 960
gatgagaacc agagtataaa ccaccaaatg gctcaggaag atgctcagcg tctctatcaa 1020
gctggtgagg ggagactagg gaccgatgaa tcttgcttta acatgatcct tgccacaaga 1080
agctttcctc agctgagagc taccatggag gcttattcta ggatggctaa tcgagacttg 1140
ttaagcagtg tgagccgtga gttttccgga tatgtagaaa gtggtttgaa gaccatcttg 1200
cagtgtgccc tgaaccgccc tgccttcttt gctgagaggc tctactatgc tatgaaaggt 1260
gctggcacag atgactccac cctggtccgg attgtggtca ctcgaagtga gattgacctt 1320
gtacaaataa aacagatgtt cgctcagatg tatcagaaga ctctgggcac aatgattgca 1380
ggtgacacga gtggagatta ccgaagactt cttctggcta ttgtgggcca gtaggaggga 1440
ttttttttt tttaatgaaa aaaaaatttc tattcatagc ttatccttca gagcaatgac 1500
ctgcatgcag caatatcaaa catcagctaa ccgaaagagc tttctgtcaa ggaccgtatc 1560
agggtaatgt gcttggtttg cacatgttgt tattgcctta attctaattt tattttgttc 1620
tctacataca atcaatgtaa agccatatca caatgataca gtaatattgc aatgtttgta 1680
aaccttcatt cttactagtt tcattctaat caagatgtca aattgaataa aaatcacagc 1740
aatctttgaa aaaaaaaaa aaaaaaaaa 1769

<210> 299
<211> 463
<212> DNA
<213> Homo sapiens

<400> 299
cgcgccgcca caatggtgcg catgaatgtc ctggcagatg ctctcaagag tatcaacaat 60
gccgaaaaga gaggcaaacg ccaggtgctt attaggccgt gctccaaagt catcgtccgg 120
tttctcactg tgatgatgaa gcatggttac attggcgaat ttgaaatcat tgatgaccac 180
agagctggga aaattgttgt gaacctcaca ggcaggctaa acaagtgtgg ggtgatcagc 240
cccagatttg acgtgcaact caaagacctg gaaaaatggc agaataatct gcttccatcc 300
cgccagtttg gtttcattgt actgacaacc tcagctggca tcatggacca tgaagaagca 360
agacgaaaac acacaggagg gaaaatcctg ggattctttt tctagggatg taatacatat 420
atttacaaat aaaatgcctc atggacaaaa aaaaaaaaa aaa 463

<210> 300
<211> 703
<212> DNA
<213> Homo sapiens

<400> 300
gcgagaatga agactattct cagcaatcag actgtcgaca ttccagaaaa tgtcgacatt 60
actctgaagg gacgcacagt tatcgtgaag ggccccagag gaaccctgcg gagggacttc 120
aatcacatca atgtagaact cagccttctt ggaaagaaaa aaaagaggct ccgggttgac 180
aaatggtggg gtaacagaaa ggaactggct accgttcgga ctatttgtag tcatgtacag 240
aacatgatca agggtgttac actgggcttc cgttacaaga tgaggtctgt gtatgctcac 300
ttccccatca acgttgttat ccaggagaat gggtctcttg ttgaaatccg aaatttcttg 360
ggtgaaaaat acatccgcag ggttcggatg agaccaggtg ttgcttgttc agtatctcaa 420
gcccagaaag atgaattaat ccttgaagga aatgacattg agcttgtttc aaattcagcg 480
gctttgattc agcaagccac aacagttaaa aacaaggata tcaggaaatt tttggatggt 540
atctatgtct ctgaaaaagg aactgttcag caggctgatg aataagatct aagagttacc 600
tggctacaga aagaagatgc cagatgacac ttaagaccta cttgtgatat ttaaatgatg 660
caataaaaga cctattgatt tgggaaaaaa aaaaaaaaaa aaa 703

<210> 301
<211> 887
<212> DNA
<213> Homo sapiens

<400> 301
gcgggggcca ggggtgggcg cgggagacgg ggcggtaccc ggggtgggag agggacgggg 60
agggggcgag gggcggaggc cgagggggca gggggggtgg gcggcggcca gtgtttacag 120
atgagcttta actgccgcct caggcgtgga gacggagacc ccgcagcccg gcggcgcctc 180
agcccttcaa cgacagtatt gagtggtcag gttacaataa accggagaga aaaggtccgc 240
ttgcactttt tttagttttc ttatttttag acaccccctcc cctccagggt gatctttaaa 300
aaagcaaaac aaaaaacacg acttttccag ccgctcagcc gtttttttcct ttcgtccgaa 360
gccgttttct gatttgactt ttctcgccgg ccggtctcag gccgcacaga cgttccagag 420
gaggagggtg acatttttac tcccttttttg gggctaacca tttatgcttt tgtacatcaa 480
ccgtgcgcgg ccggaggggg gcagggggc gggggccgag gggcgttcca atcaaatttc 540
taactttctg ttaattatta atccccctttt tactgcggtt tctgttgtca tttttaaaat 600
tttttttaatt tttttttttt ttttactttt actttttacc tcttgtgtat atgtagggaa 660
tttataggga aatatgtact ttatggaata aattttaaga actaaaatat attttatttt 720
aaataaagta atggaccttt aatcttacgc agctaaatta ctgattatat atttgctgag 780
ctgatttaag ggttaaaaaa attgtatcaa gagttttatt ttttgacttc aaagccttgt 840
taataaagcc tcttttatac atgtgaaaaa aaaaaaaaaa aaaaaaa 887

<210> 302
<211> 905
<212> DNA
<213> Homo sapiens

<400> 302
gttctagatc gcgagtggcc gcccttttt tttttttttt tgcaggaagg aattccattt 60
attgtggatg cattttcaca atatatgttt attggagcga tccattatca gtgaaaagta 120
tcaagtgttt ataaaatttt taggaatggc agattcacag aacatgctag tcagcctgca 180
gtattcctcc agaagagcta accagggcag ggctggcatg agaagtgaca tctgcgttac 240
aaagtctatc ttcctcataa gtctgtaaag agcaattgaa tcttctagct ttagcaaacc 300
taagccaaag gaaggaaagc cacgaagaat gcagaagtca aaccctcatg acaaagtagg 360
cacaagtcta caataagcta aatcagaatt tacaaataca agtgtcccag gtagcattga 420
ctcccgtcat tggagtgaaa tggatcaaag tttgaattaa ggcctatggt aaggtaacat 480
tgctttgttg tacttttgaa caagagctcc tcctgatcac tattacatat ttttctagaa 540
aatctaaagt tcagaagaga atgtatcact gctgactttt attccaatat ttggatggag 600
taagttttag ggtagaattt tgttcagttt ggatttaatc ttttgaaaag taaattcctt 660
gtttactggt ttgactataa ttctctgtta tctttacgag gtaaaactgc aagctgacta 720
gcatgttctg tgaatctgcc attcctaaaa attttataaa cacttgatac ttttcactga 780
taatggatcg ctccaataaa catatattgt gaaaatgcat ccacaataaa tggaattcct 840
tcctgcaaaa aaaaaaaaaa aaaaaaaaaa aaaaaaaaaa aaaaaaaaaa aaaaaaaaaa 900
aaaaa 905

<210> 303
<211> 1832
<212> DNA
<213> Homo sapiens

<400> 303
tcagcaaagg gaaatcaccc tgaaaggttt tagaaatggt agttttggag ttttggtggc 60
aaccaatgtt gctgcacgtg ggttagacat ccctgaggtt gatttggtta tacaaagctc 120
tccaccaaag gatgtagagt cctacattca tcgatccggg cggacaggca gagctggaag 180
gacgggggtg tgcatctgct tttatcagca caaggaagaa tatcagttag tacaagtgga 240
gcaaaaagcg ggaattaagt tcaaacgaat aggtgttcct tctgcaacag aaataataaa 300
agcttccagc aaagatgcca tcaggctttt ggattccgtg cctcccactg ccattagtca 360
cttcaaacaa tcagctgaga agctgataga ggagaaggga gctgtggaag ctctggcagc 420
agcactggcc catatttcag gtgccacgtc cgtagaccag cgctccttga tcaactcaaa 480
tgtgggtttt gtgaccatga tcttgcagtg ctcaattgaa atgccaaata ttagttatgc 540
ttggaaagaa cttaaagagc agctgggcga ggagattgat tccaaagtga agggaatggt 600
tttctcaaa ggaaagctgg gtgtttgctt tgatgtacct accgcatcag taacagaaat 660
acaggagaaa tggcatgatt cacgacgctg gcagctctct gtggccacag agcaaccaga 720
actggaagga ccacgggaag gatatggagg cttcagggga cagcgggaag gcagtcgagg 780
cttcagggga cagcgggacg gaaacagaag attcagagga cagcgggaag gcagtagagg 840
cccgagagga cagcgatcag gaggtggcaa caaaagtaac agatcccaaa acaaaggcca 900
gaagcggagt ttcagtaaag catttggtca ataattagaa atagaagatt tatatagcaa 960
aaagagaatg atgtttggca atatagaact gaacattatt tttcatgcaa agttaaaagc 1020
acattgtgcc tcctttgac cacttgccaa gtccctgtct ctttcagaca cagacaagct 1080
tcatttaaat tatttcatct gatcattatc atttataact ttattgttac ttcatcagtt 1140
tttcctttg aaaggtgtat gaattcatta catttttatt ctaatgtatt atctgtagat 1200
tagaagataa aatcaagcat gtatctgcct atactttgtg agttcacctg tctttatact 1260
caaaagtgtc ccttaatagt gtccttccct gaaataaata cctaagggag tgtaacagtc 1320

tctggaggac cactttgagc ctttggaagt taaggtttcc tcagccacct gccgaacagt 1380
ttctcatgtg gtcctattat ttgtctactg agacttaata ctgagcaatg ttttgaaaca 1440
agatttcaaa ctaatctggg ttgtaataca gtttatacca gtgtatgctc tagacttgga 1500
agatgtagta tgtttgatgt ggattaccta tacttatgtt cgttttgata catttttagc 1560
ttctcattat aaggtgattc atgctttagt gaattcttca tagatagtat atataaaagt 1620
acattttaat agaaagccag ggttttaagg aatttcacat gtataaggtg gctccatagc 1680
tttatttgta agtaggctgg ataaatggtg cttaaatggt aatgtactcc acttcttcct 1740
attggaagat taacattatt taccaagaag gacttaaggg agtaaggggc gcagattagc 1800
attgctcaag agtatgtaaa aaaaaaaaaa aa 1832

<210> 304
<211> 1824
<212> DNA
<213> Homo sapiens

<400> 304
ggcaggacag aagacttcag ctgccctgtc cacagtgggc tctgccatca gcaggaagct 60
tggagacatg aggaactctg cgaccttcaa gtcgtttgag gaccgagttg ggaccataaa 120
gtctaaggtt gtgggtgaca gagagaacgg cagtgacaac ctcccttcct cagcggggag 180
tggtgacaag cccctgtcgg atcccgcacc tttctaagcc tgtggttgct tcacccgctg 240
cagagcacac gcaacccagc ctcagcatca cagccgcagc tctgttcagc ggagcagcca 300
gccagggcgg atgagcagag ccggccctga ggacagtcct gcccatccac gcggagatgt 360
ggctgccgcg tttgcatgaa tttggagaaa acaggcttgt acacagatgt tttacactca 420
cgtttgtaga tgaaacagat cactgtgctg tccttcctag gggtgcagga agtggacagg 480
gcggagggtt tgaaagaata ttgagccaaa gcccaggctc cctttgggaa tcatgttagc 540
ccatcagaat gttgaaggat tgaagagttc taagcgtaaa ataagtggca ttttctgact 600
tcttcctcct cctccttccc tgactcacag aaggaatgca atcacccagc aagtcctacc 660
tgttacgcaa ttttttatct caaaatgccg aacgagaaaa ctgtccattt tctgagaccc 720
ccagaaagga aactgaccct cagcagctgc ctgattgtta cgcgaatcta gctttaacgg 780
aagcaaattc attatttttt aaatgcagtg gacttttcaa aaagtttaaa ttaggcaaag 840
cagctttagc ctcatagaat attatttctt tggactcaag ctgaaataca agccttacat 900
tgccttatgc tttatttctt tctaattttt atatgtatat agatgagggt tccttaatgg 960
ttgtgagcat tgtgtggaat tttacacctg gcctgcgtgg cagcctcttc cagttgaggt 1020
gttttatgtc acgcacactc catcccagtg tacaaaacct gcttctcttc tcaaccgtgg 1080
cagctcccgc tggctcctat gccctgccct aaagggctct tgagcctctg ggaatgggag 1140
gggccaagag aaggaaaacc ctgtctttag caccctttaa aagaactgtg ccccccttct 1200
cagtgctgcc tttgcatggg cctggcccgg ctcacattcg tcagtgactc caaccctcct 1260
gcttgctgta cttgggatga aacgacccca caggtcaagt ggagggtggg gcgtgggcat 1320
cagccaggat tgccgttaca gtctttttct caggagctac aaagatctct tcctgttact 1380
aaatggtcgc accccagcag cctctctcgc acaccggggc cctgcatgtc agatggcgtg 1440
gtctgcaggg ggagctctgt gccttagtgg ctcttggcag gacactgagg gcctgcctgt 1500
ggtgtgcccg gctctgccac tcccgggagg ggaagggctg ctcagctcaa ggtgtcctgt 1560
tcggtagagc aagtgtcctc tgacagccgt gtccccggac agttcagaca cccttgggga 1620
tggcactcca cacacgacag agatgcaggg gccagggaag cccagcgctc ggtgcccttc 1680
gtccagggtt aaaatcggcc tgtggggtgt ggtgagaagg caggttgtgc gggtgttgac 1740
cgatgtatct tttccttaaa gttattataa taatgggtaa tttgtcaata aagcattcct 1800
ttgggggaaa aaaaaaaaaa aaaa 1824

<210> 305
<211> 759
<212> DNA
<213> Homo sapiens

<400> 305
cgatgggcat ctctcgggac aactggcaca agcgccgcaa aaccgggggc aagagaaagc 60
cctaccacaa gaagcggaag tatgagttgg ggcgcccagc tgccaacacc aagattggcc 120
cccgccgcat ccacacagtc cgtgtgcggg gaggtaacaa gaaataccgt gccctgaggt 180
tggacgtggg gaatttctcc tggggctcag agtgttgtac tcgtaaaaca aggatcatcg 240
atgttgtcta caatgcatct aataacgagc tggttcgtac caagaccctg gtgaagaatt 300
gcatcgtgct catcgacagc acaccgtacc gacagtggta cgagtcccac tatgcgctgc 360
ccctgggccg caagaaggga gccaagctga ctcctgagga agaagagatt ttaaacaaaa 420
aacgatctaa aaaaattcag aagaaatatg atgaaaggaa aaagaatgcc aaaatcagca 480
gtctcctgga ggagcagttc cagcagggca agcttcttgc gtgcatcgct tcaaggccgg 540
gacagtgtgg ccgagcagat ggctatgtgc tagagggcaa agagttggag ttctatctta 600
ggaaaatcaa ggcccgcaaa ggcaaataaa tccttgtttt gtcttcaccc atgtaataaa 660
ggtgtttatt gttttgttcc caaaaaaaaa aaaaaaaaaa aaaaaaaaaa aaaaaaaaaa 720
aaaaaaaaaa aaaaaaaaaa aaaaaaaaaa aaaaaaaaa 759

<210> 306
<211> 938
<212> DNA
<213> Homo sapiens

<400> 306
gtcgtcgggg tttcctgctt caacagtgct tggacggaac ccggcgctcg ttccccaccc 60
cggccggccg cccatagcca gccctccgtc acctcttcac cgcaccctcg gactgcccca 120
aggcccccgc cgccgctcca gcgccgcgca gccaccgccg ccgccgccgc ctctccttag 180
tcgccgccat gacgaccgcg tccacctcgc aggtgcgcca gaactaccac caggactcag 240
aggccgccat caaccgccag atcaacctgg agctctacgc ctcctacgtt tacctgtcca 300
tgtcttacta ctttgaccgc gatgatgtgg ctttgaagaa ctttgccaaa tactttcttc 360
accaatctca tgaggagagg gaacatgctg agaaactgat gaagctgcag aaccaacgag 420
gtggccgaat ctttcttcag gatatcaaga aaccagactg tgatgactgg gagagcgggc 480
tgaatgcaat ggagtgtgca ttacatttgg aaaaaaatgt gaatcagtca ctactggaac 540
tgcacaaact ggccactgac aaaaatgacc cccatttgtg tgacttcatt gagacacatt 600
acctgaatga gcaggtgaaa gccatcaaag aattgggtga ccacgtgacc aacttgcgca 660
agatgggagc gcccgaatct ggcttggcgg aatatctctt tgacaagcac accctgggag 720
acagtgataa tgaaagctaa gcctcgggct aatttcccca tagccgtggg gtgacttccc 780
tggtcaccaa ggcagtgcat gcatgttggg gtttccttta ccttttctat aagttgtacc 840
aaaacatcca cttaagttct ttgatttgta ccattccttc aaataaagaa atttggtaaa 900
aaaaaaaaaa aaaaaaaaaa aaaaaaaaaa aaaaaaaa 938

<210> 307

<211> 1281
<212> DNA
<213> Homo sapiens

<400> 307
gcgtgatgtc tcacagaaag ttctccgctc ccagacatgg gtccctcggc ttcctgcctc 60
ggaagcgcag cagcaggcat cgtgggaagg tgaagagctt ccctaaggat gacccatcca 120
agccggtcca cctcacagcc ttcctgggat acaaggctgg catgactcac atcgtgcggg 180
aagtcgacag gccgggatcc aaggtgaaca agaaggaggt ggtggaggct gtgaccattg 240
tagagacacc acccatggtg gttgtgggca ttgtgggcta cgtggaaacc cctcgaggcc 300
tccggacctt caagactgtc tttgctgagc acatcagtga tgaatgcaag aggcgtttct 360
ataagaattg gcataaatct aagaagaagg cctttaccaa gtactgcaag aaatggcagg 420
atgaggatgg caagaagcag ctggagaagg acttcagcag catgaagaag tactgccaag 480
tcatccgtgt cattgcccac acccagatgc gcctgcttcc tctgcgccag aagaaggccc 540
acctgatgga gatccaggtg aacggaggca ctgtggccga gaagctggac tgggcccgcg 600
agaggcttga gcagcaggta cctgtgaacc aagtgtttgg gcaggatgag atgatcgacg 660
tcatcggggt gaccaagggc aaaggctaca aaggggtcac cagtcgttgg cacaccaaga 720
agctgccccg caagacccac cgaggcctgc gcaaggtggc ctgtattggg gcatggcatc 780
ctgctcgtgt agccttctct gtggcacgcg ctgggcagaa aggctaccat caccgcactg 840
agatcaacaa gaagatttat aagattggcc agggctacct tatcaaggac ggcaagctga 900
tcaagaacaa tgcctccact gactatgacc tatctgacaa gagcatcaac cctctgggtg 960
gctttgtcca ctatggtgaa gtgaccaatg actttgtcat gctgaaaggc tgtgtggtgg 1020
gaaccaagaa gcgggtgctc accctccgca agtccttgct ggtgcagacg aagcggcggg 1080
ctctggagaa gattgacctt aagttcattg acaccacctc caagtttggc catggccgct 1140
tccagaccat ggaggagaag aaagcattca tgggaccact gaagaaagac cgaattgcaa 1200
aggaagaagg agcttaatgc caggaacaga ttttgcagtt ggtggggtct caataaaatt 1260
attttccact gaaaaaaaaa a 1281

<210> 308
<211> 1698
<212> DNA
<213> Homo sapiens

<400> 308
gtttgaagca aacaggcagc gcgcgacaat ggcggtcgct cgtgcagctt tggggccatt 60
ggtgacgggt ctgtacgacg tgcaggcttt caagtttggg gacttcgtgc tgaagagcgg 120
gctttcctcc cccatctaca tcgatctgcg gggcatcgtg tctcgaccgc gtcttctgag 180
tcaggttgca gatattttat tccaaactgc ccaaaatgca ggcatcagtt ttgacaccgt 240
gtgtggagtg ccttatacag ctttgccatt ggctacagtt atctgttcaa ccaatcaaat 300
tccaatgctt attagaagga aagaaacaaa ggattatgga actaagcgtc ttgtagaagg 360
aactattaat ccaggagaaa cctgtttaat cattgaagat gttgtcacca gtggatctag 420
tgttttggaa actgttgagg ttcttcagaa ggagggcttg aaggtcactg atgccatagt 480
gctgttggac agagagcagg gaggcaagga caagttgcag gcgcacggga tccgcctcca 540
ctcagtgtgt acattgtcca aaatgctgga gattctcgag cagcagaaaa aagttgatgc 600
tgagacagtt gggagagtga agaggtttat tcaggagaat gtctttgtgg cagcgaatca 660
taatggttct cccctttcta taaaggaagc acccaaagaa ctcagcttcg gtgcacgtgc 720
agagctgccc aggatccacc cagttgcatc gaagcttctc aggcttatgc aaaagaagga 780

gaccaatctg tgtctatctg ctgatgtttc actggccaga gagctgttgc agctagcaga 840
tgctttagga cctagtatct gcatgctgaa gactcatgta gatattttga atgattttac 900
tctggatgtg atgaaggagt tgataactct ggcaaaatgc catgagttct tgatatttga 960
agaccggaag tttgcagata taggaaacac agtgaaaaag cagtatgaag gaggtatctt 1020
taaaatagct tcctgggcag atctagtaaa tgctcacgtg gtgccaggct caggagttgt 1080
gaaaggcctg caagaagtgg gcctgccttt gcatcggggg tgcctcctta ttgcggaaat 1140
gagctccacc ggctccctgg ccactgggga ctacactaga gcagcggtta gaatggctga 1200
ggagcactct gaatttgttg ttggttttat ttctggctcc cgagtaagca tgaaaccaga 1260
atttcttcac ttgactccag gagttcagtt ggaagcagga ggagataatc ttggccaaca 1320
gtacaatagc ccacaagaag ttattggcaa acgaggttcc gatatcatca ttgtaggtcg 1380
tggcataatc tcagcagctg atcgtctgga agcagcagag atgtacagaa aagctgcttg 1440
ggaagcgtat ttgagtagac ttggtgtttg agtgcttcag atacattttt cagatacaat 1500
gtgaagacat tgaagatatg tggtcccctg aaagtcactg gctggaaata atccaattat 1560
tcctgcttgg attcttccac agggcctgtg taagaatggg ttctggagtt ctcatggtct 1620
ttaggaaata ttgagtaatt tgtaatcacc gcattgatac tataataagt tcattcttaa 1680
aaaaaaaaaa aaaaaaa 1698

<210> 309
<211> 1102
<212> DNA
<213> Homo sapiens

<400> 309
gttataaatg gttataaagc tcctgttact catattagtt atttacatca aaaagctttt 60
agaaaatggt acgaggtaac caattcttgt catggtgaaa tctgattgag taaccaagca 120
gttttactat tctggtgctg cttcataaca aaaatgaaaa gctgcatgca tctacagcag 180
gcatggattg tttatgtcgt atgatatcct ttattaagta agttcactta tagtatttct 240
ataatttgat tcattgccgt aatagagcca tgtaggaaat gcactgattg catgttattg 300
tggcaagaat atcctaaatg tcattaaaat cctccaacat gatggatcta cttatggtct 360
tgtttgttga catgacaaat taacattctt atagttacat ctggaaatga gcatttgaaa 420
tagataatcc tttaagcctt gtggcaaaat ttttgtggct tttgtttaac tttgaaaggt 480
tattatgcac taaccttttt tggtggctaa ttagggttta aatacagaaa caagatttca 540
aataaaactg tctttggcag tgagtaaata gcatattttg aagtagagtt gtatactttt 600
tcataagatg tttgggaatt tttttcctga agtaataatt tattccacat ctacatcagt 660
gaaagctatc tacctatcct gagtctatct taaaggaaaa aaagaaaaaa accttatctc 720
ttgcccttat tttgaatttt ccactctttc attaatttgt tttaagctcc gtgttggaaa 780
aaaggggtag tgcattttaa attgaccttc atacgctttt aaaataagac aaatctactt 840
gataatgtac ctttatttga tctcaagttg tataaaacca ataaatttgt gttactgcag 900
tagtaatctt atgcacacgg tgatttcatg ttatatatgc aaagtaggca actgttttct 960
tagttacaga agtttcaagc ttcacttttg tgcagtagaa acaaaagtag gctacagtct 1020
gtgccatgtt gatgtacagt ttctgaaatt gttttacaag actttgataa taaaaccctt 1080
aaacttaaaa aaaaaaaaaa aa 1102

<210> 310
<211> 519
<212> DNA

<213> Homo sapiens

<400> 310
tcaaaagtca aggcatcatt taaaataatc tgatttcaga caaatgctgt gtggaaacat 60
ctatcctata gatcatccta ttcttatgtg tctttggtta tcagatcaat tacagaataa 120
ttgtgttgtg atattgtgtc ctaaattgct cattaatttt tatttacaga ttgaaaaaga 180
gggaccgtgt aaagaaaatg gaaaataaat atctttcaaa gactctttta gataaacacg 240
atgaggcaaa atcaggttca ttcattcaac gatagtttct aaacagtact taaatagcgg 300
ttggaaaacg tagccttcat tttatgattt tttcatatgt ggaaatctat tacatgtaat 360
acaaaacaaa catgtagttt gaaggcggtc agatttcttt gagaaatctt tgtagagtta 420
attttatgga aattaaaatc agaattaaat gctaaaaaaa aaaaaaaaaa aaaaaaaaaa 480
taaaaaaaaa aaaaaataaa aaaaaaaaaa aaaaaaaaa 519

<210> 311
<211> 2335
<212> DNA
<213> Homo sapiens

<400> 311
ccgaggtggc tgctggtggg ggctcctggg acgacaggct gcgcaggctc atctccccca 60
acctgggggt cgtgttcttc aacgcctgcg aggccgcgtc gcggctggcg cgcggcgagg 120
atgaggcgga gctggcgctg agcctcctgg cgcagctggg catcacgcct ctgccactca 180
gccgcggccc cgtgccagcc aaacccaccg tgctcttcga gaagatgggc gtgggccggc 240
tggacatgta tgtgctgcac ccgccctccg ccggcgccga gcgcacgctg gcctctgtgt 300
gcgccctgct ggtgtggcac cccgccggcc ccggcgagaa ggtggtgcgc gtgctgttcc 360
ccggttgcac cccgcccgcc tgcctcctgg acggcctggt ccgcctgcag cacttgaggt 420
tcctgcgaga gcccgtggtg acgccccagg acctggaggg gccggggcga gccgagagca 480
aagagagcgt gggctcccgg gacagctcga agagagaggg cctcctggcc acccacccta 540
gacctggcca ggagcgccct ggggtggccc gcaaggagcc agcacgggct gaggccccac 600
gcaagactga gaaagaagcc aaggcccccc gggagttgaa gaaagacccc aaaccgagtg 660
tctcccggac ccagccgcgg gaggtgcgcc gggcagcctc ttctgtgccc aacctcaaga 720
agacgaatgc ccaggcggca cccaagcccc gcaaagcgcc cagcacgtcc cactctggct 780
tcccgccggt ggcaaatgga ccccgcagcc cgcccagcct ccgatgtgga gaagccagcc 840
cccccagtgc agcctgcggc tctccggcct cccagctggt ggccacgccc agcctggagc 900
tggggccgat cccagccggg gaggagaagg cactggagct gcctttggcc gccagctcaa 960
tcccaaggcc acgcacaccc tcccctgagt cccaccggag ccccgcagag ggcagcgagc 1020
ggctgtcgct gagcccactg cggggcgggg aggccgggcc agacgcctca cccacagtga 1080
ccacacccac ggtgaccacg ccctcactac ccgcagaggt gggctccccg cactcgaccg 1140
aggtggacga gtccctgtcg gtgtcctttg agcaggtgct gccgccatcc gcccccacca 1200
gtgaggctgg gctgagcctc ccgctgcgtg gcccccgggc gcggcgctcg gcttccccac 1260
acgatgtgga cctgtgcctg gtgtcaccct gtgaatttga gcatcgcaag gcggtgccaa 1320
tggcaccggc acctgcgtcc cccggcagct cgaatgacag cagtgcccgg tcacaggaac 1380
gggcaggtgg gctgggggcc gaggagacgc cacccacatc ggtcagcgag tccctgccca 1440
ccctgtctga ctcggatccc gtgcccctgg cccccggtgc ggcagactca gacgaagaca 1500
cagagggctt tggagtccct cgccacgacc ctttgcctga ccccctcaag gtcccccccac 1560
cactgcctga cccatccagc atctgcatgg tggaccccga gatgctgccc cccaagacag 1620
cacggcaaac ggagaacgtc agccgcaccc ggaagcccct ggcccgcccc aactcacgcg 1680

ctgccgcccc caaagccact ccagtggctg ctgccaaaac caaggggctt gctggtgggg 1740
accgtgccag ccgaccactc agtgcccgga gtgagcccag tgagaaggga ggccgggcac 1800
ccctgtccag aaagtcctca acccccaaga ctgccactcg aggcccgtcg gggtcagcca 1860
gcagccggcc cggggtgtca gccaccccac ccaagtcccc ggtctacctg gacctggcct 1920
acctgcccag cgggagcagc gcccacctgg tggatgagga gttcttccag cgcgtgcgcg 1980
cgctctgcta cgtcatcagt ggccaggacc agcgcaagga ggaaggcatg cgggccgtcc 2040
tggacgcgct actggccagc aagcagcatt gggaccgtga cctgcaggtg accctgatcc 2100
ccactttcga ctcggtggcc atgcatacgt ggtacgcaga gacgcacgcc cggcaccagg 2160
cgctgggcat cacggtgttg ggcagcaaca gcatggtgtc catgcaggat gacgccttcc 2220
cggcctgcaa ggtggagttc tagccccatc gccgacacgc cccccactca gcccagcccg 2280
cctgtcccta gattcagcca catcagaaat aaactgtgac ttccaaaaaa aaaaa 2335

<210> 312
<211> 1027
<212> DNA
<213> Homo sapiens

<400> 312
ggggcggcgg gttggtctac gctgtgcgcg gcggacgtcg gaggcagcgg ggagcggagc 60
ggggccgccg gggcctctcc agggccgcag cggcagcagt tgggcccccc gccccggccg 120
gcggaccgaa gaacgcagga agggggccgg ggggacccgc ccccggccgg ccgcagccat 180
gaactccaac gtggagaacc taccccccgca catcatccgc ctggtgtaca aggaggtgac 240
gacactgacc gcagacccac ccgatggcat caaggtcttt cccaacgagg aggacctcac 300
cgacctccag gtcaccatcg agggccctga ggggacccca tatgctggag gtctgttccg 360
catgaaactc ctgctgggga aggacttccc tgcctcccca cccaagggct acttcctgac 420
caagatcttc cacccgaacg tgggcgccaa tggcgagatc tgcgtcaacg tgctcaagag 480
ggactggacg gctgagctgg gcatccgaca cgtactgctg accatcaagt gcctgctgat 540
ccaccctaac cccgagtctg cactcaacga ggaggcgggc cgcctgctct tggagaacta 600
cgaggagtat gcagctcggg cccgtctgct cacagagatc cacgggggcg ccggcgggcc 660
cagcggcagg gccgaagccg gtcgggccct ggccagtggc actgaagctt cctccaccga 720
ccctggggcc ccagggggcc cgggaggggc tgagggtacc atggccaaga agcatgctgg 780
cgagcgcgat aagaagctgg cggccaagaa aaagacggac aagaagcggg cgctgcggcg 840
gctgtagtgg gctctcttcc tccttccacc gtgaccccaa cctctcctgt cccctccctc 900
caactctgtc tctaagttat ttaaattatg gctggggtcg gggagggtac agggggcact 960
gggacctgga tttgtttttc taaataaagt tggaaaagca aaaaaaaaaa aaaaaaaaaa 1020
aaaaaaa 1027

<210> 313
<211> 1068
<212> DNA
<213> Homo sapiens

<400> 313
gcggagcttg ttactggtta cttggcctca tggcggtccg agcttcgttc gagaacaact 60
gtgagatcgg ctgctttgcc aagctcacca acacctactg tctggtagcg atcggaggct 120
cagagaactt ctacagtgtg ttcgagggcg agctctccga taccatcccc gtggtgcacg 180

cgtctatcgc cggctgccgc atcatcgggc gcatgtgtgt ggggaacagg cacggtctcc 240
tggtacccaa caataccacc gaccaggagc tgcaacacat tcgcaacagc ctcccagaca 300
cagtgcagat taggcgggtg gaggagcggc tctcagcctt gggcaatgtc accacctgca 360
atgactacgt ggccttggtc cacccagact tggacaggga gacagaagaa attctggcag 420
atgtgctcaa ggtggaagtc ttcagacaga cagtggccga ccaggtgcta gtaggaagct 480
actgtgtctt cagcaatcag ggagggctgg tgcatcccaa gacttcaatt gaagaccagg 540
atgagctgtc ctctcttctt caagtccccc ttgtggcggg gactgtgaac cgaggcagtg 600
aggtgattgc tgctgggatg gtggtgaatg actggtgtgc cttctgtggc ctggacacaa 660
ccagcacaga gctgtcagtg gtggagagtg tcttcaagct gaatgaagcc cagcctagca 720
ccattgccac cagcatgcgg gattccctca ttgacagcct cacctgagtc accttccaag 780
ttgttccatg ggctcctggc tctggactgt ggccaacctt ctccacattc cgcccaatct 840
gtaccggatg ctggcaggga ggtggcagag agctcactgg gactgagggg ctgggcaccc 900
aaccctttc cacctgtgct tatcgcctgg atctatcatt actgcaaaaa cctgctctgt 960
tgtgctggct ggcaggccct gtggctgctg gctgagggtt ctgctgtcct gtgccacccc 1020
attaaagtgc agttccctcc ggaaaaaaaa aaaaaaaaaa aaaaaaa 1068

<210> 314
<211> 810
<212> DNA
<213> Homo sapiens

<400> 314
tgcaaatatc caagaagagg aagtttgtcg ctgatggcat cttcaaagct gaactgaatg 60
agtttcttac tcgggagctg gctgaagatg gctactctgg agttgaggtg cgagttacac 120
caaccaggac agaaatcatt atcttagcca ccagaacaca gaatgttctt ggtgagaagg 180
gccggcggat tcgggaactg actgctgtag ttcagaagag gtttggcttt ccagagggca 240
gtgtagagct ttatgctgaa aaggtggcca ctagaggtct gtgtgccatt gcccaggcag 300
agtctctgcg ttacaaactc ctaggagggc ttgctgtgcg gagggcctgc tatggtgtgc 360
tgcggttcat catggagagt ggggccaaag gctgcgaggt tgtggtgtct gggaaactcc 420
gaggacagag ggctaaatcc atgaagtttg tggatggcct gatgatccac agcggagacc 480
ctgttaacta ctacgttgac actgctgtgc gccacgtgtt gctcagacag ggtgtgctgg 540
gcatcaaggt gaagatcatg ctgccctggg acccaactgg taagattggc cctaagaagc 600
ccctgcctga ccacgtgagc attgtggaac ccaaagatga gatactgccc accaccccca 660
tctcagaaca gaagggtggg aagccagagc cgcctgccat gccccagcca gtccccacag 720
cataacaggg tctccttggc agctgtattc tggagtctgg atgttgctct ctaaagacct 780
ttaataaaat tttgtacaaa gaaaaaaaaa 810

<210> 315
<211> 2505
<212> DNA
<213> Homo sapiens

<400> 315
cgtttgcacc tcgctgctcc agcctctggg gcgcattcca accttccagc ctgcgacctg 60
cggagaaaaa aaaattactt attttcttgc cccatacata ccttgaggcg agcaaaaaat 120
taaattttaa ccatgaggga aatcgtgcac atccaggctg gtcagtgtgg caaccagatc 180

ggtgccaagt tctgggaggt gatcagtgat gaacatggca tcgaccccac cggcacctac 240
cacggggaca gcgacctgca gctggaccgc atctctgtgt actacaatga agccacaggt 300
ggcaaatatg ttcctcgtgc catcctggtg gatctagaac ctgggaccat ggactctgtt 360
cgctcaggtc cttttggcca gatctttaga ccagacaact ttgtatttgg tcagtctggg 420
gcaggtaaca actgggccaa aggccactac acagagggcg ccgagctggt tgattctgtc 480
ctggatgtgg tacggaagga ggcagagagc tgtgactgcc tgcagggctt ccagctgacc 540
cactcactgg gcgggggcac aggctctgga atgggcactc tccttatcag caagatccga 600
gaagaatacc ctgatcgcat catgaatacc ttcagtgtgg tgccttcacc caaagtgtct 660
gacaccgtgg tcgagcccta caatgccacc ctctccgtcc atcagttggt agagaatact 720
gatgagacct attgcattga caacgaggcc ctctatgata tctgcttccg cactctgaag 780
ctgaccacac caacctacgg ggatctgaac caccttgtct cagccaccat gagtggtgtc 840
accacctgcc tccgtttccc tggccagctc aatgctgacc tccgcaagtt ggcagtcaac 900
atggtcccct tcccacgtct ccatttcttt atgcctggct ttgcccctct caccagccgt 960
ggaagccagc agtatcgagc tctcacagtg ccggaactca cccagcaggt cttcgatgcc 1020
aagaacatga tggctgcctg tgacccccgc cacggccgat acctcaccgt ggctgctgtc 1080
ttccgtggtc ggatgtccat gaaggaggtc gatgagcaga tgcttaacgt gcagaacaag 1140
aacagcagct actttgtgga atggatcccc aacaatgtca agacagccgt ctgtgacatc 1200
ccacctcgtg gcctcaagat ggcagtcacc ttcattggca atagcacagc catccaggag 1260
ctcttcaagc gcatctcgga gcagttcact gccatgttcc gccggaaggc cttcctccac 1320
tggtacacag gcgagggcat ggacgagatg gagttcaccg aggctgagag caacatgaac 1380
gacctcgtct ctgagtatca gcagtaccag gatgccaccg cagaagagga ggaggatttc 1440
ggtgaggagg ccgaagagga ggcctaaggc agagccccca tcacctcagg cttctcagtt 1500
cccttagccg tcttactcaa ctgccccttt cctctccctc agaatttgtg tttgctgcct 1560
ctatcttgtt ttttgttttt tcttctgggg ggggggtcta gaacagtgcc tggcacatag 1620
taggcgctca ataaatactt gtttgttgaa tgtctcctct ctctttccac tctgggaaac 1680
ctaggtttct gccattctgg gtgaccctgt atttctttct ggtgcccatt ccatttgtcc 1740
agttaatact tcctcttaaa aatctccaag aagctgggtc tccagatccc atttagaacc 1800
aaccaggtgc tgaaaacaca tgtagataat ggccatcatc ctaagcccaa agtagaaaat 1860
ggtagaaggt agtgggtaga agtcactata taaggaaggg gatgggattt tccattctaa 1920
aagttttgga gagggaaatc caggctatta aagtcactaa atttctaagt atgtccattt 1980
cccatctcag cttcaaggga ggtgtcagca gtattatctc cactttcaat ctccctccaa 2040
gctctactct ggaggagtct gtcccactct gtcaagtgga atccttccct ttccaactct 2100
acctccctca ctcagctcct ttcccctgat cagagaaagg gatcaagggg gttgggaggg 2160
gggaaagaga ccagccttgg tccctaagcc tccagaaacg tcttcttaat ccccaccttt 2220
tcttactccc aaaaaagaat gaacacccct gactctggag tggtgtatac tgccacatca 2280
gtgtttgagt cagtccccag aggagagggg aaccctcctc catctttttt gcaacatctc 2340
atttcttcct tttgctgttg cttcccccct cacacacttg gttttgttct atcctacatt 2400
tgagatttct attttatgtt gaacttgctg cttttttttca tattgaaaag atgacatcgc 2460
cccaagagcc aaaaataaat gggaattgaa aaaaaaaaaa aaaaa 2505

<210> 316
<211> 1588
<212> DNA
<213> Homo sapiens

<400> 316
gttgccgaga gatggatgag cagattagac tgatggacca gaacctgaag tgtctgagtg 60

ctgctgaaga aaagtactct caaaaagaag ataaatatga ggaagaaatc aagattctta 120
ctgataaact caaggaggca gagacccgtg ctgagtttgc tgagagatcg gtagccaagc 180
tggaaaagac aattgatgac ctggaagata aactgaaatg caccaaagag gagcacctct 240
gtacacaaag gatgctggac cagaccctgc ttgacctgaa tgagatgtag aacgccccag 300
tcccaccctg ctgctgctcc tccctctgac ccagactccg cctgaggcca gcctgcggga 360
agctgacctt taactgaggg ctgatcttta actggaaggc tgctttctcc tttcaccacc 420
ccctccttcc ctgtgtcttt ttcgccaaac tgtctctgcc tcttcccgga gaatccagct 480
gggctagagg ctgagcacct ttggaaacaa catttaaggg aatgtgagca caatgcataa 540
tgtctttaaa aagcatgttg tgatgtacac attttgtaat tacctttttt gttgttttgt 600
agcaaccatt tgtaaaacat tccaaataat tccacagtcc tgaagcagca atcgaatccc 660
tttctcactt ttggaaggtg acttttcacc ttaatgcata ttcccctctc catagaggag 720
aggaaaaggt gtaggcctgc cttaccgaga gccaaacaga gcccagggag actccgctgt 780
gggaaacctc attgttctgt acaaagtact agctaaacca gaaaggtgat tccaggagga 840
gttagccaaa caacaacaaa aacaaaaaat gtgctgttca agttttcagc tttaagatat 900
ctttggataa tgttatttct attttttatt tttttcatta gaagttacca aattaagatg 960
gtaagacctc tgagaccaaa attttgtccc atctctaccc cctcacaact gcttacagaa 1020
tggatcatgt ccccttatg ttgaggtgac cacttaattg ctttcctgcc tccttgaaag 1080
aaagaaagaa agaagactgt gtttttgcca ctgatttagc catgtgaaac tcatctcatc 1140
acccttttct gggtttgaag ctgctgtctc tagaagtgcc atctcaattg tgctttgtat 1200
cagtcagtgc tggagaaatc ttgaatagct tatgtacaaa actttttaaa ttttatatta 1260
ttttgaaact ttgctttggg tttgtggcac cctggccacc ccatctggct gtgacagcct 1320
ctgcagtccg tgggctggca gtttgttgat cttttaagtt tccttcccta cccagtcccc 1380
attttctggt aaggtttcta ggaggtctgt taggtgtaca tcctgcagct tattggctta 1440
aaatgtactc tccttttatg tggtctcttt ggggccgatt gggagaaaga gaaatcaata 1500
gtgcaactgt tttgatactg aatattgaca agtgtctttt tgaaataaag aaccagtccc 1560
tccaaccctc aaaaaaaaaa aaaaaaa 1588

<210> 317
<211> 1831
<212> DNA
<213> Homo sapiens

<400> 317
gttgaggact acgcgcggcc caggcctggg ccccccgctg ctccaggccg cgctgggcct 60
tgggcgggct gggtggcact ggcctgcggg ccgggcggcg agcggggggc gcgggcgggc 120
ctggctgcag cccacgggcc gggagacggg tgtgcaggtg tacaacagcc tcaccgggag 180
gaaggaaccc ctaatcgtgg cgcacgccga agccgcctcc tggtatagct gtggaccaac 240
tgtatatgat catgcgcacc ttggccatgc ttgctcatat gttagatttg atatcattcg 300
aaggatccta accaaggttt ttggatgcag catagtcatg gtgatgggta ttacagatgt 360
agatgataaa atcatcaaaa gagccaatga gatgaatatt tccccccgctt ccctcgccag 420
tctttatgag gaagacttca agcaggacat ggcagccctg aaggttctcc cacccacggt 480
gtacctgagg gtaaccgaaa atattcctca gataatttct ttcattgaag gaatcattgc 540
ttcgtgggaa cgcttattca acggcaaaag gcaatgtcta cttcgatctg aaagtctaga 600
ggagacaaag tatggcaaaa ttggtcggcg tggtccctgg tccagtccgg agaccagcgg 660
acttctgaca agccgtcatg ccaatgactt cgccctgtgg aaggcggcca aaccccagga 720
ggtgttctgg gcctctccct ggggacccgg gaggccgggc tggcacatcg agtgctctgc 780
catcgctagt atggtatttg gaagtcaact ggatatccat tcaggtggga tagatttagc 840

ttttccacat catgagaacg aaattgcaca gtgcgaagtc tttcatcagt gcgagcagtg 900
gggaaattat tttctgcatt ctgggcattt gcacgccaaa ggcaaagaag aaaaaatgtc 960
caaatcatta aagaactaca ttactattaa ggactttctg aagacctttt cccccgatgt 1020
cttccggttc ttctgcctgc ggagcagcta ccgctcagcc atcgactaca gtgacagcgc 1080
catgctccaa gctcagcagc tgctcctggg gctgggctct ttcctggagg acgcacgtgc 1140
ctacatgaag ggcagctgg cctgcggctc cgtcagggaa gcgatgctgt gggagaggct 1200
ctccagcacc aagagggccg tgaaggcggc cttggcagat gattttgaca cacccagggt 1260
ggttgatgcc atcctgggcc ttgcacacca cgggaatgga cagctcaggg cgtccctgaa 1320
ggaacctgaa gggccgagaa gtcctgctgt gtttggtgcc atcatctctt actttgaaca 1380
gtttttgaa actgttggaa tttctctggc aaatcaacag tacgtttcag gagacggcag 1440
cgaggctacc ttgcatggtg tggtggacga gctggtgcgg ttccggcaga aggtccggca 1500
gtttgcgctg gccatgcccg aggccacggg ggacgcccgg cggcagcagc tcctagaaag 1560
gcagcccctg ctggaagcat gcgacaccct gcgccggggc ctgactgccc acggcatcaa 1620
catcaaggac agaagcagta caacatccac gtgggaactg ctggatcaaa ggacaaaaga 1680
ccaaaaatca gcgggctgag gatggagcac agccatgaac ctgctcacga caagacgcac 1740
ccatgcttct cagggtcaag gctttatgtt aaagcttcct gtcggggctg ctaggtcagc 1800
attaaagtaa ggcaaccaaa aaaaaaaaa a 1831

<210> 318
<211> 3476
<212> DNA
<213> Homo sapiens

<400> 318
gccgccgctg aggaaggctg tagtgtcggg gccgaagcgg acagggaatt ggaggagctt 60
ctggaaagat gccctcttcg cttcccaaga gaagtttttc caggaactat tcgacagtga 120
actggcttcc caagccactg cggagttcga gaaggcaatg aaggagttgg ctgaggaaga 180
accccacctg gtggagcagt tccaaaagct ctcagaggct gcagggagag tgggcagtga 240
tatgacctcc caacaagaat tcacttcttg cctaaaggaa acactaagtg gattagccaa 300
aaatgccact gaccttcaga actccagcat gtcggaagaa gagctgacca aggccatgga 360
ggggctaggc atggacgaag gggatgggga agggaacatc ctccccatca tgcagagtat 420
tatgcagaac ctactctcca aggatgtgct gtacccatca ctgaaggaga tcacagaaaa 480
gtatccagaa tggttgcaga gtcatcggga atctctacct ccagagcagt ttgaaaaata 540
tcaggagcag cacagcgtca tgtgcaaaat atgtgagcag tttgaggcag agacccccac 600
agacagtgaa accactcaaa aggctcgttt tgagatggtg ctggatctta tgcagcagct 660
acaagattta ggccatcctc caaaagagct ggctggagag atgcctcctg gcctcaactt 720
tgacctggat gccctcaatc tttcgggccc accaggtgcc agtggtgaac agtgtctgat 780
catgtgaaac acaacacgtt ttcctctctg agtcccagct atggggaaca tctggagtca 840
gcagaaccat tgggacctga ggcaggagtg tcacctgcgg gagaagtctg cccgctgccc 900
tctgtcatcc cattcaagat tgtgccatac cagctgaggt tttcctctg tctctctagg 960
aatagggtct gtttcacagg ccatttctgt gaaccctact ccattgtggt ttctgccact 1020
atcaaagttc cagctacctg caaggtgaag gaaggcatcc cttttggggc atgcactttc 1080
tttcctttct caaaataatg ttatatgtgg ccacactgat gttcaccttt acgtccaggg 1140
tctttgtgcc ttgtctctac tccctctctt ggatctgggg aggaggggca gagacctggg 1200
actctgtatt tctatagttc tcctggcaga gcctttgaga atggggagaa acagcctggg 1260
ctggggctac aggtctgtca ctatgctctc ttgccttcag acagaccatt ctgaattctc 1320
taaagggaaa gggcttttgc atctaatcac aatagagttg aaagagaggc cttaggattc 1380

tcctctctct aggtgctgag ccctcacctc cctgttccag gctgagaact caaatggtta 1440
ccctgcttct tcctacaatg ctgtgtgata tgggtgaacc cagcccctga ccttcctcta 1500
tcccctgccc atcctccctt ttacctcctc tctttttaa acacctgttt atcccaacct 1560
ttttgagctc aagctgtgat aaagaagggc ccatcctatt tcccctcatc tagtccattt 1620
acgattctca ctgactcccc gtcttcctgg cagacacaaa taaacccagt gtcaggtcta 1680
ggaaattaat ggctattctt ccccagatac attctggctt atttgagata catgattctc 1740
ttagaatcct gtcccttggt tcaggaaagt agcttggaaa aggagtaggg gtatagcttg 1800
ggtccctttt cctgcaaggc cccatggggc agaatataat aaatattctg agtgaggagt 1860
gtggtctttt tctgatcttc ctcagcttcc gtaagttgca gagtgaggta tattaggaga 1920
ctagttctac acaatattgt aatgctgggt tccatcaaca cccaccttcc acaactcagt 1980
ctgcacctca gttggcaaag gagactggat ggccatcttt cctcatgttc ccttgagtat 2040
ttcaatgtag aaagcccttc aagtggtatt atattttaac cttttacatt attgttatta 2100
atgttagtaa tatattgtta tgttttctaa attatttttc tttaagctga cgtggctttt 2160
tttctgtggc tcccagtggg tctacggacc ttggctgaca tatgttggta ggtactctgg 2220
tcagctcagc tggctgtcct ggttcactca gaagataagt ctctccaaag caaattcaca 2280
tgcattatga gtcgctttga gcttctgaca tgtcacttgc ccgaggtta aaacttttca 2340
ccccttgaag accttacatg ttttatggta ttggtgagga aggaaatgtt ctcaaggtct 2400
caggctattt gggaaattcc aactcctata ccttaccaga gcatggaaga gcccagatct 2460
gaatgtaaaa cgtctctgtt ctgccagaga tggaaaaaat acaggtatac ttgtgatata 2520
gtcatggggc ttcagtgtca ctattttctc cttaaagctc cagccaaaaa ctggacaagg 2580
atagagagga ggagggaaga acaaaagagc ccttctctat gaaccttgtg ccttctgtcc 2640
taccagtttt cttttacaga ttctcacttc tgctagccta gccagggctt actccaggaa 2700
tctaaataga tgccctagtc cactttatct ttgttcccaa ggcactcatt tttattttga 2760
ttttgattga atgtgagcag gttgacctca ggtcacactt tgttccaaaa acttttggaa 2820
ttattccagg acttgtggtg gagttatggt actctagggc agtctttctc aaactatgta 2880
tggtaaagga ccaggttttt tgttttccag tccttcactt atcaatatgc attcctattg 2940
ccgatgacag gtatggagtt cacactgtgt gctgccgacc cggcaagttt gacagcaccc 3000
aaactggcca gactgttctg taggttaagt ccattgatca tgtacttgga tatcacagca 3060
acattgaaat gctaaaaagt ttttaaacac tctcaatttc taattcacca tgtcacagac 3120
tggtgaaaaa aaaaaaaagg tgttcactga ccagcacaag tctgcagatc atctttgagt 3180
agcactgttt tggggccctc ggtctctctg aagaccctag cagaactgat acctacctgt 3240
atctcttgtt ctctcctatt tgagtttcac ttccagagaa cttgttcttc agcaagaatg 3300
tgtcactagt aaggacatct ctagcatttc tctagccttc cttttctgct gctcaaaaat 3360
aatcgttaca aagcttaggt ttaagctgta tatgaaatat ttatgcgact ctcaaacttt 3420
aaaggagttg ctcctttgtt ccaaaattaa atgtgttaga taaaaaaaaa aaaaaa 3476

<210> 319
<211> 1665
<212> DNA
<213> Homo sapiens

<400> 319
caacgacggc agccccgccg gctactacct gaaggagtcc aggggcagcc ggcggtggct 60
cctcttcctg gaaggcggct ggtactgctt caaccgcgag aactgcgact ccagatacga 120
caccatgcgg cgcctcatga gctcccggga ctggccgcgc actcgcacag gcacagggat 180
cctgtcctca cagccggagg agaacccta ctggtggaac gcaaacatgg tcttcatccc 240
ctactgctcc agtgatgttt ggagcggggc ttcatccaag tctgagaaga acgagtacgc 300

cttcatgggc gccctcatca tccaggaggt ggtgcgggag cttctgggca gagggctgag 360
cggggccaag gtgctgctgc tggccgggag cagcgcgggg ggcaccgggg tgctcctgaa 420
tgtggaccgt gtggctgagc agctggagaa gctgggctac ccagccatcc aggtgcgagg 480
cctggctgac tccggctggt tcctggacaa caagcagtat cgccacacag actgcgtcga 540
cacgatcacg tgcgcgccca cggaggccat ccgccgtggc atcaggtact ggaacggggt 600
ggtcccggag cgctgccgac gccagttcca ggagggcgag gagtggaact gcttctttgg 660
ctacaaggtc tacccgaccc tgcgctgccc tgtgttcgtg gtgcagtggc tgtttgacga 720
ggcacagctg acggtggaca acgtgcacct gacggggcag ccggtgcagg agggcctgcg 780
gctgtacatc cagaacctcg gccgcgagct gcgccacaca ctcaaggacg tgccggccag 840
ctttgccccc gcctgcctct cccatgagat catcatccgg agccactgga cggatgtcca 900
ggtgaagggg acgtcgctgc cccgagcact gcactgctgg gacaggagcc tccatgacag 960
ccacaaggcc agcaagaccc ccctcaaggg ctgccccgtc cacctggtgg acagctgccc 1020
ctggccccac tgcaacccct catgccccac cgtccgagac cagttcacgg ggcaagagat 1080
gaacgtggcc cagttcctca tgcacatggg cttcgacatg cagacggtgg cccagccgca 1140
gggactggag cccagtgagc tgctggggat gctgagcaac ggaagctagg cagactgtct 1200
ggaggaggag ccggcactga ggggcccaga cacccgctgc cccagtgcca cctcaccccc 1260
caccagcagg ccctcccgtc tcttcgggac agggccccag ccgtcccccc tgtctgggtc 1320
tgcccactgc cctcctgccc cggctttccc tgcccctctc ccacagccca gccagagaca 1380
agggacctgc tgtcatcccc atctgtggcc tgggggtcct tcctgacaac gagggggtag 1440
ccagaagaga agcactggat tcctcagtcc accagctcag acagcaccca ccggccccac 1500
ccatcaagcc cttttatatt attttataaa gtgacttttt tattacttta attttttaaa 1560
aaaaggaaaa taagaatata tgatgaatga tattgttttg taacttttta aaaatgattt 1620
taaagagaca aaaaagaaaa aaaaaaaaaa aaaaaaaaaa aaaaa 1665

<210> 320
<211> 1571
<212> DNA
<213> Homo sapiens

<400> 320
tgctgttgtg atggtggtaa gggaacgggc ctggctctgg cccctgacgc aggaacatgg 60
agctgatcca ggacacctcc cgcccgccac tggagtacgt gaagggggtc ccgctcatca 120
agtactttgc agaggcactg gggcccctgc agagcttcca ggcccggcct gatgacctgc 180
tcatcagcac ctaccccaag tccggcacca cctgggtgag ccagattctg gacatgatct 240
accagggtgg tgacctggag aagtgtcacc gagctcccat cttcatgcgg gtgcccttcc 300
ttgagttcaa agccccaggg attccctcag ggatggagac tctgaaagac acaccggccc 360
cacgactcct gaagacacac ctgcccctgg ctctgctccc ccagactctg ttggatcaga 420
aggtcaaggt ggtctatgtt gcccgcaacg caaaggatgt ggcagtttcc tactaccact 480
tctaccacat ggccaaggtg caccctgagc ctgggacctg ggacagcttc ctggagaagt 540
tcatggtcgg agaagtgtcc tacggatcct ggtaccagca cgtgcaggag tggtgggagc 600
tgagccgcac ccaccctgtt ctctacctct tctatgaaga catgaaggag aaccccaaaa 660
gggagattca aaagatcctg gagtttgtgg ggcactccct gccagaggag accgtggact 720
tcatggttca gcacacgtcg ttcaaggaga tgaagaagaa ccctatgacc aactacacca 780
ccgtccccca ggagttcatg gaccacagca tctcccccttcatgaggaaa ggcatggctg 840
gggactggaa gaccaccttc accgtggcgc agaatgagcg cttcgatgcg gactatgcgg 900
agaagatggc aggctgcagc ctcagcttcc gctctgagct gtgagagggg ctcctggggt 960
cactgcagag ggagtgtgcg aatcaaacct gaccaagcgg ctcaagaata aaatatgaat 1020

tgagggcctg ggacggtagg tcatgtctgt aatcccagca atttggaggc tgaggtggga 1080
ggatcatttg agcccaggag ttcgagacca acctgggcaa catagtgaga ttctgttaaa 1140
aaaataaaat aaaataaaac caatttttaa aaagagaata aaatatgatt gtgggccagg 1200
cagagtggct catgcctgta atcccagcaa tttgagaagt tgaggctaga ggatcactgg 1260
aggacaggag tttgggacca gcctgttcaa cattacaaga catcatccct acaaaaattt 1320
gagaaaatta tctgtacgtg atggtgggca cctgtagtcc caactacttg acaagtgaag 1380
gcaggaggat cgcctgagcc agggaggtta tggctgcagt tggctgactg ggctaatcca 1440
ctcaagcctg agggacagag caaatcttgc ttgagaaata aataaaatac aatttactta 1500
acataaatta tgattcagga ccagtctggc caacatggtg aaaccccgtc tctactaaaa 1560
aaaaaaaaaa a 1571

<210> 321
<211> 1549
<212> DNA
<213> Homo sapiens

<400> 321
gctgggtcct tcggcaggag gaggaagatg gagcccagca ccgcggcccg ggcttgggcc 60
ctcttttggt tgctgctgcc cttgcttggc gcggtttgcg ccagcggacc ccgcacctta 120
gtgctgctgg acaacctcaa cgtgcgggag actcattcgc ttttcttccg gagcctgaag 180
gaccggggct ttgagctcac attcaagacc gctgatgacc ccagcctgtc tctcataaag 240
tatggggaat tcctctatga caatctcatc attttctccc cttcggtaga agatttgga 300
ggcaacatca acgtggagac catcagtgcc tttattgacg gcggaggcag tgtgctggta 360
gctgccagct ccgacattgg tgaccctctt cgagagctgg gcagtgagtg cgggattgag 420
tttgacgagg agaaaacggc tgtcattgac catcacaact atgacatctc agaccttggc 480
cagcatacgc tcatcgtggc tgacactgag aacctgctga aggccccaac catcgttggg 540
aaatcatctc taaatcccat cctctttcga ggtgttggga tggtggccga tcctgataac 600
cctttggtgc tggacatcct gacgggctct tccacctctt actccttctt cccggacaag 660
cctatcaccc agtatccaca tgcggtgggg aagaacaccc tcctcattgc tgggctccag 720
gccaggaaca atgcccgcgt catcttcagc ggctccctcg acttcttcag cgactccttc 780
ttcaactcag cagtgcagaa ggcggcgccc ggctcccaga ggtattccca gacaggcaac 840
tatgaactag ctgtggccct ctcccgctgg gtgttcaagg aggagggtgt cctccgtgtg 900
gggcctgtgt cccatcatcg ggtgggcgag acagccccac ccaatgccta cactgtcact 960
gacctagtgg agtatagcat cgtgatccag cagctctcaa atggcaaatg ggtccccttt 1020
gatggcgatg acattcagct ggagtttgtc cgcattgatc cttttgtgag gaccttcctg 1080
aagaagaaag gtggcaaata cagtgttcag ttcaagttgc ccgacgtgta tggtgtattc 1140
cagtttaaag tggattacaa ccggctaggc tacacacacc tgtactcttc cactcaggta 1200
tccgtgcggc cactccagca cacgcagtat gagcgcttca tcccctcggc ctacccctac 1260
tacgccagcg ccttctccat gatgctgggg ctcttcatct tcagcatcgt cttcttgcac 1320
atgaaggaga aggagaagtc cgactgaggg gctagagccc tctccgcaca gcgtggagac 1380
ggggcaggga ggggggttat taggattggt ggttttgttt tgctttgttt aaagccgtgg 1440
gaaaatggca caactttacc tctgtgggag atgcaacact gagagccaag ggtgggagt 1500
tgggataatt tttatataaa agaagttttt ccactttgaa ttgctaaaa 1549

<210> 322
<211> 2064

<212> DNA
<213> Homo sapiens

<400> 322
ctttgctccc ctgccgcctt ctagtgggcc gcctggtggt ggacttgtcc tgtgggatgg 60
agaccctcca tacatggggt tcaaaggtcc tggggtattc ctggatcttc aggacatctg 120
cctaccctca ggtttcccag gcctcgggtg gggaggcatc agatccctgg ccaacctgct 180
atccacccca gggtttagac ctctcttccc gtgagggaac agagtagtag cttcatttgt 240
ggcagattat ctgccttgtg ataaacttta gtggcctccc tttctgtttt gccataatct 300
gcctcctccc ccactcccat acccatctac aaagcagcct tgaaaataac cacccctgtg 360
gttcagtgtg ggtcatgatc tgtcaatcca ggcttagtac atttccaaat ggaattacca 420
gaaggatggg catcaaactg ggaaaatgat ttggtggctt gctagccgtg ttcctttttc 480
tcccaaggca ttcgttcaga tgtgcagtgt tggcagaccc aggggatgcc atttacctgg 540
cccactggtc gagatttggt catcccagga acttgggctt cagaaaagat gtttctgggc 600
tttggaccag attctggctg gtctgtggtt tgcccctcct gccacaccca gtggctttca 660
ttgggctttt ttcctctagt tattgtcagg aacagtgggg aatggcaggt attgcatttg 720
gtgaacacac tggataactg gaatcagagc cgtgtgactg agggaagagg ccttcacagc 780
ttcaatgcag aatgggatga ttatctgccc tgaaacgaag gatgcttctt tgaggggagt 840
tgggaccaat actgggaaca gcaccgctcc tggcttctct ggctttgctt ctctctgaag 900
taatttagac ttcgtgaacc ttacatactg gctccatctg tatattggga gagcctgtcc 960
ctctcagcct cacagtagtg aagttaggaa gcatggagtc cacagtgttc tttgaaagaa 1020
tcaaggcacc aagtccatgc atttacattt ctctcttgag atcagtcctt ttgttagcag 1080
cctgcaattc tgaccacggc aaccaaaaat ttctgaccgt ggcaaccaaa aatcaagcgc 1140
gaggaaacct gggggtttat ttggaggtgg gagaggatgt cccttcttct ggtaggacta 1200
tgtctggcta cattgtcttt ccattttttt ctgattgtcc ccttgccccc ctccctttc 1260
tgggggcaca gagggcatgg caattttgtt tcctagcttt ccatattcct actgcagtgc 1320
tttcttcaga atacttgttc agatcggatt cttggctgtg agggcaacca gctgctttcc 1380
tgtttcttta aaagtggatc ccacactgcc gtgtcctgca cagtgagttc tgcgttgtag 1440
aaaccatcta gcagtggtga ctcagggagt ggcctctgct cggtatgggg cctctgcaac 1500
atgaggttat ggggcttcta cgctgtgggg tcaaaggaga catcatgatc cctactggca 1560
gaaagagcag agccccagag tgggttccac ttacggggtc agtgtctttt ctgagatatt 1620
cctcggaacc acatttaaat tctttttcat attgtttgca taataattgc cttctagtgc 1680
ctactttata ggactgagag gatttaaatg agataatcca tgttatggat ttaacacagc 1740
ctctggcaca agtctaaatg ttgcatgtaa gtgttaacta ttatactgga aagaaggctc 1800
agttccttga tttaggtgtg ggagaaaaat atatatatat tttgagacca gccctggcca 1860
acatggtgaa actcatgtct acgaaaaata caaaagttag cctggtgttg gtggcgcatg 1920
cctatagttg cagctactcg ggaggctgag acacaggaat cacttgaacc cgggaggcgg 1980
aggttgcagg gagccgagat cacaccactg cactccagcc tgggtaacag agtgagatac 2040
tgtctcaaaa aaaaaaaaaa aaaa 2064

<210> 323
<211> 1317
<212> DNA
<213> Homo sapiens

<400> 323
gggtaatctc tactttccta tactgccaaa gaatgtgagg aagaaatggg actctttggt 60

tatttattga tgcgactgta aattggtaca gtatttctgg agggcaattt ggtaaaatgc 120
atcaaaagac ttaaaaatac ggacgtactt tgtgctggga actctacatc tagcaatttc 180
tctttaaaac catatcagag atgcatacaa agaattatat ataaagaagg gtgtttaata 240
atgatagtta taataataaa taattgaaac aatctgaatc ccttgcaatt ggaggtaaat 300
tatgtcttag ttataattag attgtgaatc agccaactga aaatcctttt tgcatatttc 360
aatgtcctaa aaagacacgg ttgctctata tatgaagtga aaaaaggata tggtagcatt 420
ttatagtact agttttgctt taaaatgcta tgtaaatata caaaaaaact agaaagaaat 480
atatataacc ttgttattgt atttggggga gggatactgg gataattttt attttctttg 540
aatctttctg tgtcttcaca tttttctaca gtgaatttaa tcaaatagta aagttgttgt 600
aaaaataaaa gtggatttag aaagatccag ttcttgaaaa cactgtttct ggtaatgaag 660
cagaatttaa gttggtaata ttaaggtgaa tgtcatttaa gggagttaca tctttattct 720
gctaaagaag aggatcattg atttctgtac agtcagaaca gtacttgggt ttgcaacagc 780
tttctgagaa aagctaggtg tttaatagtt taactgaaag tttaactatt taaaagacta 840
aatgcacatt ttatggtatc tgatatttta aaaagtaatg tttgattctc ctttttatga 900
gttaaattat tttatacgag ttggtaattt ttgcttttta ataaagtgga agcttgcttt 960
tttaactctt tttttattgt tattttatag aaatgctttt tgttggccgg gcacagttgc 1020
tcatccatgt aatcccagca ctgtgggagg ccgagacggg tggatcacaa ggtcaggaga 1080
tcgagaccat cctggctaat gcgttgaaac tccgtctctg ctaagaatac aaaaaattag 1140
ctgggcgtgg tggtgggcac ctgtagtccc agctactcag gaggctgagg caggagaatg 1200
gtgtgaacct gggaggtgga gcttgcagtg agcagagctt gcagtgagac gagcttgtgc 1260
cactgcactc cagcctgggc aacagagtaa gactcagtct caaaaaaaaa aaaaaaa 1317

<210> 324
<211> 1483
<212> DNA
<213> Homo sapiens

<400> 324
ttttcaatct ttttgatctc acgaccactt tttaaaaaat gtatatcttt ttttgtgtgt 60
tgttcttgtt gttgttgttg tttgagacag ggtcttgctc tgtcgcccag gctggagttc 120
agtggcacta acgtggctca tgccgcctca acctcctaga ctcaagtgac cctccctcct 180
cagcctccca agtatctggg atcaaaggtg tgaaccacca ggcctggcta attttttttt 240
atttttgta gagatgacat ttctccatgt tgcccaggct ggtttcaaac tcctgggctc 300
aagcaagcct caccctccca aagtggtggg attacaagta tgagccacca cacctggcca 360
ggaccactgg acactcttaa aaatgatgaa agacttcatg gagaagcttg tattcatgtg 420
gattatagct atcaattgtt actgtattag acatttaaaa gaaaatagaa atgaggaaaa 480
tgaatttatt taaaaatagt aaacccaaat acatgtgaac ataaatcaca tttttatgaa 540
aagatcacta ttttccacaa ttaaaaattg aggtttatgt agtttttgag atctctttaa 600
tgtctggcat aatagaatac agctggattc tcatgtatgc ttctgcacaa tcttttgtga 660
tttttttttc tttttttttg agacggagtc ttgctctgtc acccaggctg gagtgcagtg 720
gtgccatctc agctcagtgc aacctccact gccggggttc aagcgattat cctgcctccc 780
gcatgaaatg gggttttgcc atgttggcca ggcttgtctc gaactcctga cctcaggtga 840
gccactgtgt ccggccctgt tgtggtatgt tgaagtatat aaagaaaatc caggttcaca 900
cggatacata tttttaaatg gagaaatatt ggaatagcct tttcagataa ttatggatat 960
tcttctttga tactgtacaa aatttggcaa gtgatagttc cttcaaagat agctgcagtg 1020
tggaatctga aatcctatca aggaagtttt tgtactctgt taaactaaaa atccactgat 1080
ccgttttaca ctttatttat gtgaagagtg gtaactttta cccatgcatg attttgtaat 1140

atcctgtatt ggtcatttgg aaaatactgg tttactgaat tatgcagttc ttccaaatat 1200
tgacacattt ctttatataa atatcaagtc acatttgtta atatcacaac tgatatcaga 1260
aaagtcccat ggtaaatgca aatttcacaa aattctaatg tttccttgga atcttgaatt 1320
ttatcattgg caacaaatac agttttcctt gaaatgacag cctcactttt tttaattttt 1380
gagtgagaag atacctgcca aatattcaag tctgaatggc gattgcttac cagtcattct 1440
ttcatgttaa aatggtcttc ctccatttaa aaaaaaaaaa aaa 1483

<210> 325
<211> 1067
<212> DNA
<213> Homo sapiens

<400> 325
gacggtcatc gattacaacg gggaacgcac gctggatggt tttaagaaat tcctggagag 60
cggtggccag gatggggcag gggatgatga cgatctcgag gacctggaag aagcagagga 120
gccagacatg gaggaagacg atgatcagaa agctgtgaaa gatgaactgt aatacgcaaa 180
gccagacccg ggcgctgccg agacccctcg ggggctgcac acccagcagc agcgcacgcc 240
tccgaagcct gcggcctcgc ttgaaggagg gcgtcgccgg aaacccaggg aacctctctg 300
aagtgacacc tcacccctac acaccgtccg ttcacccccg tctcttcctt ctgcttttcg 360
gtttttggaa agggatccat ctccaggcag cccaccctgg tggggcttgt ttcctgaaac 420
catgatgtac tttttcatac atgagtctgt ccagagtgct tgctaccgtg ttcggagtct 480
cgctgcctcc ctcccgcggg aggtttctcc tctttttgaa aattccgtct gtgggatttt 540
tagacatttt tcgacatcag ggtatttgtt ccaccttggc caggcctcct cggagaagct 600
tgtcccccgt gtgggaggga cggagccgga ctggacatgg tcactcagta ccgcctgcag 660
tgtcgccatg actgatcatg gctcttgcat ttttgggtaa atggagactt ccggatcctg 720
tcagggtgtc ccccatgcct ggaagaggag ctggtggctg ccagccctgg gtcccggcac 780
aggcctgggc cttccccttc cctcaagcca gggctcctcc tcctgtcgtg ggctcattgt 840
gaccactggc ctctctacag cacggcctgt ggcctgttca aggcagaacc acgacccttg 900
actcccgggt ggggaggtgg ccaaggatgc tggagctgaa tcagacgctg acagttcttc 960
aggcatttct atttcacaat cgaattgaac acattggcca aataaagttg aaattttccc 1020
ccccaaaaaa aaaaaaaaaa aaaaaaaaaa aaaaaaaaaa aaaaaaa 1067

<210> 326
<211> 915
<212> DNA
<213> Homo sapiens

<400> 326
aggagccgca gggccgtagg cagccatggc gcccagccgg aatggcatgg tcttgaagcc 60
ccacttccac aaggactggc agcggcgcgt ggccacgtgg ttcaaccagc cggcccgtaa 120
gatccgcaga cgtaaggccc ggcaagccaa ggcgcgccgc atcgccccgc gccccgcgtc 180
gggtcccatc cggcccatcg tgcgctgccc cacggttcgg taccacacga aggtgcgcgc 240
cggccgcggc ttcagcctgg aggagctcag ggtggccggc attcacaaga aggtggcccg 300
gaccatcggc atttctgtgg atccgaggag gcggaacaag tccacggagt ccctgcaggc 360
caacgtgcag cggctgaagg agtaccgctc caaactcatc ctcttcccca ggaagccctc 420
ggcccccaag aagggagaca gttctgctga agaactgaaa ctggccaccc agctgaccgg 480

accggtcatg cccgtccgga acgtctataa gaaggagaaa gctcgagtca tcactgagga 540
agagaagaat ttcaaagcct tcgctagtct ccgtatggcc cgtgccaacg cccggctctt 600
cggcatacgg gcaaaaagag ccaaggaagc cgcagaacag gatgttgaaa agaaaaaata 660
aagccctcct ggggacttgg aatcagtcgg cagtcatgct gggtctccac gtggtgtgtt 720
tcgtgggaac aactgggcct gggatggggc ttcactgctg tgacttcctc ctgccagggg 780
atttggggct ttcttgaaag acagtccaag ccctggataa tgctttactt tctgtgttga 840
agcactgttg gttgtttggt tagtgactga tgtaaaacgg ttttcttgtg gggaaaaaaa 900
aaaaaaaaaa aaaaa 915

<210> 327
<211> 2338
<212> DNA
<213> Homo sapiens

<400> 327
agcgcacgtc ggcagtcggc tccctcgttg accgaatcac cgacctctct ccccagctgt 60
atttccaaaa tgtcgctttc taacaagctg acgctggaca agctggacgt taaagggaag 120
cgggtcgtta tgagagtcga cttcaatgtt cctatgaaga acaaccagat aacaaacaac 180
cagaggatta aggctgctgt cccaagcatc aaattctgct tggacaatgg agccaagtcg 240
gtagtcctta tgagccacct aggccggcct gatggtgtgc ccatgcctga caagtactcc 300
ttagagccag ttgctgtaga actcaaatct ctgctgggca aggatgttct gttcttgaag 360
gactgtgtag gcccagaagt ggagaaagcc tgtgccaacc cagctgctgg gtctgtcatc 420
ctgctggaga acctccgctt tcatgtggag gaagaaggga agggaaaaga tgcttctggg 480
aacaaggtta aagccgagcc agccaaaata gaagctttcc gagcttcact ttccaagcta 540
ggggatgtct atgtcaatga tgcttttggc actgctcaca gagcccacag ctccatggta 600
ggagtcaatc tgccacagaa ggctggtggg tttttgatga agaaggagct gaactacttt 660
gcaaaggcct tggagagccc agagcgaccc ttcctggcca tcctgggcgg agctaaagtt 720
gcagacaaga tccagctcat caataatatg ctggacaaag tcaatgagat gattattggt 780
ggtggaatgg cttttacctt ccttaaggtg ctcaacaaca tggagattgg cacttctctg 840
tttgatgaag agggagccaa gattgtcaaa gacctaatgt ccaaagctga gaagaatggt 900
gtgaagatta ccttgcctgt tgactttgtc actgctgaca agtttgatga gaatgccaag 960
actggccaag ccactgtggc ttctggcata cctgctggct ggatgggctt ggactgtggt 1020
cctgaaagca gcaagaagta tgctgaggct gtcactcggg ctaagcagat tgtgtggaat 1080
ggtcctgtgg gggtatttga atgggaagct tttgcccggg gaaccaaagc tctcatggat 1140
gaggtggtga aagccacttc taggggctgc atcaccatca taggtggtgg agacactgcc 1200
acttgctgtg ccaaatggaa cacggaggat aaagtcagcc atgtgagcac tgggggtggt 1260
gccagtttgg agctcctgga aggtaaagtc cttcctgggg tggatgctct cagcaatatt 1320
tagtactttc ctgcctttta gttcctgtgc acagccccta agtcaactta gcattttctg 1380
catctccact tggcattagc taaaaccttc catgtcaaga ttcagctagt ggccaagaga 1440
tgcagtgcca ggaaccctta aacagttgca cagcatctca gctcatcttc actgcaccct 1500
ggatttgcat acattcttca agatcccatt tgaatttttt agtgactaaa ccattgtgca 1560
ttctagagtg catatattta tattttgcct gttaaaaaga aagtgagcag tgttagctta 1620
gttctctttt gatgtaggtt attatgatta gctttgtcac tgtttcacta ctcagcatgg 1680
aaacaagatg aaattccatt tgtaggtagt gagacaaaat tgatgatcca ttaagtaaac 1740
aataaaagtg tccattgaaa ccgtgatttt tttttttttc ctgtcatact ttgttaggaa 1800
gggtgagaat agaatcttga ggaacggatc agatgtctat attgctgaat gcaagaagtg 1860
gggcagcagc agtggagaga tgggacaatt agataaatgt ccattcttta tcaagggcct 1920

actttatggc agacattgtg ctagtgcttt tattctaact tttattttta tcagttacac 1980
atgatcataa tttaaaaagt caaggcttat aacaaaaaag ccccagccca ttcctcccat 2040
tcaagattcc cactccccag aggtgaccac tttcaactct tgagtttttc aggtatatac 2100
ctccatgttt ctaagtaata tgcttatatt gttcacttcc ttttttttta ttttttaaag 2160
aaatctattt cataccatgg aggaaggctc tgttccacat atatttccac ttcttcattc 2220
tctcggtata gttttgtcac aattatagat tagatcaaaa gtctacataa ctaatacagc 2280
tgagctatgt agtatgctat gattaaattt acttatgtaa aaaaaaaaaa aaaaaaa 2338

<210> 328
<211> 2519
<212> DNA
<213> Homo sapiens

<400> 328
gcgtgtcagg tggttgcgga gctgaatcat attctaagaa ctcagccact caggtatcca 60
ccatggtgct gggtcctgaa cagaagatgt cagatgacag tgtttctgga gatcatgggg 120
agtctgccag tcttggtaac atcaaccctg cctatagtaa tccctctctt tcacagtccc 180
ctggggactc agaggagtac ttcgccactt actttaatga gaagatctcc attcctgagg 240
aggagtactc ttgttttagc tttcgtaaac tctgggcttt caccggacca ggttttctta 300
tgagcattgc ctacctggat ccaggaaata ttgaatccga tttgcagtct ggagcagtgg 360
ctggatttaa gttgctctgg atccttctgt tggccacccct tgtggggctg ctgctccagc 420
ggcttgcagc tagactggga gtggttactg ggctgcatct tgctgaagta tgtcaccgtc 480
agtatcccaa ggtcccacga gtcatcctgt ggctgatggt ggagttggct atcatcggct 540
cagacatgca agaagtcatt ggctcagcca ttgctatcaa tcttctgtct gtaggaagaa 600
ttcctctgtg ggtggcgtt ctcatcacca ttgcagatac ttttgtattt ctcttcttgg 660
acaaatatgg cttgcggaag ctagaagcat tttttggctt tctcatcact attatggccc 720
tcacatttgg atatgagtat gttacagtga aacccagcca gagccaggta ctcaagggca 780
tgttcgtacc atcctgttca ggctgtcgca ctccacagat tgaacaggct gtgggcatcg 840
tgggagctgt catcatgcca cacaacatgt acctgcattc tgccttagtc aagtctagac 900
aggtaaaccg gaacaataag caggaagttc gagaagccaa taagtacttt ttcattgaat 960
cctgcattgc actctttgtt tccttcatca tcaatgtctt tgttgtctca gtctttgctg 1020
aagcattttt tgggaaaacc aacgagcagg tggttgaagt ctgtacaaat accagcagtc 1080
ctcatgctgg cctctttcct aaagataact cgacactggc tgtggacatc tacaaagggg 1140
gtgttgtgct gggatgttac tttgggcctg ctgcactcta catttgggca gtggggatcc 1200
tggctgcagg acagagctcc accatgacag gaacctattc tggccagttt gtcatggagg 1260
gattcctgaa cctaaagtgg tcacgctttg cccgagtggt tctgactcgc tctattgcca 1320
tcatccccac tctgcttgtt gctgtcttcc aagatgtaga gcatctaaca gggatgaatg 1380
actttctgaa tgttctacag agcttacagc ttccctttgc tctcataccc atcctcacat 1440
ttacgagctt gcggccagta atgagtgact ttgccaatgg actaggctgg cggattgcag 1500
gaggaatctt ggtccttatc atctgttcca tcaatatgta ctttgtagtg gtttatgtcc 1560
gggacctagg gcatgtggca ttatatgtgg tggctgctgt ggtcagcgtg gcttatctgg 1620
gctttgtgtt ctacttgggt tggcaatgtt tgattgcact gggcatgtcc ttcctggact 1680
gtgggcatac gtgccatctg ggattgacag ctcagcctga actctatctt ctgaacacca 1740
tggacgctga ctcacttgtg tctagatgac tgacagcctg agagactcta taagaacatg 1800
tttttctaag ccctttttgt gccaggtgtc ccgttaacgt ctctgttagt tcaaaggtga 1860
gttttgttca gacgttttga acaaaaggca aagatttcct catgggaagg gtgttcaaaa 1920
ctgacagcta taaatgtagg tcagagaccc acccacctca taacagtcat acactcccag 1980

agttaaacga ttggcctctg atggccacac acccctatgg gcttgtgtct tggactctgg 2040
gatttaaaag atatatatgt atatatattt atgtaaacct gagcatctca gtttgggtag 2100
agttttaagg ttatatgaaa ctgatagaac ttttgtattt tttttttaaa tattgttttg 2160
atatatcaga tatttgtttt gtctggatca caagtgagaa agaaaagaga ataatgctct 2220
ttttcacaat gaactggtca aattgactat cttgtaaagt gatactttac tatgtcagtg 2280
aaatttcagt ttgattttag tcaaccagat tatatccttt gtatctactg atattaacac 2340
atcatattaa cacatcattt cttaaaaaaa catttcttct gtttggcaat cataattaac 2400
tctggttatc agcctatttt gtaattattg tctttgtcgt cacttttctt gaattgtttt 2460
ctagtcatta aacagatatg aaggcaaaaa aaaaaaaaaa aaaaaaaaaa aaaaaaaa 2519

<210> 329
<211> 1623
<212> DNA
<213> Homo sapiens

<400> 329
cccccagacc attatggcct ccctgcagtt tgtgatctct ttgcctgtat gcagcttaaa 60
gttgataaag agaagtggtt atatcgaact cctctacaga tgtgaaggaa tggacaagag 120
ttgagcagcc tttctgctga ttatcacaca tcatgagctg agtgactgca gcttgccaaa 180
tctttgtgtt tctgggtctg accaattagc ttagttcttc tcctgcctaa ttttgaacta 240
gtaaagcaaa gtgagtcatc agattatgag ttactgttta aaagaaaaat gctgtttatt 300
catgctgagg tgattcagtt ccctccttct tacagaagta ttttaattca ccccacacta 360
gaaatgcagc atctttgtgg acgtcttttt cacaagcctc caaggctcct tagattgggt 420
cgttactaaa agtacattaa aacactcttg tttatcgaag tatattgatg tattctaaag 480
ctagtaaact tccctaacgt ttaattgccc tacagatgct tctcttgctg tgggttttct 540
tttgttagtg gtctgaaata attattttcc tgttctatta atacatagtg tattttgcac 600
aaaaaaatta acctggtcaa tagtgattac caaaatatat attaataatc ttggcaattt 660
ttgacattaa ttatgaaaca ttttagccca cgttagttct acattattct tcacttaaac 720
tcagctactg caaattttgt ctttctgtaa atgttattaa aatatccagt gagctcttta 780
gaaggactca gtattatttc aagactattt ttgaggtaat tctagccttt taaaatattc 840
tacagaccta cggggcttaa aagaacccca gtaccgacta agcaaatagg caaaagacat 900
gttggaaatg tagtatagta cttgaaacag tcactatcat agggataatt ggtgcatcct 960
gtgtaaatgg aagctgagct tgacacctgg tgcttttaag tagggataaa gtcatcctct 1020
cactgcaagc acagcatacc tgtacctcca aaagtgacgt tttagtgaac aggccgtttt 1080
caacacttgt gccttggggt gttcattgaa gctttgtgaa aactactgat gttttctcag 1140
tctccttaaa gttacgtcca tgctttaaaa tgtctgtgta ggagagaagt ggggtttata 1200
atgttttctc taagatatct ttgctgcttt ccagactttg aaactattaa gcttcttaac 1260
tgcctcttac cggaaatact tctggggaaa cttcatggtc ccaaaatgtc attgccatac 1320
agcttcacta gagttctttg aaccacagct gaaaagagct ttgtattatt ttttaattcc 1380
ctccccagat atcatttagg agtattatat aaaggtggtg ggcaaaaaca atgtaaggag 1440
cctttccagt tatcttgagt tgcagctctg tagtttcttg aggccaaaca cactgtattt 1500
tacaagtcaa aatataattt acattaatca ctatgttaat gagtatgtaa aacattcttt 1560
tgcattgatg aattttgtat ctgcttccat taaaagcata acagccataa aaaaaaaaaa 1620
aaa 1623

<210> 330

<211> 3379
<212> DNA
<213> Homo sapiens

<400> 330
ctggcctggg cctgaagtga gtgagaggca catgaagaga agtattcaag tatttataca 60
gataggaatc aagataatca acaatgtctg tcactgagga agacctgtgc caccatatga 120
aagtagtagt tcgtgtacgt ccggaaaaca ctaaagaaaa agcagctgga tttcataaag 180
tggttcatgt tgtggataaa catatcctag tttttgatcc caaacaagaa gaagtcagtt 240
ttttccatgg aaagaaaact acaaatcaaa atgttataaa gaaacaaaat aaggatctta 300
aatttgtatt tgatgctgtt tttgatgaaa cgtcaactca gtcagaagtt tttgaacaca 360
ctactaagcc aattcttcgt agttttttga atggatataa ttgcacagta cttgcctatg 420
gtgccactgg tgctgggaag acccacacta tgctaggatc agctgatgaa cctggagtga 480
tgtatctaac aatgttacac ctttacaaat gcatggatga gattaaagaa gagaaaatat 540
gtagtactgc agtttcatat ctggaggtat ataatgaaca gattcgtgat ctcttagtaa 600
attcagggcc acttgctgtc cgggaagata cccaaaaagg ggtggtcgtt catggactta 660
ctttacacca gcccaaatcc tcagaagaaa ttttacattt attggataat ggaaacaaaa 720
acaggacaca acatcccact gatatgaatg ccacatcttc tcgttctcat gctgttttcc 780
aaatttactt gcgacaacaa gacaaaacag caagtatcaa tcaaaatgtc cgtattgcca 840
agatgtcact cattgacctg gcaggatctg agcgagcaag tacttccggt gctaagggga 900
cccgatttgt agaaggcaca aatattaata gatcactttt agctcttggg aatgtcatca 960
atgccttagc agattcaaag agaaagaatc agcatatccc ttacagaaat agtaagctta 1020
ctcgcttgtt aaaggattct cttggaggaa actgtcaaac tataatgata gctgctgtta 1080
gtccttcctc tgtattctac gatgacacat ataacactct taagtatgct aaccgggcaa 1140
aggacattaa atcttctttg aagagcaatg ttcttaatgt caataatcat ataactcaat 1200
atgtaaagat ctgtaatgag cagaaggcag agattttatt gttaaaagaa aaactaaaag 1260
cctatgaaga acagaaagcc ttcactaatg aaaatgacca agcaaagtta atgatttcaa 1320
accctcagga aaaagaaatc gaaaggtttc aagaaatcct gaactgcttg ttccagaatc 1380
gagaagaaat tagacaagaa tatctgaagt tggaaatgtt acttaaagaa aatgaactta 1440
aatcattcta ccaacaacag tgccataaac aaatagaaat gatgtgttct gaagacaaag 1500
tagaaaaggc cactggaaaa cgagatcata gacttgcaat gttgaaaact cgtcgctcct 1560
acctggagaa aaggagggag gaggaattga agcaatttga tgagaatact aattggctcc 1620
atcgtgtcga aaaagaaatg ggactcttaa gtcaaaacgg tcatattcca aaggaactca 1680
agaaagatct tcattgtcac catttgcacc tccagaacaa agatttgaaa gcacaaatta 1740
gacatatgat ggatctagct tgtcttcagg aacagcaaca caggcagact gaagcagtat 1800
tgaatgcttt acttccaacc ctaagaaaac aatattgcac attaaaagaa gccggcctgt 1860
caaatgctgc ttttgaatct gacttcaaag agatcgaaca tttggtagag aggaaaaaag 1920
tggtagtttg ggctgaccaa actggcgaac aaccaaagca aaacgatcta cccgggattt 1980
ctgttcttat gacctttca caacttggac cagttcagcc tattccttgt tgctcatctt 2040
caggtggaac taatctggtt aagattccta cagaaaaaag aactcggaga aaactaatgc 2100
catctccctt gaaaggacag catactctaa agtctccacc atctcaaagt gtgcagctca 2160
atgattctct tagcaaagaa cttcagccta ttgtatatac accagaagac tgtagaaaag 2220
cttttcaaaa tccgtctaca gtaaccttaa tgaaaccatc atcatttact acaagttttc 2280
aggctatcag ctcaaacata aacagtgata attgtctgaa aatgttgtgt gaagtagcta 2340
tccctcataa tagaagaaaa gaatgtggac aggaggactt ggactctaca tttactatat 2400
gtgaagacat caagagctcg aagtgtaaat tacccgaaca agaatcacta ccaaatgata 2460
acaaagacat tttacaacgg cttgatcctt cttcattctc aactaagcat tctatgcctg 2520
taccaagcat ggtgccatcc tacatggcaa tgactactgc tgccaaaagg aaacggaaat 2580

taacaagttc tacatcaaac agttcgttaa ctgcagacgt aaattctgga tttgccaaac 2640
gtgttcgaca agataattca agtgagaagc acttacaaga aaacaaacca acaatggaac 2700
ataaaagaaa catctgtaaa ataaatccaa gcatggttag aaaatttgga agaaatattt 2760
caaaaggaaa tctaagataa atcacttcaa aaccaagcaa aatgaagttg atcaaatctg 2820
cttttcaaag tttatccaat accctttcaa aaatatattt aaaatctttg aaagaagacc 2880
catcttaaag ctaagtttac ccaagtactt tcagcaagca gaaaaatgaa actctttgtt 2940
ttcttctttt gtgttctaaa aaaataaaat ttcaaaagaa aaggttgtct tttaagtttt 3000
ttaaatattt gttgcctttt aaaatccctg agtgtaagtt accatggtgg cagcttagtt 3060
ttactatgcc acaacaagtt gactaggaca ttttagtaaa tggtagtgag ttaaattatc 3120
tttattattt tttaaaaata agaatttaga agtggtaaaa ttatggccca agatgtattt 3180
ggttctctat tatgttttga tacattattt taatcatata tatgactttc cttttcaaaa 3240
atactttaat gtacaagtgt aaatatatgt gcccataaaa tcattgtaaa tattatttag 3300
tcatcacaaa taaaatattg tcccttgcta cttgatatat taaagatgta gattttaaag 3360
tgaaaaaaaa aaaaaaaaa 3379

<210> 331
<211> 964
<212> DNA
<213> Homo sapiens

<400> 331
tctcaaccct gtgagagtcc atattgagat tggcccagat ggaagagtga cgggtgaagc 60
agatgttgag tttgctactc atgaagaagc tgtggcagct atgtccaaag acagggccaa 120
tatgcagcac agatatatag aactcttctt gaattcaaca acaggggcca gcaatggggc 180
gtatagcagc caggtgatgc aaggcatggg ggtgtctgct gcccaggcca cttacagtgg 240
cctggagagc cagtcagtga gtggctgtta cggggccggc tacagtgggc agaacagcat 300
gggtggctat gactagtttt gttaggaaca tttgagttac ttcaatcatt ttcacaggca 360
gccaacaagc aattaagagc agttataata gaggaagctg ggggacccat tttgcaccat 420
gagtttgtga aaaatctgga ttaaaaaatt acctcttcag tgttttctca tgcaaaattt 480
tcttctagca tgtgataatg agtaaactaa aactattttc agcttttctc aattaacatt 540
ttggtagtat acttcagagt gatgttatct aagtttaagt agtttaagta tgttaaatgt 600
ggatctttta caccacatca cagtgaacac actggggaga cgtgcttttt tggaaaactc 660
aaaggtgcta gctccctgat tcaaagaaat atttctcatg tttgttcatt ctagtttata 720
ttttcattta aaatccttta ggttaagttt aagcttttta aaagttagtt ttgagaattg 780
agacacaata ctaatactgt aggaattggt gaggccttga cttaaaactt tctttgtact 840
gtgatttcct tttgggtgta ttttgctaag tgaaacttgt taaatttttt gttaactaaa 900
tttttttctt aaaataaaga ctttttcaca aaaaaaaaaa aaaaaaaaaa aaaaaaaaaa 960
aaaa 964

<210> 332
<211> 1937
<212> DNA
<213> Homo sapiens

<400> 332
gcctcccgct cgccctgaac ccagtgcctg cagccatggc tcccggccag ctcgccttat 60

ttagtgtctc tgacaaaacc ggccttgtgg aatttgcaag aaacctgacc gctcttggtt 120
tgaatctggt cgcttccgga gggactgcaa aagctctcag ggatgctggt ctggcagtca 180
gagatgtctc tgagttgacg ggatttcctg aaatgttggg gggacgtgtg aaaactttgc 240
atcctgcagt ccatgctgga atcctagctc gtaatattcc agaagataat gctgacatgg 300
ccagacttga tttcaatctt ataagagttg ttgcctgcaa tctctatccc tttgtaaaga 360
cagtggcttc tccaggtgta actgttgagg aggctgtgga gcaaattgac attggtggag 420
taaccttact gagagctgca gccaaaaacc acgctcgagt gacagtggtg tgtgaaccag 480
aggactatgt ggtggtgtcc acggagatgc agagctccga gagtaaggac acctccttgg 540
agactagacg ccagttagcc ttgaaggcat tcactcatac ggcacaatat gatgaagcaa 600
tttcagatta tttcaggaaa cagtacagca aaggcgtatc tcagatgccc ttgagatatg 660
gaatgaaccc acatcagacc cctgcccagc tgtacacact gcagcccaag cttcccatca 720
cagttctaaa tggagcccct ggatttataa acttgtgcga tgctttgaac gcctggcagc 780
tggtgaagga actcaaggag gctttaggta ttccagccgc tgcctctttc aaacatgtca 840
gcccagcagg tgctgctgtt ggaattccac tcagtgaaga tgaggccaaa gtctgcatgg 900
tttatgatct ctataaaacc ctcacaccca tctcagcggc atatgcaaga gcaagagggg 960
ctgataggat gtcttcattt ggtgattttg ttgcattgtc cgatgtttgt gatgtaccaa 1020
ctgcaaaaat tatttccaga gaagtatctg atggtataat tgccccagga tatgaagaag 1080
aagccttgac aatactttcc aaaaagaaaa atggaaacta ttgtgtcctt cagatggacc 1140
aatcttacaa accagatgaa aatgaagttc gaactctctt tggtcttcat ttaagccaga 1200
agagaaataa tggtgtcgtc gacaagtcat tatttagcaa tgttgttacc aaaaataaag 1260
atttgccaga gtctgccctc cgagacctca tcgtagccac cattgctgtc aagtacactc 1320
agtctaactc tgtgtgctac gccaagaacg ggcaggttat cggcattgga gcaggacagc 1380
agtctcgtat acactgcact cgccttgcag gagataaggc aaactattgg tggcttagac 1440
accatccaca agtgctttcg atgaagttta aaacaggagt gaagagagca gaaatctcca 1500
atgccatcga tcaatatgtg actggaacca ttggcgagga tgaagatttg ataaagtgga 1560
aggcactgtt tgaggaagtc cctgagttac tcactgaggc agagaagaag gaatgggttg 1620
agaaactgac tgaagtttct atcagctctg atgccttctt ccctttccga gataacgtag 1680
acagagctaa aaggagtggt gtggcgtaca ttgcggctcc ctccggttct gctgctgaca 1740
aagttgtgat tgaggcctgc gacgaactgg gaatcatcct cgctcatacg aaccttcggc 1800
tcttccacca ctgattttac cacacactgt tttttggctt gcttatgtgt aggtgaacag 1860
tcacgcctga aactttgagg ataacttttt aaaaaaataa aacagtatct cttaatcact 1920
ggaaaaaaaa aaaaaaa 1937

<210> 333
<211> 2029
<212> DNA
<213> Homo sapiens

<400> 333
cggacgcgtg ggctgaagtg tattgaaaca aaatgattat ttcataatgt ttgaattgct 60
tccaaactgc atgttattta ttctaaactc tccctctgat aggataccga gacctagaga 120
agttaagaaa acaagcccca gatcaatcac attgctcctg acagcaccta accttctaga 180
ttccaagtcc aatggttttc ctggtactat gatgctagta gatcttaaaa aataataatc 240
tagatctaca ctatccaata tggtagccac tggccacatg tgattttagc tcttgatatg 300
tgtttaattc gaatttagat gtgccgtaag tgtaaaatgc acactgaatt tcaaagactt 360
agtatgaaaa aaaaaagtat ctcaattttt attacatgaa gtgataacct agaaacactg 420
gacgaagtaa atattataaa aattaatttt acatatttcc ttttatttaa tgtggctact 480

agaaacttat gtatggctca catatttcta ctgaacagca ctgatatatt tggcaatata 540
cagggtatac attatatata tatatatata ttctatgtaa atagttcatt aaataattcc 600
tatagtaaaa tatgagaaac aggaaactca tatccaactt ggaaaagttt caaatataat 660
acttattaag aaatacaggt tctattcttc aggtactctt acctagaatg aattaaggat 720
atgtagaaac tcttatgtta cttataaaat aataaagaga tactgtaggt ctctacagag 780
atgctggagt gtccctatag tacctctaga gtatagtata gagatactat aggatctctt 840
tactatatag atatatggta tagactatag aggtatctct agactatata ttatttatac 900
tctactatcc tatatagtat ctctatatag agagataata tatatactcc atatagtata 960
ctctagagat gctataagtt tctgtagtat ctttatagta gagataatag ggagggataa 1020
tagtataggg acgtactata tacctatagt atctctataa gtacacaagt tcccccatta 1080
atcatctgtt ttcagttgtt aaccaaataa ggtattatgc aggctcatct ttttatataa 1140
gaagaaaaga ctgaatgctc cctatgactt cattttctcc ctgttcccac taccatgtcc 1200
ccaaacatac tgacacacaa agaaaatata aatacccctg attcttttag ccttaaactt 1260
agggcctcat cctaaacatc ttaagctcag catctattcc aaaggaactc tttaatgata 1320
acgtttcttt gtaaagagac gaatatcatc tggggtttcc tctgaaatgt agatgaacct 1380
cacttcagtt ctttgtgtac taaaaagacc cacccttctc acccatgtgg cttttgtctt 1440
tccttgtgtt atgtaataag tgatggggta gaaggatggg gggtggagga agctacatca 1500
tggagagagg aggtagaaga cagctgtgga aaatttggat ggtggaagac tagaatctac 1560
taccatttac atgctaatga aaaccagaaa tggtcttttt tcccccgttt gatctgattt 1620
gaaagtttgt gacttttgtc tagtatgtca ttttaccccc tgtgtttttc tcattgttgt 1680
tagctataaa aactctttga aattctcaaa ataaaggaag gtttctttca ggaataggag 1740
ggctgaggtc aagctgtgaa actgcattct tcatagtggg tctattgggg tggaaaaccc 1800
gcctttgaag taatagaagg tagaagcccg gaaggaaaca ggtctggccc tggatccagg 1860
aaaataaaga aagaaataat tggagatgta gcttagtact tagaattatc ccagaagtga 1920
acaacatcag taaggtctgg gcttccccct aacgtaacct cttatgaggg tttcaacctc 1980
acatccatat atcttttaaa aaaaaaaaaa aaaaaaaaaa aaaaaaaaa 2029

<210> 334
<211> 2923
<212> DNA
<213> Homo sapiens

<400> 334
ccagcctgga cgacagagca gactccatct caaaaaaaaa aaaaaaatgg tcaccccttt 60
tgctcctaaa tcaccctcaa agtaaaagag aacaagaaac agaagcagaa atccatattt 120
agtgaaataa gacaacacct gtagctccaa acctgtagaa gcagatccca gagaaaagca 180
ggccagttct ctcctggaat cccagaaagt cccaggaatt ggaggcttca gtgctgcagt 240
agggagggga ctaaaaacaa agtctgtata tggagcagta agacctccgg gttctcatcc 300
cccatcctgt gtgctctcgg gtgactagcc ctctccctct ccaggtttag cttctggaga 360
aattaaatca aagaggctct agaactgggg atggcaggca ttgagtgcag ggagtgagtt 420
gccagaggct gagagcagag agatttagtg gcagtgggca gagcaaacag caaaatgact 480
gccctcttcc ctggccttgc tccagaaact gagcagccag acatacacac tcccagaagg 540
cagttggagg tccccccggg caatcagaac cacccacaga gaagacctcc agatactgac 600
atttagaagc ctctgaccaa agagctgcct ggccacccac tggcttactc gctgacaggc 660
catgctcctg ccgtacacac cgatcctatc agtcatcctc atgggctcct ttccagactt 720
ctgaggaaag cttccaacat gaagagggag ttcaaaacaa acagagagag agagagagag 780
agagagagag agagagagag agagagaaaa gaaatataaa caatgcaggg agcaaaagat 840

aacttcaaat aaactcaaaa tattcttctg ggagagatga gacaatattg catacataaa 900
acaagaagag gatgctatca ctaaatttta aaaaaaagta acagaaagta agaaagacct 960
ctaagaaatg tttctgaaat gaggtataat tttttttttt ttgaggggga gtcttgctct 1020
gtcacccagg ctggagcgca gtggcgtgat ctcggctcac tgcaacctcc gtctcccggg 1080
ttcaagtgat tctcctgcct cagcttcctg agtagctggg attacaggca tgcgccacca 1140
tgcctggcta attttgcatt tttagtagag acggggtttc accatgttgg ccaggctggt 1200
ctgaaactcc cgacctcagg tgatctgccc acctcagcct cccaaagtgc tgggattaca 1260
ggcatgagct accgtgcctg gcctgtgtat ttttggagac agagtcttgc tctgttgccc 1320
aggctggagg gcagtggtgc aaactctgcc tcccgggttc aagcgattct cgtgcgtcac 1380
ctcccaagta gctggggcta gaggcatgtg ccaccatgtc tggttacttt ttatataaac 1440
cttcagttta atcttttagc ttactgctgt gtttttcagc tttattcact ttgctttttt 1500
tctatatttt cgtttcaact caacttttaa aaaatcatac ttgtatattt gtatatttat 1560
atttattttc caagacctca tttgcctctt tttcataatg ggctattatt gctccatggt 1620
acagaatctt ctgttctttc ctaaactatt agttattttt aaaatatttg ttaactttct 1680
ccccccttgct agttctcagg tacccgcttt ctcccagagt gctggctttc cttaattatt 1740
tgctgacttg tgctggtgtg taacccttca tacttaggta tttctatttg tctgactgct 1800
gatttgattc caatccagtg tttcttctga ttcgtggaga agagacgaca acgctgtgag 1860
gctctggttt tggtggcttg tctgggtgtg ggagctcctt gtcatcatgg gatttttagc 1920
tccctgggtc gccatcctac atggccactc tcctgccgat gctgcccgct actcagcagg 1980
aggggaagtc gagaccacct ccttaacctt acagacattg attgtgagct tggagcacct 2040
tccgtgactg gaccacccat gcagtggtcc tttgcttttt gcatttttaa ctgcaatttt 2100
ccccaagagt cttccctaat acagtctctt aggaattgac ggtgggatta aacaaggcat 2160
gtttctgcac tgaatatggt ctatgggttg ccactttgca acctcagctt taaggcttat 2220
ttcctcccag gaaactgatg ttaacttttt aagttaaaaa tgtgtatata agtaatacat 2280
gtttattttg gaaattagaa aatacaggcc aggtgtggtg gctcatgcct gtaatcccag 2340
catttggga ggttgaggcg ggaggattgc ctgggcccgg gagtttgaga ccagcctggg 2400
caacataggg aaattccatc tctacaaaaa aattaaacat tggctgggtg tggtggtgcc 2460
cacctgtggt cacagctact cgggagactg aggtgggagg atagcttggg caggggaggt 2520
tgaggctaca gtgagccacg atcacaccaa tgtgacacag tgagaccctg tctcaaaaaa 2580
aaaaaaaaag aaagaaaata taggtaaaca caaagacaaa aaagcagggc atggtggctc 2640
acacctgtaa ttctagcacc ttgggaggct gaggcaggag gatcacttga ggtcaggaac 2700
tcgagacgag accaggctgg gcagcatggc aggaccccat ctctataaaa agtacaaaaa 2760
ttagcagggc acggtggtgt gcacctgtgg tcctgctact tgggaagttg aggtgggagg 2820
atcacctgag ccctggaggt tgagggtgct gtgagccatg atggcaccac tccactccag 2880
tccaggtgaa agagccagat cctgtctcaa aaaaaaaaaa aaa 2923

<210> 335
<211> 2283
<212> DNA
<213> Homo sapiens

<400> 335
cgcctccagg ccccttcccg cgccgcgacg cacgctgccc cggaaggccg cggcgctgta 60
gtgcggcgcc ccaggttctt tagtggaaga acgcgaagcg aggatgagtg atccgtggag 120
gcagtaacag gcgcggcgag ggagaagtga ttcccgaaga atcaaggctg ggccggaccc 180
ggtggcctgg caacaggtaa taagagaaat gaagccaaca ggtacagacc caaggatctt 240
atctatagct gctgaagttg caaaaagccc tgagcagaat gtccctgtta tactgttgaa 300

gttaaaagaa ataataaaca tcacaccttt aggaagctca gagttgaaga aaatcaaaca 360
agatatatat tgttatgatc tcattcaata ttgcctcttg gtcctcagtc aagattattc 420
tcgaatccag ggtggttgga ctacaatttc ccagcttaca cagatattaa gccattgctg 480
tgtgggcttg gagccaggag aagatgcaga ggaattttac aatgaattac ttccatcagc 540
tgcagaaaat tttctagttt tggggagaca attacaaaca tgttttatca atgcagctaa 600
ggctgaagaa aaagatgaat tactacactt tttccaaatt gtgactgatt ctctcttctg 660
gcttttggga ggccatgttg aacttattca gaatgtacta caaagtgatc atttcttaca 720
tttactgcaa gctgacaatg tccaaatagg atctgcagtc atgatgatgc tacagaatat 780
attacagatc aacagtggtg atttactcag aataggaaga aaagccctgt attcaatttt 840
agatgaagtt attttcaagc ttttttcaac tcctagtcca gttataagaa gtactgctac 900
aaaactccta ctgttgatgg ctgaatccca tcaggaaatt ttgattttac tgagacaaag 960
tacctgctac aaaggactca gacgtctact aagtaaacag gaaactggga ctgaattcag 1020
tcaagaactt agacagcttg ttggcctttt aagcccaatg gtctatcagg aagtagaaga 1080
gcagatccaa acgatcaaag atgttgctgg agataaatag gcagaaggaa gaagaggacc 1140
tcaaattaca attgcaactt caaagacaga gagccatgag actttcccga gaattgcagc 1200
tgagtatgct cgaaatagtt catccaggtc aggtggagaa acactatcgg gaaatggaag 1260
agaaatcagc actgattatc cagaaacatt ggagagggta cagggaaagg aaaaattttc 1320
accaacagag gcagtctctc atagagtata aagcagctgt cacacttcaa agagcagcgc 1380
ttaaattcct agcgaagtac cgtaagaaaa agaaactatt tgctccttgg cgaggactcc 1440
aagaactcac tgatgcacgc cgagttgaac tgaagaaacg agtggatgac tatgtcagaa 1500
gacatttggg ctctccaatg tcagatgtgg tcagtaggga gctccatgcc caagctcaag 1560
aacgactgca acactacttt atgggcaggg ccctagaaga gcgagcccag cagcacagag 1620
aagctctgat agcacagatc agcaccaacg ttgaacagct aatgaaggca ccaagtctga 1680
aggaggcaga agggaaagaa cctgagctct tcctaagtag atccaggcct gtggcagcca 1740
aggccaagca ggcccatctc acaaccctga agcacataca agcaccctgg tggaagaagc 1800
ttggagaaga atctggagat gagattgatg ttccaaagga tgagcttagt atagaattag 1860
aaaatttatt cattggtgga accaaaccac cttagtgagt aaccctaaga attgacacaa 1920
atctcatatt ttaggagatt atattggttc tgcctctggc atgctggtag actagggcca 1980
tcctaactta ttattttcca gaggttctcc tccagacaag acctgcagta agcaaagagt 2040
tatattctac ctctctctca attttctttt tcttttctct gtatcctcat ccttagccac 2100
acacagattt gtgtggcttt tattgtagaa ctaaacttag catagtgttc tgttgtttac 2160
atgaagtgtg tttttctttg gtttcttctg ttttccaact aaatattttt ttctaaataa 2220
atattttcaa caattgattt gaaaaaaaaa aaaaaaaaaa aaaaaaaaaa aaaaaaaaaa 2280
aaa 2283

<210> 336
<211> 2765
<212> DNA
<213> Homo sapiens

<400> 336
gggagacgtg gtgccgctgc gggctcgctc tgccgtgcgc taggcttggt gggaaggcct 60
gttctcgagt ccgcgctttt cgtcaccgcc atgtcgggag gtggtgtgat tcgtggcccc 120
gcagggaaca acgattgccg catctacgtg ggtaacttac ctccagacat ccgaaccaag 180
gacattgagg acgtgttcta caaatacggc gctatccgcg acatcgacct caagaatcgc 240
cgcgggggac cgcccttcgc cttcgttgag ttcgaggacc cgcgagacgc ggaagacgcg 300
gtgtatggtc gcgacggcta tgattacgat gggtaccgtc tgcgggtgga gtttcctcga 360

agcggccgtg gaacaggccg aggcggcggc gggggtggag gtggccggag ctccccgagg 420
tcgctatggc ccccatccag gcggtctgaa aacagagtgg ttgtctctgg actgcctcca 480
agtggaagtt ggcaggattt aaaggatcac atgcgtgaag caggtgatgt atgttatgct 540
gatgtttacc gagatggcac tggtgtcgtg gagtttgtac ggaaagaaga tatgacctat 600
gcagttcgaa aactggataa cactaagttt agatctcatg agggagaaac tgcctacatc 660
cgggttaaag ttgatgggcc cagaagtcca agttatggaa gatctcgatc tcgaagccgt 720
agtcgtagca gaagccgtag cagaagcaac agcaggagtc gcagttactc cccaaggaga 780
agcagaggat caccacgcta ttctccccgt catagcagat ctcgctctcg tacataagat 840
gattggtgac actttttgta gaacccatgt tgtatacagt tttcctttat tcagtacaat 900
cttttcattt tttaattcaa actgttttgt tcagaatggg ctaaagtgtt gaattgcatt 960
cttgtaatat ccccttgctc ctaacatcta cattcccttc gtgtctttga taaattgtat 1020
tttaagtgat gtcatagaca ggattgttta aatttagtta actccatact cttcagactg 1080
tgatattgtg taaatgtcta tctgccctgg tttgtgtgaa ctgggatgtt gggggtgttt 1140
gtggttatct tacctgggga agttcttatg tttatcttgc ttttcatgtg tctttctgta 1200
gacatatctg aagagatgga ttaagaatgc tttggattaa ggattgtgga gcacatttca 1260
atcattttag gattgtcaaa aggaggattg aggaggatca gatcaataat ggaggcaatg 1320
gtatgactcc aagtgctatt gtcacagatg aaattggcag tattgacctt atactaaaag 1380
gcaggggtta aaaatgatta tatacatttt ccttaaaaca cttgcaaaca ttttattcag 1440
ttgtctttag ctacaattgc tttgcttttt aaaccttggc aattgtggca aaattatatt 1500
gcccattttg tagcaactta ttttgctccc ttccccccat ttttgtttta atagggacta 1560
atgtgggaag aactggctaa tttgtcacag tgcttagtta caactgttaa tgtgtgacct 1620
gctgttggtg tacatgtggg tacagggtgt ttttaaatcc aacaagatag agtataatat 1680
caatactgct aaatctgcat gtcctctgtg tgactgatag agcgttgcta tttcattttt 1740
ttaagacaaa atgaaagcaa aatatagagt tccaatgtat tggtgtagat aatctagttg 1800
ggaatacttt taagtctcac cttccccttt aaactaatat tcataattgg ttcatatgtt 1860
taaaagactt taatttacaa attaaattgc aaatgggagc attagattta gtttagact 1920
taggtgggta gcaatgccag taaacttaaa ttacgtaact tcttgcaacc acgaaacctg 1980
taatacgctg tacagtaaca agtgttggca ttatcagttg aactgtaaat acaaaatgct 2040
tcttccaatt agtctctatg atgattaagt ttctaaaatt tatctgaaca ccattcagaa 2100
acttgttttg gggaatttga tagttattga tgtgcatctg ttaaactgat gacagacata 2160
actcatcatt ccccagaaac cttttttgat tacagtatct aacattttgc ctcctctttt 2220
ttggttttgc tggttataaa ggtttggatt ggagagggct cactggatcc caatccttgg 2280
agctggatca ttggattcaa atcataatgt ggataggata gggaggatga attacccagg 2340
attcatggag cgggatcaga ttaccaggaa cataggagtg gattcctgcc ccaaccaaac 2400
cgcattcgtg tggatttttt tattcaactt aattggctat tccaaagatt ttttttttcc 2460
tatttttgac gattggagcc cttaagatgc acgatggaat tgtgttttgc gtttttttggt 2520
aaaaggagca aagcgaggac ctggagataa acgctggagc aatctccttg gaaggattca 2580
gcacgagtag atggtaaaca tttaaagggg aaaggggggg tttgtttaaa atagtaaatc 2640
agtaagtcac ttctaaattt aaagaaaaca aaattggagt tgaagaataa gtaggtttcc 2700
aattggctat tgccgttttc tttgaaaaaa taaacatttt ttaaaaaact aaaaaaaaaa 2760
aaaaa 2765

<210> 337
<211> 1567
<212> DNA
<213> Homo sapiens

<400> 337
ggggtcctgc caccgcgcca cttggcctgc ctccgtcccg ccgcgccact tcgcctgcct 60
ccgtcccccg cccgccgcgc catgcctgtg gccggctcgg agctgccgcg ccggcccttg 120
ccccccgccg cacaggagcg ggacgccgag ccgcgtccgc cgcacgggga gctgcagtac 180
ctggggcaga tccaacacat cctccgctgc ggcgtcagga aggacgaccg cacgggcacc 240
ggcaccctgt cggtattcgg catgcaggcg cgctacagcc tgagagatga attccctctg 300
ctgacaacca aacgtgtgtt ctggaagggt gttttggagg agttgctgtg gtttatcaag 360
ggatccacaa atgctaaaga gctgtcttcc aagggagtga aaatctggga tgccaatgga 420
tcccgagact ttttggacag cctgggattc tccaccagag aagaagggga cttgggccca 480
gtttatggct tccagtggag gcattttggg gcagaataca gagatatgga atcagattat 540
tcaggacagg gagttgacca actgcaaaga gtgattgaca ccatcaaaac caaccctgac 600
gacagaagaa tcatcatgtg cgcttggaat ccaagagatc ttcctctgat ggcgctgcct 660
ccatgccatg ccctctgcca gttctatgtg gtgaacagtg agctgtcctg ccagctgtac 720
cagagatcgg gagacatggg cctcggtgtg cctttcaaca tcgccagcta cgccctgctc 780
acgtacatga ttgcgcacat cacgggcctg aagccaggtg actttataca cactttggga 840
gatgcacata tttacctgaa tcacatcgag ccactgaaaa ttcagcttca gcgagaaccc 900
agacctttcc caaagctcag gattcttcga aaagttgaga aaattgatga cttcaaagct 960
gaagactttc agattgaagg gtacaatccg catccaacta ttaaaatgga aatggctgtt 1020
tagggtgctt tcaaaggagc tcgaaggata ttgtcagtct ttaggggttg ggctggatgc 1080
cgaggtaaaa gttctttttg ctctaaaaga aaaaggaact aggtcaaaaa tctgtccgtg 1140
acctatcagt tattaatttt taaggatgtt gccactggca aatgtaactg tgccagttct 1200
ttccataata aaaggctttg agttaactcc ctgagggtat ctgacaatgc tgaggttatg 1260
aacaaagtga ggagaatgaa atgtatgtgc tcttagcaaa aacatgtatg tgcatttcaa 1320
tcccacgtac ttataaagaa ggttggtgaa tttcccaagc tatttttgga atatttttag 1380
aatattttaa gaatttccca agctattccc tcaaatttga gggagctgag taaccccatc 1440
gatcatgatg tagagtgtgg ttatgaactt taaagttata gttgttttat atgttgctat 1500
aataaagaag tgttttgcat tcgtcaaaaa aaaaaaaaaa aaaaaaaaaa aaaaaaaaaa 1560
aaaaaaa 1567

<210> 338
<211> 2224
<212> DNA
<213> Homo sapiens

<400> 338
caactctcca caacctttta ttacatcaag aaggagctaa aatggcagtg cgtttagctg 60
gtgggctgca gaaaatggtt gccttgctca acaaaacaaa tgttaaattc ttggctatta 120
cgacagactg ccttcaaatt ttagcttatg gcaaccaaga aagcaagctc atcatactgg 180
ctagtggtgg accccaagct ttagtaaata taatgaggac ctatacttac gaaaaactac 240
tgtggaccac aagcagagtg ctgaaggtgc tatctgtctg ctctagtaat aagccggcta 300
ttgtagaagc tggtggaatg caagctttag gacttcacct gacagatcca agtcaacgtc 360
ttgttcagaa ctgtctttgg actctcagga atctttcaga tgctgcaact aaacaggaag 420
ggatggaagg tctccttggg actcttgttc agcttctggg ttcagatgat ataaatgtgg 480
tcacctgtgc agctggaatt ctttctaacc tcacttgcaa taattataag aacaagatga 540
tggtctgcca agtgggtggt atagaggctc ttgtgcgtac tgtccttcgg gctggtgaca 600
gggaagacat cactgagcct gccatctgtg ctcttcgtca tctgaccagc cgacaccaag 660
aagcagagat ggcccagaat gcagttcgcc ttcactatgg actaccagtt gtggttaagc 720

tcttacaccc accatcccac tggcctctga taaaggctac tgttggattg attcgaaatc 780
ttgccctttg tcccgcaaat catgcacctt tgcgtgagca gggtgccatt ccacgactag 840
ttcagttgct tgttcgtgca catcaggata cccagcgccg tacgtccatg ggtgggacac 900
agcagcaatt tgtggagggg gtccgcatgg aagaaatagt tgaaggttgt accggagccc 960
ttcacatcct agctcgggat gttcacaacc gaattgttat cagaggacta aataccattc 1020
cattgtttgt gcagctgctt tattctccca ttgaaaacat ccaaagagta gctgcagggg 1080
tcctctgtga acttgctcag gacaaggaag ctgcagaagc tattgaagct gagggagcca 1140
cagctcctct gacagagtta cttcactcta ggaatgaagg tgtggcgaca tatgcagctg 1200
ctgttttgtt ccgaatgtct gaggacaagc cacaagatta caagaaacgg ctttcagttg 1260
agctgaccag ctctctcttc agaacagagc caatggcttg gaatgagact gctgatcttg 1320
gacttgatat tggtgcccag ggagaacccc ttggatatcg ccaggatgat cctagctatc 1380
gttcttttca ctctggtgga tatggccagg atgccttggg tatggacccc atgatggaac 1440
atgagatggg tggccaccac cctggtgctg actatccagt tgatgggctg ccagatctgg 1500
ggcatgccca ggacctcatg gatgggctgc ctccaggtga cagcaatcag ctggcctggt 1560
ttgatactga cctgtaaatc atcctttagg taagaagttt taaaaagcca gtttgggtaa 1620
aatactttta ctctgcctac agaacaaaga cttggttggt agggtgggag tggtttaggc 1680
tatttgtaaa tctgccacaa aaacaggtat atactttgaa aggagatgtc ttggaacatt 1740
ggaatgttct cagatttctg gttgttatgt gatcatgtgt ggaagttatt aactttaatg 1800
tttttgcca cagcttttgc aacttaatac tcaaatgagt aacatttgct gttttaaaca 1860
ttaatagcag cctttctctc tttatacagc tgtattgtct gaacttgcat tgtgattggc 1920
ctgtagagtt gctgagaggg ctcgaggggt gggctggtat ctcagaaagt gcctgacaca 1980
ctaaccaagc tgagtttcct atgggaacaa ttgaagtaaa ctttttgttc tggtcccttt 2040
tggtcgagga gtaacaatac aaatggattt tgggagtgac tcaagaagtg aagaatgcac 2100
aagaatggat cacaaaaaaa aaaaaaaaaa aaaaaaaaaa aaaaaaaaaa aaaaaaaaaa 2160
aaaaaaaaaa aaaaaaaaaa aaaaaaaaaa aaaaaaaaaa aaaaaaaaaa aaaaaaaaaa 2220
aaaa 2224

<210> 339
<211> 854
<212> DNA
<213> Homo sapiens

<400> 339
gggacacagc cggccaggag aaattcggtg gactgagaga tggctattat atccaagccc 60
agtgtgccat cataatgttt gatgtaacat cgagagttac ttacaagaat gtgcctaact 120
ggcatagaga tctggtacga gtgtgtgaaa acatccccat tgtgttgtgt ggcaacaaag 180
tggatattaa ggacaggaaa gtgaaggcga aatccattgt cttccaccga aagaagaatc 240
ttcagtacta cgacatttct gccaaaagta actacaactt tgaaaagccc ttcctctggc 300
ttgctaggaa gctcattgga gaccctaact tggaatttgt tgccatgcct gctctcgccc 360
caccagaagt tgtcatggac ccagctttgg cagcacagta tgagcacgac ttagaggttg 420
ctcagacaac tgctctcccg gatgaggatg atgacctgtg agaatgaagc tggagcccag 480
cgtcagaagt ctagttttat aggcagctgt cctgtgatgt cagcggtgca gcgtgtgtgc 540
cacctcatta ttatctagct aagcggaaca tgtgcttcat ctgtgggatg ctgaaggaga 600
tgagtgggct ttcggagtga atgtggcagt ttaaaaaata acttcattgt ttggacctgc 660
atatttagct gttttggaac gcagttgatt ccttgagttt catatataag actgctgcag 720
tcacatccca atattcagtg gtgaaatctt gtttgttact gtcattccca ttccttttcg 780
tttagaatca gaataaagtt gtatttcaaa tatttaaaaa aaaaaaaaaa aaaaaaaaaa 840

aaaaaaaaaa aaaa 854

<210> 340
<211> 1816
<212> DNA
<213> Homo sapiens

<400> 340
gaaagatgga ggaccatcag cacgtgccca tcgacatcca gaccagcaag ctgctcgatt 60
ggctggtgga cagaaggcac tgcagcctga aatggcagag tctggtgctg acgatccgcg 120
agaagatcaa tgctgccatc caggacatgc cagagagcga agagatcgcc cagctgctgt 180
ctgggtccta cattcactac tttcactgcc taagaatcct ggaccttctc aaaggcacag 240
aggcctccac gaagaatatt tttggccgat actcttcaca gcggatgaag gattggcagg 300
agattatagc tctgtatgag aaggacaaca cctacttagt ggaactctct agcctcctgg 360
ttcggaatgt caactatgag atcccctcac tgaagaagca gattgccaag tgccagcagc 420
tgcagcaaga atacagccgc aaggaggagg agtgccaggc aggggctgcc gagatgcggg 480
agcagttcta ccactcctgc aagcagtatg gcatcacggg cgaaaatgtc cgaggagaac 540
tgctggccct ggtgaaggac ctgccgagtc agctggctga gattggggca gcggctcagc 600
agtccctggg ggaagccatt gacgtgtacc aggcgtctgt ggggtttgtg tgtgagagcc 660
ccacagagca ggtgttgcca atgctgcggt tcgtgcagaa gcggggaaac tcaacggtgt 720
acgagtggag gacagggaca gagccctctg tggtggaacg accccacctc gaggagcttc 780
ctgagcaggt ggcagaagat gcgattgact ggggcgactt tggggtagag gcagtgtctg 840
aggggactga ctctggcatc tctgccgagg ctgctggaat cgactggggc atcttcccgg 900
aatcagattc aaaggatcct ggaggtgatg ggatagactg gggagacgat gctgttgctt 960
tgcagatcac agtgctggaa gcaggaaccc aggctccaga aggtgttgcc aggggcccag 1020
atgccctgac actgcttgaa tacactgaga cccggaatca gttccttgat gagctcatgg 1080
agcttgagat cttcttagcc cagagagcag tggagttgag tgaggaggca gatgtcctgt 1140
ctgtgagcca gttccagctg gctccagcca tcctgcaggg ccagaccaaa gagaagatgg 1200
ttaccatggt gtcagtgctg gaggatctga ttggcaagct taccagtctt cagctgcaac 1260
acctgtttat gatcctggcc tcaccaaggt atgtggaccg agtgactgaa ttcctccagc 1320
aaaagctgaa gcagtcccag ctgctggctt tgaagaaaga gctgatggtg cagaagcagc 1380
aggaggcact tgaggagcag gcggctctgg agcctaagct ggacctgcta ctggagaaga 1440
ccaaggagct gcagaagctg attgaagctg acatctccaa gaggtacagc gggcgccctg 1500
tgaacctgat gggaacctct ctgtgacacc ctccgtgttc ttgcctgccc atcttctccg 1560
cttttgggat gaagatgata gccagggctg ttgttttggg gcccttcaag gcaaaagacc 1620
aggctgactg gaagatggaa agccacagga aggaagcggc acctgatggt gatcttggca 1680
ctctccatgt tctctacaag aagctgtggt gattggccct gtggtctatc aggcgaaaac 1740
cacagattct ccttctagtt agtatagcgg acttaataaa agaggaaaaa actcttgctt 1800
caaaaaaaaa aaaaaa 1816

<210> 341
<211> 696
<212> DNA
<213> Homo sapiens

<400> 341

gaaaaaacaa agagagacat taacaggttt ctgagtgtgg ccagtcttca aggacttatt 60
catgaaggca ccatgacttc tttgtgcatg gccatgacag aggagcagca taagtctgtg 120
gtcatcgatt gcagcagctc ccagcctcag ttctgcaatg caggaagtaa ccggttttgt 180
gaggattgga tgcaagcttt tttaaatggt gccaaaggag gtaacccttt tcttttccga 240
caagtactgg agaactttaa actaaaggcc atacaagaca caaacaattt gaagagattt 300
atccgacagg cagaaatgaa tcattatgct ttgtttaaat gttacatgtt cctaaagaac 360
tgtggtagtg gagatatact tttgaagatt gttaaagtgg aacatgaaga aatgcctgaa 420
gccaaaaatg tgatagctgt ccttgaagaa ttcatgaaag aagctcttga ccaaagtttt 480
tgatcatatg ttttgagata attgtatgat caagttgtat atttaagtct tagtgtttga 540
aattgcagtt ataattgttc ataggattgc tatttaagat gatttgaaac tcaatccaga 600
ttttcttttt gtattttacc aatttaactt aaataaaaat ctgaagaaca aaaaaaaaaa 660
aaaaaaaaaa aaaaaaaaaa aaaaaaaaaa aaaaaa 696

<210> 342
<211> 4912
<212> DNA
<213> Homo sapiens

<400> 342
ctttttgtt tttgagactc catcccaggc aggagtgcag tggcacgatc ttggctcact 60
gcagcctctg cctcccaggt tcaagcgact ctcctgcccc agtgacccaa gtagctggga 120
ttacaggcat gcgcccacta atttttgtat ttattgtaga gacagggttt catcatgttg 180
cccaggctgc tctcaaactc ctgatccacc ctcaagcaat ccaccttcct tggcctccca 240
aagtgctggg attacaggca tgagccaccc tgcgtccggc ccaaaattta gtgacttaaa 300
acaagtattt attatctcac agtttctgct ggccaggagt tcagaagcag cttagacatt 360
tctcacaaag ttgctgtcat ctgaaggctt ggctggagct ggaggatgct cttccaagct 420
cactcagatt ggggaccaca aattcagtgc ccaccggatt gtcttagcag cctcgatccc 480
gtatttccat gctatgttta caaatgacat gatggagtgc aagcaggatg agattgtaat 540
gcaaggaatg gacccaagtg ccctggaggc tctgatcaac tttgcctaca acggcaacct 600
tgccattgac cagcaaaatg tccagtcatt gctgatgggg gcgagcttcc tgcagctgca 660
gagcatcaaa gacgcctgct gcacattcct tcgagaacgg cttcacccaa aaaactgcct 720
gggtgtgcgc cagtttgctg agacaatgat gtgtgctgtg ctgtacgacg ctgccaacag 780
cttcatccac cagcactttg tggaggtgtc catgtcagaa gagttcctgg ccctgccctt 840
ggaagacgtg cttgagctgg tgtctcggga tgagctgaat gtcaaatctg aggagcaggt 900
ctttgaagct gcattggcct gggtcagata cgaccgggag cagaggggtc cctacctgcc 960
tgagctgctg tccaatatcc gcctgcccct ctgtcggccc cagttccttt cagacagagt 1020
acagcaggat gacctggtgc gttgctgcca caaatgcagg gacctggtag acgaagcaaa 1080
ggactaccac ctcatgccag agcgccggcc ccacctgcca gctttcagaa cccggccacg 1140
ctgctgcaca tccatcgctg gacttatcta cgctgtaggg ggcctcaact cagcaggtga 1200
ttccctgaat gtggtggaag tgttcgaccc cattgccaat tgctgggaga gatgccgtcc 1260
catgacaaca gcccgcagcc gcgttggcgt ggctgtggtg aacgggcttc tctatgccat 1320
cggaggatat gacggccagc tacggctgag cactgtggag gcctacaacc cggagacaga 1380
cacatggacc agagtgggga gcatgaatag caagagaagt gccatgggga cagtcgtgct 1440
ggatgggcag atctacgtct gtgggggcta cgatggcaac tcttccctca gctccgtgga 1500
gacctactca cctgagacgg acaaatggac agtggtgacc tcgatgagct cgaatcgcag 1560
tgctgctggg gttacagtct ttgagggcag gatatatgtg tcaggcggcc atgatggttt 1620
gcagatcttc agcagtgtgg aacactacaa ccaccacaca gccacctggc accctgcagc 1680

tggcatgctc aacaagcgct gccggcacgg agccgcctcc ctggggagca agatgtttgt 1740
ctgcgggggc tacgatggct ctggcttcct cagcattgcc gagatgtaca gctctgtggc 1800
agaccagtgg tgcctgattg tccccatgca cacgcgcagg agccgggtct ccctggtggc 1860
cagctgtggg cgcctctacg ctgttggggg ctacgacgga cagtcaaacc taagctcagt 1920
ggagatgtat gacccagaga cagactgctg gacattcatg gcccccatgg cgtgccatga 1980
gggaggggtc ggtgtgggct gcatccctct cctcaccatc taaggcagag gatgggatgt 2040
ggtggggcag ggatctggta cagacatagg cgcttccttc caggaacagt ccctcaggag 2100
aggcagtgga ccagaagaga tggcgaaacg tgagctcgcc ggaggtacag tttttccagg 2160
tgcttaagcc ctccccaact gtgccaccct tgtgaccttc aggcttgggt catcaagatg 2220
cacagcatgg aacacaagct cctctggatc ctgcagctgg tgacatggaa ctgttttctg 2280
gtccacatga acacaggctc catccaggcc cagctcctac ccaccgcctc tctgtgggcc 2340
agctgttcac agaaggcctt ccatctgatg ctccccatcg cctgcttgct ctccagccga 2400
gtctggccaa tttgccatgg ggaggctgca gtgtccaagc ctgctggaaa ctgggatgta 2460
gctggggacg aaaggacaga cccaagcgtt ctccctgcct gagatggtgt ggccacagca 2520
gtggaaggct gcacacaggc acattccttc ttccacagtg gggcaccaag gattctgtcc 2580
tcattgctgg gtaagcaggg agaagagaag ttttccccat gtctaatttt gggatttcag 2640
tgaggccttt tccatctgtc caggagaaca gaagggaaaa aaagatactt gaaagaaact 2700
gaaggaaatt taaacaaaga aacacttgaa agaaactgga aagaaaaata attttttat 2760
gtgaacaaat tttgcaagaa gaaaaaagca taaaagacac taacggcaaa tctatgttta 2820
aatggaaaat cgtctaactg gagaagggcg gtatccaccc cacattcgga tcccagggtc 2880
ctgaggcctc gcattgagct gggggttccc tctgagcccc agtgtgtgtg gaatcagtgc 2940
actcttgact gggcctgtag taaggtgctc atggggtttg tcttctcacc caccatcaga 3000
ggacttttaa aatcataggc gtagagagtt agctatctgc tgaattactg ccactcttct 3060
tggtgggggc tcctagctgt ggctgggggc tccaggcgcc cctgtgatta cctcctactg 3120
ccaccatggc gctcattcag attccccact ctcactaaca ttgcttcctt ttttgaccag 3180
caggaaacag caggtctggc cagattctca cttgcccatc aatctcgttc ttggatgatt 3240
tccctcattg tgatgcttct ggggcacgtt gaccatatgc acctctagaa cctaaccagg 3300
gcttccttct accagctgtg ggcgggcttg gtctggtaac cttgtctgct ctgccattcc 3360
actgctcctc catccactcg ccaatcccaa gagtctggcc tccctccagc cctgggcaga 3420
ctgaccagca aggtggacct ttacattcaa gcacagctgg cttttatgac ataaagaact 3480
aaaggccgaa agaatctctt gctgctgcaa agaacagatt ttatatttct tcctctaatc 3540
ttggcaaatg acctttacct tttggaaaga tttcatattg cttcctcctc cctggatagg 3600
acctaatgta gcacagcggg actcaaagag gaggacattt tctcttgcca gtgcactggg 3660
cagtggggct gtccttcaac tgctgctgcc aaaattggtt ttctaaaatt cttccagtag 3720
agactaaaag aagattcaat tcctgtaacc caagactgag tcttagggct ccagtctcca 3780
cctgcttggt ttcctatcct ttgctgcctg cctggggtgg cctggaagcc tgttcagaaa 3840
ggcacaatgt ggagcctggg gtgtctcccc caccccagga ccgtcaggtt taccagtgtg 3900
tgcaatcgcc atgtattcag agggaagtac ctttgttacc tacaacttag gagctaggcc 3960
tctgctacaa gcacttgaaa atgatatttt tatttttaac gtctcaacaa tctgatatcg 4020
gatgtcgttt aacctgggct cgtggtaggg ctccagcatt tctccctcct tcctggtttg 4080
cctgtagggg tagactcgga aggtgggtgg ggtgtgcatt tcctgttagg agtgtatcag 4140
tgcttgtctt attataagcc cctttctttt gtgaatttga agtagcacca acaagcctgg 4200
attgtgaagg tattaagaat cggtctgtgg gctactgagt gggtccttag gatactggcc 4260
cagattttgc cactgggtat ggcagatcat tttctaccat ggcctgctgc tcttgtagtg 4320
gacttcctga gtccaatccc acctcctggt gtagaattta cactgctgca cctgaggtcg 4380
atgtttcaaa gtaagatcaa gccagtgttt tgatctgggc tctgagcaca agtcaggaaa 4440
caccaacata ttcacactct cccagtaggt tcctcagtcc gatggtgaat ggctattcgt 4500
aaatggctgg tctggctctt tggtgttgga gcctttccaa tagccccatg aaaagaagca 4560

tcacccaagg atattgtaaa aaggatgtaa caaggagata gggtagacat tgtactcagt 4620
gggccttggg gcctagccca gctctgagca gaggactgtg gcattcactg tccttgagtg 4680
tttcaccttc ttggataaca cacgggcctt ctcttctgga tttcatcaga gattacagcc 4740
agatgggggc tgaagaccat cctcttgacc acagaggtgt gactgtggga attcctccca 4800
atttatggtt tcccagaaaa tcttagttcc ttttatttat agaatgcatg tcttttgtgt 4860
taagaaacca aagagaaata aagagaacac tcctaataaa aaaaaaaaaa aa 4912

<210> 343
<211> 2731
<212> DNA
<213> Homo sapiens

<400> 343
gacacaagac caagtttgaa acagatgcaa ataaactaaa atacagctct aatgtctttt 60
tctgctattc tgtctccctt ttcctctttg tctgtaaatg taaggaattt gagacaaaga 120
ggaaaaggga gacagaatag cagaatcttg actttgatag tcaagatctt gttcaagact 180
tggcacttga tctttttgta aacctagtat tatgttatgg tcaagtggga agcatgaatt 240
taaatgtagt atttaaaaga ttacttggtc ttggctttcc aatctgtggg agtgtttgga 300
tcattttata cacacacaca cacacacaca cacacacaca caccttttt tttttttgat 360
atgaggtctt accctgtcac ccagactaga gtgcagtggc gcaatcttag ctcactgcaa 420
cctctgccta cctggctcaa gggatcctcc cacctcagcc tccagagtag ctgggactac 480
aggcacatgc caccaagccc agctaatttt tcatattttt ggtagagatg gggtttcact 540
ctgttgccca ggctggtctc aaactcctga gctcaagtga tccacccacc ttggccttcc 600
aaagtgctgg gattacaggt gtgagctact gcacctggta cattattata atttaataat 660
agtgacagga aaaggtaaaa attgcaagca acaataataa ccaataatgc agaaatacat 720
ataaaatgtt aactgtaaag gcagatattg ttgcctatat cagccaatgt agatcttctg 780
gttttatact tactggtata ctataaatga aatttagtga cagcatactg tataccaaac 840
tagagtttac ccattgttat ttcttattta gatttttaga tacattgtct tttgattcta 900
acattggcat tatcttaatt taatctctgt atatttttct tctttttgga aatatgcgtt 960
tgtaaaagct taccttataa gctactatgg tgaccttggc ctatcagatg ggtgttctct 1020
taaaccgttt tatactgctt tataccagac tcaaaattgc caacatagtt atctgctaat 1080
tctcaataac ttaaagccac attattttg ctagagtaat tagtactaca ttgtttattt 1140
ctagatgcac tttaatattc atgtcaccag agtaatttag ttttactgtt ttattaatct 1200
gattgattaa gtctatccca atatgtctct gcagttgggt gacccaatat gtctctgcag 1260
ttgggtgacc caatatgtct ctgcagttgg gtgactacag aaataattca tgcagattac 1320
ctcgctattt gtatcatatt atatacaaat gacatactct agtgcatttg aaatgtaatt 1380
taatggaatc ttgtagttta caaaattaag attgtcttta aagtaaatca ttattccttt 1440
aatttactcc tctcatgacc cctgtaactg tttttcactt cagttcaaca tacatttatg 1500
ccttgcctcc tgtataccag ccagtaggtg ctggggttgc aaaataacta agatcaaccc 1560
atacatgctg gaagctctca atctagtagg agaggataga cacgaaagct cataatttgg 1620
ttggtttggt tttgagacag ggcagtctct gtcgccagct ggaggcagcg gcatgatctc 1680
agctcactgc agctttgacc tcccaggctg aagtgaccct ttcacctcag tacccaaaag 1740
tagctggaat tacaggtgcc cacctgccag aaaactcagt tttacttaaa ttatttattt 1800
tcaacgtgta gctcatgaag atactaactt taaatggcag ggagtagatt gagaatcatc 1860
atggaagacc ttttttttt tttcttgaga tggagtctca ctccgtcacc caagctggag 1920
tgcaatggca tgatcttggc tcactgcatt ctccacttct gggttcaagt gattctccta 1980
cctcagcctc ccgagtagct gggattacag gcatgcagca ccacacccag ctaatttttt 2040

tatttttagt agagatgggg tttcaccatg ttggccaggc tggtctaaaa ctcctgacct 2100
caggtgatcc acctgcctca gcctcccaaa gtgctgggat tacaggtgtg ggccaccaca 2160
cctggcccat catggaagac tttctgatgg tgatgtttga attgggtttt ggagagtgaa 2220
ttagaatgtt ttgaactaat acagtaaagg acagtagaaa gaaggaacaa catggacaga 2280
gaccttaagg catgaaatgt cattctgtat tcagttagac gtttagtctt gatagaagga 2340
ttttttcctt agaacagatt acacctatat gataagattt tattttgttt ttatttaata 2400
atagttcagt taaaatataa gcccaaaatt gctccataaa atttggcagc agttatgcta 2460
ttgacagcat ataaaaagca ctcaatcgag ctaggtgcag tggctcatgg ctgtaattcc 2520
agcactttgg gaggccccag caaaaggatc acttgattca aggaatttga gaccagtntg 2580
ggcaacatgg caagactcta tctgtatgaa aaaaaaaatt ttttaattag ctgggaatag 2640
tgatgtgtgc ccttgcagtg agctgtgatt tgctctactg cactccacct tgagtagcag 2700
agtgaaactg tctttgaaaa aaaaaaaaaa a 2731

<210> 344
<211> 561
<212> DNA
<213> Homo sapiens

<400> 344
gcttaaactt cagataccca agctgttcat aaattagtga ctacataaga tagttctggt 60
tttacgacta tagtgtgaca ctggttgagt cagtcagctt ctctgggcct catctgtaaa 120
aggaaatgga gagcaacatt atctatactc cctccctccc tctatttctc cctcccttcc 180
ttcctccttc ccttcctccc tttcttcctc ccttctccct ttctctctcc ctccctgcct 240
ccctcccttt cttccttctc tgcctccttc cctgtgattg gggaaaataa tagctcaagt 300
aattttaaaa aaattcaatt tagttatttc tttagcatat tactaaagac ttagactcct 360
aagactttcc gtaagggaaa ctagatgtgc tttgtagtgc agtcctaatt tttaaaattt 420
aaggagccca ggcatggtgg ctcacgccta tagtctcaga actttggatg gctcaggcgg 480
aaggattgct tgagcccagg aatttgagac cagcctgggc aacatagcaa aacctcatct 540
ttaccaaaaa aaaaaaaaaa a 561

<210> 345
<211> 3443
<212> DNA
<213> Homo sapiens

<400> 345
cctttttttt tttttttac caaaaaaggg gggaaaaagg atattagttt tgttatcttc 60
ctcaattaca aaatgcctgt tttttactct ctgtcacaca aaccctgata aacatgcttc 120
tttatcgatt agcacagttg ggcttatact ttttgtacag tatgccagtg gaacatcaga 180
tgctgaacac tagcacatgc tgtgattttg caatccctgc ccacatcaca cacttgatat 240
catttgtggg aggtcatgtg ggttggccta cacactggca agtaaactca ttgatttgga 300
caatgagtca ctgacaatga ggatgttatt ttattttagc aggtttgtgc caacgtcatt 360
gcagaggcaa gaagaagaag ggatatgaat gatacactga cagataccaa cttaataaat 420
aaaaagcttg ccaatcttga aaaaaaagga acatggactt aacaccttgc aaagatttta 480
agtattttac atattctacc aagcatgtta tatagccaaa gcctgtaatc tctttctggc 540
attaatctgg ctttttaaaa aaattctgtt ttgtttttgt tttattaaaa tcatggcctc 600

tggaattcat tatcagtttc tctccactta aaccaagaga gtactgctgg ttgcaaaagg 660
atgttagaaa tcatccccca ttgtgctgtt ttctctaata atttgaagaa gggtgttatt 720
tgctatgtgt atacactgtc tccaaaatac attaactcct tgtaaaggcc tgacgccagt 780
tgtattagtc tgttctcaca ttgttataaa gaaatacctg agattgggta atttataaga 840
aaagaggttt aattgtctca cgggtctgca ggttgtacag gaagcatggc agcatctgct 900
tctggggagg cctggggaaa cttacaatca tggcagaagg caaaagtggg gacaggcaca 960
ccacatggcc agagcaggag caagagagag agacggaggg gaggtaccac acgcttaaat 1020
aactacatct taggagaact cattcactgt catgaggaca gtaccatggg gatggtgctg 1080
aaccattcat aagaacctac ccctgtgatc taaccacctc cctgcaggcc ccacctccaa 1140
cactggggat tatatttcaa catgcgattt tgagcaggag gacacatcca aactgtatca 1200
ccagtattcc tccaagttgc tgtcctctgc atagagtgct ttttcccaga tacttaaagg 1260
taaaggggcc aaagtatccc acccacaaaa tctgtcctca gtatcccctg catcctcatg 1320
cttcagagtc actcttattt ctcaggtgaa atgagcaggt acgtatgact gtgatgtcac 1380
ttaaatacaa aaaaatacaa aaaaaaaatt caaacaaaaa taaataagaa atgagcaggt 1440
acggctgcct gggcaccaca ccagagaagc tcagaggcaa ggtccaccca ccccagggag 1500
aaacgctgag gatcaaactg gtgaagagca ggccacattg cactttcctc ttgctcttgt 1560
tcacaagctg ttcctcagaa atcacaggag atgtaaaggt ctactggttt gctccctgag 1620
ggaccttctg gaaacttcat gggcctctag gcaatcaaaa ggtcttcttt ttcaccagcc 1680
ccaatctttg gaggaaacag agctttgagg aagaaaaagc aggaaaacca ggcacctggt 1740
ctagaaaagt cagtgaaggg ggtccacagt ggagctttcc accatactat tctgaggctg 1800
cttcacaaag accaaagctc ctgcctaaag gttttgcctg ctccccaggc atccagacat 1860
cactggcttt ttcctactcc aacccaagaa aaggattctg tctaatcaca agtccaattg 1920
gctgtctctc ttgtctgacc aaaatcctgt tctcgccaaa cagaagcact gctatctact 1980
tccatctccc cagcctactg tgtacaaagc atgccttgcc acctgccccc ctcatctttt 2040
tgttcatgtg agaatgcatg ggtcccagga gcttcggtga gaggacagag aaaaatggga 2100
aattccatta ccaaaacatt ttagagaaag aagaaaaggc aaaggagaat ggaacgattg 2160
ttgctacttg ctggcagatt aaaagaactg atgaagaact ttcctgcttt ctccgggtgc 2220
actggctcac gcttgcgatc ccacactttg gggggccgag gcaagcagat tgcctgagct 2280
caggagttca agaccagcca gggcaacaca gtgaaacccc atctctacta aaatacaaaa 2340
aattagccag gcgtggcagc atgcgcctgt aatcccagct actcgggagg ctgaggcagg 2400
agaatcgctt gaacctggga agcaggggtt gcagtgagca gagactgggt cactgcactc 2460
tagcctgggt gacagagcaa gactctaact ccaaaaaaca aaaaaaaaat ctttcctgct 2520
ttctctgcct atatactaag taatccacat gagatagtcc catgattata aacactgcct 2580
aaagaaggat ttaaaaataa acagacattt aaaattttta tagagaactt tttacaatca 2640
agatgaccag gcactgttaa gaaaatgaaa agagctttgt tatataactt atacctctcc 2700
caaaattcta aactatttat cctacttgga aaatatctca tataatctac ctgctagtct 2760
ggtccagttt tctaatcatt ttttgctaac aagaagtgtt ttcttatata caatataatt 2820
cttttatttc catttaagcc aatctttttg ttccatctct taagaatttg agaaaatgtg 2880
ggtttgcttc ctctttccaa cttagttaac caacttaata tgagtcatcc cttggttttt 2940
ctctaaattc agtaatcagc cttcacagat ctcacttaac agattcacag atctcttaac 3000
catttttcag gcttcagaat ttcctctttg ccacccttaa tatgaacagt taacctgggt 3060
ttatctacgc ttttacatca aatccaaact gctttgtttt tagggcctcc agaacccaat 3120
cttgctttcc cacaacctta tttctatgtt ttgtcccttc tataaactaa atatactccc 3180
attgctaatt aagctattcc ctcatccctc cagtttagac agtcttctca ctttatttaa 3240
tcccaatcca tctccacatg gccgcttcaa caccagtata tccaccaaaa aacatttgcc 3300
aatgtccccc atccagaatg atctcctttc atattgcagg cagaaaccag ggtgagtggt 3360
tcagtcatgg ctgtgtggtt gaatgtgctg gttctgttag cactgcagca tctttttagg 3420
ttctgactag ttctagatcg cga 3443

<210> 346
<211> 1358
<212> DNA
<213> Homo sapiens

<400> 346
gttccaaccc agggggaaaa atgcggcctt tgactgaaga ggagacccgt gtcatgtttg 60
agaagatagc gaaatacatt ggggagaatc ttcaactgct ggtggaccgg cccgatggca 120
cctactgttt ccgtctgcac aacgaccggg tgtactatgt gagtgagaag attatgaagc 180
tggccgccaa tatttccggg gacaagctgg tgtcgctggg gacctgcttt ggaaaattca 240
ctaaaaccca caagtttcgg ttgcacgtca cagctctgga ttaccttgca ccttatgcca 300
agtataaagt ttggataaag cctggtgcag agcagtcctt cctgtatggg aaccatgtgt 360
tgaaatctgg tctgggtcga atcactgaaa atacttctca gtaccagggc gtggtggtgt 420
actccatggc agacatccct ttgggttttg ggtggcagc caaatctaca caagactgca 480
gaaaagtaga ccccatggcg attgtggtat ttcatcaagc agacattggg gaatatgtgc 540
ggcatgaaga gacgttgact taaaacgaag ccattccaag gacagacggc tgtatggaaa 600
ggccgagctt tgtttcctgt gtttgtgtgg actccaccat catgttgaat tttgtcaaca 660
ctctggcctc ttcagggact tcttatttac tgtactctct atcactgaca aatgcaggct 720
ggattcttat tatatacaga gatggctcaa aaatggggtt tcagatcttt gtgacgaaat 780
agaatactgt ttcatatttg aatcagaggg cttcttgttc tgagaaatag gttcaaaatc 840
attggaacca ggaacaagaa tagcttattg ttatctgtga taacactgtt ttctaaacac 900
aaggattttc tttttttatta atatgcaaca tagacattgc cataacagaa taataaacca 960
catgtggggt tttaaaaatg aaatttggct aataggagca attcagctat ttttctatac 1020
agtaattggt gtgtggtata gaagaaaaac gggttcaaac ccccacttctg ccacctacca 1080
gctatatggc cttgaatgag tcattcagct ttaataaggt tcattttctt ctgtttaaaa 1140
agacacaaaa cttgaaaatc agctttggcc atctacctga gaattagaaa gtctgatttt 1200
tggaattaga aatcatgatt gtaggctggg cacagtggct cgcgcctgta atcccagcac 1260
tttgggaggc caaggcggac ggatcacttg aggttaggag tttgagacca gcctggccaa 1320
catggtgaaa ccccatctct actaaaaaaa aaaaaaaa 1358

<210> 347
<211> 1047
<212> DNA
<213> Homo sapiens

<400> 347
ctggatacca tttgttgaaa gatgttatta ctctagctga acttacaaga gactttagac 60
cagggatcta aattacagtg gccttagtga ccttgtcctt atcttcttag gacagctgag 120
aagccactgg gacttagagc ctttaaaagg agattaactg tcccaaaagg atctttgcta 180
ctgaccagca gacacttctt ccttcagtag cctttcatac tgtgttgagt aacaccctag 240
ggtgtccatt aaagttttga gttttaccta gggcccagag ccatgaatca ggattctgtc 300
tacatgattc gtgttttcat tggtgtcaaa atacaaaagc caaagttctg gctatgaatt 360
gttaacttgg aagaaatact aactgccacc acttattaag tgcctactgt gtgccaggct 420
ctgaactagg tgcttcatat acattatcct aaattatctc aacatatgag gtaggtgttt 480
taatttttat tttatagaac ttggtgtgtt tgactgttaa gctatggggc tagagagagg 540

gtttgatccc aggtccctct gtgcttttgc tgctgagcca cacaacctct catttcaaaa 600
acactttcaa aatgctaaca tattctaatt cactctaggc caccaaaaac tttaatacta 660
atatctgatt tgtaaatgac ttaatgtatc cttgacccta tcagctgaat ttaatgaaat 720
attcctctct gctgtgaaat tttaccagta tagtatttgg tctagtgaca gagcgagact 780
ccgtctacac acacacacac acacacacac acatccttcc tcctctaacc ccaaactaag 840
atcacagaag gtgatccagt cagagaacag agggaaatct taccaggaag ggcttaagta 900
cacttttttt taaaacagct ttattgtttt taaagcctac aatttgataa gccttgacat 960
atgtatacct gtgaaagcat caccacaatc aagacactgg acatatctat cacaccccat 1020
cctaaaaaaa aaaaaaaaaa aaaaaaa 1047

<210> 348
<211> 1306
<212> DNA
<213> Homo sapiens

<400> 348
tccaaaaagg gtcagtctac ctcccgccat aaaaaactca tgttcaagac agaagggcct 60
gactcagact gacattctcc acttcttgtt ccccactgac agcctcccac ccccatctct 120
ccctcccctg ccattttggg ttttgggtct ttgaaccctt gcttgcaata ggtgtgcgtc 180
agaagcaccc aggacttcca tttgctttgt cccggggctc cactgaacaa gttggcctgc 240
actggtgttt tgttgtgggg aggaggatgg ggagtaggac ataccagctt agattttaag 300
gtttttactg tgagggatgt ttgggagatg taagaaatgt tcttgcagtt aagggttagt 360
ttacaatcag ccacattcta ggtaggggcc cacttcaccg tactaaccag ggaagctgtc 420
cctcactgtt gaattttctc taacttcaag gcccatatct gtgaaatgct ggcatttgca 480
cctacctcac agagtgcatt gtgagggtta atgaaataat gtacatctgg ccttgaaacc 540
accttttatt acatggggtc tagaacttga ccccccttgag ggtgcttgtt ccctctccct 600
gttggtcggt gggttggtag tttctacagt tgggcagctg gttaggtaga gggagttgtc 660
aagtctctgc tggcccagcc aaaccctgtc tgacaacctc ttggtgaacc ttagtaccta 720
aaaggaaatc tcaccccatc ccacaccctg gaggatttca tctcttgtat atgatgatct 780
ggatccacca agacttgttt tatgctcagg gtcaatttct tttttttttt tttttttttt 840
ttttcttttt ctttgagact gggtctcgct ttgttgccca ggctggagtg gagtggcgtg 900
atcttggctt actgcagcct ttgcctcccc ggctcgagca gtcctgcctc agcctccgga 960
gtagctggga ccacaggttc atgccaccat ggccagccaa cttttgcatg ttttgtaaag 1020
atggggtctc acagtgttgc ccaggctggt ctcaaactcc tgggctcagg cgatccacct 1080
gtctcagcct cccagagtgc tgggattaca attgtgagcc accacgtcca gctggaaggg 1140
tcaacatctt ttacattctg caagcacatc tgcattttca ccccaccctt cccctccttc 1200
tccctttttta tatcccattt ttatatcgat ctcttatttt acaataaaac tttgctgcca 1260
aaaaaaaaaa aaaaaaaaaa aaaaaaaaaa aaaaaaaaaa aaaaaa 1306

<210> 349
<211> 341
<212> DNA
<213> Homo sapiens

<400> 349
agaaaataag ccattcctca taccaatata ggatcagctc cttgacctct gaggggcagg 60

agtgcttcct ggtgtgtgta ttagaatccc ttcctgcctt gtttcatggc agtgaaatgc 120
ctcttggtcc tgtccaagtg tatctttcac tgatttctga atcatgttct agttgcttga 180
ccctgccaca tgggtccagt gttcatctga gcataactgt actaaatcct ttttccatat 240
cagtataata aaggagtgat gtgcaataaa aaaaaaaaaa aaaaaaaaaa aaaaaaaaaa 300
aaaaaaaaaa aaaaaaaaaa aaaaaaaaaa aaaaaaaaaa a 341

<210> 350
<211> 791
<212> DNA
<213> Homo sapiens

<400> 350
ggcacctgta gtcccagcta ctcgggaggc tgaggcagga aaatctcttg aacctgggag 60
gcagaggttg cagtgagccg agattgtgcc actgcactct agcctagctg acagagtgag 120
gctccgtcct aaaaaaaaaa aaaagtaaat atctgttgat gaaaaaactg acacttccta 180
tgggtttacc tcctttcctt ccgttgtttt ctctttggta tccctcacgc gttttcccct 240
ctccgctgca gtcacctatt tcccacttgt ttttcttctc tccttcttct ttttcttatt 300
gtgtcctccc tgccaccagt cacaggcttg tggtctacaa ataatgctgg tttgggttta 360
ttttaaaaca tctaacatga gatcagtgcc tgctttttaa agaagcatta cattatgtat 420
tagttataca aattattaga caatgtctta tctttatttt attgttttac acatagaaca 480
gagactattt ggagcctttg gaataacatt ccagcgtata aatataaatg aaatagtttg 540
gcaaattaac tctctccagg ggtcatctag aaatatgatt ctgtcatcag atagaaattc 600
tattgctaga gtcctttagc cagcaaatag atttctatg cttggtgagc aaattcatca 660
caaatttgaa gctagttaca aataaaataa aataaaataa attaattaaa aagaaatttt 720
aaaaatccca acttacagtt taaaaagaag aaaagtggaa aaaaaaaaca atgaacaaaa 780
aaaaaaaaaa a 791

<210> 351
<211> 1474
<212> DNA
<213> Homo sapiens

<400> 351
ttcagcagtt agctaaatta caagatcgag aatggttaac agaacttttt caacaaagca 60
aggtcaatat gcagaaaatg ctcccagaaa ttgatcagaa taaggaccgc atgttggaga 120
ttttggaagg aaagggactg agtttcttat tcccactcct caaattggag aaggaactgt 180
tgaagcaaat aaagttggat ccatcccctc aaaccatata taaatggatt aaagataaca 240
tctctcccaa acttcatgta gataaaggat ttgtgaacat cttaatgact agcttcttac 300
agtacatttc tagtgaagta aacccccccca gcgatgaaac agattcatcc tctgctcctt 360
ccaaagaaca gttagagcag gaaaaacaac tactactatc tttcaagcca gtaatgcaga 420
aatttcttca tgatcacgtt gatctacaag tcagtgccct gtatgctctc caggtgcact 480
gctataacag caacttccca aaaggcatgt tacttcgctt ttttgtgcac ttctatgaca 540
tggaaattat tgaagaagaa gctttcttgg cttggaaaga agatataacc caagagtttc 600
cgggaaaagg caaggctttg ttccaggtga atcagtggct aacctggtta gaaactgctg 660
aagaagaaga atcagaggaa gaagctgact aaagaaccag ccaaagcctt aaattgtgca 720
aaacatactg ttgctatgat gtaactgcat ttgacctaac cactgcgaaa attcattccg 780

ctgtaatgtt ttcacaatat ttaaagcaga agcacgtcag ttaggatttc cttctgcata 840
aggttttttt gtagtgtaat gtcttaatca tagtctacca tcaaatattt taggagtatc 900
tttaatgttt agatagtata ttagcagcat gcaataatta catcataagt tctcaagcag 960
aggcagtcta ttgcaaggac cttctttgct gccagttatc ataggctgtt ttaagttaga 1020
aaactgaata gcaacactga atactgtaga aatgcacttt gctcagtaat acttgagttg 1080
ttgcaatatt tgattatcca tttggttgtt acagaaaaat tcttaactgt aattgatggt 1140
tgttgccgta atagtatatt gcctgtattt ctacctctag taatgggctt tatgtgctag 1200
attttaatat ccttgagcct gggcaagtgc acaagtcttt ttaaaagaaa catggtttac 1260
ttgcacaaaa ctgatcagtt ttgagagatc gttaatgccc ttgaagtggt ttttgtgggt 1320
gtgaaacaaa tggtgagaat ttgaattggt ccctcctatt atagtattga aattaagtct 1380
acttaattta tcaagtcatg ttcatgccct gattttatat acttgtatct atcaataaac 1440
attgtgatac ttgaaaaaaa aaaaaaaaaa aaaa 1474

<210> 352
<211> 2932
<212> DNA
<213> Homo sapiens

<220>
<221> misc_feature
<222> (1332)
<223> "n" may be "a" , "c", "g", or "t".

<400> 352
ccagcctgga cgacagagca gactccatct caaaaaaaaa aaaaaaatgg tcaccccttt 60
tgctcctaaa tcaccctcaa agtaaaagag aacaagaaac agaagcagaa atccatattt 120
agtgaaataa gacaacacct gtagctccaa acctgtagaa gcagatccca gagaaaagca 180
ggccagttct ctcctggaat cccagaaagt cccaggaatt ggaggcttca gtgctgcagt 240
agggagggga ctaaaaacaa agtctgtata tggagcagta agacctccgg gttctcatcc 300
cccatcctgt gtgctctcgg gtgactagcc ctctccctct ccaggtttag cttctggaga 360
aattaaatca aagaggctct agaactgggg atggcaggca ttgagtgcag ggagtgagtt 420
gccagaggct gagagcagag agatttagtg gcagtgggca gagcaaacag caaaatgact 480
gccctcttcc ctggccttgc tccagaaact gagcagccag acatacacac tcccagaagg 540
cagttggagg tccccccggg caatcagaac cacccacaga gaagacctcc agatactgac 600
atttagaagc cttctgacca aagagcttgc ctggccaccc actggcttac tcgctgacag 660
gccatgctcc tgccgtacac accggatcct atcagtcatc ctcatgggct cctttccaga 720
cttctgagga aagcttccaa catgaagagg gagttcaaaa caaacagaga gagagagaga 780
gagagagaga gagagagaga gagagagaga aaagaaatat aaacaatgca gggagcaaaa 840
gataacttca aataaactca aaatattctt ctgggagaga tgagacaata ttgcatacat 900
aaaacaagaa gaggatgcta tcactaaatt ttaaaaaaaa gtaacagaaa gtaagaaaga 960
cctctaagaa atgtttctga aatgaggtat aatttttttt tttttgaggg ggagtcttgc 1020
tctgtcaccc aggctggagc gcagtggcgt gatctcggct cactgcaacc tccgtctccc 1080
gggttcaagt gattctcctg cctcagcttc ctgagtagct gggattacag gcatgcgcca 1140
ccatgcctgg ctaattttgc atttttagta gagacggggt ttcaccatgt tggccaggct 1200
ggtctgaaac tcccgacctc aggtgatctg cccacctcag cctcccaaag tgctgggatt 1260
acaggcatga gctaccgtgc ctggcctgtg tatttttgga gacagagtct tgctctgttg 1320
cccaggctgg anggcagtgg tgcaactcta aactctgcct cccgggttca agcgattctc 1380

gtgcgtcacc tcccaagtag ctggggctag aggcatgtgc caccatgtct ggttactttt 1440
tatataaacc ttcagtttaa tcttttagct tactgctgtg tttttcagct ttattcactt 1500
tgcttttttt ctatattttc gtttcaactc aacttttaaa aaatcatact tgtatatttg 1560
tatatttata tttattttcc aagacctcat ttgcctcttt ttcataatgg gctattattg 1620
ctccatggta cagaatcttc tgttctttcc taaactatta gttattttta aaatatttgt 1680
taactttctc cccctgcta gttctcaggt acccgctttc tcccagagtg ctggctttcc 1740
ttaattattt gctgacttgt gctggtgtgt aacccttcat acttaggtat ttctatttgt 1800
ctgactgctg atttgattcc aatccagtgt ttcttctgat tcgtggagaa gagacgacaa 1860
cgctgtgagg ctctggtttt ggtggcttgt ctgggtgtgg gagctccttg tcatcatggg 1920
atttttagct ccctgggtcg ccatcctaca tggccactct cctgccgatg ctgcccgcta 1980
ctcagcagga ggggaagtcg agaccacctc cttaacctta cagacattga ttgtgagctt 2040
ggagcacctt ccgtgactgg accacccatg cagtggtcct ttgctttttg catttttaac 2100
tgcaattttc cccaagagtc ttccctaata cagtctctta ggaattgacg gtgggattaa 2160
acaaggcatg tttctgcact gaatatggtc tatgggttgc cactttgcaa cctcagcttt 2220
aaggcttatt tcctcccagg aaactgatgt taacttttta agttaaaaat gtgtatataa 2280
gtaatacatg tttattttgg aaattagaaa atacaggcca ggtgtggtgg ctcatgcctg 2340
taatcccaac attttgggag gttgaggcgg gaggattgcc tgggcccggg agtttgagac 2400
cagcctgggc aacataggga aattccatct ctacaaaaaa attaaacatt ggctgggtgt 2460
ggtggtgccc acctgtggtc acagctactc gggagactga ggtgggagga tagcttgggc 2520
aggggaggtt gaggctacag tgagccacga tcacaccaat gtgacacagt gagaccctgt 2580
ctcaaaaaaa aaaaaaaaga aagaaaatat aggtaaacac aaagacaaaa aagcagggca 2640
tggtggctca cacctgtaat tctagcacct tgggaggctg aggcaggagg atcacttgag 2700
gtcaggaact cgagacgaga ccaggctggg cagcatggca ggaccccatc tctataaaaa 2760
gtacaaaaat tagcagggca cggtggtgtg cacctgtggt cctgctactt gggaagttga 2820
ggtgggagga tcacctgagc cctggaggtt gagggtgctg tgagccatga tggcaccact 2880
ccactccagt ccaggtgaaa gagccagatc ctgtctcaaa aaaaaaaaaa aa 2932

<210> 353
<211> 1254
<212> DNA
<213> Homo sapiens

<400> 353
gagagcgggg cctacggcgc ggccaaggcg ggcggctcct tcgacctgcg gcgcttcctg 60
acgcagccgc aggtggtggc gcgcgccgtg tgcttggtct tcgccttgat cgtgttctcc 120
tgcatctatg gtgagggcta cagcaatgcc cacgagtcta agcagatgta ctgcgtgttc 180
aaccgcaacg aggatgcctg ccgctatggc agtgccatcg gggtgctggc ctccctggcc 240
taccagcgct acaaggctgg cgtggacgac ttcatccaga attacgttga ccccactccg 300
gaccccaaca ctgcctacgc ctcctaccca ggtgcatctg tggacaacta ccaacagcca 360
cccttcaccc agaacgcgga gaccaccgag ggctaccagc cgccccctgt gtactgagcg 420
gcggttagcg tgggaagggg gacagagagg gccctcccct ctgccctgga ctttcccatg 480
agcctcctgg aactgccagc ccctctcttt cacctgttcc atcctgtgca gctgacacac 540
agctaaggag cctcatagcc tggcgggggc tggcagagcc acaccccaag tgcctgtgcc 600
cagagggctt cagtcagccg ctcactcctc cagggcactt ttaggaaagg gtttttagct 660
agtgtttttc ctcgctttta atgacctcag ccccgcctgc agtggctaga agccagcagg 720
tgcccatgtg ctactgacaa gtgcctcagc ttccccccgg cccgggtcag gccgtgggag 780
ccgctattat ctgcgttctc tgccaaagac tcgtgggggc catcacacct gccctgtgca 840

gcggagccgg accaggctct tgtgtcctca ctcaggtttg cttcccctgt gcccactgct 900
gtatgatctg ggggccacca ccctgtgccg gtggcctctg ggctgcctcc cgtggtgtga 960
gggcggggct ggtgctcatg gcacttcctc cttgctccca cccctggcag cagggaaggg 1020
ctttgcctga caacacccag ctttatgtaa atattctgca gttgttactt aggaagcctg 1080
gggagggcag gggtgcccca tggctcccag actctgtctg tgccgagtgt attataaaat 1140
cgtgggggag atgcccggcc tgggatgctg tttggagacg gaataaatgt tttctcattc 1200
aaaaaaaaaa aaaaaaaaaa aaaaaaaaaa aaaaaaaaaa aaaaaaaaaa aaaa 1254

<210> 354
<211> 1324
<212> DNA
<213> Homo sapiens

<400> 354
cctttccaag ggagtggttg tgtgatcgcc atcttaggga aaagatgttc tcgtccgtgg 60
cgcacctggc gcgggcgaac cccttcaaca cgccacatct gcagctggtg cacgatggtc 120
tcggggacct ccgcagcagc tccccagggc ccacgggcca gccccgccgc cctcgcaacc 180
tggcagccgc cgccgtggaa gagtacagtt gtgaatttgg ctccgcgaag tattatgcac 240
tgtgtggctt tggtggggtc ttaagttgtg gtctgacaca cactgctgtg gttcccctgg 300
atttagtgaa atgccgtatg caggtggacc cccaaaagta caagggcata tttaacggat 360
tctcagttac acttaaagag gatggtgttc gtggtttggc taaaggatgg gctccgactt 420
tccttggcta ctccatgcag ggactctgca agtttggctt ttatgaagtc tttaaagtct 480
tgtatagcaa tatgcttgga gaggagaata cttatctctg gcgcacatca ctatatttgg 540
ctgcctctgc cagtgctgaa ttctttgctg acattgccct ggctcctatg gaagctgcta 600
aggttcgaat tcaaacccag ccaggttatg ccaacacttt gagggatgca gctcccaaaa 660
tgtataagga agaaggccta aaagcattct acaagggggt tgctcctctc tggatgagac 720
agataccata caccatgatg aagttcgcct gctttgaacg tactgttgaa gcactgtaca 780
agtttgtggt tcctaagccc cgcagtgaat gttcaaagcc agagcagctg gttgtaacat 840
ttgtagcagg ttacatagct ggagtctttt gtgcaattgt ttctcaccct gctgattctg 900
tggtatctgt gttgaataaa gaaaaaggta gcagtgcttc tctggtcctc aagagacttg 960
gatttaaagg tgtatggaag ggactgtttg cccgtatcat catgattggt accctgactg 1020
cactacagtg gtttatctat gactccgtga aggtctactt cagacttcct cgccctcctc 1080
cacccgagat gccagagtct ctgaagaaga agcttgggtt aactcagtag ttagatcaaa 1140
gcaaatgtgg actgaatctg cttgttgatc agtgttgaag aaagtgcaaa aggaactttt 1200
atatatttga cagtgtagga aattgtctat tcctgatata attactgtag tactcttgct 1260
taaggcaaga gtttcagatt tactgttgaa ataaacccaa ctcttcatga aaaaaaaaaa 1320
aaaa 1324

<210> 355
<211> 2303
<212> DNA
<213> Homo sapiens

<400> 355
cggacgcgtg ggcggcgatc gcggcctgag gctgctcccg gacaagggca acgagcgttt 60
cgtttggact tctcgacttg agtgcccgcc tccttcgccg ccgcctctgc agtcctcagc 120

gcagtctttc cacaggagcc agcatacttc ctgaacatgg agagtgttgt tcgccgctgc 180
ccattcttat cccgagtccc ccaggccttt ctgcagaaag caggcaaatc tctgttgttc 240
tatgcccaaa actgccccaa gatgatggaa gttggggcca agccagcccc tcgggcattg 300
tccactgcag cagtacacta ccaacagatc aaagaaaccc ctccggccag tgagaaagac 360
aaaactgcta aggccaaggt ccaacagact cctgatggat cccagcagag tccagatggc 420
acacagcttc cgtctggaca ccccttgcct gccacaagcc agggcactgc aagcaaatgc 480
cctttcctgg cagcacagat gaatcagaga ggcagcagtg tcttctgcaa agccagtctt 540
gagcttcagg aggatgtgca ggaaatgaat gccgtgagga aagaggttgc tgaaacctca 600
gcaggcccca gtgtggttag tgtgaaaacc gatggagggg atcccagtgg actgctgaag 660
aacttccagg acattatgca aaagcaaaga ccagaaagag tgtctcatct tcttcaagat 720
aacttgccaa aatctgtttc cacttttcag tatgatcgtt tctttgagaa aaaaattgat 780
gagaaaaaga atgaccacac ctatcgagtt tttaaaactg tgaaccggcg agcacacatc 840
ttccccatgg cagatgacta ttcagactcc ctcatcacca aaaagcaagt gtcagtctgg 900
tgcagtaatg actacctagg aatgagtcgc cacccacggg tgtgtggggc agttatggac 960
actttgaaac aacatggtgc tggggcaggt ggtactagaa atatttctgg aactagtaaa 1020
ttccatgtgg acttagagcg ggagctggca gacctccatg ggaaagatgc cgcactcttg 1080
ttttcctcgt gctttgtggc caatgactca accctcttca ccctggctaa gatgatgcca 1140
ggctgtgaga tttactctga ttctgggaac catgcctcca tgatccaagg gattcgaaac 1200
agccgagtgc caaagtacat cttccgccac aatgatgtca gccacctcag agaactgctg 1260
caaagatctg acccctcagt ccccaagatt gtggcatttg aaactgtcca ttcaatggat 1320
ggggcggtgt gcccactgga agagctgtgt gatgtggccc atgagtttgg agcaatcacc 1380
ttcgtggatg aggtccacgc agtggggctt tatggggctc gaggcggagg gattggggat 1440
cgggatggag tcatgccaaa aatggacatc atttctggaa cacttggcaa agcctttggt 1500
tgtgttggag ggtacatcgc cagcacgagt tctctgattg acaccgtacg gtcctatgct 1560
gctggcttca tcttcaccac ctctctgcca cccatgctgc tggctggagc cctggagtct 1620
gtgcggatcc tgaagagcgc tgagggacgg gtgcttcgcc gccagcacca gcgcaacgtc 1680
aaactcatga gacagatgct aatggatgcc ggcctccctg ttgtccactg ccccagccac 1740
atcatccctg tgcgggttgc agatgctgct aaaaacacag aagtctgtga tgaactaatg 1800
agcagacata acatctacgt gcaagcaatc aattacccta cggtgccccg gggagaagag 1860
ctcctacgga ttgcccccac ccctcaccac acaccccaga tgatgaacta cttccttgag 1920
aatctgctag tcacatggaa gcaagtgggg ctggaactga agcctcattc ctcagctgag 1980
tgcaacttct gcaggaggcc actgcatttt gaagtgatga gtgaaagaga gaagtcctat 2040
ttctcaggct tgagcaagtt ggtatctgct caggcctgag catgacctca attatttcac 2100
ttaaccccag gccattatca tatccagatg gtcttcagag ttgtctttat atgtgaatta 2160
agttatatta aattttaatc tatagtaaaa acatagtcct ggaaataaat tcttgcttaa 2220
atggtgaaaa aaaaaaaaaa aaaaaaaaaa aaaaaaaaaa aaaaaaaaaa aaaaaaaaaa 2280
aaaaaaaaaa aaaaaaaaaa aaa 2303

<210> 356
<211> 361
<212> PRT
<213> Homo sapiens

<400> 356
Met Phe Ser Ser Val Ala His Leu Ala Arg Ala Asn Pro Phe Asn Thr
1 5 10 15

Pro His Leu Gln Leu Val His Asp Gly Leu Gly Asp Leu Arg Ser Ser
20 25 30

Ser Pro Gly Pro Thr Gly Gln Pro Arg Arg Pro Arg Asn Leu Ala Ala
35 40 45

Ala Ala Val Glu Glu Tyr Ser Cys Glu Phe Gly Ser Ala Lys Tyr Tyr
50 55 60

Ala Leu Cys Gly Phe Gly Gly Val Leu Ser Cys Gly Leu Thr His Thr
65 70 75 80

Ala Val Val Pro Leu Asp Leu Val Lys Cys Arg Met Gln Val Asp Pro
85 90 95

Gln Lys Tyr Lys Gly Ile Phe Asn Gly Phe Ser Val Thr Leu Lys Glu
100 105 110

Asp Gly Val Arg Gly Leu Ala Lys Gly Trp Ala Pro Thr Phe Leu Gly
115 120 125

Tyr Ser Met Gln Gly Leu Cys Lys Phe Gly Phe Tyr Glu Val Phe Lys
130 135 140

Val Leu Tyr Ser Asn Met Leu Gly Glu Glu Asn Thr Tyr Leu Trp Arg
145 150 155 160

Thr Ser Leu Tyr Leu Ala Ala Ser Ala Ser Ala Glu Phe Phe Ala Asp
165 170 175

Ile Ala Leu Ala Pro Met Glu Ala Ala Lys Val Arg Ile Gln Thr Gln
180 185 190

Pro Gly Tyr Ala Asn Thr Leu Arg Asp Ala Ala Pro Lys Met Tyr Lys
195 200 205

Glu Glu Gly Leu Lys Ala Phe Tyr Lys Gly Val Ala Pro Leu Trp Met
210 215 220

Arg Gln Ile Pro Tyr Thr Met Met Lys Phe Ala Cys Phe Glu Arg Thr
225 230 235 240

Val Glu Ala Leu Tyr Lys Phe Val Val Pro Lys Pro Arg Ser Glu Cys
245 250 255

Ser Lys Pro Glu Gln Leu Val Val Thr Phe Val Ala Gly Tyr Ile Ala
260 265 270

Gly Val Phe Cys Ala Ile Val Ser His Pro Ala Asp Ser Val Val Ser
275 280 285

Val Leu Asn Lys Glu Lys Gly Ser Ser Ala Ser Leu Val Leu Lys Arg
290 295 300

Leu Gly Phe Lys Gly Val Trp Lys Gly Leu Phe Ala Arg Ile Ile Met
305 310 315 320

Ile Gly Thr Leu Thr Ala Leu Gln Trp Phe Ile Tyr Asp Ser Val Lys
325 330 335

Val Tyr Phe Arg Leu Pro Arg Pro Pro Pro Pro Glu Met Pro Glu Ser
340 345 350

Leu Lys Lys Lys Leu Gly Leu Thr Gln
355 360

<210> 357
<211> 640
<212> PRT
<213> Homo sapiens

<400> 357
Met Glu Ser Val Val Arg Arg Cys Pro Phe Leu Ser Arg Val Pro Gln
1 5 10 15

Ala Phe Leu Gln Lys Ala Gly Lys Ser Leu Leu Phe Tyr Ala Gln Asn
20 25 30

Cys Pro Lys Met Met Glu Val Gly Ala Lys Pro Ala Pro Arg Ala Leu
35 40 45

Ser Thr Ala Ala Val His Tyr Gln Gln Ile Lys Glu Thr Pro Pro Ala
50 55 60

Ser Glu Lys Asp Lys Thr Ala Lys Ala Lys Val Gln Gln Thr Pro Asp
65 70 75 80

Gly Ser Gln Gln Ser Pro Asp Gly Thr Gln Leu Pro Ser Gly His Pro
85 90 95

Leu Pro Ala Thr Ser Gln Gly Thr Ala Ser Lys Cys Pro Phe Leu Ala
100 105 110

Ala Gln Met Asn Gln Arg Gly Ser Ser Val Phe Cys Lys Ala Ser Leu
115 120 125

Glu Leu Gln Glu Asp Val Gln Glu Met Asn Ala Val Arg Lys Glu Val
130 135 140

Ala Glu Thr Ser Ala Gly Pro Ser Val Val Ser Val Lys Thr Asp Gly
145 150 155 160

Gly Asp Pro Ser Gly Leu Leu Lys Asn Phe Gln Asp Ile Met Gln Lys
165 170 175

Gln Arg Pro Glu Arg Val Ser His Leu Leu Gln Asp Asn Leu Pro Lys
180 185 190

Ser Val Ser Thr Phe Gln Tyr Asp Arg Phe Phe Glu Lys Lys Ile Asp
195 200 205

Glu Lys Lys Asn Asp His Thr Tyr Arg Val Phe Lys Thr Val Asn Arg
210 215 220

Arg Ala His Ile Phe Pro Met Ala Asp Asp Tyr Ser Asp Ser Leu Ile
225 230 235 240

Thr Lys Lys Gln Val Ser Val Trp Cys Ser Asn Asp Tyr Leu Gly Met
245 250 255

Ser Arg His Pro Arg Val Cys Gly Ala Val Met Asp Thr Leu Lys Gln
260 265 270

His Gly Ala Gly Ala Gly Gly Thr Arg Asn Ile Ser Gly Thr Ser Lys
275 280 285

Phe His Val Asp Leu Glu Arg Glu Leu Ala Asp Leu His Gly Lys Asp
290 295 300

Ala Ala Leu Leu Phe Ser Ser Cys Phe Val Ala Asn Asp Ser Thr Leu
305 310 315 320

Phe Thr Leu Ala Lys Met Met Pro Gly Cys Glu Ile Tyr Ser Asp Ser
325 330 335

Gly Asn His Ala Ser Met Ile Gln Gly Ile Arg Asn Ser Arg Val Pro
340 345 350

Lys Tyr Ile Phe Arg His Asn Asp Val Ser His Leu Arg Glu Leu Leu
355 360 365

Gln Arg Ser Asp Pro Ser Val Pro Lys Ile Val Ala Phe Glu Thr Val
370 375 380

His Ser Met Asp Gly Ala Val Cys Pro Leu Glu Glu Leu Cys Asp Val
385 390 395 400

Ala His Glu Phe Gly Ala Ile Thr Phe Val Asp Glu Val His Ala Val
405 410 415

Gly Leu Tyr Gly Ala Arg Gly Gly Gly Ile Gly Asp Arg Asp Gly Val
420 425 430

Met Pro Lys Met Asp Ile Ile Ser Gly Thr Leu Gly Lys Ala Phe Gly
435 440 445

Cys Val Gly Gly Tyr Ile Ala Ser Thr Ser Ser Leu Ile Asp Thr Val
450 455 460

Arg Ser Tyr Ala Ala Gly Phe Ile Phe Thr Thr Ser Leu Pro Pro Met
465 470 475 480

Leu Leu Ala Gly Ala Leu Glu Ser Val Arg Ile Leu Lys Ser Ala Glu
485 490 495

Gly Arg Val Leu Arg Arg Gln His Gln Arg Asn Val Lys Leu Met Arg
500 505 510

Gln Met Leu Met Asp Ala Gly Leu Pro Val Val His Cys Pro Ser His
515 520 525

Ile Ile Pro Val Arg Val Ala Asp Ala Ala Lys Asn Thr Glu Val Cys
530 535 540

Asp Glu Leu Met Ser Arg His Asn Ile Tyr Val Gln Ala Ile Asn Tyr
545 550 555 560

Pro Thr Val Pro Arg Gly Glu Glu Leu Leu Arg Ile Ala Pro Thr Pro
565 570 575

His His Thr Pro Gln Met Met Asn Tyr Phe Leu Glu Asn Leu Leu Val
580 585 590

Thr Trp Lys Gln Val Gly Leu Glu Leu Lys Pro His Ser Ser Ala Glu
595 600 605

Cys Asn Phe Cys Arg Arg Pro Leu His Phe Glu Val Met Ser Glu Arg
610 615 620

Glu Lys Ser Tyr Phe Ser Gly Leu Ser Lys Leu Val Ser Ala Gln Ala
625 630 635 640

<210> 358
<211> 9
<212> PRT
<213> Artificial Sequence

<220>
<223> Description of Artificial Sequence:Designed peptide recognized by HLA-A2 restricted cytotoxic T lymphocytes

<400> 358
Gln Ile Gly Ala Lys Phe Trp Glu Val
1 5

<210> 359
<211> 9
<212> PRT
<213> Artificial Sequence

<220>
<223> Description of Artificial Sequence:Designed peptide recognized by HLA-A2 restricted cytotoxic T lymphocytes

<400> 359
Phe Met Pro Gly Phe Ala Pro Leu Thr
1 5

<210> 360
<211> 10
<212> PRT
<213> Artificial Sequence

<220>
<223> Description of Artificial Sequence:Designed peptide recognized by HLA-A2 restricted cytotoxic T lymphocytes

<400> 360
Thr Leu Leu Val Ala Val Phe Gln Asp Val
1 5 10

<210> 361
<211> 10
<212> PRT
<213> Artificial Sequence

<220>
<223> Description of Artificial Sequence:Designed peptide recognized by HLA-A2 restricted cytotoxic T lymphocytes

<400> 361
Val Ala Tyr Leu Gly Phe Val Phe Tyr Leu
1 5 10

<210> 362
<211> 10
<212> PRT
<213> Artificial Sequence

<220>
<223> Description of Artificial Sequence:Designed peptide recognized by HLA-A2 restricted cytotoxic T lymphocytes

<400> 362
Leu Leu Pro Thr Leu Arg Lys Gln Tyr Cys
1 5 10

<210> 363
<211> 9
<212> PRT
<213> Artificial Sequence

<220>
<223> Description of Artificial Sequence:Designed peptide recognized by HLA-A2 restricted cytotoxic T lymphocytes

<400> 363
Met Val Tyr Asp Leu Tyr Lys Thr Leu
1 5

<210> 364

<211> 10
<212> PRT
<213> Artificial Sequence

<220>
<223> Description of Artificial Sequence:Designed peptide recognized by HLA-A2 restricted cytotoxic T lymphocytes

<400> 364
Gly Leu Cys Lys Phe Gly Phe Tyr Glu Val
1 5 10

<210> 365
<211> 9
<212> PRT
<213> Artificial Sequence

<220>
<223> Description of Artificial Sequence:Designed peptide recognized by HLA-A2 restricted cytotoxic T lymphocytes

<400> 365
Phe Gly Phe Tyr Glu Val Phe Lys Val
1 5

<210> 366
<211> 9
<212> PRT
<213> Artificial Sequence

<220>
<223> Description of Artificial Sequence:Designed peptide recognized by HLA-A2 restricted cytotoxic T lymphocytes

<400> 366
Leu Gln Trp Phe Ile Tyr Asp Ser Val
1 5

<210> 367
<211> 10
<212> PRT
<213> Artificial Sequence

<220>
<223> Description of Artificial Sequence:Designed peptide recognized by HLA-A2 restricted cytotoxic T lymphocytes

<400> 367
Ala Leu Ala Pro Met Glu Ala Ala Lys Val
1 5 10

<210> 368
<211> 10
<212> PRT
<213> Artificial Sequence

<220>
<223> Description of Artificial Sequence:Designed peptide recognized by HLA-A2 restricted cytotoxic T lymphocytes

<400> 368
Arg Thr Val Glu Ala Leu Tyr Lys Phe Val
1 5 10

<210> 369
<211> 9
<212> PRT
<213> Artificial Sequence

<220>
<223> Description of Artificial Sequence:Designed peptide recognized by HLA-A2 restricted cytotoxic T lymphocytes

<400> 369
Val Leu Ser Cys Gly Leu Thr His Thr
1 5

<210> 370
<211> 9
<212> PRT
<213> Artificial Sequence

<220>
<223> Description of Artificial Sequence:Designed

peptide recognized by HLA-A2 restricted cytotoxic T lymphocytes

<400> 370
Ala Leu Leu Phe Ser Ser Cys Phe Val
1 5

<210> 371
<211> 10
<212> PRT
<213> Artificial Sequence

<220>
<223> Description of Artificial Sequence:Designed peptide recognized by HLA-A2 restricted cytotoxic T lymphocytes

<400> 371
Phe Leu Ser Arg Val Pro Gln Ala Phe Leu
1 5 10

<210> 372
<211> 10
<212> PRT
<213> Artificial Sequence

<220>
<223> Description of Artificial Sequence:Designed peptide recognized by HLA-A2 restricted cytotoxic T lymphocytes

<400> 372
Met Leu Leu Ala Gly Ala Leu Glu Ser Val
1 5 10

<210> 373
<211> 10
<212> PRT
<213> Artificial Sequence

<220>
<223> Description of Artificial Sequence:Designed peptide recognized by HLA-A2 restricted cytotoxic T lymphocytes

<400> 373
Leu Leu Gln Asp Asn Leu Pro Lys Ser Val
1 5 10

<210> 374
<211> 9
<212> PRT
<213> Artificial Sequence

<220>
<223> Description of Artificial Sequence:Designed peptide recognized by HLA-A2 restricted cytotoxic T lymphocytes

<400> 374
Leu Met Ser Arg His Asn Ile Tyr Val
1 5

<210> 375
<211> 10
<212> PRT
<213> Artificial Sequence

<220>
<223> Description of Artificial Sequence:Designed peptide recognized by HLA-A2 restricted cytotoxic T lymphocytes

<400> 375
Ser Leu Ile Asp Thr Val Arg Ser Tyr Ala
1 5 10

<210> 376
<211> 10
<212> PRT
<213> Artificial Sequence

<220>
<223> Description of Artificial Sequence:Designed peptide recognized by HLA-A2 restricted cytotoxic T lymphocytes

<400> 376
Phe Leu Gln Lys Ala Gly Lys Ser Leu Leu
1 5 10

<210> 377
<211> 9
<212> PRT
<213> Artificial Sequence

<220>
<223> Description of Artificial Sequence:Designed peptide recognized by HLA-A2 restricted cytotoxic T lymphocytes

<400> 377
Leu Leu Phe Ser Ser Cys Phe Val Ala
1 5

<210> 378
<211> 9
<212> PRT
<213> Artificial Sequence

<220>
<223> Description of Artificial Sequence:Designed peptide recognized by HLA-A2 restricted cytotoxic T lymphocytes

<400> 378
Gly Leu Leu Lys Asn Phe Gln Asp Ile
1 5

<210> 379
<211> 9
<212> PRT
<213> Artificial Sequence

<220>
<223> Description of Artificial Sequence:Designed peptide recognized by HLA-A2 restricted cytotoxic T lymphocytes

<400> 379
Ser Val Trp Cys Ser Asn Asp Tyr Leu
1 5

<210> 380

<211> 10
<212> PRT
<213> Artificial Sequence

<220>
<223> Description of Artificial Sequence:Designed peptide recognized by HLA-A2 restricted cytotoxic T lymphocytes

<400> 380
Leu Leu Val Thr Trp Lys Gln Val Gly Leu
1 5 10

<210> 381
<211> 10
<212> PRT
<213> Artificial Sequence

<220>
<223> Description of Artificial Sequence:Designed peptide recognized by HLA-A2 restricted cytotoxic T lymphocytes

<400> 381
Val Ala Asn Asp Ser Thr Leu Phe Thr Leu
1 5 10

<210> 382
<211> 974
<212> DNA
<213> Homo sapiens

<400> 382
gaaacaagtt gaagtacctg gctttcctcc gcaagcggat gaacaccaac ccttcccgag 60
gcccctacca cttccgggcc cccagccgca tcttctggcg gaccgtgcga ggtatgctgc 120
cccacaaaac caagcgaggc caggccgctc tggaccgtct caaggtgttt gacggcatcc 180
cacctcccta cgacaagaaa aagcggatgg tggttcctgc tgccctcaag gtcgtgcgtc 240
tgaagcctac aagaaagttt gcctatctgg ggcgcctggc tcacgaggtt ggctggaagt 300
accaggcagt gacagccacc ctggaggaga agaggaaaga gaaagccaag atccactacc 360
ggaagaagaa acagctcatg aggctacgga aacaggccga gaagaacgtg gagaagaaaa 420
ttgacaaata cacagaggtc ctcaagaccc acggactcct ggtctgagcc caataaagac 480
tgttaattcc tcatgcgttg cctgcccttc ctccattgtt gccctggaat gtacgggacc 540
caggggcagc agcagtccag gtgccacagg cagccctggg acataggaag ctgggagcaa 600
ggaaagggtc ttagtcactg cctcccgaag ttgcttgaaa gcactcggag aattgtgcag 660
gtgtcattta tctatgacca ataggaagag caaccagtta ctatgagtga aagggagcca 720
gaagactgat tggagggccc tatcttgtga gtggggcatc tgttggactt cccacctggt 780

catatactct gcagctgtta gaatgtgcaa gcacttgggg acagcatgag cttgctgttg 840
tacacagggt atttctagaa gcagaaatag actgggaaga tgcacaacca aggggttaca 900
ggcatcgccc atgctcctca cctgtatttt gtaatcagaa ataaattgct tttaaagaaa 960
aaaaaaaaaa aaaa 974

<210> 383
<211> 821
<212> DNA
<213> Homo sapiens

<400> 383
ggggccgggg ccgaggccgc ggagctcgcg gaggcaaggc cgaggataag gagtggatgc 60
ccgtcaccaa gttgggccgc ttggtcaagg acatgaagat caagtccctg gaggagatct 120
atctcttctc cctgcccatt aaggaatcag agatcattga tttcttcctg gggcctctc 180
tcaaggatga ggttttgaag attatgccag tgcagaagca gacccgtgcc ggccagcgca 240
ccaggttcaa ggcatttgtt gctatcgggg actacaatgg ccacgtcggt ctgggtgtta 300
agtgctccaa ggaggtggcc accgccatcc gtggggccat catcctggcc aagctctcca 360
tcgtccccgt gcgcagaggc tactgggggga acaagatcgg caagccccac actgtccctt 420
gcaaggtgac aggccgctgc ggctctgtgc tggtacgcct catccctgca cccaggggca 480
ctggcatcgt ctccgcacct gtgcctaaga agctgctcat gatggctggt atcgatgact 540
gctacacctc agcccggggc tgcactgcca ccctgggcaa cttcgccaag gccacctttg 600
atgccatttc taagacctac agctacctga cccccgacct ctggaaggag actgtattca 660
ccaagtctcc ctatcaggag ttcactgacc acctcgtcaa gacccacacc agagtctccg 720
tgcagcggac tcaggctcca gctgtggcta caacataggg tttttataca agaaaaataa 780
agtgaattta gcgtgaaaaa aaaaaaaaaa aaaaaaaaaa a 821

<210> 384
<211> 741
<212> DNA
<213> Homo sapiens

<400> 384
gcggcgtgag aagccatgag cagcaaagtc tctcgcgaca ccctgtacga ggcggtgcgg 60
gaagtcctgc acgggaacca gcgcaagcgc cgcaagttcc tggagacggt ggagttgcag 120
atcagcttga agaactatga tccccagaag gacaagcgct tctcgggcac cgtcaggctt 180
aagtccactc cccgccctaa gttctctgtg tgtgtcctgg gggaccagca gcactgtgac 240
gaggctaagg ccgtggatat cccccacatg gacatcgagg cgctgaaaaa actcaacaag 300
aataaaaaac tggtcaagaa gctggccaag aagtatgatg cgtttttggc ctcagagtct 360
ctgatcaagc agattccacg aatcctcggc ccaggtttaa ataaggcagg aaagttccct 420
tccctgctca cacacaacga aaacatggtg gccaaagtgg atgaggtgaa gtccacaatc 480
aagttccaaa tgaagaaggt gttatgtctg gctgtagctg ttggtcacgt gaagatgaca 540
gacgatgagc ttgtgtataa cattcacctg gctgtcaact tcttggtgtc attgctcaag 600
aaaaactggc agaatgtccg ggccttatat atcaagagcc ccatgggcaa gccccagcgc 660
ctatattaag gcccatttga ataaattcta tttcccgttt aaaaaaaaaa aaaaaaaaaa 720
aaaaaaaaaa aaaaaaaaaa a 741

<210> 385
<211> 142
<212> PRT
<213> Homo sapiens

<400> 385
Met Asn Thr Asn Pro Ser Arg Gly Pro Tyr His Phe Arg Ala Pro Ser
1 5 10 15

Arg Ile Phe Trp Arg Thr Val Arg Gly Met Leu Pro His Lys Thr Lys
20 25 30

Arg Gly Gln Ala Ala Leu Asp Arg Leu Lys Val Phe Asp Gly Ile Pro
35 40 45

Pro Pro Tyr Asp Lys Lys Lys Arg Met Val Val Pro Ala Ala Leu Lys
50 55 60

Val Val Arg Leu Lys Pro Thr Arg Lys Phe Ala Tyr Leu Gly Arg Leu
65 70 75 80

Ala His Glu Val Gly Trp Lys Tyr Gln Ala Val Thr Ala Thr Leu Glu
85 90 95

Glu Lys Arg Lys Glu Lys Ala Lys Ile His Tyr Arg Lys Lys Lys Gln
100 105 110

Leu Met Arg Leu Arg Lys Gln Ala Glu Lys Asn Val Glu Lys Lys Ile
115 120 125

Asp Lys Tyr Thr Glu Val Leu Lys Thr His Gly Leu Leu Val
130 135 140

<210> 386
<211> 233
<212> PRT
<213> Homo sapiens

<400> 386
Met Pro Val Thr Lys Leu Gly Arg Leu Val Lys Asp Met Lys Ile Lys
1 5 10 15

Ser Leu Glu Glu Ile Tyr Leu Phe Ser Leu Pro Ile Lys Glu Ser Glu
20 25 30

Ile Ile Asp Phe Phe Leu Gly Ala Ser Leu Lys Asp Glu Val Leu Lys

35 40 45

Ile Met Pro Val Gln Lys Gln Thr Arg Ala Gly Gln Arg Thr Arg Phe
50 55 60

Lys Ala Phe Val Ala Ile Gly Asp Tyr Asn Gly His Val Gly Leu Gly
65 70 75 80

Val Lys Cys Ser Lys Glu Val Ala Thr Ala Ile Arg Gly Ala Ile Ile
85 90 95

Leu Ala Lys Leu Ser Ile Val Pro Val Arg Arg Gly Tyr Trp Gly Asn
100 105 110

Lys Ile Gly Lys Pro His Thr Val Pro Cys Lys Val Thr Gly Arg Cys
115 120 125

Gly Ser Val Leu Val Arg Leu Ile Pro Ala Pro Arg Gly Thr Gly Ile
130 135 140

Val Ser Ala Pro Val Pro Lys Lys Leu Leu Met Met Ala Gly Ile Asp
145 150 155 160

Asp Cys Tyr Thr Ser Ala Arg Gly Cys Thr Ala Thr Leu Gly Asn Phe
165 170 175

Ala Lys Ala Thr Phe Asp Ala Ile Ser Lys Thr Tyr Ser Tyr Leu Thr
180 185 190

Pro Asp Leu Trp Lys Glu Thr Val Phe Thr Lys Ser Pro Tyr Gln Glu
195 200 205

Phe Thr Asp His Leu Val Lys Thr His Thr Arg Val Ser Val Gln Arg
210 215 220

Thr Gln Ala Pro Ala Val Ala Thr Thr
225 230

<210> 387
<211> 217
<212> PRT
<213> Homo sapiens

<400> 387
Met Ser Ser Lys Val Ser Arg Asp Thr Leu Tyr Glu Ala Val Arg Glu
1 5 10 15

Val Leu His Gly Asn Gln Arg Lys Arg Arg Lys Phe Leu Glu Thr Val
20 25 30

Glu Leu Gln Ile Ser Leu Lys Asn Tyr Asp Pro Gln Lys Asp Lys Arg
35 40 45

Phe Ser Gly Thr Val Arg Leu Lys Ser Thr Pro Arg Pro Lys Phe Ser
50 55 60

Val Cys Val Leu Gly Asp Gln Gln His Cys Asp Glu Ala Lys Ala Val
65 70 75 80

Asp Ile Pro His Met Asp Ile Glu Ala Leu Lys Lys Leu Asn Lys Asn
85 90 95

Lys Lys Leu Val Lys Lys Leu Ala Lys Lys Tyr Asp Ala Phe Leu Ala
100 105 110

Ser Glu Ser Leu Ile Lys Gln Ile Pro Arg Ile Leu Gly Pro Gly Leu
115 120 125

Asn Lys Ala Gly Lys Phe Pro Ser Leu Leu Thr His Asn Glu Asn Met
130 135 140

Val Ala Lys Val Asp Glu Val Lys Ser Thr Ile Lys Phe Gln Met Lys
145 150 155 160

Lys Val Leu Cys Leu Ala Val Ala Val Gly His Val Lys Met Thr Asp
165 170 175

Asp Glu Leu Val Tyr Asn Ile His Leu Ala Val Asn Phe Leu Val Ser
180 185 190

Leu Leu Lys Lys Asn Trp Gln Asn Val Arg Ala Leu Tyr Ile Lys Ser
195 200 205

Pro Met Gly Lys Pro Gln Arg Leu Tyr
210 215

<210> 388
<211> 9
<212> PRT
<213> Artificial Sequence

<220>
<223> Description of Artificial Sequence:Designed peptide recognized by HLA-A26 restricted cytotoxic

T lymphocytes

<400> 388
Leu Val Leu Asp Gly Arg Gly His Leu
1 5

<210> 389
<211> 9
<212> PRT
<213> Artificial Sequence

<220>
<223> Description of Artificial Sequence:Designed peptide recognized by HLA-A26 restricted cytotoxic T lymphocytes

<400> 389
His Leu Leu Gly Arg Leu Ala Ala Ile
1 5

<210> 390
<211> 9
<212> PRT
<213> Artificial Sequence

<220>
<223> Description of Artificial Sequence:Designed peptide recognized by HLA-A26 restricted cytotoxic T lymphocytes

<400> 390
Ala Ile Val Ala Lys Gln Val Leu Leu
1 5

<210> 391
<211> 9
<212> PRT
<213> Artificial Sequence

<220>
<223> Description of Artificial Sequence:Designed peptide recognized by HLA-A26 restricted cytotoxic T lymphocytes

<400> 391

Val Leu Leu Gly Arg Lys Val Val Val
1 5

<210> 392
<211> 9
<212> PRT
<213> Artificial Sequence

<220>
<223> Description of Artificial Sequence:Designed peptide recognized by HLA-A26 restricted cytotoxic T lymphocytes

<400> 392
Ala Phe Leu Arg Lys Arg Met Asn Thr
1 5

<210> 393
<211> 9
<212> PRT
<213> Artificial Sequence

<220>
<223> Description of Artificial Sequence:Designed peptide recognized by HLA-A26 restricted cytotoxic T lymphocytes

<400> 393
His Phe Arg Ala Pro Ser Arg Ile Phe
1 5

<210> 394
<211> 9
<212> PRT
<213> Artificial Sequence

<220>
<223> Description of Artificial Sequence:Designed peptide recognized by HLA-A26 restricted cytotoxic T lymphocytes

<400> 394
Val Leu Lys Thr His Gly Leu Leu Val
1 5

<210> 395
<211> 9
<212> PRT
<213> Artificial Sequence

<220>
<223> Description of Artificial Sequence:Designed peptide recognized by HLA-A26 restricted cytotoxic T lymphocytes

<400> 395
Pro Val Thr Lys Leu Gly Arg Leu Val
1 5

<210> 396
<211> 9
<212> PRT
<213> Artificial Sequence

<220>
<223> Description of Artificial Sequence:Designed peptide recognized by HLA-A26 restricted cytotoxic T lymphocytes

<400> 396
Lys Ile Met Pro Val Gln Lys Gln Thr
1 5

<210> 397
<211> 9
<212> PRT
<213> Artificial Sequence

<220>
<223> Description of Artificial Sequence:Designed peptide recognized by HLA-A26 restricted cytotoxic T lymphocytes

<400> 397
Val Thr Gly Arg Cys Gly Ser Val Leu
1 5

<210> 398
<211> 9

<212> PRT
<213> Artificial Sequence

<220>
<223> Description of Artificial Sequence:Designed peptide recognized by HLA-A26 restricted cytotoxic T lymphocytes

<400> 398
Arg Leu Ile Pro Ala Pro Arg Gly Thr
1 5

<210> 399
<211> 9
<212> PRT
<213> Artificial Sequence

<220>
<223> Description of Artificial Sequence:Designed peptide recognized by HLA-A26 restricted cytotoxic T lymphocytes

<400> 399
Asp Leu Trp Lys Glu Thr Val Phe Thr
1 5

<210> 400
<211> 9
<212> PRT
<213> Artificial Sequence

<220>
<223> Description of Artificial Sequence:Designed peptide recognized by HLA-A26 restricted cytotoxic T lymphocytes

<400> 400
His Leu Val Lys Thr His Thr Arg Val
1 5

<210> 401
<211> 9
<212> PRT
<213> Artificial Sequence

<220>
<223> Description of Artificial Sequence:Designed peptide recognized by HLA-A26 restricted cytotoxic T lymphocytes

<400> 401
His Thr Arg Val Ser Val Gln Arg Thr
1 5

<210> 402
<211> 9
<212> PRT
<213> Artificial Sequence

<220>
<223> Description of Artificial Sequence:Designed peptide recognized by HLA-A26 restricted cytotoxic T lymphocytes

<400> 402
Arg Thr Gln Ala Pro Ala Val Ala Thr
1 5

<210> 403
<211> 9
<212> PRT
<213> Artificial Sequence

<220>
<223> Description of Artificial Sequence:Designed peptide recognized by HLA-A26 restricted cytotoxic T lymphocytes

<400> 403
Thr Leu Tyr Glu Ala Val Arg Glu Val
1 5

<210> 404
<211> 9
<212> PRT
<213> Artificial Sequence

<220>
<223> Description of Artificial Sequence:Designed peptide recognized by HLA-A26 restricted cytotoxic

T lymphocytes

<400> 404
Glu Thr Val Glu Leu Gln Ile Ser Leu
1 5

<210> 405
<211> 9
<212> PRT
<213> Artificial Sequence

<220>
<223> Description of Artificial Sequence:Designed peptide recognized by HLA-A26 restricted cytotoxic T lymphocytes

<400> 405
Lys Val Asp Glu Val Lys Ser Thr Ile
1 5

<210> 406
<211> 9
<212> PRT
<213> Artificial Sequence

<220>
<223> Description of Artificial Sequence:Designed peptide recognized by HLA-A26 restricted cytotoxic T lymphocytes

<400> 406
Thr Ile Lys Phe Gln Met Lys Val Leu
1 5

<210> 407
<211> 9
<212> PRT
<213> Artificial Sequence

<220>
<223> Description of Artificial Sequence:Designed peptide recognized by HLA-A26 restricted cytotoxic T lymphocytes

<400> 407

Lys Val Leu Cys Leu Ala Val Ala Val
1 5

<210> 408
<211> 9
<212> PRT
<213> Artificial Sequence

<220>
<223> Description of Artificial Sequence:Designed peptide recognized by HLA-A26 restricted cytotoxic T lymphocytes

<400> 408
Ser Thr Met Gly Lys Pro Gln Arg Leu
1 5

## Description

### FIELD OF THE INVENTION

The present invention relates to a tumor antigen, and more particularly to a polypeptide or a peptide recognized by tumor-specific cytotoxic T lymphocytes, a polynucleotide encoding the polypeptide or the peptide and a complementary strand thereto, a recombinant vector comprising the polynucleotide, a transformant comprising the recombinant vector, an antibody against the polypeptide or the peptide, a compound having any interaction with the polypeptide or the peptide and/or an HLA or with the polynucleotide, a cytotoxic T lymphocyte inducer consisting of the peptide and/or the polypeptide, and a pharmaceutical composition comprising the same, and a method for producing the polypeptide and the peptide, a method for screening for the compound, a method for inducing cytotoxic T lymphocytes using the polypeptide and/or the peptide, a method for measuring the polypeptide or the peptide or the polynucleotide encoding the same, and a reagent kit used for the screening method or the measuring method.

### BACKGROUND OF THE INVENTION

The immune system, particularly cytotoxic T lymphocytes that participate in a cellular immunity play an important role in the exclusion of cancer *in vivo.* Infiltration of cytotoxic T lymphocytes exhibiting cytotoxicity against tumor cells has been detected at the tumor site of a cancer patient (Arch. Surg., 126:200-205, 1990.) A target molecule (tumor antigen) of the tumor-specific cytotoxic T lymphocytes was first discovered in a melanoma. A tumor antigen generated in a tumor cell is degraded in the cell into a peptide (tumor antigen peptide) consisting of 8 to 11 amino acids, which binds to a human leukocyte antigen (HLA) molecule that is the major histocompatibility complex antigen to be presented on the surface of the tumor cell. The cytotoxic T lymphocytes recognize a complex consisting of an HLA molecule and the tumor antigen peptide, and damage the tumor cell. In other words, the cytotoxic T lymphocytes recognize the tumor cells in an HLA-restricted manner.

HLA is a cell membrane antigen, and is expressed on almost all eukaryotic cells. HLA is mainly classified into class I antigen and class II antigen. The HLA recognized by the cytotoxic T lymphocytes together with an antigen peptide belongs to class I antigens. HLA class I antigens are further classified into HLA-A, HLA-B, HLA-C and so on. HLA-A, HLA-B, and HLA-C are expressed on each human eukaryotic cell in various amounts. It has been reported that HLA has genetic polymorphism. For example, HLA-A has diversity such as A1, A2, A24, A26, A31, and so on. HLA-B has diversity such as B8, B27, B46, and so on. HLA-C has diversity such as Cw3, Cw6, and so on. In this context, the type of HLA is not same in each individual. The HLA-A2 allele is found in approximately 40% of Japanese, approximately 53% of Chinese, approximately 49% of Northern Caucasians, approximately 38% of Southern Caucasians, and approximately 23% of African Blacks. The HLA-A26 allele is found in approximately 22% of Japanese, approximately 16% of Koreans, and approximately 8% of Northern Caucasians (Imanishi, T. et al., HLA 1991, 1: 1065-1220, 1992, Oxford, Oxford Scientific publications.)

When a complex consisting of an HLA class I antigen and a tumor antigen peptide is recognized by cytotoxic T lymphocytes, the type of HLA is also recognized thereby. The amino acid sequence of the tumor antigen peptide capable of binding to HLA molecule is well-known to have a motif (a specific sequence) that differs depending on each type of HLA.

In recent years, molecules, such as a tumor rejection antigen gene, a T cell receptor (TCR), and so, which are involved in specific immunity, have been identified in melanoma, esophageal cancer, and other cancers, and a specific immunotherapy of advanced cancer or metastatic cancer has been studied using the peptide (Science, 254: 1643-1647, 1991; J. Exp. Med, 183:1185-1192, 1996; J. Immunol., 163:4994-5004, 1999; Proc. Natl. Acad. Sci. USA, 92:432-436, 1995; Science, 269:1281-1284, 1995; J. Exp. Med, 186:785-793, 1997.)

Now, in Europe and in the United States, cancer vaccine therapy has been developed in which cytotoxic T lymphocytes are activated by an administration of a tumor antigen in a cancer patient. Results from a clinical test of a melanoma specific tumor antigen have been reported. For example, administration of a melanoma antigen gp-100 peptide subcutaneously to melanoma patients along with administering interleukin-2 (IL-2) intravenously gave a tumor regression in 42% of the patients (Nature Medicine, 4:321, 1998.) In this way, by utilizing a tumor antigen as a vaccine, an effective treatment against cancer can be achieved.

However, almost all of the identified tumor antigens are derived from melanomas. Tumor antigens derived from epithelial cancers and adenocarcinomas, which occur at high incidence rates, have been reported for such specific immunotherapy only in a few papers. In addition, in view of the diversity of cancer, an identical tumor antigen may not be expressed in the same degree in all cancer cells. Variations of the type or the tissue of cancer cells gives variations of the type or the amount of a tumor antigen being expressed in the same. In addition, the type of HLA varies in each individual because of the polymorphism of HLA gene, so that the type of a tumor antigen peptide capable of working in each individual is considered to be different. Naturally, cancer vaccine therapy by activating the cytotoxic T lymphocytes using one kind of tumor antigen has a therapeutic effect on cancer having the tumor antigen. However, in order to induce and/or activate specific cytotoxic T lymphocytes in cancer therapy and obtain a high therapeutic effect corresponding to the diversity of cancer, it is important to discover and use many novel tumor antigens.

## SUMMARY OF THE INVENTION

An embodiment of the present invention is a peptide consisting of an amino acid sequence selected from the group consisting of SEQ ID NO:1 to SEQ ID NO:213, SEQ ID NO:358 to SEQ ID NO:381, and SEQ ID NO:388 to SEQ ID NO:408 in the sequence listing.

Another embodiment of the present invention is a polypeptide consisting of an amino acid sequence selected from the group consisting of SEQ ID NO:215 to SEQ ID NO:288 (excluding SEQ ID NO:228, SEQ ID NO:236, SEQ ID NO:261, and SEQ ID NO:269), SEQ ID NO:356, SEQ ID NO:357, SEQ ID NO:385, and SEQ ID NO:386 in the sequence listing.

Still another embodiment of the present invention is a peptide consisting of an amino acid sequence selected from the group consisting of SEQ ID NO:1 to SEQ ID NO:213, SEQ ID NO:358 to SEQ ID NO:381, and SEQ ID NO: 388 to SEQ ID NO:408 in the sequence listing, wherein the peptide is recognized by a cytotoxic T lymphocyte and/or induces a cytotoxic T lymphocyte.

Yet another embodiment of the present invention is the peptide, wherein the peptide is recognized by a cytotoxic T lymphocyte in an HLA-A2-restricted manner or HLA-A26-restricted manner and/or induces a cytotoxic T lymphocyte in an HLA-A2-restricted manner or HLA-A26-restricted manner when it is recognized by a cytotoxic T lymphocyte and/or induces a cytotoxic T lymphocyte.

An additionally, embodiment of the present invention is a polypeptide consisting of an amino acid sequence selected from the group consisting of SEQ ID NO:214 to SEQ ID NO:288, SEQ ID NO:356, SEQ ID NO:357, and SEQ ID NO:385 to SEQ ID NO:387 in the sequence listing, wherein the polypeptide is recognized by a cytotoxic T lymphocyte and/or induces a cytotoxic T lymphocyte.

An embodiment of the present invention is a polypeptide, wherein the polypeptide is recognized by the cytotoxic T lymphocyte in an HLA-A2-restricted manner or HLA-A26-restricted manner and/or induces the cytotoxic T lymphocyte in an HLA-A2-restricted manner or HLA-A26-restricted manner when it is recognized by the above cytotoxic T lymphocyte and/or induces the cytotoxic T lymphocyte.

Another embodiment of the present invention is a medicament consisting of one or more of peptides that consist of an amino acid sequence selected from the group consisting of SEQ ID NO:1 to SEQ ID NO:213, SEQ ID NO:358 to SEQ ID NO:381, and SEQ ID NO:388 to SEQ ID NO:408 in the sequence listing, and/or, one or more of polypeptides that consist of an amino acid sequence selected from the group consisting of SEQ ID NO:214 to SEQ ID NO:288, SEQ ID NO:356, SEQ ID NO:357, and SEQ ID NO:385 to SEQ ID NO:387 in the sequence listing.

Yet another embodiment of the present invention is a cancer vaccine comprising one or more of peptides that consist of an amino acid sequence selected from the group consisting of SEQ ID NO:1 to SEQ ID NO:213, SEQ ID, NO:358 to SEQ ID NO:381, and SEQ ID NO:388 to SEQ ID NO:408 in the sequence listing, and/or, one or more of polypeptides that consist of an amino acid sequence selected from the group consisting of SEQ ID NO:214 to SEQ ID NO:288, SEQ ID NO:356, SEQ ID NO:357, and SEQ ID NO:385 to SEQ ID NO:387 in the sequence listing.

Still another embodiment of the present invention is the cancer vaccine, wherein the vaccine is used for treating one or more of cancers selected from the group consisting of colon cancer, esophageal cancer, oral squamous cell cancer, renal cancer, pulmonary cancer, gynecological cancer, and prostate cancer.

An additional embodiment of the present invention is an agent for inducing a cytotoxic T lymphocyte, wherein the agent comprising one or more of peptides that consist of an amino acid sequence selected from the group consisting of SEQ ID NO:1 to SEQ ID NO:213, SEQ ID NO:358 to SEQ ID NO:381, and SEQ ID NO:388 to SEQ ID NO:408 in the sequence listing, and/or, one or more of polypeptides that consist of an amino acid sequence selected from the group consisting of SEQ ID NO:214 to SEQ ID NO:288, SEQ ID NO:356, SEQ ID NO:357, and SEQ ID NO:385 to SEQ ID NO:387 in the sequence listing.

An embodiment of the present invention is also a method for inducing a cytotoxic T lymphocyte, wherein the method comprises using one or more of peptides that consist of an amino acid sequence selected from the group consisting of SEQ ID NO:1 to SEQ ID NO:213, SEQ ID NO:358 to SEQ ID NO:381, and SEQ ID NO:388 to SEQ ID NO:408 in the sequence listing, and/or, one or more of polypeptides that consist of an amino acid sequence selected from the group consisting of SEQ ID NO:214 to SEQ ID NO:288, SEQ ID NO:356, SEQ ID NO:357, and SEQ ID NO: 385 to SEQ ID NO:387 in the sequence listing.

Another embodiment of the present invention is a polynucleotide encoding a peptide or polypeptide consisting of an amino acid sequence selected from the group consisting of SEQ ID NO:1 to SEQ ID NO:288, SEQ ID NO:356 to SEQ ID NO:381, and SEQ ID NO:385 to SEQ ID NO:408 in the sequence listing, or a complementary strand thereof.

Still another embodiment of the present invention is a polynucleotide consisting of a nucleotide sequence selected from a group consisting of SEQ ID NO:290 to SEQ ID NO:355 (excluding SEQ ID NO:299 and SEQ ID NO: 332) and SEQ ID NO:382 to SEQ ID NO:384 in the sequence listing, or a complementary strand thereof.

Yet another embodiment of the present invention is a polynucleotide consisting of a nucleotide sequence selected from a group consisting of SEQ ID NO:289 to SEQ ID NO:355 and SEQ ID NO:382 to SEQ ID NO:384 in the sequence listing, or a complementary strand thereof, wherein the polypeptide encoded by the polynucleotide induces a cytotoxic T lymphocyte and/or is recognized by a cytotoxic T lymphocyte, or the complementary strand thereof.

An additional embodiment of the present invention is the polynucleotide or the complementary strand thereof, wherein a polypeptide encoded by the polynucleotide induces a cytotoxic T lymphocyte in an HLA-A2-restrcited manner or HLA-A26-restricted manner and/or is recognized by a cytotoxic T lymphocyte in an HLA-A2-restrcited manner or HLA-A26-restricted manner when it induces a cytotoxic T lymphocyte and/or is recognized by a cytotoxic T lymphocyte.

An embodiment of the present invention is a polynucleotide that hybridizes to the polynucleotide or the complementary strand thereof under stringent conditions.

Another embodiment of the present invention is a recombinant vector comprising the polynucleotide or the complementary strand thereof.

Still another embodiment of the present invention is a recombinant vector, wherein the recombinant vector is a recombinant expression vector.

Yet another embodiment of the present invention is a transformant transformed with the recombinant vector.

An additional embodiment of the present invention is a method for producing the peptide or the polypeptide, wherein the method comprises culturing a transformant transformed with the recombinant vector.

An embodiment of the present invention is an antibody immunologically recognizing the peptide or the polypeptide.

Another embodiment of the present invention is a method for screening for a compound that enhances recognition of the peptide or the polypeptide at least by an HLA-A2-restricted or HLA-A26-restricted cytotoxic T lymphocyte through interacting with the peptide or the polypeptide and/or a HLA-A2 molecule or HLA-A26 molecule, and/or a compound that enhances expression of the polynucleotide or the complementary strand thereof through interacting with the same, wherein the method comprises using at least one selected from the group consisting of the peptide, the polypeptide, the polynucleotide or the complementary strand thereof, the recombinant vector, the transformant and the antibody.

Still anther embodiment of the present invention is a compound that is obtained by the screening method.

Yet another embodiment of the present invention is a compound that enhances recognition of at least one of the peptide and/or the polypeptide by an HLA-A2-restricted or HLA-A26-restricted cytotoxic T lymphocyte, or a compound that enhances the expression of the polynucleotide or the complementary strand thereof through interacting with the same.

An additional embodiment of the present invention is a pharmaceutical composition used for treating cancers, wherein the composition comprising at least one member selected from the group consisting of the peptide, the polypeptide, the polynucleotide or the complementary strand thereof, the recombinant vector, the transformant, the antibody, and the compound.

An embodiment of the present invention is a method for measuring quantitatively or qualitatively the peptide, the polypeptide, or the polynucleotide.

Another embodiment of the present invention is the measuring method, wherein the method is used in an examination for a cancer disease.

Still another embodiment of the present invention is a reagent kit for use in the screening method or the measuring method, wherein the reagent kit comprises at least one member selected from the group consisting of the peptide, the polypeptide, the polynucleotide or the complementary strand thereof, and the antibody.

An additional embodiment of the present invention is the reagent kit, wherein the reagent kit is used in an examination for a cancer disease.

## BRIEF DESCRIPTION OF THE DRAWINGS

Fig. 1 illustrates that cDNA clone 2 was recognized by an HLA-A2-restricted cytotoxic T lymphocyte OK-CTLd, and enhanced the interferon-$\gamma$ (IFN-$\gamma$) production from OK-CTLd.

Fig. 2 illustrates that cDNA clone 29 was recognized by an HLA-A2-restricted cytotoxic T lymphocyte OK-CTLd, and enhanced IFN-$\gamma$ production from OK-CTLd.

Fig. 3 illustrates that cDNA clone 40 was recognized by an HLA-A2-restricted cytotoxic T lymphocyte OK-CTLd, and enhanced IFN-$\gamma$ production from OK-CTLd.

Fig. 4 illustrates that peptides derived from cDNA clone 2 were recognized by an HLA-A2-restricted cytotoxic T lymphocyte OK-CTLd, and enhanced IFN-γ production from OK-CTLd.

Fig. 5 illustrates that peptides derived from cDNA clone 29 were recognized by an HLA-A2-restricted cytotoxic T lymphocyte OK-CTLd, and enhanced IFN-γ production from OK-CTLd.

Fig. 6 illustrates that peptides derived from cDNA clone 40 were recognized by an HLA-A2-restricted cytotoxic T lymphocyte OK-CTLd, and enhanced IFN-γ production from OK-CTLd.

Fig. 7 illustrates the cytotoxicity of a subline of an HLA-A2-restricted cytotoxic T lymphocyte OK-CTLd and T lymphocyte antigen receptor Vβ usages (TCR Vβ usages.) Fig. 7a illustrates the cytotoxicity of a pooled OK-CTLd subline against T2 cells pulsed with each of five peptides or with a peptide derived from human immunodeficiency virus (HIV) used as a negative control (n.c.). In this figure, the Y-axis denotes percentage of $^{51}$Cr released from T2 cells, i.e., specific cytolytic activity; and the X-axis denotes the ratio of effector cells to target cells (E/T ratio.) Fig. 7b illustrates TCR Vβ usages of peripheral blood mononuclear cells (OK-PBMC) derived from a cancer patient OK, OK-CTLd-6, OK-CTLd-9, or sublines that were established from OK-CTLd, which were pooled as shown in the figure. In the figure, "non-reactive sublines" denote sublines that do not respond to the peptides, and numbers shown in the upper side denotes types of TCR Vβ.

Fig. 8 illustrates that peripheral blood mononuclear cells (PBMC), derived from a cancer patient, recognized each cDNA clone or each peptide in an HLA-A2-restricted manner. Fig. 8a illustrates that PBMC (OK-PBMC) derived from a cancer patient recognized COS-7 cells, into which a plasmid carrying each cDNA clone was co-transfected with HLA-A0207, resulting in enhancement of IFN-γ production from the PBMC. Fig. 8b illustrates that PBMC derived from a cancer patient recognized COS-7 cells, into which HLA-A0207 was transfected and pulsed with a peptide, resulting in enhancement of IFN-γ production from the PBMC.

Fig. 9 illustrates the expression of clone 10, clone 30, clone 41, and clone 108 in various cells such as Panc-1 cells, SW620 cells, peripheral blood mononuclear cells (PBMC) and PHA-blast at the mRNA level.

Fig. 10 illustrates that each of cDNA clones, derived from a human colon cancer, such as SW620-cl.48 (Fig. 10a) and SW620-cl.121 (Fig. 10b) was recognized by an HLA-A2-restricted cytotoxic T lymphocyte OK-CTLd, and enhanced IFN-γ production from OK-CTLd.

Fig. 11 illustrates that peptides derived from cDNA clone SW620-cl.48, which is derived from a human colon cancer, were recognized by HLA-A2-restricted cytotoxic T lymphocyte OK-CTLd, and enhanced IFN-γ production from OK-CTLd.

Fig. 12 illustrates that each of cDNA clones, derived from a human esophageal cancer, such as KE4-cl.17, KE4-cl. 18, and KE4-cl.21, was recognized by an HLA-A26-restricted cytotoxic T lymphocyte KE4-CTL, and enhanced IFN-γ production from KE4-CTL.

Fig. 13 illustrates that peptides derived from a cDNA clone KE4-cl.21, which is derived from a human esophageal cancer, were recognized by an HLA-A26-restricted cytotoxic T lymphocyte KE4-CTL, and enhanced IFN-γ production from KE4-CTL. Fig. 13a and Fig. 13b show the results of two experiments. In Fig. 13a, '-•-' denotes KE4-21·P28, '-▲-' denotes KE4-21·P29, '-■-' denotes KE4-21·P39, and '-o-' denotes KE4-21·P40. In Fig. 13b, '-•-' denotes KE4-21·P28, '-▲-' denotes KE4-21·P38, '-o-' denotes KE4-21·P40, and '-■-' denotes KE4-21·P47.

Fig. 14 illustrates that cytotoxic T lymphocytes were induced and/or activated in an HLA-A26-restricted manner from peripheral blood mononuclear cells (PBMC) derived from a cancer patient, by peptides derived from cDNA clones KE4-cl.18 and KE4-cl.21 that are derived from a human esophageal cancer. In the figure, 'Pt' denotes a patient. Fig. 14a illustrates that PBMC, which had been previously stimulated with a peptide, recognized the corresponding peptide, resulting in enhancement of IFN-γ production from the PBMC. In Fig. 14a, '*' denotes that the Two-tailed Student's T-test showed a significant difference ($P < 0.05$) in data. Fig. 14b illustrates cytotoxicity against $HLA-A26^+$ esophageal cancer (KE4), $HLA-A26^-$ esophageal cancer (KE3), and PHA-blast, which were examined by a 6-hour $^{51}$Cr release test after culturing for 21 days of PBMC derived from a cancer patient together with a peptide. The Y-axis denotes percentage of specific lysis while the X-axis denotes ratios of effector cells against target cells (E/T ratio.) In Fig. 14b, '*' denotes that the Two-tailed Student's T-test showed a significant difference ($P < 0.05$) between the percentage of lysis of KE4 tumor cells and those of KE3 tumor cells

## DETAILED DESCRIPTION OF THE INVENTION

### Isolation and identification of tumor antigen gene

OK-CTL is an HLA-A2-restricted tumor-specific cytotoxic T lymphocyte, which is activated by recognizing a tumor antigen peptide together with an HLA-A2 that is the major type for Japanese among HLA-A molecules, has been established in the present invention from tumor infiltrating lymphocytes (which, hereinafter, may be abbreviated to TIL) of a colon cancer patient according to the method described in the literature [Gomi, S. et al., J. Immunol. (1999) 163: 4994-5004]. OK-CTL obtained is a T lymphocyte in which 80% of the cells have a phenotype of $CD3^+CD4^-CD8^+$ and the remaining have a phenotype of $CD3^+CD4^+CD8^-$. OK-CTL recognized all of the tested $HLA-A2^+$ tumor cells and produced interferon-γ (IFN-γ) showing an adequate cytotoxicity, while it did not lyse HLA-A2⁻tumor cells, Epstein-Barr virus-transformed autologous B cells (EBV-B), and autologous PHA-blastoid T cells. The cytotoxicity of OK-CTL was inhibited by a monoclonal antibody (mAb) against HLA class I, CD8 or HLA-A2. These results revealed that OK-CTL activates by recognizing tumor cells in an HLA-A2 restricted manner to show cytotoxicity, wherein HLA-A2 is an HLA class I antigen. OK-CTLd that is one of sublines of OK-CTL was used in the present invention. Cryopreserved OK-CTLd was incubated with 100 U/ml of interleukin-2 (which, hereinafter, may be abbreviated to IL-2) for 2 weeks or more for use in the experiments described herein.

A tumor antigen that is recognized by OK-CTLd and can activate OK-CTLd was isolated/identified from a cDNA library of human colon cancer cell line SW620 (HLA-A0201/A2402) by using the gene expression cloning method [Shichijo, S. et al., J. Exp. Med. (1998) 187: 277-288]. A gene encoding the tumor antigen was identified by co-transfecting a cDNA of human colon cancer cell line SW620 together with a cDNA of HLA-A0207 into COS-7 cells, followed by selecting a cell that enhances IFN-γ production from OK-CTLd among cells expressing the transfected gene. The method will be described in more detail in the following Examples. As a result, sixty-seven cDNA clones were obtained which encode gene products recognized by OK-CTLd in an HLA-A2-restricted manner. Hereafter, "gene product" denotes a polypeptide comprising an amino acid sequence being coded by each gene. The nucleotide sequences of the obtained cDNA clones were determined by the dideoxynucleotide sequencing method. These nucleotide sequences are shown in the sequence listing as SEQ ID NO:289 to SEQ ID NO:355. Among these, it was revealed that clone 76 (SEQ ID NO:346) is identical to clone 78 (SEQ ID NO:295.) The above genes were registered in DNA Data Bank of Japan (DDBJ) of National Institute of Genetics (Tables 1 to 7.)

A homology search for these clones using the known database resulted in the finding of genes, derived from human, that are highly homologous to fifty-seven clones among the above obtained sixty-seven genes (see Tables 1 to 7 below.) However, no highly homologous gene was found with respect to nine genes, i.e., clone 4, clone 37, clone 58, clone 71, clone 79, clone 87, clone 89, clone 92, and clone 97. With respect to clone 30, a highly homologous gene was found in murine genes but not in human genes. Although the nucleotide sequences highly homologous to the genes according to the present invention and the deduced amino acid sequences thereof are disclosed, it has not been reported and not been disclosed that these genes encode tumor antigens, even in the open database of National Center for Biotechnology Information (NCBI) when it was browsed on November 2, November 6, and November 7; 2001.

Tumor rejection antigen genes that have been cloned so far, such as those of melanoma, include relatively many genes encoding unusual proteins that contain mutant antigens. However, the genes obtained by the present invention include many genes highly homologous to various enzymes and molecules involved in transcription, translation, and so on. The kinds of the proteins having such homology are similar to those of antigens detected by the Serological analysis of recombinant cDNA expression libraries (SEREX.) The SEREX method permits detecting antigens by using antibodies appearing in a patient's serum [Sahin, U., et al., Proc. Natl. Acad. Sci. USA, 92: 11810-11813, 1995]. In such a method, often detected are antigens that induce humoral immunity. Approximately 1,500 tumor antigens have been identified so far using the method.

Amino acid sequences (SEQ ID NO:214, SEQ ID NO:228, SEQ ID NO:269, SEQ ID NO:261, and SEQ ID NO:236) encoded by five genes (clone 12, clone 65, clone 81, clone 86, and clone 100) among the genes obtained by the present invention are identical to those encoded by known genes ID456, ID629, ID1226, ID163, and ID116, respectively, that were identified by the SEREX method and disclosed in the SEREX database (http://www-ludwig.unil.ch/SEREX.html). Moreover, nucleotide sequences (SEQ ID NO:289, SEQ ID NO:299, and SEQ ID NO:332) of clone 12, clone 82, and clone 86, are identical to those of ID456, ID1197, and ID163, respectively. In addition, it was revealed that clone 32, clone 41, clone 74, and clone 87 are partially homologous to ID1072, ID1233, ID979, and ID567, respectively.

Although the SEREX method can be used to detect tumor antigens, only MAGE-1, tyrosinase, and NY-ESO-1, were so far identified as tumor antigens capable of inducing both cellular and humoral immunities among approximately 1,500 tumor antigens or more that were detected by the method. All of those identified as tumor antigens by the SEREX method do not always induce and/or activate cytotoxic T lymphocytes (which, hereinafter, may be abbreviated to CTL) that are involved in the cellular immunity. The SEREX database disclosed the nucleotide sequences of the above genes and the amino acid sequences of their gene products, while it was not disclosed in the database browsed on June 10, 2002 that the gene products are recognized by CTL and, induce and/or activate CTL.

The present invention revealed for the first time that gene products of the genes shown in Tables 1 to 7 including the above genes (clone 12, clone 65, clone 81, clone 82, clone 86, and clone 100) are recognized by CTL in an HLA-A2-restricted manner and can induce and/or activate CTL.

The above genes obtained by the present invention are genes encoding tumor antigens that are recognized by a tumor-specific CTL in an HLA-A2-restricted manner, which are recognized by CTL in an HLA-A26-restricted manner and can induce and/or activate CTL when expressed in a cell. Amino acid sequences encoded by these genes are shown as SEQ ID NO:214 to SEQ ID NO:288, SEQ ID NO:356, and SEQ ID NO:357 of in the sequence listing (see Tables 1 to 7.) Clone 76 was identical to clone 78, so that it was revealed that amino acid sequences (SEQ ID NO:277 and SEQ ID NO:220) encoded by both genes are also identical each other.

Table 1

| cDNA clone (base length, bp) | SEQ ID NO: | polypeptide encoded by cDNA□@@@(AA length) | SEQ ID NO: | Homologous genes [accession number of GenBank] |
|---|---|---|---|---|
| 12 (1280) [AB062273] | 289 | PP 12 (335) | 214 | glyceraldehyde- 3-phosphate dehydrogenase [XM_006959] |
| 40 (2978) [AB062293] | 290 | PP 40 (599) | 215 | ATP-binding cassette, sub- family E(OABP), member1 (ABCE1) [XM_003555] |
| 43 (1218) [AB062294] | 291 | PP 43 (101) | 216 | ubiquitin- homology domain protein PIC1 [U61397] ubiquitin- like1 (sentrin) [BC006462] |
| 2 (825) [AB062123] | 292 | PP 2 (249) | 217 | ribosomal protein S6 [NM_001010] FLJ23534 fis [AK027187] |
| 11 (1978) [AB062128] | 293 | PP 11 (184) | 218 | member of Ras oncogene family (RAP1B) [BC000176] |
| 8 (895) [AB062126] | 294 | PP 8 (162) | 219 | transcription factor BTF 3. [X74070] |
| 78 (1358) [AB062479] | 295 | PP 78 (180) | 220 | CGI-37 [AF132971] HSPC031 [XM_007837] |
| 67 (2033) [AB062395] | 296 | PP 67 (166) | 221 | NOF1 [U39400] chromosome 11 open reading frame 4 [BC004378] |
| 9 (1059) [AB062127] | 297 | PP 9 (194) | 222 | putative oncogene [XM_016246] inosine triphosphate pyrophosphatase [AF219116] |
| 95 (1769) [AB062429] | 298 | PP 95 (466) | 223 | Annexin A7 [BC002632] |

Table 2

| cDNA clone (base length, bp) | SEQ ID NO: | polypeptide encoded by cDNA□@@@(AA length) | SEQ ID NO: | Homologous genes [accession number of GenBank] |
|---|---|---|---|---|
| 82 (463) [AB062400] | 299 | PP 82 (130) | 224 | ribosomal protein S15a [X84407] [BC001697] |

Table 2 (continued)

| cDNA clone (base length, bp) | SEQ ID NO: | polypeptide encoded by cDNA□@@@(AA length) | SEQ ID NO: | Homologous genes [accession number of GenBank] |
|---|---|---|---|---|
| 103 (703) [AB062431] | 300 | PP 103 (192) | 225 | ribosomal protein L9 [BC004206] |
| 4 (887) [AB062435] | 301 | PP 4 (67) | 226 | [AL365207] |
| 14 (905) [AB062484] | 302 | PP 14 (66) | 227 | caldesmon, 3'UTR [AJ223812] [AC090497] |
| 65 (1832) [AB062394] | 303 | PP 65 (145) | 228 | Gu protein [U41387] DEAD/H box polypeptide 21(DDX21) [NM_004728] |
| 69 (1824) [AB062396] | 304 | PP 69 (49) | 229 | tumor protein D52- like 2 [XM_009688] |
| 83 (759) [AB062401] | 305 | PP 83 (208) | 230 | ribosomal protein S8 [NM_001012] |
| 84 (938) [AB062402] | 306 | PP 84 (183) | 231 | ferritin heavy polypeptide 1 [M11146] [BC000857] |
| 32 (1281) [AB062291] | 307 | PP 32 (403) | 232 | ribosomal protein L3 [NM_000967] |
| 21 (1698) [AB062285] | 308 | PP 21 (480) | 233 | uridine monophosphate synthetase [NM_000373] |
| 33 (1102) [AB062486] | 309 | PP 33 (86) | 234 | splicing factor, arginine/ serine-rich 11 [XM_001835] |
| 68 (519) [AB062487] | 310 | PP 68 (54) | 235 | glutaminyl- peptide cyclotransferase [NM_012413] |

Table 3

| cDNA clone (base length, bp) | SEQ ID NO: | polypeptide encoded by cDNA□@@@(AA length) | SEQ ID NO: | Homologous genes [accession number of GenBank] |
|---|---|---|---|---|
| 100 (2335) [AB062430] | 311 | PP 100 (672) | 236 | FLJ10669 [XM_009301] [BC006358] |
| 73 (1027) [AB062397] | 312 | PP 73 (222) | 237 | ubiquitin carrier protein (E2-EPF) [NM_014501] [BC004236] |

Table 3 (continued)

| cDNA clone (base length, bp) | SEQ ID NO: | polypeptide encoded by cDNA□@@@(AA length) | SEQ ID NO: | Homologous genes [accession number of GenBank] |
|---|---|---|---|---|
| 27 (1068) [AB062289] | 313 | PP 27 (245) | 238 | integrin beta 4 binding protein [BC001119] |
| 26 (810) [AB062288] | 314 | PP 26 (117) | 239 | IMR-90 ribosomal protein S3 [U14992] [BC003577] |
| 56 (2505) [AB062393] | 315 | PP 56 (444) | 240 | *f* Àtublin [AF141349] [BC002347] |
| 5 (1588) [AB062125] | 316 | PP 5 (92) | 241 | tropomyosin 4 [BC000771] [X04588] |
| 10 (1831) [AB062436] | 317 | PP 10 (453) | 242 | FLJ22118 [NM_024537] |
| 22 (3476) [AB062286] | 318 | PP 22 (209) | 243 | peroxisomal farnesylated protein [NM_002857] |
| 30 (1665) [AB062438] | 319 | PP 30 (354) | 244 | 8 days embryo of MUS musculus [AK019987] |
| 88 (1571) [AB062428] | 320 | PP 88 (295) | 245 | sulfotransferase family, cytosolic1A phenol-preferring member1 [NM_001055] |
| 45 (1549) [AB062391] | 321 | PP 45 (439) | 246 | dolichyl-diphosphooligosaccharide-protein glycosyltransferase [BC002594] |

Table 4

| cDNA clone (base length, bp) | SEQ ID NO: | polypeptide encoded by cDNA□@@@ (AA length) | SEQ ID NO: | Homologous genes [accession number of GenBank] |
|---|---|---|---|---|
| 58 (2064) | 322 | PP 58-F3 (56) | 247 | - |
| | | PP 58-F2 (46) | 248 | |
| 18 (1317) [AB062485] | 323 | PP 18-F1 (61) | 249 | methylthioadenosine phosphorylase [XM_011800] |
| | | PP 18-F2 (73) | 250 | |
| | | PP 18-F3 (43) | 251 | |
| 87 (1483) | 324 | PP 87 (30) | 252 | - |

Table 4 (continued)

| cDNA clone (base length, bp) | SEQ ID NO: | polypeptide encoded by cDNA□@@@ (AA length) | SEQ ID NO: | Homologous genes [accession number of GenBank] |
|---|---|---|---|---|
| 24<br>(1067)<br>[AB062434] | 325 | PP 24<br>(87) | 253 | prolyl 4- hydroxylase beta- subunit and disulfide isomerase [M22806] |
| 46<br>(915)<br>[AB062392] | 326 | PP 46<br>(211) | 254 | ribosomal protein L13 [NM_000977] |
| 110<br>(2338)<br>[AB062432] | 327 | PP 110<br>(417) | 255 | phosphoglycerate kinase 1 (PGK1) [XM_010102] |
| 20<br>(2519)<br>[AB062284] | 328 | PP 20<br>(568) | 256 | natural resistance-associated macrophage protein 2 [AF064484] |
| 6<br>(1623) | 329 | PP 6- F1 (46) | 257 | DKFZp762E1112 [AL162047] |
| | | PP 6- F2 (36) | 258 | |
| 108<br>(3379)<br>[AB062483] | 330 | PP 108<br>(898) | 259 | DKFZp434G2226 [NM_031217] |
| 23<br>(964)<br>[AB062287] | 331 | PP 23<br>(71) | 260 | heterogeneous nuclear ribonucleoprotein F [BC004254] [L28010] |

Table 5

| cDNA clone (base length, bp) | SEQ ID NO: | polypeptide encoded by cDNA□@@@(AA length) | SEQ ID NO: | Homologous genes [accession number of GenBank] |
|---|---|---|---|---|
| 86<br>(1937)<br>[AB062403] | 332 | PP 86<br>(592) | 261 | 5- amnoinidazole- 4- carboxamide- 1- beta- D- ribonucleotide transformylase/ inosinicase [D82348] |
| 89<br>(2029)<br>[AB062482] | 333 | PP 89<br>(62) | 262 | - |
| 92<br>(2923)<br>[AB062488] | 334 | PP 92<br>(43) | 263 | - |
| 85<br>(2283)<br>[AB062481] | 335 | PP 85- F2 (303) | 264 | KIAA0036 [NM_014642] [BC005806] |
| | | PP 85- F3 (264) | 265 | |

Table 5 (continued)

| cDNA clone (base length, bp) | SEQ ID NO: | polypeptide encoded by cDNA□@@@(AA length) | SEQ ID NO: | Homologous genes [accession number of GenBank] |
|---|---|---|---|---|
| 3 (2765) [AB062124] | 336 | PP 3 (248) | 266 | Human SF2p33 [M69040] splicing factor, arginine/serine- rich 2 (SC- 35) [BC006181] |
| 29 (1567) [AB062290] | 337 | PP 29 (313) | 267 | thymidylate synthetase [NM 001071] [XM 008753] |
| 35 (2224) [AB062292] | 338 | PP 35 (511) | 268 | catenin beta 1(CTNNB1) [NM001904] [XM_003222] |
| 81 (854) [AB062399] | 339 | PP 81 (128) | 269 | RAN, member RAS oncogene family [BC004272] |
| 114 (1816) [AB062433] | 340 | PP 114 (506) | 270 | FLJ13660, similar to Rattus CDK5 activator_binding protein [AK023722] FLJ20253 [AK000260] |
| 19 (696) [AB062437] | 341 | PP 19 (136) | 271 | protein kinase Njmu- R1 [XM_015338] |

Table 6

| cDNA clone (base length, bp) | SEQ ID NO: | polypeptide encoded by cDNA (AA length) | SEQ ID NO: | Homologous genes [accession number of GenBank] |
|---|---|---|---|---|
| 74 (4912) [AB062478] | 342 | PP 74 (509) | 272 | KIAA0795 [AB018338] |
| 41 (2731) [AB062477] | 343 | PP 41-F1 (49) | 273 | FLJ20489 [AK000496] |
| | | PP 41-F3 (109) | 274 | |
| 79 (561) [AB062480] | 344 | PP 79 (54) | 275 | [AC008088] |
| 36 (3443) | 345 | PP 36 (66) | 276 | FLJ22245 [AK025898] FLJ20489 [AK000496] |
| 76 (1358) [AB062398] | 346 | PP 76 (180) | 277 | HSPC031 [XM_007837] |
| 37 (1047) | 347 | PP 37 (34) | 278 | Intron of UDP- N-acetylglucosamine 2-epimerase gene [AF317635] |

Table 6 (continued)

| cDNA clone (base length, bp) | SEQ ID NO: | polypeptide encoded by cDNA (AA length) | SEQ ID NO: | Homologous genes [accession number of GenBank] |
|---|---|---|---|---|
| 38<br>(1306) | 348 | PP 38-F1 (168) | 279 | 3'UTR of tumor protein P53<br>(Li- Fraumenisyndrome)<br>[BC003596] |
| | | PP 38- F3 (158) | 280 | |
| 70<br>(341) | 349 | PP 70<br>(43) | 281 | Phosphoglycerate mutase 1 (PGAM1)<br>[XM_017950] |
| 71<br>(791) | 350 | PP 71<br>(61) | 282 | - |
| 75<br>(1474) | 351 | PP 75<br>(207) | 283 | eukaryotic translation initiation factor 4 gamma, 2 (E1F4G2)<br>[XM_006326] |
| 97<br>(2932) | 352 | PP 97-F1 □105 | 284 | - |
| | | PP 97- F2 (91) | 285 | |
| | | PP 97- F3 (75) | 286 | |
| 53<br>(1254) | 353 | PP 53- F1 (83) | 287 | synaptogyrin 2 (SYNGR2)<br>[NM_004710] |
| | | PP 53- F2 (117) | 288 | |

Table 7

| cDNA clone (base length, bp) | SEQ ID NO: | polypeptide encoded by cDNA□@@@(AA length) | SEQ ID NO: | Homologous genes [accession number of GenBank] |
|---|---|---|---|---|
| SW620-cl.48<br>(1324) | 354 | SW620-48-PP<br>(361) | 356 | solute carrier family 25 (mitochondrial carrier; phosphate carrier), member 3 (SLC25A3)<br>[XM_039619] |
| SW620-cl.121<br>(2303) | 355 | SW620-121-PP<br>(640) | 357 | aminolevulinate, delta-, synthase 1 (ALAS1)<br>[NM_000688] |

In addition, an HLA-A26-restricted tumor-specific cytotoxic T lymphocyte KE4-CTL, from an esophageal cancer patient (HLA-A2601/2402), which is activated by recognizing HLA-A26 and a tumor antigen peptide has been established [Nakao, M. et al., Cancer Res., 55: 4248-4252, 1995]. Moreover, tumor antigens recognized by KE4-CTL were isolated/identified from the cDNA library of human esophageal cancer cell line KE4 (HLA-A2601/2402) in a manner similar to one described above. Three cDNA clones encoding tumor antigens recognized by KE4-CTL in an HLA-A26-restricted manner were obtained by co-transfecting cDNA of a human esophageal cancer cell line KE4 and cDNA of HLA-A2601 into VA13 cells, followed by selecting clones that enhance IFN-γ production from KE4-CTL from cells in which the transfected genes were expressed. These nucleotide sequences and deduced amino acid sequences, were determined in a manner similar to one described above and are shown as SEQ ID NO:382 to SEQ ID NO:384 and SEQ ID NO:385 to SEQ ID NO:387 in the sequence listing, respectively. These genes were registered in DNA Data Bank of Japan (DDBJ) of National Institute of Genetics (Table 8.) In addition, as described in Table 8, genes highly homologous to these genes were found by homology search. Among these, the nucleotide sequence of KE4-cl.21 is identical to that of ribosomal protein L10a except for the 640$^{th}$ nucleotide, and both deduced amino acid sequences were identical each other. Although nucleotide sequences of these highly homologous genes and deduced amino acid sequences thereof are disclosed, it has not been reported that these encode tumor antigens and not been disclosed even in the open database of NCBI when it was browsed on November 7; 2001. The genes thus obtained are those encoding tumor antigens recognized by tumor-specific CTL in an HLA-A26-restricted manner, which are recognized by CTL in an HLA-A26-restricted manner when expressed in a cell and can induce and/or activate CTL.

Table 8

| cDNA clone (base length, bp) | SEQ ID NO: | polypeptide encoded by cDNA□@@@ (AA length) | SEQ ID NO: | Homologous genes [accession number of GenBank] |
|---|---|---|---|---|
| KE4-cL17 (974) [AB082924] | 382 | KE4-17-PP (142) | 385 | ribosomal protein L13a [NM_012423] [XM_027885] |
| KE4-cl. 18 (821) [AB082925] | 383 | KE4-18-PP (233) | 386 | ribosomal protein S2 [BC001795] |
| KE4- cl.21 (741) [AB082926] | 384 | KE4- 21- PP (217) | 387 | ribosomal protein L10a [BC006791] [AAH06791] |

In the present specification, "a tumor antigen" means a protein or a peptide that is recognize by tumor-specific cytotoxic T lymphocytes and/or is capable of inducing tumor-specific cytotoxic T lymphocytes, which is present in a tumor cell. Moreover, a "tumor antigen peptide" means a peptide that is generated as a result of degradation of the tumor antigen in a tumor cell, which is recognize by tumor-specific cytotoxic T lymphocytes through being presented on a cell surface by binding to an HLA molecule and/or is capable of inducing tumor-specific cytotoxic T lymphocytes. In addition, the site of the amino acid sequence that is capable of inducing and/or activating tumor-specific cytotoxic T lymphocytes, which is present in a tumor antigen, is called "a tumor antigen epitope (tumor antigen determinant.)"

"Recognize" herein means that a subject distinguishes an object from others and cognates it, for example, binds to the object cognized. Particularly, in the present specification, that CTL recognize the tumor cells or the tumor antigen peptides means that CTL binds through a T cell receptor (which, hereinafter, may be abbreviated to TCR) to the tumor antigen peptides that are presented by HLA molecules.

"Activate" herein means to enhance or to make it work further a thing or a state that has an activity or an action. Particularly, in the present specification, activation of CTL means that CTL recognizes an antigen being presented by an HLA molecule to produce IFN-γ or to show cytotoxicity against the target cells recognized thereby.

"Induce" herein means to generate an activity or an action from a thing or a state that are in a phase merely having the activity or the action. Particularly, in the present specification, induction of an antigen-specific CTL means to make CTL, which specifically recognizes a certain antigen, differentiate and/or proliferate *in vitro* or *in* vivo. Moreover in the present specification, the inducer of cytotoxic T lymphocytes means an agent which changes the state where CD8-positive T lymphocytes specifically recognizing a certain antigen is absent or present in a very low degree, to the state where the cytotoxic T lymphocytes recognizing the antigen is present in a very degree.

Identification of tumor antigen peptide CTL

In order to obtain a tumor antigen peptide from amino acid sequences encoded by the above genes, an HLA-A2 binding motif (a specific sequence) was searched using the homepage (http://bimas.dcrt.nih.gov/molbio/hla_bind/) of Bioinformatics & Molecular Analysis Section (BIMAS), and an amino acid sequence suitable for the motif was specified based on the amino acid sequences encoded by the above genes and the amino acid sequences of gene products of genes highly homologous to the above genes. Based on the result, various peptides of 9-mer and 10-mer having an HLA-A2-binding motif were designed and synthesized.

Each synthetic peptide was pulsed to T2 cells expressing HLA-A2, followed by culturing the T2 cells with OK-CTLd to measure IFN-γ production from OK-CTLd. Peptides recognized by OK-CTLd were selected using the amount of IFN-γ production as an index. Among 628 peptides synthesized, 237 peptides (SEQ ID NO:1 to SEQ ID NO: 213 and SEQ ID NO:358 to SEQ ID NO:381) were recognized by OK-CTLd and enhanced IFN-γ production from OK-CTLd in a dose dependent manner of the peptide. Among these peptides, P77 (SEQ ID NO:20) and P79 (SEQ ID NO:21) derived from clone 78 were identical to P656 (SEQ ID NO:211) and P658 (SEQ ID NO:212) derived from clone 76, respectively. Thus, 235 of tumor antigen peptides were obtained that are recognized by CTL in an HLA-A2-restricted manner and can activate the CTL.

Various peptides of 9-mer and 10-mer capable of binding to HLA-A26 were designed and synthesized based on the amino acid sequences of the above tumor antigen (SEQ ID NO:385 to SEQ ID NO:387) recognized by CTL in an HLA-A26-restricted manner, according to the method described in the literature [J. Exp. Med., 184: 735-740, 1996). With respect to the gene KE4-cl.17, peptides related to the amino acid sequences encoded by genes highly homologous to the gene were also designed and synthesized. Each synthetic peptide described above was pulsed to VA13 cells that were made to express HLA-A26, followed by culturing the VA13 cells with KE4-CTL to measure IFN-γ production from KE4-CTL for use as an index so as to select peptides recognized by KE4-CTL in an HLA-A26-restricted manner. Twenty-one peptides (SEQ ID NO:388 to SEQ ID NO:408) among the synthesized peptides were recognized by KE4-CTL and enhanced IFN-γ production from KE4-CTL in a dose dependent manner of each peptide. Thus, twenty-one tumor antigen peptides were obtained that are recognized by CTL in an HLA-A26-restricted manner and can activate the CTL.

<u>Specificity of TCR recognizing peptide</u>

CTL recognize a complex comprising a tumor antigen peptide and an HLA molecule through a TCR that is expressed on the cell surface thereof. It was reported that the diversity of TCR is over $10^{17}$. Investigation using 480 OK-CTLd sublines established from a parent cell line OK-CTLd, revealed that the recognition of peptide by the sublines depends on the kind of subline (see Table 9.) Moreover, TCR Vβ of these OK-CTLd sublines recognizing each peptide was different in each subline (see Fig. 7b.) These results revealed that CTL having different TCR Vβ shows different specificity against each peptide. These results further revealed that even CTL established from a tumor-infiltrating lymphocyte of one patient is a population consisting of cells having different TCR, whence it can recognize various antigens and peptides.

<u>Induction and/or activation of CTL in peripheral blood mononuclear cells derived from a cancer patient</u>

It was investigated whether forty-five gene products among the above gene products, which are recognized by OK-CTLd in an HLA-A2-restricted manner, are recognized by a peripheral blood mononuclear cell (which, hereinafter may be abbreviated to PBMC) derived from an HLA-A2$^+$ colon cancer patient. As a result, the PBMC derived from one colon cancer patient, who provided TIL for use to establish OK-CTL, recognized six gene products (clone 20, clone 43, clone 56, clone 84, clone 86, and clone 108.) The PBMC derived from another colon cancer patient recognized four gene products (clone 20, clone 84, clone 86, and clone 108.) On the other hand, these clones were not recognized by PBMC derived from a metastatic melanoma patient, PBMC derived from a healthy donor, or a cytotoxic T lymphocyte GK-CTL established from a lung cancer tissue-infiltrating lymphocyte.

In addition, peptides derived from these gene products, P430, P431, P434, and P440 (SEQ ID NO:360, SEQ ID NO:361, SEQ ID NO:144, and SEQ ID NO:146) derived from clone 20, P45 (SEQ ID NO:10) derived from clone 43, P288 (SEQ ID NO:358) and P289 (SEQ ID NO:359) derived from clone 56, P184 (SEQ ID NO:65) derived from clone 84, P485 (SEQ ID NO:166) and P486 (SEQ ID NO:363) derived from clone 86, P449 (SEQ ID NO:153) and P463 (SEQ ID NO:362) derived from clone 108 were recognized by at least one of the PBMC derived from the both colon cancer patients described above and enhanced IFN-γ production from the PBMC. A cell that recognizes the above gene product or the above peptide and produces IFN-γ was an HLA-A2-restricted CD8-positive T lymphocyte in PBMC.

As described above, it was revealed that CTL precursor recognizing the above tumor antigen and peptides derived therefrom in an HLA-A2-restricted manner is present in PBMC of a colon cancer patient. These tumor antigens and peptides derived therefrom would be able to induce and/or activate a tumor-specific CTL in an HLA-A2-restricted manner in this patient. Therefore, tumor antigens shown in Tables 1 to 7 and peptides derived therefrom would be suitable for use in the specific immunotherapy for HLA-A2$^+$ colon cancer patients.

Moreover, among the above peptides recognized by KE4-CTL in an HLA-A26-restricted manner, CTL-inducing ability of PBMC derived from HLA-A26$^+$ patients (oral squamous cell cancer, four cases; renal cancer, one case; and pulmonary cancer, one case) was investigated with respect to the following six peptides that strongly activated KE4-CTL: peptides KE4-18·P5 (SEQ ID NO:395), KE-4-18·P22 (SEQ ID NO:399), and KE4-18·P25 (SEQ ID NO:400) derived from KE4-cl.18 as well as KE4-21·P28 (SEQ ID NO:403), KE4-21·P29 (SEQ ID NO:404) and KE4-21·P40 (SEQ ID NO:407) derived from KE4-cl.21. As a result, the above six peptides were recognized by PBMC derived from the cancer patients and significantly enhanced IFN-γ production from the PBMC, while PBMC derived from a healthy donor did not enhance IFN-γ production. In addition, PBMC stimulated with KE4-18□P22, KE4-21□P28, or KE4-21□P29 showed strong cytotoxicity against HLA-A26$^+$ cancer cells (KE4), while showing a weak cytotoxicity against HLA-A26$^-$ cancer cells (KE3) or not responding to an HLA-A26$^+$ PHA-blast.

Thus, tumor antigens shown in Table 8 and peptides derived therefrom can induce and/or activate a tumor-specific CTL in an HLA-A26-stricted manner in PBMC of a cancer patient, so that they would be applicable to use in the specific immunotherapy for HLA- A26$^+$ cancer patients.

Polypeptide and peptide

In the present specification, "a polypeptide" means a long chain peptide of arbitrary peptides comprising two or more amino acids bound to each other by a peptide bond or by a modified peptide bond. For example, a protein is included in the definition of polypeptide herein. Moreover, a short chain peptide sometimes called an oligopeptide or an oligomer is simply called "a peptide" herein. Amino acid sequence may be given both in one-letter symbols and three-letter symbols hereafter.

A polypeptide according the present invention is a polypeptide encoded by one of the above genes obtained from human colon cancer cell line SW620 or human esophageal cancer cell line KE4, preferably a polypeptide consisting of an amino acid sequence described in any one of those of SEQ ID NO:214 to SEQ ID NO:288, SEQ ID NO: 356, SEQ ID NO:357, and SEQ ID NO:385 to SEQ ID NO:387 in the sequence listing. A polypeptide consisting of an amino acid sequence of SEQ ID NO:214, SEQ ID NO:228, SEQ ID NO:236, SEQ ID NO:261, or SEQ ID NO:269 is identical to a polypeptide already disclosed in SEREX database. Moreover, a polypeptide consisting of the amino acid sequence of SEQ ID NO:387 is identical to a polypeptide disclosed in GenBank.

These polypeptides can be used for tumor antigens that induce and/or activate CTL. For example, a polypeptide consisting of an amino acid sequence described in any one of those of SEQ ID NO:214 to SEQ ID NO:288, SEQ ID NO:356, and SEQ ID NO:357 in the sequence listing is recognized by an HLA-A2-restricted antigen-specific CTL and, hence, can be used for a tumor antigen that induces and/or activates the CTL. Moreover, a polypeptide consisting of an amino acid sequence described in any one of those from SEQ ID NO:385 to SEQ ID NO:387 is recognized by an HLA-A26-restricted antigen-specific CTL and can be used for a tumor antigen that induces and/or activates the CTL. In addition, these polypeptides can be used for materials to specify a tumor antigen epitope and to obtain a tumor antigen peptide.

Tumor antigen peptides according to the present invention can be obtained by selecting ones that are recognized by CTL and/or induce CTL, from peptides designed based on amino acid sequences of the above polypeptides. The selection can be carried out as shown in the Examples described hereinafter, for example, by culturing a peptide-pulsed antigen-presenting cell together with an HLA-A2-restricted or HLA-A26-restricted CTL such as OK-CTLd or KE4-CTL, followed by measuring IFN-γ production from the activated CTL as an index to select ones. A peptide according to the present invention may be the peptide having a property of a tumor antigen epitope presented on the surface of an antigen-presenting cell through binding to HLA-A2 or HLA-A26 and recognized by CTL. The peptide consists of at least about 5 or more, preferably about 7 or more, and more preferably 9 to 10 amino acid residues. Particularly preferably, the peptide is one consisting of an amino acid sequence described in any one of those of SEQ ID NO:1 to SEQ ID NO:213, SEQ ID NO:358 to SEQ ID NO:381, and SEQ ID NO:388 to SEQ ID NO:408 in the sequence listing. These peptides can be used as a tumor antigen peptide for activating and/or inducing CTL. For example, a peptide consisting of an amino acid sequence described in any one of those of SEQ ID NO:1 to SEQ ID NO:213 and SEQ ID NO:358 to SEQ ID NO:381 in the sequence listing is recognized by an HLA-A2-restricted antigen-specific CTL, and hence can be used as a tumor antigen peptide for activating and/or inducing the CTL. Moreover, a peptide consisting of an amino acid sequence described in any one of those from SEQ ID NO:388 to SEQ ID NO:408 in the sequence listing is recognized by an HLA-A26-restricted antigen-specific CTL, and hence can be used as a tumor antigen peptide for activating and/or inducing the CTL.

For inducing and/or activating CTL, one of the above-described polypeptide or peptide may be used or they may be used in combination of two or more. CTL is a cell population consisting of several kinds of peptide-specific CTL, so that it is recommended to use these peptides preferably in combination of two or more.

A polypeptide or peptide, which has one or several amino acid(s) with a mutation such as deletion, substitution, addition, or insertion introduced into the polypeptide or peptide specified as above, and is recognized by or is capable of inducing at least the HLA-A2-restricted or HLA-A26-restricted CTL, is also included within the scope of the present invention. A polypeptide or peptide having such mutation(s) can be a naturally existing polypeptide or peptide, or can be a polypeptide or peptide in which such mutation(s) is introduced. A means for introducing mutations such as a deletion, substitution, addition, or insertion is well-known and, for example, Ulmer's technique (Science, 219:666, 1983) can be employed. When introducing such mutation, in view of preventing a change of the fundamental properties (such as the physical properties, activity, or immunological activity) of the polypeotide or peptide, mutual substitution among, for example, amino acids having similar properties (polar amino acids, non-polar amino acids, hydrophobic amino acids, hydrophilic amino acids, positively charged amino acids, negatively charged amino acids, aromatic amino acids, and so on) can be carried out. In addition, some modification, such as modification of the constitutive amino group or carboxyl group, can be made on these polypeptides or peptides to such an extent that there is no notable change of their function.

Polynucleotide

A polynucleotide according to the present invention is one encoding the above polypeptide or peptide that is recognized by CTL and/or induces CTL in an HLA-A2-restricted or HLA-A26-restricted manner, or a complementary strand thereof. It is preferably a polynucleotide encoding a peptide consisting of an amino acid sequence described in any one of those of SEQ ID NO:1 to SEQ ID NO:213, SEQ ID NO:358 to SEQ ID NO:381, and SEQ ID NO:388 to SEQ ID NO:408, or encoding a polypeptide consisting of an amino acid sequence described in any one of those of SEQ ID NO:214 to SEQ ID NO:288, SEQ ID NO:356, SEQ ID NO:357, and SEQ ID NO:385 to SEQ ID NO:387 in the sequence listing, or a complementary strand thereof. Exemplified is a polynucleotide consisting of a nucleotide sequence described in any one of those of SEQ ID NO:289 to SEQ ID NO:355 and SEQ ID NO:382 to SEQ ID NO:384 in the sequence listing, or its complementary strand. Among these polynucleotides, a polynucleotide consisting of a nucleotide sequence described in any one of those of SEQ ID NO:289, SEQ ID NO:299, and SEQ ID NO:332 is identical to those that have already disclosed in SEREX database.

A polynucleotide according to the present invention may consist of at least about 15 or more, preferably about 21 to 30 or more nucleotides, wherein the nucleotides correspond to a region encoding a tumor antigen epitope in the amino acid sequence of the polypeptides according to the present invention, or a complementary strand thereof. Selection of such a useful polynucleotide and determination of the nucleotide sequence thereof can be carried out, for example, by employing well-known protein expression systems to confirm the ability of the expressed peptide or polypeptide to induce and/or activate CTL.

The above polynucleotide is recognized by CTL in an HLA-A2-restricted or HLA-A26-restricted manner when expressed in a cell having HLA-A2 or HLA-A26, and hence can induce and/or activate CTL. For example, a polynucleotide consisting of a nucleotide sequence described in any one of those from SEQ ID NO:289 to SEQ ID NO:355 in the sequence listing can be recognized by an HLA-A2-restricted antigen-specific CTL and/or can induce the CTL when expressed in a cell having HLA-A2. In addition, a polynucleotide consisting of a nucleotide sequence described in any one of those from SEQ ID NO:382 to SEQ ID NO:384 can be recognized by an HLA-A26-restricted antigen-specific CTL and/or can induce the CTL when expressed in a cell having HLA-A26.

Moreover, a polynucleotide that hybridizes to the above-described polynucleotide under stringent conditions is also included in the scope of the present invention. Preferably such is a polynucleotide that a polypeptide or peptide encoded thereby is recognized by CTL in an HLA-A2-restricted or HLA-A26-restricted manner and/or induces the CTL. In the case where the polynucleotide molecule is a DNA molecule, "a DNA molecule that hybridizes to a DNA molecule under stringent conditions" can be obtained, for example, by the method described in "Molecular Cloning: A Laboratory Manual (edited by Sambrook et al, Cold spring Harbor Laboratory Press, New York, 1989.)" "To hybridize under stringent conditions" herein means that a positive hybridizing signal is still observed even under the condition in which, for example, hybridization is carried out in a solution containing 6 X SSC, 0.5% SDS, and 50% formamide at 42°C and then, washing is carried out in a solution containing 0.1 X SSC and 0.5% SDS at 68°C. A polynucleotide that hybridizes to the above polynucleotide under stringent conditions, which encodes a polypeptide or peptide that is recognized by CTL in an HLA-A2-restricted or HLA-A26-restricted manner and/or induces CTL, can be selected, for example, by employing well-known protein expression systems to confirm the ability of the expressed polypeptide or peptide to induce and/or activate CTL. The selection can be carried out as shown in the Examples described hereinafter, for example, by culturing a peptide-pulsed antigen-presenting cell together with an HLA-A2-restricted or HLA-A26-restricted CTL such as OK-CTLd or KE4-CTL and so on, followed by measuring IFN-γ production from the activated CTL for use as an index to select ones.

The above-described polynucleotide may have a poly(A) structure in its 3'-terminal. The number of poly(A) does not have any influence on the site encoding the amino acid acting as a tumor antigen, so that the number of poly (A) of the polynucleotide is not limited.

All of the above-described polynucleotides provide genetic information useful for producing a polypeptide or a peptide according to the present invention or can be also utilized as a reagent and a standard of a nucleic acid.

Recombinant vector

A recombinant vector can be obtained by inserting the above-described polynucleotide into an adequate DNA vector. The DNA vector used is properly selected in accordance with the kind of host and the purpose of use. The DNA vector may be a naturally existing one and also may be one that lacks a part of its DNA other than that necessary for replication. For example, vectors can be exemplified as those derived from a chromosome, an episome, and a virus, for example, vectors derived from a bacterial plasmid, derived from a bacteriophage, derived from a transposon, derived from an enzyme episome, derived from an inserting element, and derived from an enzyme chromosome element, for example, vectors derived from a virus such as baculovirus, papovavirus, SV40, vacciniavirus, adenovirus, fowlpox virus, pseudorabies virus, and retrovirus, and vectors prepared by combination of them, for example, vectors derived from the genetic element of the plasmid and the bacteriophage, for example, a cosmid and a phagemid. Further, an expression vector and a cloning vector etc. can be used in accordance with the desired purpose.

The recombinant vector, which comprises the constitutional elements of the desired gene sequence and a gene sequence possessing information relating to replication and regulation, such as a promoter, a ribosome-binding site, a terminator, a signal sequence, an enhancer, and so on, can be prepared by combining them using well-known methods. As a method for inserting the polynucleotide according to the present invention into the above-described DNA vector, well-known methods can be employed. For example, a method can be used, wherein an appropriate restriction enzyme is chosen for treating a DNA to cleave it at a specific site, and then, the DNA is mixed with the DNA used as a vector treated in the same way, followed by ligating with a ligase. A desired recombinant vector can also be obtained by ligating an adequate linker to the desired polynucleotide followed by inserting the resultant molecule into a multi-cloning site of a vector suitable for a purpose.

(Transformant)

The DNA vector in which the above-described polynucleotide has been inserted can be used to obtain a transformant by transforming a well-known host such as *Escherichia coli,* yeast, *Bacillus subtillis,* an insect cell, or a mammalian cell therewith by well-known methods. In the case of carrying out the transformation, a more preferable system is exemplified by the method for integrating the gene in the chromosome, in view of achieving stability of the gene. However, an autonomous replication system using a plasmid can be conveniently used. Introduction of the DNA vector into the host cell can be carried out by standard methods such that described in "Molecular Cloning: A Laboratory Manual" (ed. by Sambrook et al., Cold Spring Harbor Laboratory Press, Cold Spring Harbor, New York, 1989.) Concretely, calcium phosphate transfection, DEAE-dextran-mediated transfection, microinjection, cation lipid-mediated transfection, electroporation, transduction, scrape loading, ballistic introduction and infection can be employed.

Using an expression vector as a DNA vector for the above-described transformant, a polypeptide or a peptide according to the present invention can be provided. A transformant, transformed with a DNA expression vector comprising the above-described polynucleotide, is cultured under well-known culture conditions suitable for each host. Culturing may be conducted by using indicators, such as a function of the polypeptide or a peptide according to the present invention that is expressed by the transformant, particularly at least the activity to induce and/or activate CTL, or the peptide or the amount of the peptide produced in the host or outside of the host. Subculturing or batch culturing may be also carried out using an amount of the transformant in the culture as an indicator.

Production of the polypeptide or peptide

A polypeptide or a peptide according to the present invention can be produced by a genetic engineering technique as above by using the vector or the transformant described above. Moreover, it can also be produced by a general method known in peptide chemistry. For example, "Peptide Synthesis (Maruzen) 1975" and "Peptide Synthesis, Interscience, New York, 1996" are exemplified. However, any widely known method can be used.

A polypeptide or peptide according to the present invention can be purified and collected by a method, such as gel filtration chromatography, ion column chromatography, affinity chromatography, and the like, in combination, or by fractionation means on the basis of a difference in solubility using ammonium sulfate, alcohol, and the like, using CTL-inducing and/or activating ability of the polypeptide or the peptide as an indicator. More preferably used is a method, wherein the polypeptide or the peptides are specifically adsorbed and collected by using polyclonal antibodies or monoclonal antibodies, which are prepared against the polypeptide or the peptides based on the information of their amino acid sequences.

Antibody

An antibody according to the present invention is prepared by using the above-described polypeptide or peptide as an antigen. An antigen may be the above-described polypeptide or peptide itself, or its fragment that is composed of at least 5, more preferably at least 8 to 10 amino acids. In order to prepare the antibody specific to the above-described polypeptide or peptide, a region consisting of the amino acid sequence intrinsic to the above-described polypeptide or peptide is desirably used. The amino acid sequence is not necessarily homologous to the amino acid sequence of the polypeptide or the peptide, but is preferably a site exposed to the outside of a stereo-structure of the polypeptide or the peptide. In such a case, it is sufficient that the amino acid sequence of the exposed site is consecutive in the exposed site, even if it may be discrete in its primary structure. The antibody is not limited as long as it binds or recognizes the polypeptide or the peptide immunologically. The presence or absence of the binding or the recognition can be determined by a well-known antigen-antibody binding reaction.

In order to produce an antibody, a well-known method for antibody production can be employed. For example, the antibody is obtained by administration of the polypeptide or peptide according to the present invention to an animal in the presence or absence of an adjuvant with or without linking such to a carrier so as to induce humoral immunity and/or cell-mediated immunity. Any carrier can be used as long as it is not harmful to the host and is capable of enhancing immunogenicity. For example, cellulose, a polymerized amino acid, albumin, keyholelimpet hemocyanine (KLH), and the like are exemplified. An adjuvant can be exemplified by Freund's complete adjuvant (FCA), Freund's incomplete adjuvant (FIA), Ribi (MPL), Ribi (TDM), Ribi (MPL+TDM), Bordetella pertussis vaccine, muramyl-dipeptide (MDP), aluminum adjuvant (ALUM), and a combination thereof. As an animal used for immunization, a mouse, rat, rabbit, goat, horse, and so on, is preferably used.

A polyclonal antibody can be obtained from serum of an animal subjected to the above-described immunological means by a well-known method for collecting antibodies. A preferable means is exemplified by immunoaffinity chromatography.

A monoclonal antibody can be produced by collecting antibody-producing cells (for example, a lymphocyte derived from a spleen or a lymph node) from the animal subjected to the above-described immunological method, followed by introducing a well-known transformation technique with a permanently proliferating cell (for example, myeloma strain such as P3X63Ag8 cells.) For example, the antibody-producing cells are fused with the permanent proliferating cells by a well-known method to prepare hybridomas. Then, the hybridomas are subjected to cloning, followed by selecting ones producing the antibody that recognizes specifically the above-described polypeptide or peptide to collect the antibody from a culture solution of the hybridoma.

The polyclonal antibody or the monoclonal antibody thus obtained, which recognizes and binds to the above-described polypeptide or peptide, can be utilized as an antibody for purification, a reagent, a labeling marker and so on.

Method for screening for a compound

The above-described polypeptide or peptide, the polynucleotide encoding the same or the complementary strand thereof, the cell transformed based on the information concerning the amino acid sequence and nucleotide sequence, or the antibody immunologically recognizing the same, provide an effective means for screening a substance capable of enhancing the recognition of the same by CTL, when using them solely or in combination with each other. The screening method can be constructed utilizing a well-known screening system. For example, there can be a system in which HLA-A2 restricted CTL (such as OK-CTL) is cultured together with an antigen-presenting cell that is pulsed with a tumor antigen peptide followed by measuring an amount of IFN-γ produced from the CTL for using as an indicator to detect the recognition of the peptide by the CTL. Addition of a test substance to the system allows one to select a substance capable of enhancing the recognition of the above-described polypeptide or peptide by HLA-A2 restricted CTL. Using HLA-A26-restricted CTL (such as KE4-CTL) in place of HLA-A2-restricted CTL in the system, allows one to select a substance capable of enhancing the recognition of the above polypeptide or peptide by HLA-A26-restricted CTL. This system describes one screening method, but the screening method according to the present invention is not limited thereto.

A compound obtained by the above-described screening method is also part of the present invention. The compound may be a compound that interacts with the above-described polypeptide or peptide recognized by CTL in an HLA-A2 restricted manner, for example, a polypeptide or peptide consisting of the amino acid sequence of any one of those of SEQ ID NO:1 to SEQ ID NO:213 and SEQ ID NO:358 to SEQ ID NO:381, and/or HLA-A2 molecule, and enhances the recognition of the polypeptide or the peptide by HLA-A2 restricted CTL. Further, it may be a compound that interacts with the polynucleotide encoding the above-described polypeptide or peptide recognized by CTL in an HLA-A2 restricted manner, and enhances the expression of the same.

A compound may be also provided that interacts with the above-described polypeptide or peptide recognized by CTL in an HLA-A26 restricted manner, for example, a polypeptide consisting the amino acid sequence of any one of those of SEQ ID NO:385 to SEQ ID NO:387 or a peptide consisting the amino acid sequence of any one of those of SEQ ID NO:388 to SEQ ID NO:408, and/or HLA-A26 molecule, and enhances the recognition of the polypeptide or the peptide by HLA-A26 restricted CTL. Further, a compound may be provided that enhances the expression of the polynucleotide encoding the above-described polypeptide or peptide.

The compound thus selected can be used in a pharmaceutical composition by further selecting ones in consideration of the balance of biological usefulness and toxicity.

Pharmaceutical composition

A polypeptide or peptide provided in the present invention can be used as a tumor antigen or tumor antigen peptide to induce and/or activate a tumor-specific CTL. In other words, a medicament consisting of one or more selected from the above polypeptides and peptides, a method for inducing CTL comprising using one or more selected from the above polypeptides and peptides, and an inducer of CTL comprising one or more selected from the above polypeptides and peptides, are also included in the scope of the present invention.

Moreover, the present invention provides a pharmaceutical composition that contains at least one selected from a group consisting of the above-described polypeptide and peptide, the polynucleotide encoding the polypeptide and peptide, or the complementary strand thereof, the recombinant vector prepared based on the information of these amino acid sequence and nucleotide sequence, the cell transformed by the recombinant vector, the antibody immunologically recognizing the polypeptide or peptide, the compound enhancing the recognition of CTL through interaction with the polypeptide and peptide, and the compound enhancing the expression of the polynucleotide through interaction therewith, when using them solely or in combination with each other. The pharmaceutical composition is useful for treating cancers. The above polypeptide or peptide obtained from colon cancer cell line SW620 is useful, for example, for treating colon cancer. The above polypeptide or peptide obtained from esophageal cancer cell line KE4 is useful, for example, for treating esophageal cancer, oral squamous cell cancer, and renal cancer. The above polypeptide or peptide is also applicable to various cancers such as gynecological cancers (e.g., breast cancer, endometrial cancer, cervical cancer, ovarian cancer) and prostate cancer. HLA-A2 allele is found in approximately 40% of Japanese, approximately 53% of Chinese, approximately 49% of North Caucasians, approximately 38% of South Caucasians, and approximately 23% of African Blacks, while HLA-A26 allele is found in approximately 22% of Japanese, approximately 16% of Korean, and approximately 8% of North Caucasians [Imanishi, T. et al., HLA 1991, 1: 1065-1220, 1992, Oxford, Oxford Scientific publications]. Therefore, the above medicament and pharmaceutical composition would be effective for many patients.

Concretely, for example, a medicament consisting of one or more selected from the group consisting of the above polypeptide and peptide, and a pharmaceutical composition consisting of one or more selected from the group consisting of the above polypeptide and peptide, can be used as so-called "cancer vaccine". "Cancer vaccine", herein, means a medicament capable of damaging a cancer cell selectively by inducing and/or enhancing a specific immunological response to a cancer cell. The amount of administration thereof can be determined depending on the degree of the recognition of the polypeptide or the peptide by CTL. Generally, as an active ingredient, the amount ranges 0.01 mg to 100 mg/day/adult human body, preferably 0.1 mg to 10 mg/day/adult human body. Administration can be carried out according to well-known methods for administrating a peptide for medical use, preferably subcutaneously, intravenously, or intramuscularly. When administering, in order to induce and/or enhance an immunological response, a polypeptide and/or peptide according to the present invention can be used in the presence or absence of an appropriate adjuvant with or without linking such to a carrier. Any carriers can be used as long as it is not harmful to the human body and can enhance the immunogenicity, such as cellulose, a polymerized amino acid, albumin, and so on. An adjuvant generally used for the peptide vaccination can be used for the present invention, such as Freund's incomplete adjuvant (FIA), aluminum adjuvant (ALUM), Bordetella pertussis vaccine, mineral oil, and so on. Moreover, the formulation can be appropriately selected from well-known methods for formulating peptides.

Alternately, an effective action of a cancer vaccine can also be obtained by inducing and/or activating CTL in a mononuclear cell fraction that is collected from the peripheral blood of a patient, followed by returning the fraction, back into the blood of the patient. Culture conditions, such as the concentration of mononuclear cells and the concentration of the polypeptide or the peptide according to the present invention, and the culture period, can be determined using simple experiments. Further, a substance, such as interleukin 2 (IL-2) having an ability to induce the growth of lymphocytes may be added to the culture.

In the case of using the above-described polypeptide or the peptide as a cancer vaccine, using even only one polypeptide or one peptide is effective as a cancer vaccine. However, plural kinds of the above-described polypeptide or peptide can be used in combination. Recently, it has been reported that multi-peptide based immunotherapy is effective [Miyagi, Y. et al., Clin. Cancer Res., 7: 3950-3962, 2001] [Lee, P. et al., J. Clin. Oncol., 19: 3836-3847, 2001] [Nestle, F. O. et al., Nat. Med., 4: 328-332, 1998]. CTL of a cancer patient is a cell population consisting of plural kinds of peptide-specific CTL, so that using plural kinds of polypeptides or peptides as an anti-cancer vaccine may give a higher effect than using only one kind. Especially in a cancer patient having both HLA-A26 and HLA-A2, a higher effect may be sometimes obtained when using the combination of a polypeptide or peptide recognized by an HLA-A26-restricted CTL and a polypeptide or peptide recognized by an HLA-A2-restricted CTL.

A polynucleotide encoding the above-described polypeptide or peptide, preferably the peptide, or its complementary strand can be used for the gene therapy of a cancer. Both a method in which one of these polynucleotides is carried on a vector and the obtained vector is directly transduced into a body for expression and a method in which a cell is collected from a human followed by introducing the polynucleotide into the cell in vitro for expression, can be utilized. Retrovirus, adenovirus, vaccinia virus, and so on, are known as a vector. Recommended is retrovirus. With respect to these viruses, replication-deficient viruses are used. Although the amount of administration is variable depending on the degree of the recognition of the polypeptide or peptide by CTL, it is generally 0.1 μg to 100 mg/day/adult human body, preferably 1 μg to 50 mg/day/human adult body, as an amount of DNA encoding a polypeptide or peptide according to the present invention. This amount is administered once every several days to several months.

Assay method and reagent

The above-described polypeptide or peptide, the polynucleotide encoding the polypeptide or peptide or its complementary strand, and the antibody that immunologically recognizes the polypeptide or the peptide, can be used independently for a diagnostic marker or a reagent, etc. When used as a reagent, the reagent can contain buffer(s), salt(s), stabilizing agent(s), and/or antiseptic(s.) Moreover, the present invention also provides a reagent kit comprising one container or more filled with one of them or more. For the preparation thereof, it is sufficient to use a well-known means for their preparation according to each of peptide, polypeptide, polynucleotide, antibody, and so on.

These reagents and reagent kits can be used for a screening method according to the present invention or can be used for the quantitative or qualitative measurement of a polypeptide or peptide according to the present invention or a polynucleotide encoding any one of these. A method for the measurement can be constructed by using well-known method(s) for those skilled in the art. Available methods include radioimmunoassay, competitive binding assay, Western blot analysis, ELISA, and so on. In addition, nucleic acids can be detected or quantified at an RNA level by using, for example, amplification, PCR, RT-PCR, RNase protection, Northern blotting and other hybridization methods.

The sample subjected to measurement is exemplified by the cells derived from an individual human body, blood, urine, saliva, spinal fluid, tissue biopsy, or autopsy material, and the like. The nucleic acid subjected to measurement is obtained from the each sample described above by a well-known method for nucleic acid preparation. For the nucleic acid, genomic DNA can be directly used for detection, or it may be enzymatically amplified by using PCR or any other amplification method before the analysis. RNA or cDNA may be similarly used.

In comparing with a normal genotype, a deletion or insertion of a gene can be detected by measuring the change in size of the above amplification product. Hybridizing the amplified product with the labeled DNA encoding the above-described polypeptide can permit identifying point mutations.

Approximately 87% of the above genes obtained from colon cancer cell line SW620 are more highly (about twice) expressed in colon cancers compared with normal colon tissues. For example, it was revealed that clone 10, clone 30, and clone 108, are selectively expressed in colon cancer cells (see Example 6.)

Although mRNA of KE4-cl.18 obtained from esophageal cancer cell line KE4 was expressed in all of the tested cancer cells and non-malignant tumor cell lines (PHA blastoid T cell and VA13), it was not expressed in normal tissues other than testis, muscle, and peripheral blood mononuclear cells. Moreover, although mRNA of KE4-cl.21 was expressed in all of the tested cancer cells and non-malignant tumor cell lines as well as normal tissue, the expression level in the normal tissue was low. It was revealed that these mRNAs are over-expressed especially in cancer cells in the head and the neck, and non-malignant proliferating cells.

Therefore, detecting mutation of, reduction of, and increase in the polypeptide or peptide according to the present invention and the DNA encoding any one of these, by the above-described measuring method, makes it possible to diagnose diseases, to which the polypeptide or peptide or DNA is associated, such as epithelial cell cancer and adenocarcinoma, and so on.

Examples

Although the present invention is described concretely by Examples below, the present invention is not limited to these examples.

Example 1

Establishment of HLA-A2-restricted CTL

An HLA-A2-restricted tumor-specific CTL was established from TIL of a colon cancer patient (HLA-A0207/3101, -B46/51, -Cw1) according to the method described in the literature [Int. J. Cancer, 81: 459-466, 1999] [J. Immunol., 163: 4997-5004, 1999]. TIL obtained from the colon tumor patient was cultured for a long period up to 50 days or longer with the addition of 100 U/ml of recombinant human interleukin 2 (IL-2.) Every 7 days, a portion of TIL activated by IL-2 was collected and cultured together with various kinds of tumor cells or normal cells to evaluate its CTL activity by measuring IFN-γ production and by measuring $^{51}$Cr released from the cancer cells (Gomi, S. et al., J. Immunol., 163:4997-5004, 1999.) IFN-γ was measured by an enzyme-linked immunosorbent assay (ELISA.) At day 58 after the start of the culture, CTL showing HLA-A2-restricted and tumor-specific CTL activity (which may be called as OK-CTL), was obtained. OK-CTL obtained is a cell population in which 80% of the cells have a phenotype of $CD3^+CD4^-CD8^+$ and 20% of the cells have a phenotype of $CD3^+CD4^+CD8^-$. The above-established CTLs were frozen in small portions and preserved until use.

OK-CTL recognized HLA-A0201$^+$ pancreatic adenocarcinoma cells, Panc-1, HLA-A0201$^+$ colon adenocarcinoma cells SW620, HLA-A0206+ squamous cell carcinoma (SCC) cells KE3, and HLA-A0207+ oral SCC cells CA9-22, resulting in production of IFN-γ, and showed adequate cytotoxicity against these cells. However, it did not show any cytotoxicity against HLA-A2- tumor cells, Epstein-Barr virus-transformed autologous B cells (EBV-B), and phytohemagglutinin (PHA) blast autologous T cells. Moreover, OK-CTL lysed all of the tested HLA-A2+ tumor cells (HLA-A0201+ breast adenocarcinoma R27, primary hepatocellular carcinoma HAK-2, melanoma SK-MEL-5, astrocytoma SF126, HLA-A0206+ pulmonary adenocarcinoma PC9, HLA-A0207+ pulmonary adenocarcinoma 1-87, and cervical SCC cells OMC-4.) The activity was inhibited by anti-HLA class I monoclonal antibody, anti-CD8 monoclonal antibody or anti-HLA-A2 monoclonal antibody (monoclonal antibody may be sometimes abbreviated as "mAb" hereafter), but not inhibited by other mAbs. Thus, it was confirmed that OK-CTL is an HLA-A2-restricted CTL.

The phenotype of an HLA class I allele of the above-described tumor cells has already been reported [Gomi, S. et al., J. Immunol., 163: 4997-5004, 1999]. Moreover, the serotype of HLA class I of the above patient was determined with peripheral blood mononuclear cells (PBMC) according to the conventional method. In addition, the HLA-A2 subtype was determined by the sequence-specific oligonucleotide probe method and direct DNA sequencing. The surface phenotype of OK-CTL was analyzed by the direct immunofluorescence method with fluorescein isothiocyanate (FITC)-labeled anti-CD3 mAb, anti-CD4 mAb, anti-CD8 mAb (Nichirei), or anti-TCRαβ mAb (WT31, Becton Dickinson.) Moreover, the antibodies used for testing the HLA-restriction and specificity of OK-CTL were anti-HLA class I mAb (W6/32, IgG2a), anti-HLA-A2 mAb (BB7.2, IgG2b), anti-CD8 mAb (Nu-Ts/c, IgG2a), anti-HLA class II mAb (H-DR-1, IgG2a), and anti-CD4 (Nu-Th/i, IgG1) mAb. Anti-CD13 mAb (MCS-2, IgG1) and anti-CD14 mAb (JML-H14, IgG2a) were used as isotype-matched control mAbs.

Example 2

Identification of cDNA clone encoding HLA-A2-restricted tumor antigen

A gene encoding a tumor antigen recognized by OK-CTLd that is one of sublines of OK-CTL was isolated and identified from a cDNA library of human colon cancer cell line SW620 according to the known method [Shichijo, S. et al., J. Exp. Med., 187: 277-288, 1998]. Cryopreserved OK-CTLd was cultured in the presence of 100 U/ml of IL-2 for 2 weeks or more to use in the experiments.

First of all, a poly (A)+RNA of SW620 cells was converted to cDNA, and ligated with a SalI adapter so as to insert into the expression vector pCMV-SPORT-2 (Invitrogen Corp.). Moreover, cDNA of HLA-A0207 was obtained by the reverse transcription-polymerase chain reaction (RT-PCR) and cloned into the eukaryotic cell expression vector pCR3 (Invitrogen Corp.).

A cDNA library of SW620 cells was pooled by 100 clones, and 200 ng of the cDNA pooled in each well and 200 ng of the HLA-A0207 cDNA were mixed in 100 μl of lipofectoamine (Invitrogen Corp.)/Opti-MEM (Invitrogen Corp.) 1:200 mixture for 30 min. 50 μl of the obtained mixture was added to a simian kidney cell line COS-7 ($1\times10^4$), and incubated for 6 h for co-transduction. Then, RPMI-1640 culture medium containing 10% FCS was added to and culturing was carried out for 2 days, followed by addition of OK-CTLd ($2X10^5$) to each well. After a further 18 h incubation, 100 μl of the supernatant was collected, and IFN-γ production was measured by ELISA. In this case, as a negative control, using COS-7 cells into which the gene had not been transfected as a target, IFN-γ production from OK-CTLd was examined and the value of IFN-γ produced was subtracted as a background from that of each measurement.

A pool of cDNA library was screened with the criteria in which cDNA capable of enhancing IFN-γ production from OK-CTLd was judged as a positive one having CTL-activating ability. After confirming reproducibility of CTL-activating ability of the pool, individual clones were taken up from the pool with which CTL-activating ability was observed, and screening was further carried out to select positive clones having CTL-activating ability from an independent pool. Dose dependency in OK-CTLd activation of clones obtained was confirmed, resulting to give finally 65 clones. On the other hand, with respect to COS-7 cells into which only expression vector pCMV-SPORT-2 was co-transduced together with HLA-A0207, IFN-γ production from OK-CTLd was not enhanced. As representative data, results with clone 2, clone 29, and clone 40 are illustrated in Fig. 1, Fig. 2, and Fig. 3, respectively. Similar results were obtained also with respect to other clones.

The nucleotide sequences of the cDNA clones obtained were determined by the dideoxynucleotide sequencing method using an ABI PRISM™ 377 DNA Sequencer (Perkin-Elmer, Inc.) and a DNA sequencing kit (Perkin-Elmer, Inc.). In addition, amino acid sequences encoding the cDNA clones were deduced from the nucleotide sequences (see Tables 1 to 6 above.) It was revealed that clone 76 (SEQ ID NO:346) is identical to clone 78 (SEQ ID NO:295) among the clones obtained.

With respect to each of the genes obtained, a homology search was carried out using GenBank/DDBJ, and the results obtained are summarized in Tables 1 to 6 above. Moreover, a homology search using the SEREX database (http://www-ludwig.unil.ch/SEREX.html) revealed that amino acid sequences encoded by clone 12, clone 65, clone 81, clone 86, and clone 100 are identical to ones encoded by known genes ID456, ID629, ID1226, ID163, and ID116, respectively, which are disclosed in SEREX database. Moreover, the nucleotide sequences of clone 12, clone 82, and clone 86 are identical to those of ID456, ID1197, and ID163, respectively. In addition, it was found that clone 32, clone 41, clone 74, and clone 87 are partially homologous to ID1072, ID1233, ID979, and ID567, respectively.

Example 3

Identification of HLA-A2-restricted tumor antigen peptide

In order to obtain tumor- antigen peptides from tumor antigens encoded by genes obtained in Example 2, an HLA-A2 binding motif (a specific sequence) was searched for amino acid sequences encoded by the genes using a home page (http://bimas.dcrt.nih.gov/molbio/hla_bind/) of Bioinformatics & Molecular Analysis Section (BIMAS.) The type of HLA of OK-CTLd is HLA-A0207, so that motif search was carried out for peptides capable of binding to HLA-A0207 molecule. HLA-A0207 molecule is different from HLA-A0201 molecule only in the 123$^{rd}$ amino acid residue in the amino acid sequence, which is Y in the sequence of the former and is C in the sequence of the latter. This amino acid residue is not located in an $\alpha$-helix or $\beta$-sheet that is related with peptide binding, but is located in a coil region of the secondary structure, so that the difference of this amino acid residue does not affect the peptide binding. Therefore, a peptide that is suitable for an HLA-A0201-binding motif [Rammensee, H.-G. et al., Immunogenetics, 41: 178-228, 1995] would bind to HLA-A0207. Then, peptides capable of binding to an HLA-A0207 molecule were designed based on a result obtained by searching an HLA-A0201-binding motif, and various peptides of 9-mer and 10-mer (whose purity is 70% or higher) were synthesized by a well-known method. Moreover, with respect to clone 5, clone 23, clone 26, clone 35, clone 65, clone 81, and clone 100, peptides were designed and synthesized based on the amino acid sequences encoded by genes highly homologous to each gene.

In order to examine the recognition of the above-described peptide by CTL, T2 cells were used as an antigen-presenting cell. T2 cells express the HLA-A2 molecule on a cell surface without binding to a peptide, because of deficiency of transporters associated with antigen processing (TAP) [Cancer Res., 54: 1071-1076, 1994.] First of all, each synthesized peptide (10 $\mu$M) described above was incubated together with T2 cells under 5% $CO_2$/95% air at 37°C for 3 h to make the peptide bind to HLA-A2 expressed on the cell surface. T2 cells thus pulsed with a peptide was used as a target cell (T). In addition, OK-CTLd obtained in Example 1 was used as an effector cell (E). Target cells ($1\times10^4$) and effector cells ($2\times10^4$) were mixed (E/T ratio = 2) and incubated for 18 h, followed by collecting 100 $\mu$l of supernatant to measure IFN-$\gamma$ by ELISA. At this time, the amount of IFN-$\gamma$ produced from OK-CTLd against T2 cells that had not been pulsed with the peptide was subtracted as a background from each measurement value. As a result, 213 peptides, each consisting of amino acid sequence of SEQ ID NO:1 to 213 in the sequence listing, were recognized by OK-CTLd and enhanced IFN-$\gamma$ production from OK-CTLd in a dose dependent manner of each peptide. The obtained 213 peptides were P1 to P4 (SEQ ID NO:1 to SEQ ID NO:4) derived from clone 12; P26, P28, P34, P38 and P39 (SEQ ID NO:5 to SEQ ID NO:9) derived from clone 40; P45 (SEQ ID NO:10) derived from clone 43; P46, P48, P53 and P54 (SEQ ID NO:11 to SEQ ID NO:14) derived from clone 2; P57 and P64 (SEQ ID NO:15 and SEQ ID NO:16) derived from clone 11; P71, P73 and P75 (SEQ ID NO:17 to SEQ ID NO:19) derived from clone 8; P77, P79, P80 and P81 (SEQ ID NO:20 to SEQ ID NO:23) derived from clone 78; P85, P86 and P88 (SEQ ID NO:24 to SEQ ID NO:26) derived from clone 67; P90-P93 and P95 (SEQ ID NO:27 to SEQ ID NO:31) derived from clone 9; P98, P100, P102, P103 and P104 (SEQ ID NO:32 to SEQ ID NO:36) derived from clone 95; P110 to P112 (SEQ ID NO:37 to SEQ ID NO:39) derived from clone 82; P118, P120 and P121 (SEQ ID NO:40 to SEQ ID NO:42) derived from clone 103; P122 and P131 (SEQ ID NO:43 and SEQ ID NO:44) derived from clone 4; P132 and P133 (SEQ ID NO:45 and SEQ ID NO:46) derived from clone 14; P138 to P141, P144, P146, P149, P150 and P152 (SEQ ID NO:47 to SEQ ID NO:55) derived from clone 65; P153 (SEQ ID NO:56) derived from clone 69; P175 to P177, P179 and P180 (SEQ ID NO:57 to SEQ ID NO:61) derived from clone 83; P181 to P184 (SEQ ID NO:62 to SEQ ID NO:65) derived from clone 84; P193 to P195 and P197 (SEQ ID NO:66 to SEQ ID NO:69) derived from clone 32; P201, P214, P217 and P226 (SEQ ID NO:70 to SEQ ID NO:73) derived from clone 21; P229, P230 and P236 (SEQ ID NO:74 to SEQ ID NO:76) derived from clone 33; P242 and P243 (SEQ ID NO:77 and SEQ ID NO:78) derived from clone 68; P247, P249, P251, P255 and P259 (SEQ ID NO:79 to SEQ ID NO:83) derived from clone 100; P260 and P262 to P265 (SEQ ID NO:84 to SEQ ID NO:88) derived from clone 73; P268, P272 and P273 (SEQ ID NO:89 to SEQ ID NO:91) derived from clone 27; P277 to P280 and P282 (SEQ ID NO:92 to SEQ ID NO:96) derived from clone 26; P294, P295 and P297 (SEQ ID NO:97 to SEQ ID NO:99) derived from clone 56; P300, P302 and P303 (SEQ ID NO:100 to SEQ ID NO:102) derived from clone 5; P312, P317 and P319 (SEQ ID NO:103 to SEQ ID NO:105) derived from clone 10; P321 to P323 and P330 (SEQ ID NO:106 to SEQ ID NO:109) derived from clone 22; P333, P340, P342, P344, P347 and P348 (SEQ ID NO:110 to SEQ ID NO: 115) derived from clone 30; P354, P358 and P360 (SEQ ID NO:116 to SEQ ID NO:118) derived from clone 88; P362, P363, P367, P369, P379 and P380 (SEQ ID NO:119 to SEQ ID NO:124) derived from clone 45; P384 and P385 (SEQ ID NO:125 and SEQ ID NO:126) derived from clone 58; P388 and P389 (SEQ ID NO:127 and SEQ ID NO:128) derived from clone 18; P390 and P391 (SEQ ID NO:129 and SEQ ID NO:130) derived from clone 87; P393 and P394 (SEQ ID NO:131 and SEQ ID NO:132) derived from clone 24; P400 and P401 (SEQ ID NO:133 and SEQ ID NO:134) derived from clone 46; P406, P409, P410, P412, P418, P421 and P424 (SEQ ID NO:135 to SEQ ID NO:141) derived from clone 110; P426, P429, P434, P436, P440 and P441 (SEQ ID NO:142 to SEQ ID NO:147) derived from clone 20; P443 and P445 to P447 (SEQ ID NO:148 to SEQ ID NO:151) derived from clone 6; P448 to P450, P453, P455-P457 and P461 (SEQ ID NO:152 to SEQ ID NO:159) derived from clone 108; P470 (SEQ ID NO:160) derived from clone 23; P479, P480, P482 to P485, P489, P492 and P495 (SEQ ID NO:161 to SEQ ID NO:169) derived from clone 86; P497 (SEQ ID NO:170) derived from clone 89; P511 (SEQ ID NO:171) derived from clone 92; P515, P522 to P524, P528, P529, P532, P533, P536, P525 and P526 (SEQ ID NO:172 to SEQ ID NO:182) derived from clone 85; P538 and P539 (SEQ ID NO:183 and SEQ ID NO:184) derived from clone 3; P542, P543, P546 and P548 (SEQ ID NO:185 to SEQ ID NO:188) derived from clone 29; P550, P555 and P556 (SEQ ID NO:189 to SEQ ID NO:191) derived from clone 35; P570 and P579 (SEQ ID NO:192 and SEQ ID NO:193) derived from clone 81; P583, P587, P589, P590, P596 and P597 (SEQ ID NO:194 to SEQ ID NO:199) derived from clone 114; P603 (SEQ ID NO:200) derived from clone 19; P614, P616, P617 and P623 (SEQ ID NO:201 to SEQ ID NO:204) derived from clone 74; P634 and P636 (SEQ ID NO:205 and SEQ ID NO:206) derived from clone 41; P642 and P644 (SEQ ID NO:207 and SEQ ID NO:208) derived from clone 79; P649 and P654 (SEQ ID NO:209 and SEQ ID NO:210) derived from clone 36; and P656, P658 and P661 (SEQ ID NO:211 to SEQ ID NO:213) derived from clone 76. Among these, P77 (SEQ ID NO:20) and P79 (SEQ ID NO:21) derived from clone 78 were identical to P656 (SEQ ID NO:211) and P658 (SEQ ID NO:212) derived from clone 76, respectively.

Results with peptides derived from clone 2, clone 29, and clone 40 are representatively illustrated in Figs. 4, 5, and 6, respectively. In the figures, peptides recognized by OK-CTLd and judged as ones capable of activating OK-CTLd are shown with bold (solid) lines. A peptide (SLYNTVATL) (negative control) derived from HIV (human immunodeficiency virus) that is suitable for an HLA-A2-binding motif was not recognized by OK-CTLd.

Example 4

Specificity of TCR that recognizes a peptide

Tumor antigen peptides obtained in Example 3 are presented on the cell surface by HLA-A2 and recognized by a peptide-specific OK-CTLd clone together with HLA molecule through a T cell receptor (TCR) expressed on each clone. It is reported that the diversity of TCR is larger than $10^{17}$, and the type of HLA molecule recognized and the peptide recognized are totally depending on the TCR.

Therefore, using 480 sublines (which, hereinafter, may be called "OK-CTLd sublines") established using OK-CTLd as a parent strain, the specificity of each subline in the recognition of a tumor cell and the above peptide was examined. OK-CTLd sublines were established from a parent strain (OK-CTLd) by incubating the strain at a density of 1,000 cells/well according to the method described in the literature [Ito, M. et al., Cancer Res., 61: 2038-2046, 2001]. The recognition by OK-CTLd sublines of tumor cells (SW620, and COS-7 as a negative control) and of T2 cell pulsed with the above-described peptide was examined in a manner similar to Example 3 using IFN-γ production from the subline as an index.

As a result, one hundred one of the OK-CTLd sublines (21%) reacted with SW620 and produced a significantly higher level of IFN-γ than the amount produced in the reaction against COS-7. Results of twenty-seven sublines that showed good growth are summarized in Table 9. The symbol of "-" in Table 9 denotes that the amount of IFN-γ produced is 20 pg/ml or less. These sublines react with at least one peptide (eight sublines), maximally eight peptides (subline #70), with the average number of peptides that reacted with each subline being 2.6. Fifteen peptides shown in Table 9 were recognized by at least one subline (three peptides), maximally twenty-one sublines (P440 derived from clone 20, SEQ ID NO:146), with the average number of sublines that responded to each peptide being 4.7. Moreover, these sublines did not recognize T2 cells that were not pulsed with a peptide (which is designated as "NC" in Table 9) or T2 cells that were pulsed with a peptide derived from HIV suitable for an HLA-A2-binding motif (which is designated as "HIV" in Table 9.)

Table 9

| peptide | cDNA \ CTL subline | 9 | 13 | 24 | 28 | 32 | 33 | 34 | 38 | 39 | 40 | 43 | 46 | 48 | 49 | 51 | 54 | 56 | 61 | 65 | 66 | 68 | 70 | 72 | 81 | 89 | 91 | 96 |
|---|---|---|---|---|---|---|---|---|---|---|---|---|---|---|---|---|---|---|---|---|---|---|---|---|---|---|---|---|
| | | IFN-γ produced from OK-CTLd-9 subline (pg/ml) | | | | | | | | | | | | | | | | | | | | | | | | | | |
| P1 | #012 | - | - | - | - | - | - | - | 100 | - | - | - | - | - | - | - | - | - | - | - | - | 122 | - | - | - | - | - | - |
| P46 | #002 | - | - | - | - | - | - | 174 | - | - | - | - | - | - | - | - | - | - | - | - | - | - | - | - | - | - | - | - |
| P77 | #076 | - | - | - | - | - | - | - | 216 | - | - | - | - | - | - | - | - | - | - | - | - | - | 116 | - | - | - | - | - |
| P102 | #095 | - | - | - | - | - | - | 156 | - | - | - | 148 | - | - | - | - | - | - | - | - | - | - | - | 134 | - | - | - | - |
| P180 | #083 | - | - | - | - | - | - | - | 276 | - | - | - | - | - | - | - | - | - | - | - | - | - | 146 | - | - | - | - | 105 |
| P264 | #073 | 392 | - | - | 242 | - | - | 340 | 850 | 162 | - | - | 238 | - | - | 310 | - | - | - | - | 112 | - | 354 | - | - | - | - | - |
| P295 | #056 | 108 | - | - | - | - | - | - | - | - | - | 128 | - | - | - | - | - | - | - | - | 106 | - | 280 | 104 | - | - | 170 | - |
| P319 | #010 | - | - | - | - | - | - | 194 | - | - | - | - | 116 | - | - | - | - | - | - | - | - | - | 340 | - | - | 112 | - | - |
| P424 | #110 | - | - | - | - | - | - | 166 | 104 | - | - | - | - | - | - | - | - | - | - | - | - | - | - | - | - | - | - | 130 |
| P440 | #020 | 122 | 102 | 224 | 536 | - | 118 | 498 | 492 | 650 | 202 | - | 150 | 156 | - | - | 180 | 162 | 180 | 132 | 192 | 306 | 198 | - | 130 | 123 | 196 | - |
| P449 | #108 | - | - | - | - | - | - | - | - | - | - | - | - | - | - | - | - | - | - | - | - | - | - | 120 | - | - | - | - |
| P480 | #086 | - | - | - | - | - | - | - | - | - | - | - | - | - | - | - | - | - | - | - | - | - | 134 | - | - | - | - | - |
| P533 | #085 | - | - | 386 | - | - | - | - | - | - | - | - | - | - | - | - | - | - | - | - | 110 | - | 344 | - | - | 102 | - | - |
| P546 | #029 | - | - | - | - | - | - | - | - | - | - | - | - | - | 156 | - | - | 230 | - | - | - | - | - | - | - | - | - | - |
| P590 | #114 | 636 | 153 | 328 | 360 | 106 | - | - | - | - | 220 | - | 270 | - | - | - | - | - | 106 | - | - | - | - | - | - | - | - | - |
| HIV | | - | - | - | - | - | - | - | - | - | - | - | - | - | - | - | - | - | - | - | - | - | - | - | - | - | - | - |
| NC | | - | - | - | - | - | - | - | - | - | - | - | - | - | - | - | - | - | - | - | - | - | - | - | - | - | - | - |
| COS-7 | | - | - | - | 394 | - | - | 172 | 402 | 272 | 294 | - | 296 | 188 | 306 | 552 | - | 364 | 336 | - | 234 | 246 | 216 | - | 134 | - | 354 | - |
| SW620 | | 100 | 168 | 154 | 554 | 184 | 294 | 334 | 568 | 314 | 360 | 284 | 404 | 324 | 432 | 782 | 180 | 482 | 434 | 280 | 368 | 420 | 308 | 198 | 230 | 152 | 420 | 248 |

OK-CTLd sublines were pooled to use as effector cells, and cytotoxicity against target cells was examined

by the $^{51}$Cr release assay. As a target cell, a T2 cell was used that was pulsed with P1 (SEQ ID NO:1) derived from clone 12, P424 (SEQ ID NO:141) derived from clone 110, P440 (SEQ ID NO:146) derived from clone 20, P449 (SEQ ID NO:153) derived from clone 108, or P590 (SEQ ID NO:197) derived from clone 114, and labeled with $^{51}$Cr. Effector cells and target cells were mixed each other at an E/T ratio of 10, 20, and 40, and then incubated for 6 h, followed by measuring a radioactivity of $^{51}$Cr released in the culture supernatant. As a result, as shown in Fig. 7a, the OK-CTLd subline showed cytotoxicity at a significant level against target cells pulsed with a peptide, but did not show any cytotoxicity against target cells pulsed with a peptide derived from HIV suitable for an HLA-A2-binding motif (negative control.)

Moreover, when TCR Vβ of these OK-CTLd sublines that recognize each peptide was analyzed, it was revealed that the type differs in each subline (Fig. 7b.) TCR Vβ usages were analyzed according to the known method [Ito, M. et al., Cancer Res., 61: 2038-2046, 2001].

These results revealed that the difference of TCR Vβ makes CTL showing non-identical specificity against each peptide. On the other hand, all the tested TCR Vβ are expressed on OK-PBMC (a PBMC of a colon cancer patient who provided TIL used for establishing OK-CTL) or on PBMC of a healthy donor at a similar level.

Thus, it was revealed that even CTL established from a tumor-infiltrating lymphocyte of one patient are not homogeneous but are a cell population consisting of plural kinds of peptide-specific CTL and recognize various antigens and peptides.

Example 5

Analysis of HLA-A2-restricted CTL in PBMC of a tumor patient

Mononuclear cell fractions were prepared from the peripheral blood of three HLA-A2$^+$ cancer patients (i.e., a colon cancer patient OK who provided TIL used for establishing OK-CTL, another colon cancer patient TT, and a metastatic melanoma patient) and cultured in the presence of IL-2 for 30 days. Then, their reactivity to the cDNA clone encoding the tumor antigen, which was obtained in Example 2, was examined in a manner similar to Example 2. In addition, their reactivity to the peptide encoded by the cDNA clone was examined in a manner similar to Example 3. At this time, analysis was also carried out in a similar manner using GK-CTL established from a tumor infiltrating lymphocyte of a pulmonary cancer tissue [Gomi, S. et al., J. Immunol., 163: 4994-5004, 1999.]

Products of six genes (clone 20, clone 43, clone 56, clone 84, clone 86, and clone 108) were recognized by PBMC derived from colon cancer patient OK (which, hereinafter, may be called OK-PBMC) (Fig. 8a), and products of four genes (i.e., clone 20, clone 84, clone 86, and clone 108) were recognized by activated PBMC (which, hereinafter, may be called TT-PBMC) derived from colon cancer patient TT, and as a result IFN-γ production from each PBMC was enhanced.

Moreover, HLA-A2-binding peptides derived from these six gene products, namely three peptides, one peptide, two peptides, one peptide, one peptide, and one peptide encoded by clone 20, clone 43, clone 56, clone 84, clone 86, and clone 108, respectively, enhanced IFN-γ production from OK-PBMC in a dose dependent manner of each peptide (Fig. 8b.) These peptides are P430, P434, and P440 (SEQ ID NO:360, 144, and 146) derived from clone 20; P45 (SEQ ID NO:10) derived from clone 43;P288 (SEQ ID NO:358) and P289 (SEQ ID NO:359) derived from clone 56; P184 (SEQ ID NO:65) derived from clone 84; P485 (SEQ ID NO:166) derived from clone 86; and P449 (SEQ ID NO:153) derived from clone 108. OK-PBMC strongly reacted to P289 (SEQ ID NO:359) derived from clone 56, P449 (SEQ ID NO:153) derived from clone 108, and P485 (SEQ ID NO:166) derived from clone 86, and produced a greater amount of IFN-γ than that in the reaction to other peptides.

In addition, the IFN-γ production was inhibited by a monoclonal antibody against HLA class I antigen, CD8, or HLA-A2, but not inhibited by an anti-HLA class II antigen mAb, an anti-CD4 mAb, or an anti-CD14 mAb that was used as an isotype-matched control antibody. This revealed that the recognition of these peptides by the activated PBMC described above is restricted by HLA-A2 that is HLA-class I antigen and that PBMC activated by recognizing the peptide is CD8-positive CTL.

TT-PBMC recognized P431 (SEQ ID NO:361) derived from clone 20, P184 (SEQ ID NO:65) derived from clone 84, P486 (SEQ ID NO:363) derived from clone 86, and P463 (SEQ ID NO:362) derived from clone 108.

Among the above peptides, P288 (SEQ ID NO:358) and P289 (SEQ ID NO:359) derived from clone 56, P430 (SEQ ID NO:360) and P431 (SEQ ID NO:361) derived from clone 20, P463 (SEQ ID NO:362) derived from clone 108, and P486 (SEQ ID NO:363) derived from clone 86 did not strongly enhance IFN-γ production from OK-CTLd in the examination of Example 3, but, as described above, were recognized by a PBMC of a colon cancer patient in an HLA-A2-restricted manner and strongly enhanced IFN-γ production from the same.

Thus, it was revealed that at least products of the above genes (i.e., clone 20, clone 43, clone 56, clone 84, clone 86, and clone 108) and peptides derived from the gene products, namely, the above twelve peptides were recognized by PBMC of a colon cancer patient and can activate CTL in an HLA-2-restricted manner. On the other hand, the above gene products were not recognized by GK-CTL or activated PBMC derived from a metastatic melanoma patient or PBMC derived from a healthy donor.

Thus, it was revealed that a CTL precursor recognizing the above tumor antigens and peptides derived therefrom in an HLA-A2-restricted manner is present in PBMC of a colon cancer patient. Therefore, these tumor antigens and peptides derived therefrom would be able to induce and/or activate a tumor-specific CTL in an HLA-A2-restricted manner in this patient.

Example 6

Expression level of gene

Expression level of genes obtained in Example 2 in colon cancer (four colon cancer cell lines and two primary tumors) and normal colon tissue (two colon epithelial tissues) were examined by the SAGE analysis [Velculescu, V. E., et al. Science, 270: 484-487, 1995.] Comparison of expression level of cDNA between colon cancer and normal colon tissue revealed that approximately 87% of the above genes are more highly expressed in colon cancer than in normal colon tissue with the ratio being about twice. On the contrary, clone 11, clone 35, clone 84, clone 95, and clone 110 were more highly expressed in normal colon tissue.

In addition, expression of clone 10, clone 30, clone 41, and clone 108 in various cells was examined by the northern blot analysis. The northern blot analysis was carried out using total RNA (10 μg/lane) extracted from PBMC or PHA-blast derived from a healthy donor, SW620 (colon cancer cell line), or Panc-1 (pancreatic cancer cell line), according to the method described in the paper [Nishizaka, S. et al., Cancer Res., 60: 4830-4837, 2000.] The following $^{32}$P-labelled probe cDNAs were used: a 593 bp cDNA fragment obtained by digesting clone 10 with AccI so as to contain the 2, 028$^{th}$ to the 2,260$^{th}$ nucleotide; a 1,020 bp cDNA fragment obtained by digesting clone 30 with DraI and HincII to contain the 885$^{th}$ to the 1,904$^{th}$ nucleotide; an 812 bp cDNA fragment obtained by digesting clone 41 with AccI so as to contain the 2,709$^{th}$ to the 3,520$^{th}$ nucleotide; and a 1,109 bp cDNA fragment obtained by digesting clone 108 with AvaII so as to contain the 2,146th to the 3,254$^{th}$ nucleotide. Moreover, β-actin mRNA was examined similarly as a control concerning the expression degree. As a result, it was revealed that clone 10, clone 30, and clone 108 are expressed selectively in a colon cancer cell (Fig. 9.)

Example 7

Isolation/identification of cDNA clone encoding an HLA-A2 tumor antigen

Two new cDNA clones SW620-cl.48 (SEQ ID NO:354) and SW620-cl.121 (SEQ ID NO:355) that are recognized by OK-CTLd were obtained by screening a cDNA library pool of SW620 in a manner similar to Example 2. As illustrated in Figs. 10a and 10b, each of these cDNA clones was recognized by OK-CTLd in an HLA-A2-restricted manner and in a dose dependent manner of plasmid, and enhanced IFN-γ production from OK-CTLd. On the other hand, in the case where COS-7 cells into which only expression vector pCMV-SPORT-2 was co-transduced together with HLA-A0207, IFN-γ production from OK-CTLd was not enhanced. Thus, two cDNA clones encoding tumor antigens, which are recognized by CTL in an HLA-A2-restricted manner and can activate CTL, were obtained.

Nucleotide sequences of the cDNA clones obtained were determined in a manner similar to Example 2, and an amino acid sequence encoded by each cDNA clone was deduced from each nucleotide sequence. Moreover, a homology search was carried out for GenBank with respect to the nucleotide sequence of each clone obtained. The result is summarized in Table 7 described above.

Example 8

Identification of an HLA-A2-restricted tumor antigen peptide

Based on amino acid sequences encoded by genes SW620-cl.48 and SW620-cl.121 obtained in Example 7, various peptides of 9-mer and 10-mer suitable for an HLA-A0207 binding motif (a specific sequence) were designed and synthesized by a well-known method in a manner similar to Example 3, and were obtained with a purity of 70% or higher.

Among the synthesized peptides (1.22 ng/ml to 20 μg/ml), peptides recognized by OK-CTLd in an HLA-A2-restricted manner were selected in a manner similar to Example 3. As a result, eighteen peptides of SEQ ID NO:364 to 381 in the sequence listing were recognized by OK-CTLd in a dose dependent manner and enhanced IFN-γ production from OK-CTLd, while a peptide (SLYNTVATL) derived from HIV that was used in place of the above peptide as a negative control did not enhance IFN-γ production from CTL. The obtained eighteen peptides are SW620-48·P162, SW620-48·P163, SW620-48·P165, SW620-48·P166, SW620-48·P173, and SW620-48·P174 (SEQ ID NO:364 to 369) derived from SW620-cl.48 as well as SW620-121·P665, SW620-121·P666, SW620-121·P667, SW620-121·P668, SW620-121·P669, SW620-121·P676, SW620-121·P677, SW620-121·P678, SW620-121·P679, SW620-121-P685, SW620-121·P686, and SW620-121·P688 (SEQ ID NO:370 to 381) derived from SW620-cl.121. As representative data, Fig. 11 illustrates that six peptides derived from SW620-cl.48 were recognized by OK-CTLd in a dose dependent manner and enhanced IFN-γ production from OK-CTLd.

Example 9

Establishment of HLA-A26-restricted CTL

HLA-A26-restricted tumor-specific CTL was established from an esophageal cancer patient (HLA-A2601/2402) according to a method described in the literature [Nakao, M., Cancer Res., 55:4248-4252, 1995.] As a result, a cell line showing tumor-specific cytotoxicity in an HLA-A26-restricted manner was obtained and designated as KE4-CTL. The established CTL was cryopreserved in small portions until use.

Example 10

Isolation/identification of cDNA clone encoding an HLA-A26-restricted tumor antigen

A gene encoding a tumor antigen recognized by KE4-CTL was isolated/identified from a human esophageal cancer cell line KE4 according to a known method [J. Exp. Med., 187: 277-288, 1998.] First of all, poly(A)$^+$RNA of KE4 cells was converted to cDNA, ligated with a SalI adaptor so as to insert into the expression vector pSV-SPORT-1 (Invitrogen Corp.). Moreover, cDNA of HLA-A2601 was obtained by the reverse transcription-polymerase chain reaction (RT-PCR) and cloned into the eukaryotic cell expression vector pCR3 (Invitrogen Corp.).

A cDNA library of KE4 cells was pooled by 100 clones, and 200 ng of the cDNA pooled in each well and 200 ng of the HLA-A2601 cDNA were mixed in 100 μl of lipofectoamine (Invitrogen Corp.)/Opti-MEM (Invitrogen Corp.) 1: 200 mixture for 30 min. 50 μl of the obtained mixture was added to VA13 cells ($1\times10^4$), and incubated for 6 h for co-transduction. Then, RPMI-1640 culture medium containing 10% FCS was added thereto and culturing was carried out for 2 days, followed by addition of KE4-CTL ($2X10^5$) to each well. After a further 18 h incubation, 100 μl of the supernatant was collected, and IFN-γ production was measured by ELISA.

A pool of cDNA library was screened with the criteria in which cDNA capable of enhancing IFN-γ production from KE4-CTL was judged as a positive one having CTL-activating ability. After confirming reproducibility of CTL-activating ability of the pool with which the ability was observed, individual clones were taken up from the pool and screening was further carried out to select positive clones having CTL-activating ability from an independent pool. Dose dependency of the obtained clones was confirmed in a similar manner, resulting in giving finally three cDNA clones KE4-cl.17, KE4-cl.18, and KE4-cl.21. These cDNA clones were recognized by KE4-CTL in an HLA-A26-restricted manner and in a dose dependent manner of plasmid, and enhanced IFN-γ production from KE4-CTL (Fig. 12.) On the other hand, when VA13 cell was used into which only expression vector pSV-SPORT-1 was co-transduced together with HLA-A2601, enhancement of IFN-γ production from KE4-CTL was not observed.

Nucleotide sequences of the cDNA clones obtained were determined in a manner similar to Example 2, and an amino acid sequence encoded by each of the cDNA clones was deduced from each of the nucleotide sequences. Moreover, with respect to the nucleotide sequence of each of the clones obtained, a homology search was carried out from GenBank. The result is summarized in Table 8 described above.

Example 11

Identification of an HLA-A26-restricted tumor antigen peptide

In order to obtain tumor-antigen peptides from genes KE4-17, KE4-18, and KE4-21 encoding tumor antigens, various peptides of 9-mer and 10-mer were designed and synthesized according to a method described in the literature [J. Exp. Med., 184: 735-740, 1996] based on each amino acid sequence encoded by each gene, and were obtained at a purity of 70% or higher. With respect to gene KE4-17, peptides were designed and synthesized based on amino acid sequences encoded by genes highly homologous to the gene.

After each of the synthesized peptides was pulsed to VA13 cells in which HLA-A2601 was expressed, the resultant cell was cultured together with KE4-CTL, followed by measuring IFN-γ produced from KE4-CTL so as to select the peptides recognized by CTL in an HLA-A26-restricted manner. First of all, HLA-A2601 plasmid at 100 ng/well was added to VA13 cells, and then incubated for two days for gene-transduction, followed by incubating in the presence of each of the synthesized peptides at 1.22 ng/ml to 20 μg/ml under the 5% $CO_2$/95% air at 37°C for 4 h so as to make the peptide binding to an HLA-A26 expressed on the cell surface. VA13 cells to which the peptide was pulsed was used as a target cell (T), and KE4-CTL was used as an effector cell (E). Target cells ($1\times10^4$) and effector cells ($2\times10^4$) were mixed (E/T ratio = 2), and then incubated for 18 h. After incubation, 100 μl of the supernatant was collected to measure IFN-γ by ELISA. At this time, the value of IFN-γ produced from CTL in response to VA13 cells to which a peptide was not pulsed, was subtracted as a background from that of each measurement. This examination was carried out twice for each peptide. In each of the two experiments, cryopreserved KE4-CTL was thawed and cultured using a feeder cell before use. Namely, lots of KE4-CTL used in the two experiments were different from each other.

As a result, twenty-one peptides of SEQ ID NO:388 to 408 in the sequence listing were recognized by KE4-CTL and enhanced IFN-γ production from KE4-CTL. The twenty-one peptides obtained were KE4-17·P12 and KE4-17·P19 (SEQ ID NO:393 and SEQ ID NO:394) derived from KE4-17; KE4-17·P1, KE4-17·P3, KE4-17·P5, KE4-17·P7, and KE4-17·P11 (SEQ ID NO:388 to SEQ ID NO:392) derived from a gene highly homologous to KE4-17; KE4-18·P5, KE4-18·P9, KE4-18·P15, KE4-18·P16, KE4-18·P22, KE4-18·P25, KE4-18□P26, and KE4-18.P27 (SEQ ID NO:395 to SEQ ID NO:402) derived from KE4-18; as well as KE4-21·P28, KE4-21·P29, KE4-21·P38, KE4-21·P39, KE4-21·P40, and KE4-21·P47 (SEQ ID NO:403 to SEQ ID NO:408) derived from KE4-21. Among these, KE4-17·P3 (SEQ ID NO: 389), KE4-17·P12 (SEQ ID NO:393), KE4-18·P15 (SEQ ID NO:397), KE4-18·P27 (SEQ ID NO:402), KE4-21·P28 (SEQ ID NO:403), and KE4-21·P40 (SEQ ID NO:407) were recognized by KE4-CTL and enhanced IFN-γ production from KE4-CTL in both of the two experiments. The remaining fifteen peptides were recognized by KE4-CTL and enhanced IFN-γ production from KE4-CTL only in one of the two experiments. The reason why the two experiments gave different results could be explained as follows: CTL is a cell population consisting of various peptide-specific CTL, so that in the case where different feeder cells are used for culturing after being thawed from a frozen state as described above, CTL showing different reactivity to peptides could be obtained.

As representative data, Fig. 13 illustrates that peptides derived from KE4-21 enhanced IFN-γ production from KE4-CTL in a dose dependent manner of the peptide. Figs. 13a and 13b illustrate the results of the two experiments.

Example 12

Induction of an HLA-A26-restricted CTL in PBMC

PBMC ($1\times10^5$) obtained from HLA-$A26^+$ cancer patients (four cases of oral squamous cell cancer, one case of renal cancer, and one case of pulmonary cancer) and five healthy donors were incubated with a peptide (purity > 90%) at 10 μM in the presence of 100 U/ml of IL-2. At day 3, 6, and 9 after starting the culture, a half of the culture medium was replaced with a fresh medium containing a corresponding peptide at 20 μM. After three times of stimulation, added was VA13 cells that were pulsed with a corresponding peptide after being transfected into with HLA-A2601, and incubation was carried out for 18 h, followed by measuring concentration of IFN-γ in the culture supernatant. Statistic analysis was carried out using Two-tailed Student's T-test.

Peptides KE4-18□P5 (SEQ ID NO:395), KE4-18□P22 (SEQ ID NO:399), and KE4-18□P25 (SEQ ID NO:400) derived from KE4-cl.18 as well as peptides KE4-21·P28 (SEQ ID NO:403), KE4-21·P29 (SEQ ID NO:404) and KE4-21·P40 (SEQ ID NO:407) derived from KE4-cl.21 were used for the examination. As a result, four peptides, one peptide and four peptides among the above six peptides were recognized by PBMC of three cancer patients (Pt.1, Pt. 2, and Pt.4), respectively, and significantly enhanced IFN-γ production from the PBMC (Fig. 14a.) KE4-18·P22 and KE4-21·P28 were recognized by PBMC of three cancer patients among five cases and by PBMC of two cancer patients among six cases, respectively, and enhanced IFN-γ production from the PBMC. On the other hand, these peptides did not enhance IFN-γ production from PBMC prepared from the rest of the three cancer patients and five healthy donors. These cellular responses were inhibited by an anti-HLA class I mAb and an anti-CD8 mAb, but not inhibited by other antibodies used for the test. These results revealed that a cell that recognizes the above peptide and produces IFN-γ is an HLA class I-restricted CD8-positive T lymphocyte in PBMC.

Cytotoxicity against HLA-$A26^+$ or $A26^-$ cancer cells was examined using PBMC stimulated with these peptides by carrying out the $^{51}Cr$ release test for 6 h at three different E/T ratios. PBMC stimulated with KE4-18·P22, KE4-21·P28, or KE4-21·P29 exhibited strong cytotoxicity against an HLA-$A26^+$ cancer cell (KE4), while it exhibited a weak cytotoxicity against an HLA-$A26^-$ cancer cell (KE3), and did not react with an HLA-$A26^+$ PHA-blast (Fig. 14b.) A significant difference was observed between the cytotoxicity against KE4 cells and that against KE3 cells.

These results revealed that HLA-A26-restricted tumor antigens and peptides derived therefrom according to the present invention can induce and/or activate tumor-specific CTL in an HLA-A26-restricted manner in PBMC of a cancer patient.

Example 13

With respect to genes KE4-cl.18 and KE4-cl.21 obtained in Example 10, expression of their mRNAs in tissues and various cancer cells was examined by the northern blot analysis. The northern blot analysis was carried out by a method similar to one described in Example 6. As a probe cDNA, ribosomal protein S2 cDNA and L10a cDNA highly homologous to KE4-cl.18 and KE4-cl.21, which were labelled with $^{32}P$, were used. Similarly, β-actin mRNA was examined as a control with respect to the expression degree [Shichijo, S. et al., J. Exp. Med., 187: 277-288, 1998.]

Based on the obtained results, relative expression levels were calculated using the Equation 1 below [Shichijo, S. et al., J. Exp. Med., 187: 277-288, 1998] and summarized in Table 10.

Equation 1

Index = (concentration of S2 or L10a in a specimen/

concentration of β-actin in a specimen) × (concentration of

β-actin in KE4 cells/concentration of S2 or L10a in KE4

cells)

KE4-cl.18 was expressed in all of the cancer cells tested (i.e., KE4, KE3, Kuma-1, Kuma-3, HSC-2, HSC-3, HSC-4, OSC-20, Ca9-22, QG56, Sq-l.LC99A, LK79, 11-18, LK87, and PC-9) as well as non-malignant tumor cell lines (PHA-blast and VA13) but not expressed in normal tissues other than testis, muscle, and peripheral blood mononuclear cells, which are brain, colon, heart, kidney, liver, lung, placenta, small intestine, spleen, and stomach.

Although KE4-cl.21 was expressed in all of the tested cancer cells and non-malignant tumor cell lines as well as normal tissues, the expression level in the normal tissue was low.

It was observed that mRNAs of KE4-cl.18 and KE4-cl.21 are over-expressed especially in cancer cells in the head and neck as well as in non-malignant proliferating cell.

Table 10

| Cell lines or Tissues | Relative Index | |
|---|---|---|
| | S2 mRNA | L10a |
| ***Esophageal SCCs*** | | |
| KE4 (control) | 1.0 | 1.0 |
| KE3 | 1.6 | 0.6 |
| ***Head and Neck SCCs*** | | |
| Kuma-1 | 1.6 | 1.2 |
| Kuma-3 | 1.5 | 1.2 |
| ***Oral SCCs*** | | |
| HSC-2 | 1.1 | 1.2 |
| HSC-3 | 1.3 | 1.3 |
| HSC-4 | 1.0 | 1.1 |
| OSC-20 | 1.0 | 1.2 |
| Ca9-22 | 1.5 | 1.1 |
| ***Lung SCCs*** | | |
| QG56 | 1.0 | 1.0 |
| Sq-1 | 1.6 | 1.0 |
| ***Lung large cell carcinoma*** | | |
| LC99A | 1.2 | 2.0 |

Table 10 (continued)

| Cell lines or Tissues | Relative Index | |
|---|---|---|
| ***Lung small cell carcinoma*** | | |
| LK79 | 0.9 | 0.6 |
| ***Lung adenocarcinomas*** | | |
| 11-18 | 1.2 | 0.9 |
| LK87 | 0.8 | 1.2 |
| PC-9 | 1.2 | 1.5 |
| ***Non-malignant proliferationg cells*** | | |
| PHA-blastoid T cell | 1.5 | 1.1 |
| VA13 | 1.2 | 0.8 |
| ***Normal tissues*** | | |
| Brain | <0.2 | 1.0 |
| Colon | <0.2 | <0.2 |
| Heart | <0.2 | <0.2 |
| Kidney | <0.2 | 0.5 |
| Liver | <0.2 | 0.5 |
| Lung | <0.2 | 0.7 |
| Muscle | 0.4 | 1.0 |
| Placenta | <0.2 | <0.2 |
| Small Intestine | <0.2 | 0.3 |
| Spleen | <0.2 | 0.3 |
| Stomach | <0.2 | <0.2 |
| Testis | 0.6 | 0.6 |
| PBMC | 0.6 | 0.8 |

INDUSTRIAL APPLICABILITY

The present invention makes it possible to induce and/or activate CTL in an HLA-A2-restricted manner or HLA-A26-restricted manner so as to conduct a specific immunotherapy for treating epithelial cancers, adenocarcinomas, and the like, such as colon cancer, esophageal cancer, oral cancer, renal cancer, pulmonary cancer, gynecological cancer, and prostate cancer. At present, 160,000 new colon cancer patients are found in the United States per year. Moreover, about 58,000 patients die due to colon cancer per year, which is approximately 18% of the mortality due to cancer. The colon cancer is the third cause of death due to cancer. An HLA-A2 allele is found in approximately 40% of Japanese, approximately 53% of Chinese, approximately 49% of North Caucasians, approximately 38% of South Caucasians, and approximately 23% of African Blacks. Moreover, an HLA-A26 allele is found in approximately 22% of Japanese, approximately 16% of Korean, and approximately 8% of North Caucasians. Therefore, the present invention can make a great contribution to the treatment of cancers. Moreover, the present invention will make a great contribution also to the basic research on molecules involved in the recognition of epithelial cancers, adenocarcinomas, and the like by a T lymphocyte.

Summary of the sequence listing

SEQ ID NO:1: Designed peptide recognized by HLA-A2 restricted cytotoxic T lymphocytes
SEQ ID NO:2: Designed peptide recognized by HLA-A2 restricted cytotoxic T lymphocytes
SEQ ID NO:3: Designed peptide recognized by HLA-A2 restricted cytotoxic T lymphocytes
SEQ ID NO:4: Designed peptide recognized by HLA-A2 restricted cytotoxic T lymphocytes
SEQ ID NO:5: Designed peptide recognized by HLA-A2 restricted cytotoxic T lymphocytes

SEQ ID NO:6: Designed peptide recognized by HLA-A2 restricted cytotoxic T lymphocytes
SEQ ID NO:7: Designed peptide recognized by HLA-A2 restricted cytotoxic T lymphocytes
SEQ ID NO:8: Designed peptide recognized by HLA-A2 restricted cytotoxic T lymphocytes
SEQ ID NO:9: Designed peptide recognized by HLA-A2 restricted cytotoxic T lymphocytes
SEQ ID NO:10: Designed peptide recognized by HLA-A2 restricted cytotoxic T lymphocytes
SEQ ID NO:11: Designed peptide recognized by HLA-A2 restricted cytotoxic T lymphocytes
SEQ ID NO:12: Designed peptide recognized by HLA-A2 restricted cytotoxic T lymphocytes
SEQ ID NO:13: Designed peptide recognized by HLA-A2 restricted cytotoxic T lymphocytes
SEQ ID NO:14: Designed peptide recognized by HLA-A2 restricted cytotoxic T lymphocytes
SEQ ID NO:15: Designed peptide recognized by HLA-A2 restricted cytotoxic T lymphocytes
SEQ ID NO:16: Designed peptide recognized by HLA-A2 restricted cytotoxic T lymphocytes
SEQ ID NO:17: Designed peptide recognized by HLA-A2 restricted cytotoxic T lymphocytes
SEQ ID NO:18: Designed peptide recognized by HLA-A2 restricted cytotoxic T lymphocytes
SEQ ID NO:19: Designed peptide recognized by HLA-A2 restricted cytotoxic T lymphocytes
SEQ ID NO:20: Designed peptide recognized by HLA-A2 restricted cytotoxic T lymphocytes
SEQ ID NO:21: Designed peptide recognized by HLA-A2 restricted cytotoxic T lymphocytes
SEQ ID NO:22: Designed peptide recognized by HLA-A2 restricted cytotoxic T lymphocytes
SEQ ID NO:23: Designed peptide recognized by HLA-A2 restricted cytotoxic T lymphocytes
SEQ ID NO:24: Designed peptide recognized by HLA-A2 restricted cytotoxic T lymphocytes
SEQ ID NO:25: Designed peptide recognized by HLA-A2 restricted cytotoxic T lymphocytes
SEQ ID NO:26: Designed peptide recognized by HLA-A2 restricted cytotoxic T lymphocytes
SEQ ID NO:27: Designed peptide recognized by HLA-A2 restricted cytotoxic T lymphocytes
SEQ ID NO:28: Designed peptide recognized by HLA-A2 restricted cytotoxic T lymphocytes
SEQ ID NO:29: Designed peptide recognized by HLA-A2 restricted cytotoxic T lymphocytes
SEQ ID NO:30: Designed peptide recognized by HLA-A2 restricted cytotoxic T lymphocytes
SEQ ID NO:31: Designed peptide recognized by HLA-A2 restricted cytotoxic T lymphocytes
SEQ ID NO:32: Designed peptide recognized by HLA-A2 restricted cytotoxic T lymphocytes
SEQ ID NO:33: Designed peptide recognized by HLA-A2 restricted cytotoxic T lymphocytes
SEQ ID NO:34: Designed peptide recognized by HLA-A2 restricted cytotoxic T lymphocytes
SEQ ID NO:35: Designed peptide recognized by HLA-A2 restricted cytotoxic T lymphocytes
SEQ ID NO:36: Designed peptide recognized by HLA-A2 restricted cytotoxic T lymphocytes
SEQ ID NO:37: Designed peptide recognized by HLA-A2 restricted cytotoxic T lymphocytes
SEQ ID NO:38: Designed peptide recognized by HLA-A2 restricted cytotoxic T lymphocytes
SEQ ID NO:39: Designed peptide recognized by HLA-A2 restricted cytotoxic T lymphocytes
SEQ ID NO:40: Designed peptide recognized by HLA-A2 restricted cytotoxic T lymphocytes
SEQ ID NO:41: Designed peptide recognized by HLA-A2 restricted cytotoxic T lymphocytes
SEQ ID NO:42: Designed peptide recognized by HLA-A2 restricted cytotoxic T lymphocytes
SEQ ID NO:43: Designed peptide recognized by HLA-A2 restricted cytotoxic T lymphocytes
SEQ ID NO:44: Designed peptide recognized by HLA-A2 restricted cytotoxic T lymphocytes
SEQ ID NO:45: Designed peptide recognized by HLA-A2 restricted cytotoxic T lymphocytes
SEQ ID NO:46: Designed peptide recognized by HLA-A2 restricted cytotoxic T lymphocytes
SEQ ID NO:47: Designed peptide recognized by HLA-A2 restricted cytotoxic T lymphocytes
SEQ ID NO:48: Designed peptide recognized by HLA-A2 restricted cytotoxic T lymphocytes
SEQ ID NO:49: Designed peptide recognized by HLA-A2 restricted cytotoxic T lymphocytes
SEQ ID NO:50: Designed peptide recognized by HLA-A2 restricted cytotoxic T lymphocytes
SEQ ID NO:51: Designed peptide recognized by HLA-A2 restricted cytotoxic T lymphocytes
SEQ ID NO:52: Designed peptide recognized by HLA-A2 restricted cytotoxic T lymphocytes
SEQ ID NO:53: Designed peptide recognized by HLA-A2 restricted cytotoxic T lymphocytes
SEQ ID NO:54: Designed peptide recognized by HLA-A2 restricted cytotoxic T lymphocytes
SEQ ID NO:55: Designed peptide recognized by HLA-A2 restricted cytotoxic T lymphocytes
SEQ ID NO:56: Designed peptide recognized by HLA-A2 restricted cytotoxic T lymphocytes
SEQ ID NO:57: Designed peptide recognized by HLA-A2 restricted cytotoxic T lymphocytes
SEQ ID NO:58: Designed peptide recognized by HLA-A2 restricted cytotoxic T lymphocytes
SEQ ID NO:59: Designed peptide recognized by HLA-A2 restricted cytotoxic T lymphocytes
SEQ ID NO:60: Designed peptide recognized by HLA-A2 restricted cytotoxic T lymphocytes
SEQ ID NO:61: Designed peptide recognized by HLA-A2 restricted cytotoxic T lymphocytes
SEQ ID NO:62: Designed peptide recognized by HLA-A2 restricted cytotoxic T lymphocytes
SEQ ID NO:63: Designed peptide recognized by HLA-A2 restricted cytotoxic T lymphocytes

SEQ ID NO:64: Designed peptide recognized by HLA-A2 restricted cytotoxic T lymphocytes
SEQ ID NO:65: Designed peptide recognized by HLA-A2 restricted cytotoxic T lymphocytes
SEQ ID NO:66: Designed peptide recognized by HLA-A2 restricted cytotoxic T lymphocytes
SEQ ID NO:67: Designed peptide recognized by HLA-A2 restricted cytotoxic T lymphocytes
SEQ ID NO:68: Designed peptide recognized by HLA-A2 restricted cytotoxic T lymphocytes
SEQ ID NO:69: Designed peptide recognized by HLA-A2 restricted cytotoxic T lymphocytes
SEQ ID NO:70: Designed peptide recognized by HLA-A2 restricted cytotoxic T lymphocytes
SEQ ID NO:71: Designed peptide recognized by HLA-A2 restricted cytotoxic T lymphocytes
SEQ ID NO:72: Designed peptide recognized by HLA-A2 restricted cytotoxic T lymphocytes
SEQ ID NO:73: Designed peptide recognized by HLA-A2 restricted cytotoxic T lymphocytes
SEQ ID NO:74: Designed peptide recognized by HLA-A2 restricted cytotoxic T lymphocytes
SEQ ID NO:75: Designed peptide recognized by HLA-A2 restricted cytotoxic T lymphocytes
SEQ ID NO:76: Designed peptide recognized by HLA-A2 restricted cytotoxic T lymphocytes
SEQ ID NO:77: Designed peptide recognized by HLA-A2 restricted cytotoxic T lymphocytes
SEQ ID NO:78: Designed peptide recognized by HLA-A2 restricted cytotoxic T lymphocytes
SEQ ID NO:79: Designed peptide recognized by HLA-A2 restricted cytotoxic T lymphocytes
SEQ ID NO:80: Designed peptide recognized by HLA-A2 restricted cytotoxic T lymphocytes
SEQ ID NO:81: Designed peptide recognized by HLA-A2 restricted cytotoxic T lymphocytes
SEQ ID NO:82: Designed peptide recognized by HLA-A2 restricted cytotoxic T lymphocytes
SEQ ID NO:83: Designed peptide recognized by HLA-A2 restricted cytotoxic T lymphocytes
SEQ ID NO:84: Designed peptide recognized by HLA-A2 restricted cytotoxic T lymphocytes
SEQ ID NO:85: Designed peptide recognized by HLA-A2 restricted cytotoxic T lymphocytes
SEQ ID NO:86: Designed peptide recognized by HLA-A2 restricted cytotoxic T lymphocytes
SEQ ID NO:87: Designed peptide recognized by HLA-A2 restricted cytotoxic T lymphocytes
SEQ ID NO:88: Designed peptide recognized by HLA-A2 restricted cytotoxic T lymphocytes
SEQ ID NO:89: Designed peptide recognized by HLA-A2 restricted cytotoxic T lymphocytes
SEQ ID NO:90: Designed peptide recognized by HLA-A2 restricted cytotoxic T lymphocytes
SEQ ID NO:91: Designed peptide recognized by HLA-A2 restricted cytotoxic T lymphocytes
SEQ ID NO:92: Designed peptide recognized by HLA-A2 restricted cytotoxic T lymphocytes
SEQ ID NO:93: Designed peptide recognized by HLA-A2 restricted cytotoxic T lymphocytes
SEQ ID NO:94: Designed peptide recognized by HLA-A2 restricted cytotoxic T lymphocytes
SEQ ID NO:95: Designed peptide recognized by HLA-A2 restricted cytotoxic T lymphocytes
SEQ ID NO:96: Designed peptide recognized by HLA-A2 restricted cytotoxic T lymphocytes
SEQ ID NO:97: Designed peptide recognized by HLA-A2 restricted cytotoxic T lymphocytes
SEQ ID NO:98: Designed peptide recognized by HLA-A2 restricted cytotoxic T lymphocytes
SEQ ID NO:99: Designed peptide recognized by HLA-A2 restricted cytotoxic T lymphocytes
SEQ ID NO:100: Designed peptide recognized by HLA-A2 restricted cytotoxic T lymphocytes
SEQ ID NO:101: Designed peptide recognized by HLA-A2 restricted cytotoxic T lymphocytes
SEQ ID NO:102: Designed peptide recognized by HLA-A2 restricted cytotoxic T lymphocytes
SEQ ID NO:103: Designed peptide recognized by HLA-A2 restricted cytotoxic T lymphocytes
SEQ ID NO:104: Designed peptide recognized by HLA-A2 restricted cytotoxic T lymphocytes
SEQ ID NO:105: Designed peptide recognized by HLA-A2 restricted cytotoxic T lymphocytes
SEQ ID NO:106: Designed peptide recognized by HLA-A2 restricted cytotoxic T lymphocytes
SEQ ID NO:107: Designed peptide recognized by HLA-A2 restricted cytotoxic T lymphocytes
SEQ ID NO:108: Designed peptide recognized by HLA-A2 restricted cytotoxic T lymphocytes
SEQ ID NO:109: Designed peptide recognized by HLA-A2 restricted cytotoxic T lymphocytes
SEQ ID NO:110: Designed peptide recognized by HLA-A2 restricted cytotoxic T lymphocytes
SEQ ID NO:111: Designed peptide recognized by HLA-A2 restricted cytotoxic T lymphocytes
SEQ ID NO:112: Designed peptide recognized by HLA-A2 restricted cytotoxic T lymphocytes
SEQ ID NO:113: Designed peptide recognized by HLA-A2 restricted cytotoxic T lymphocytes
SEQ ID NO:114: Designed peptide recognized by HLA-A2 restricted cytotoxic T lymphocytes
SEQ ID NO:115: Designed peptide recognized by HLA-A2 restricted cytotoxic T lymphocytes
SEQ ID NO:116: Designed peptide recognized by HLA-A2 restricted cytotoxic T lymphocytes
SEQ ID NO:117: Designed peptide recognized by HLA-A2 restricted cytotoxic T lymphocytes
SEQ ID NO:118: Designed peptide recognized by HLA-A2 restricted cytotoxic T lymphocytes
SEQ ID NO:119: Designed peptide recognized by HLA-A2 restricted cytotoxic T lymphocytes
SEQ ID NO:120: Designed peptide recognized by HLA-A2 restricted cytotoxic T lymphocytes
SEQ ID NO:121: Designed peptide recognized by HLA-A2 restricted cytotoxic T lymphocytes

SEQ ID NO:122: Designed peptide recognized by HLA-A2 restricted cytotoxic T lymphocytes
SEQ ID NO:123: Designed peptide recognized by HLA-A2 restricted cytotoxic T lymphocytes
SEQ ID NO:124: Designed peptide recognized by HLA-A2 restricted cytotoxic T lymphocytes
SEQ ID NO:125: Designed peptide recognized by HLA-A2 restricted cytotoxic T lymphocytes
SEQ ID NO:126: Designed peptide recognized by HLA-A2 restricted cytotoxic T lymphocytes
SEQ ID NO:127: Designed peptide recognized by HLA-A2 restricted cytotoxic T lymphocytes
SEQ ID NO:128: Designed peptide recognized by HLA-A2 restricted cytotoxic T lymphocytes
SEQ ID NO:129: Designed peptide recognized by HLA-A2 restricted cytotoxic T lymphocytes
SEQ ID NO:130: Designed peptide recognized by HLA-A2 restricted cytotoxic T lymphocytes
SEQ ID NO:131: Designed peptide recognized by HLA-A2 restricted cytotoxic T lymphocytes
SEQ ID NO:132: Designed peptide recognized by HLA-A2 restricted cytotoxic T lymphocytes
SEQ ID NO:133: Designed peptide recognized by HLA-A2 restricted cytotoxic T lymphocytes
SEQ ID NO:134: Designed peptide recognized by HLA-A2 restricted cytotoxic T lymphocytes
SEQ ID NO:135: Designed peptide recognized by HLA-A2 restricted cytotoxic T lymphocytes
SEQ ID NO:136: Designed peptide recognized by HLA-A2 restricted cytotoxic T lymphocytes
SEQ ID NO:137: Designed peptide recognized by HLA-A2 restricted cytotoxic T lymphocytes
SEQ ID NO:138: Designed peptide recognized by HLA-A2 restricted cytotoxic T lymphocytes
SEQ ID NO:139: Designed peptide recognized by HLA-A2 restricted cytotoxic T lymphocytes
SEQ ID NO:140: Designed peptide recognized by HLA-A2 restricted cytotoxic T lymphocytes
SEQ ID NO:141: Designed peptide recognized by HLA-A2 restricted cytotoxic T lymphocytes
SEQ ID NO:142: Designed peptide recognized by HLA-A2 restricted cytotoxic T lymphocytes
SEQ ID NO:143: Designed peptide recognized by HLA-A2 restricted cytotoxic T lymphocytes
SEQ ID NO:144: Designed peptide recognized by HLA-A2 restricted cytotoxic T lymphocytes
SEQ ID NO:145: Designed peptide recognized by HLA-A2 restricted cytotoxic T lymphocytes
SEQ ID NO:146: Designed peptide recognized by HLA-A2 restricted cytotoxic T lymphocytes
SEQ ID NO:147: Designed peptide recognized by HLA-A2 restricted cytotoxic T lymphocytes
SEQ ID NO:148: Designed peptide recognized by HLA-A2 restricted cytotoxic T lymphocytes
SEQ ID NO:149: Designed peptide recognized by HLA-A2 restricted cytotoxic T lymphocytes
SEQ ID NO:150: Designed peptide recognized by HLA-A2 restricted cytotoxic T lymphocytes
SEQ ID NO:151: Designed peptide recognized by HLA-A2 restricted cytotoxic T lymphocytes
SEQ ID NO:152: Designed peptide recognized by HLA-A2 restricted cytotoxic T lymphocytes
SEQ ID NO:153: Designed peptide recognized by HLA-A2 restricted cytotoxic T lymphocytes
SEQ ID NO:154: Designed peptide recognized by HLA-A2 restricted cytotoxic T lymphocytes
SEQ ID NO:155: Designed peptide recognized by HLA-A2 restricted cytotoxic T lymphocytes
SEQ ID NO:156: Designed peptide recognized by HLA-A2 restricted cytotoxic T lymphocytes
SEQ ID NO:157: Designed peptide recognized by HLA-A2 restricted cytotoxic T lymphocytes
SEQ ID NO:158: Designed peptide recognized by HLA-A2 restricted cytotoxic T lymphocytes
SEQ ID NO:159: Designed peptide recognized by HLA-A2 restricted cytotoxic T lymphocytes
SEQ ID NO:160: Designed peptide recognized by HLA-A2 restricted cytotoxic T lymphocytes
SEQ ID NO:161: Designed peptide recognized by HLA-A2 restricted cytotoxic T lymphocytes
SEQ ID NO:162: Designed peptide recognized by HLA-A2 restricted cytotoxic T lymphocytes
SEQ ID NO:163: Designed peptide recognized by HLA-A2 restricted cytotoxic T lymphocytes
SEQ ID NO:164: Designed peptide recognized by HLA-A2 restricted cytotoxic T lymphocytes
SEQ ID NO:165: Designed peptide recognized by HLA-A2 restricted cytotoxic T lymphocytes
SEQ ID NO:166: Designed peptide recognized by HLA-A2 restricted cytotoxic T lymphocytes
SEQ ID NO:167: Designed peptide recognized by HLA-A2 restricted cytotoxic T lymphocytes
SEQ ID NO:168: Designed peptide recognized by HLA-A2 restricted cytotoxic T lymphocytes
SEQ ID NO:169: Designed peptide recognized by HLA-A2 restricted cytotoxic T lymphocytes
SEQ ID NO:170: Designed peptide recognized by HLA-A2 restricted cytotoxic T lymphocytes
SEQ ID NO:171: Designed peptide recognized by HLA-A2 restricted cytotoxic T lymphocytes
SEQ ID NO:172: Designed peptide recognized by HLA-A2 restricted cytotoxic T lymphocytes
SEQ ID NO:173: Designed peptide recognized by HLA-A2 restricted cytotoxic T lymphocytes
SEQ ID NO:174: Designed peptide recognized by HLA-A2 restricted cytotoxic T lymphocytes
SEQ ID NO:175: Designed peptide recognized by HLA-A2 restricted cytotoxic T lymphocytes
SEQ ID NO:176: Designed peptide recognized by HLA-A2 restricted cytotoxic T lymphocytes
SEQ ID NO:177: Designed peptide recognized by HLA-A2 restricted cytotoxic T lymphocytes
SEQ ID NO:178: Designed peptide recognized by HLA-A2 restricted cytotoxic T lymphocytes
SEQ ID NO:179: Designed peptide recognized by HLA-A2 restricted cytotoxic T lymphocytes

SEQ ID NO:180: Designed peptide recognized by HLA-A2 restricted cytotoxic T lymphocytes
SEQ ID NO:181: Designed peptide recognized by HLA-A2 restricted cytotoxic T lymphocytes
SEQ ID NO:182: Designed peptide recognized by HLA-A2 restricted cytotoxic T lymphocytes
SEQ ID NO:183: Designed peptide recognized by HLA-A2 restricted cytotoxic T lymphocytes
SEQ ID NO:184: Designed peptide recognized by HLA-A2 restricted cytotoxic T lymphocytes
SEQ ID NO:185: Designed peptide recognized by HLA-A2 restricted cytotoxic T lymphocytes
SEQ ID NO:186: Designed peptide recognized by HLA-A2 restricted cytotoxic T lymphocytes
SEQ ID NO:187: Designed peptide recognized by HLA-A2 restricted cytotoxic T lymphocytes
SEQ ID NO:188: Designed peptide recognized by HLA-A2 restricted cytotoxic T lymphocytes
SEQ ID NO:189: Designed peptide recognized by HLA-A2 restricted cytotoxic T lymphocytes
SEQ ID NO:190: Designed peptide recognized by HLA-A2 restricted cytotoxic T lymphocytes
SEQ ID NO:191: Designed peptide recognized by HLA-A2 restricted cytotoxic T lymphocytes
SEQ ID NO:192: Designed peptide recognized by HLA-A2 restricted cytotoxic T lymphocytes
SEQ ID NO:193: Designed peptide recognized by HLA-A2 restricted cytotoxic T lymphocytes
SEQ ID NO:194: Designed peptide recognized by HLA-A2 restricted cytotoxic T lymphocytes
SEQ ID NO:195: Designed peptide recognized by HLA-A2 restricted cytotoxic T lymphocytes
SEQ ID NO:196: Designed peptide recognized by HLA-A2 restricted cytotoxic T lymphocytes
SEQ ID NO:197: Designed peptide recognized by HLA-A2 restricted cytotoxic T lymphocytes
SEQ ID NO:198: Designed peptide recognized by HLA-A2 restricted cytotoxic T lymphocytes
SEQ ID NO:199: Designed peptide recognized by HLA-A2 restricted cytotoxic T lymphocytes
SEQ ID NO:200: Designed peptide recognized by HLA-A2 restricted cytotoxic T lymphocytes
SEQ ID NO:201: Designed peptide recognized by HLA-A2 restricted cytotoxic T lymphocytes
SEQ ID NO:202: Designed peptide recognized by HLA-A2 restricted cytotoxic T lymphocytes
SEQ ID NO:203: Designed peptide recognized by HLA-A2 restricted cytotoxic T lymphocytes
SEQ ID NO:204: Designed peptide recognized by HLA-A2 restricted cytotoxic T lymphocytes
SEQ ID NO:205: Designed peptide recognized by HLA-A2 restricted cytotoxic T lymphocytes
SEQ ID NO:206: Designed peptide recognized by HLA-A2 restricted cytotoxic T lymphocytes
SEQ ID NO:207: Designed peptide recognized by HLA-A2 restricted cytotoxic T lymphocytes
SEQ ID NO:208: Designed peptide recognized by HLA-A2 restricted cytotoxic T lymphocytes
SEQ ID NO:209: Designed peptide recognized by HLA-A2 restricted cytotoxic T lymphocytes
SEQ ID NO:210: Designed peptide recognized by HLA-A2 restricted cytotoxic T lymphocytes
SEQ ID NO:211: Designed peptide recognized by HLA-A2 restricted cytotoxic T lymphocytes
SEQ ID NO:212: Designed peptide recognized by HLA-A2 restricted cytotoxic T lymphocytes
SEQ ID NO:213: Designed peptide recognized by HLA-A2 restricted cytotoxic T lymphocytes
SEQ ID NO:284: "Xaa" may be "Asp" or "Glu".
SEQ ID NO:352: "n" may be "a", "c", "g" or "t".
SEQ ID NO:358: Designed peptide recognized by HLA-A2 restricted cytotoxic T lymphocytes
SEQ ID NO:359: Designed peptide recognized by HLA-A2 restricted cytotoxic T lymphocytes
SEQ ID NO:360: Designed peptide recognized by HLA-A2 restricted cytotoxic T lymphocytes
SEQ ID NO:361: Designed peptide recognized by HLA-A2 restricted cytotoxic T lymphocytes
SEQ ID NO:362: Designed peptide recognized by HLA-A2 restricted cytotoxic T lymphocytes
SEQ ID NO:363: Designed peptide recognized by HLA-A2 restricted cytotoxic T lymphocytes
SEQ ID NO:364: Designed peptide recognized by HLA-A2 restricted cytotoxic T lymphocytes
SEQ ID NO:365: Designed peptide recognized by HLA-A2 restricted cytotoxic T lymphocytes
SEQ ID NO:366: Designed peptide recognized by HLA-A2 restricted cytotoxic T lymphocytes
SEQ ID NO:367: Designed peptide recognized by HLA-A2 restricted cytotoxic T lymphocytes
SEQ ID NO:368: Designed peptide recognized by HLA-A2 restricted cytotoxic T lymphocytes
SEQ ID NO:369: Designed peptide recognized by HLA-A2 restricted cytotoxic T lymphocytes
SEQ ID NO:370: Designed peptide recognized by HLA-A2 restricted cytotoxic T lymphocytes
SEQ ID NO:371: Designed peptide recognized by HLA-A2 restricted cytotoxic T lymphocytes
SEQ ID NO:372: Designed peptide recognized by HLA-A2 restricted cytotoxic T lymphocytes
SEQ ID NO:373: Designed peptide recognized by HLA-A2 restricted cytotoxic T lymphocytes
SEQ ID NO:374: Designed peptide recognized by HLA-A2 restricted cytotoxic T lymphocytes
SEQ ID NO:375: Designed peptide recognized by HLA-A2 restricted cytotoxic T lymphocytes
SEQ ID NO:376: Designed peptide recognized by HLA-A2 restricted cytotoxic T lymphocytes
SEQ ID NO:377: Designed peptide recognized by HLA-A2 restricted cytotoxic T lymphocytes
SEQ ID NO:378: Designed peptide recognized by HLA-A2 restricted cytotoxic T lymphocytes
SEQ ID NO:379: Designed peptide recognized by HLA-A2 restricted cytotoxic T lymphocytes

SEQ ID NO:380: Designed peptide recognized by HLA-A2 restricted cytotoxic T lymphocytes
SEQ ID NO:381: Designed peptide recognized by HLA-A2 restricted cytotoxic T lymphocytes
SEQ ID NO:388: Designed peptide recognized by HLA-A26 restricted cytotoxic T lymphocytes
SEQ ID NO:389: Designed peptide recognized by HLA-A26 restricted cytotoxic T lymphocytes
SEQ ID NO:390: Designed peptide recognized by HLA-A26 restricted cytotoxic T lymphocytes
SEQ ID NO:391: Designed peptide recognized by HLA-A26 restricted cytotoxic T lymphocytes
SEQ ID NO:392: Designed peptide recognized by HLA-A26 restricted cytotoxic T lymphocytes
SEQ ID NO:393: Designed peptide recognized by HLA-A26 restricted cytotoxic T lymphocytes
SEQ ID NO:394: Designed peptide recognized by HLA-A26 restricted cytotoxic T lymphocytes
SEQ ID NO:395: Designed peptide recognized by HLA-A26 restricted cytotoxic T lymphocytes
SEQ ID NO:396: Designed peptide recognized by HLA-A26 restricted cytotoxic T lymphocytes
SEQ ID NO:397: Designed peptide recognized by HLA-A26 restricted cytotoxic T lymphocytes
SEQ ID NO:398: Designed peptide recognized by HLA-A26 restricted cytotoxic T lymphocytes
SEQ ID NO:399: Designed peptide recognized by HLA-A26 restricted cytotoxic T lymphocytes
SEQ ID NO:400: Designed peptide recognized by HLA-A26 restricted cytotoxic T lymphocytes
SEQ ID NO:401: Designed peptide recognized by HLA-A26 restricted cytotoxic T lymphocytes
SEQ ID NO:402: Designed peptide recognized by HLA-A26 restricted cytotoxic T lymphocytes
SEQ ID NO:403: Designed peptide recognized by HLA-A26 restricted cytotoxic T lymphocytes
SEQ ID NO:404: Designed peptide recognized by HLA-A26 restricted cytotoxic T lymphocytes
SEQ ID NO:405: Designed peptide recognized by HLA-A26 restricted cytotoxic T lymphocytes
SEQ ID NO:406: Designed peptide recognized by HLA-A26 restricted cytotoxic T lymphocytes
SEQ ID NO:407: Designed peptide recognized by HLA-A26 restricted cytotoxic T lymphocytes
SEQ ID NO:408: Designed peptide recognized by HLA-A26 restricted cytotoxic T lymphocytes

## Claims

1. A peptide consisting of an amino acid sequence selected from the group consisting of SEQ ID NO:1 to SEQ ID NO:213, SEQ ID NO:358 to SEQ ID NO:381, and SEQ ID NO:388 to SEQ ID NO:408 in the sequence listing.

2. A polypeptide consisting of an amino acid sequence selected from the group consisting of SEQ ID NO:215 to SEQ ID NO:288 (excluding SEQ ID NO:228, SEQ ID NO:236, SEQ ID NO:261, and SEQ ID NO:269), SEQ ID NO:356, SEQ ID NO:357, SEQ ID NO:385, and SEQ ID NO:386 in the sequence listing.

3. A peptide consisting of an amino acid sequence selected from the group consisting of SEQ ID NO:1 to SEQ ID NO:213, SEQ ID NO:358 to SEQ ID NO:381, and SEQ ID NO:388 to SEQ ID NO:408 in the sequence listing, wherein the peptide is recognized by a cytotoxic T lymphocyte and/or induces a cytotoxic T lymphocyte.

4. The peptide according to claim 3, wherein that being recognized by a cytotoxic T lymphocyte and/or inducing a cytotoxic T lymphocyte is that being recognized by a cytotoxic T lymphocyte in an HLA-A2-restricted manner or HLA-A26-restricted manner and/or inducing a cytotoxic T lymphocyte in an HLA-A2-restricted manner or HLA-A26-restricted manner.

5. A polypeptide consisting of an amino acid sequence selected from the group consisting of SEQ ID NO:214 to SEQ ID NO:288, SEQ ID NO:356, SEQ ID NO:357, and SEQ ID NO:385 to SEQ ID NO:387 in the sequence listing, wherein the polypeptide is recognized by a cytotoxic T lymphocyte and/or induces a cytotoxic T lymphocyte.

6. The polypeptide according to claim 5, wherein that being recognized by a cytotoxic T lymphocyte and/or inducing a cytotoxic T lymphocyte is that being recognized by a cytotoxic T lymphocyte in an HLA-A2-restricted manner or HLA-A26-restricted manner and/or inducing a cytotoxic T lymphocyte in an HLA-A2-restricted manner or HLA-A26-restricted manner..

7. A medicament consisting of one or more of peptides that consist of an amino acid sequence selected from the group consisting of SEQ ID NO:1 to SEQ ID NO:213, SEQ ID NO:358 to SEQ ID NO:381, and SEQ ID NO:388 to SEQ ID NO:408 in the sequence listing, and/or, one or more of polypeptides that consist of an amino acid sequence selected from the group consisting of SEQ ID NO:214 to SEQ ID NO:288, SEQ ID NO:356, SEQ ID NO:357, and SEQ ID NO:385 to SEQ ID NO:387 in the sequence listing.

**8.** A cancer vaccine comprising one or more of peptides that consist of an amino acid sequence selected from the group consisting of SEQ ID NO:1 to SEQ ID NO:213, SEQ ID NO:358 to SEQ ID NO:381, and SEQ ID NO:388 to SEQ ID NO:408 in the sequence listing, and/or, one or more of polypeptides that consist of an amino acid sequence selected from the group consisting of SEQ ID NO:214 to SEQ ID NO:288, SEQ ID NO:356, SEQ ID NO:357, and SEQ ID NO:385 to SEQ ID NO:387 in the sequence listing.

**9.** The cancer vaccine according to claim 8, wherein the vaccine is used for treating one or more of cancers selected from the group consisting of colon cancer, esophageal cancer, oral squamous cell cancer, renal cancer, pulmonary cancer, gynecological cancer, and prostate cancer.

**10.** An inducer of a cytotoxic T lymphocyte, wherein the inducer comprising one or more of peptides that consist of an amino acid sequence selected from the group consisting of SEQ ID NO:1 to SEQ ID NO:213, SEQ ID NO:358 to SEQ ID NO:381, and SEQ ID NO:388 to SEQ ID NO:408 in the sequence listing, and/or, one or more of polypeptides that consist of an amino acid sequence selected from the group consisting of SEQ ID NO:214 to SEQ ID NO:288, SEQ ID NO:356, SEQ ID NO:357, and SEQ ID NO:385 to SEQ ID NO:387 in the sequence listing.

**11.** A method for inducing a cytotoxic T lymphocyte, wherein the method comprises using one or more of peptides that consist of an amino acid sequence selected from the group consisting of any one of SEQ ID NO:1 to SEQ ID NO:213, SEQ ID NO:358 to SEQ ID NO:381 in the sequence listing, and SEQ ID NO:388 to SEQ ID NO:408, and/or, one or more of polypeptides that consist of an amino acid sequence selected from the group consisting of any one of SEQ ID NO:214 to SEQ ID NO:288, SEQ ID NO:356, SEQ ID NO:357, and SEQ ID NO:385 to SEQ ID NO:387 in the sequence listing.

**12.** A polynucleotide encoding a peptide or polypeptide consisting of an amino acid sequence selected from the group consisting of SEQ ID NO:1 to SEQ ID NO:288, SEQ ID NO:356 to SEQ ID NO:381, and SEQ ID NO:385 to SEQ ID NO:408 in the sequence listing, or a complementary strand thereof.

**13.** A polynucleotide consisting of a nucleotide sequence selected from the group consisting of SEQ ID NO:290 to SEQ ID NO:355 (excluding SEQ ID NO:299 and SEQ ID NO:332) and SEQ ID NO:382 to SEQ ID NO:384 in the sequence listing, or a complementary strand thereof.

**14.** A polynucleotide consisting of a nucleotide sequence selected from the group consisting of SEQ ID NO:289 to SEQ ID NO:355 and SEQ ID NO:382 to SEQ ID NO:384 in the sequence listing, or a complementary strand thereof, wherein a polypeptide encoded by the polynucleotide induces a cytotoxic T lymphocyte and/or is recognized by a cytotoxic T lymphocyte.

**15.** The polynucleotide or the complementary strand thereof according to claim 14, wherein that being recognized by a cytotoxic T lymphocyte and/or inducing a cytotoxic T lymphocyte is that being recognized by a cytotoxic T lymphocyte in an HLA-A2-restricted manner or HLA-A26-restricted manner and/or inducing a cytotoxic T lymphocyte in an HLA-A2-restricted manner or HLA-A26-restricted manner.

**16.** A polynucleotide that hybridizes to the polynucleotide or the complementary strand thereof according to any one of claims 12 to 15 under stringent conditions.

**17.** A recombinant vector comprising the polynucleotide or the complementary strand thereof according to any one of claims 12 to 16.

**18.** The recombinant vector according to claim 17, wherein the recombinant vector is a recombinant expression vector.

**19.** A transformant transformed with the recombinant vector according to claim 17 or 18.

**20.** A method for producing the peptide according to claim 1, 3, or 4 or the polypeptide according to claim 2, 5, or 6, wherein the method comprises culturing a transformant transformed with the recombinant vector according to claim 18.

**21.** An antibody immunologically recognizing the peptide according to claim 1, 3, or 4, or the polypeptide according to claim 2, 5, or 6.

**22.** A method for screening for a compound that enhances recognition of the peptide according to claim 4 or the polypeptide according to claim 6 at least by an HLA-A2-restricted or HLA-A26-restricted cytotoxic T cell through interacting with the peptide or the polypeptide and/or a HLA-A2 molecule or HLA-A26 molecule, and/or a compound that enhances expression of the polynucleotide or the complementary strand thereof according to any one of claims 12 to 15 through interacting with the same, wherein the method comprises using at least one selected from the group consisting of the peptide according to claim 4, the polypeptide according to claim 6, the polynucleotide or the complementary strand thereof according to any one of claims 12 to 15, the recombinant vector according to claim 17 and 18, the transformant according to claim 19 and the antibody according to claim 21.

**23.** A compound that is obtained by the screening method according to claim 22.

**24.** A compound that enhances recognition of at least one of the peptide according to claim 4 and/or the polypeptide according to claim 6 by an HLA-A2-restricted or HLA-A26-restricted cytotoxic T cell, or a compound that enhances the expression of the polynucleotide or the complementary strand thereof according to any one of claims 12 to 15 through interacting with the same.

**25.** A pharmaceutical composition used for treating cancers, the composition comprising at least one selected from the group consisting of the peptide according to claim 1, 3 or 4, the polypeptide according to claim 2, 5 or 6, the polynucleotide or the complementary strand thereof according to any of claims 12 to 15, the recombinant vector according to claim 17 or 18, the transformant according to claim 19, the antibody according to claim 21, and the compound according to claim 23 or 24.

**26.** A method for measuring quantitatively or qualitatively the peptide according to claim 1, 3, or 4, the polypeptide according to claim 2, 5, or 6, or the polynucleotide according to any one of claims 12 to 15.

**27.** The measuring method according to claim 26, wherein the method is used in an examination for a cancer disease.

**28.** A reagent kit for use in the screening method according to claim 22 or the measuring method according to claim 26, wherein the reagent kit comprises at least one selected from the group consisting of the peptide according to claim 1, 3 or 4, the polypeptide according to claim 2, 5 or 6, the polynucleotide or the complementary strand thereof according to any one of claims 12 to 15, and the antibody according to claim 21.

**29.** The reagent kit according to claim 28, wherein the reagent kit is used in an examination for a cancer disease.

Figure 1

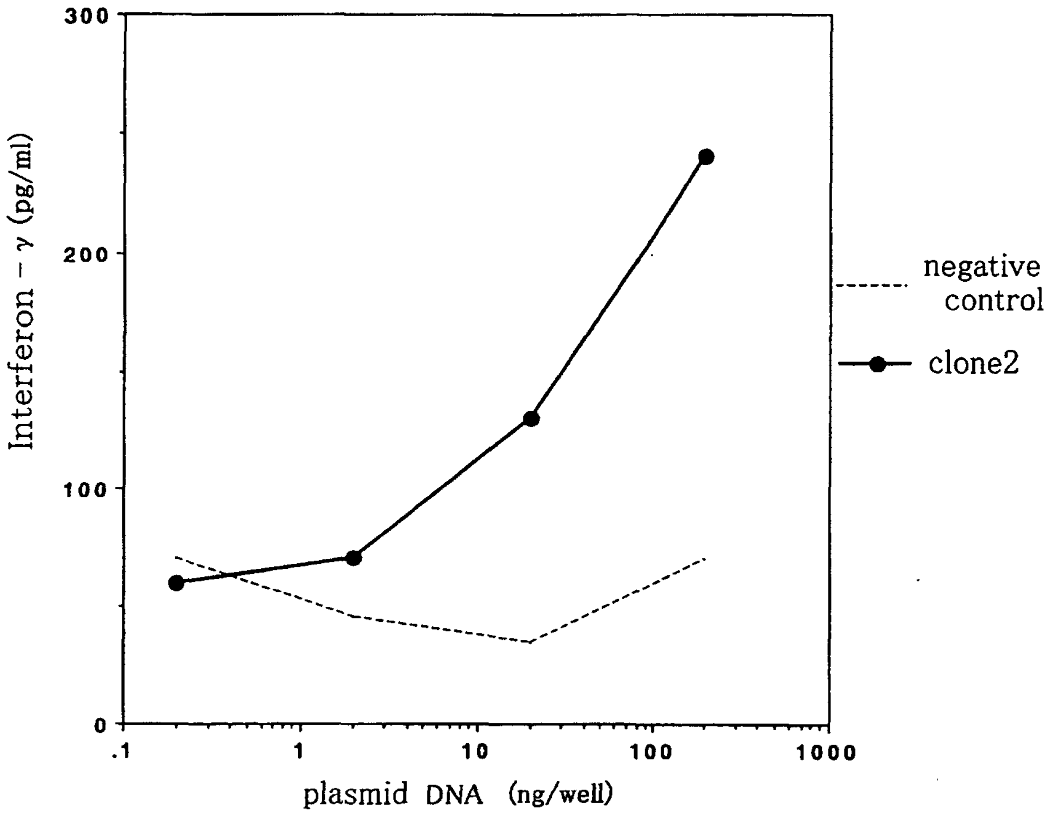


Figure 2

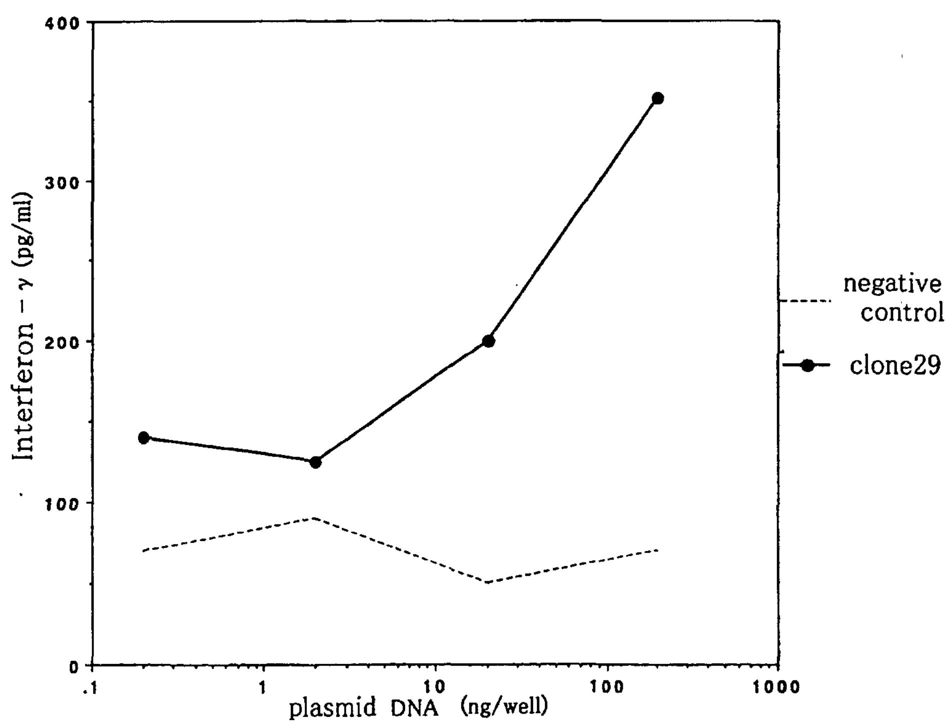

Figure 3

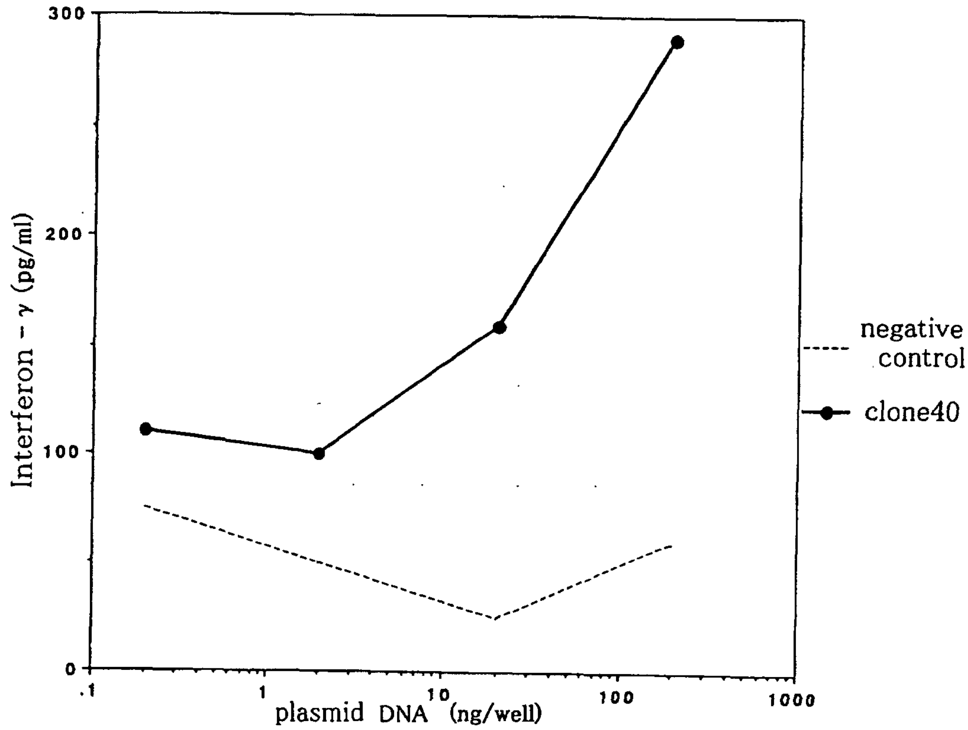


Figure 4

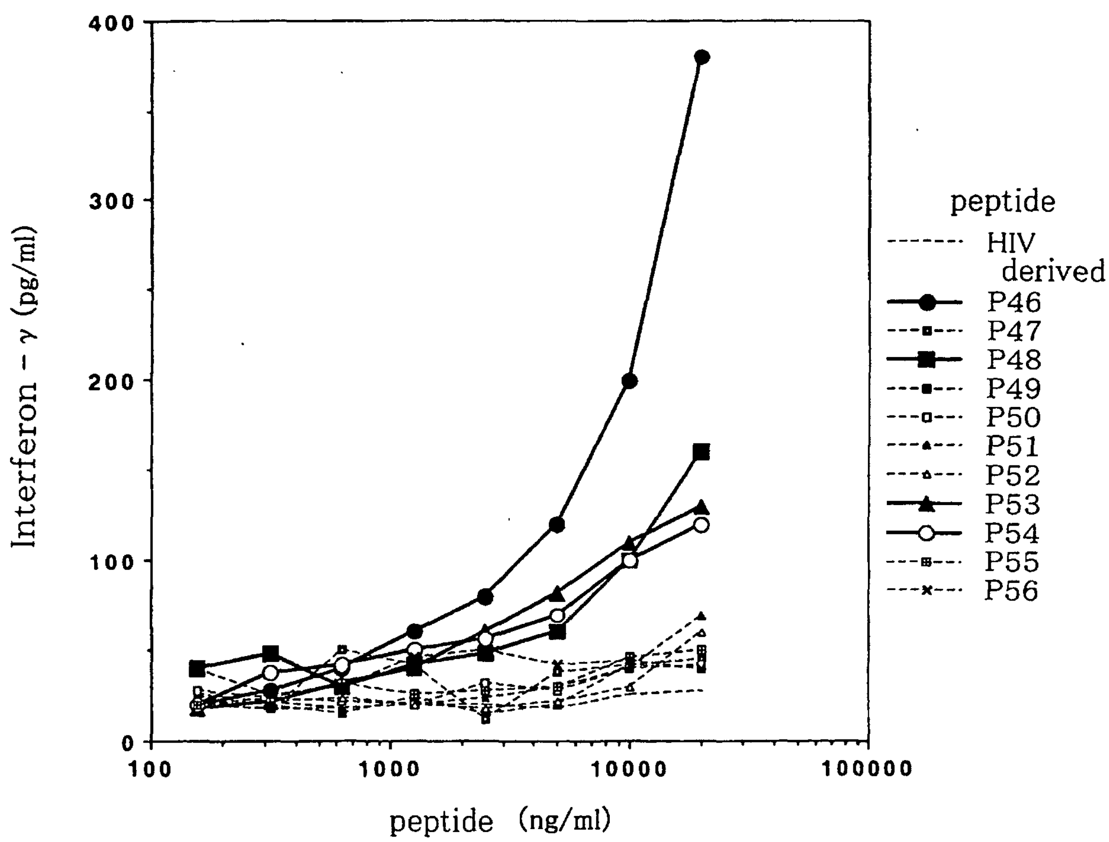

Figure 5

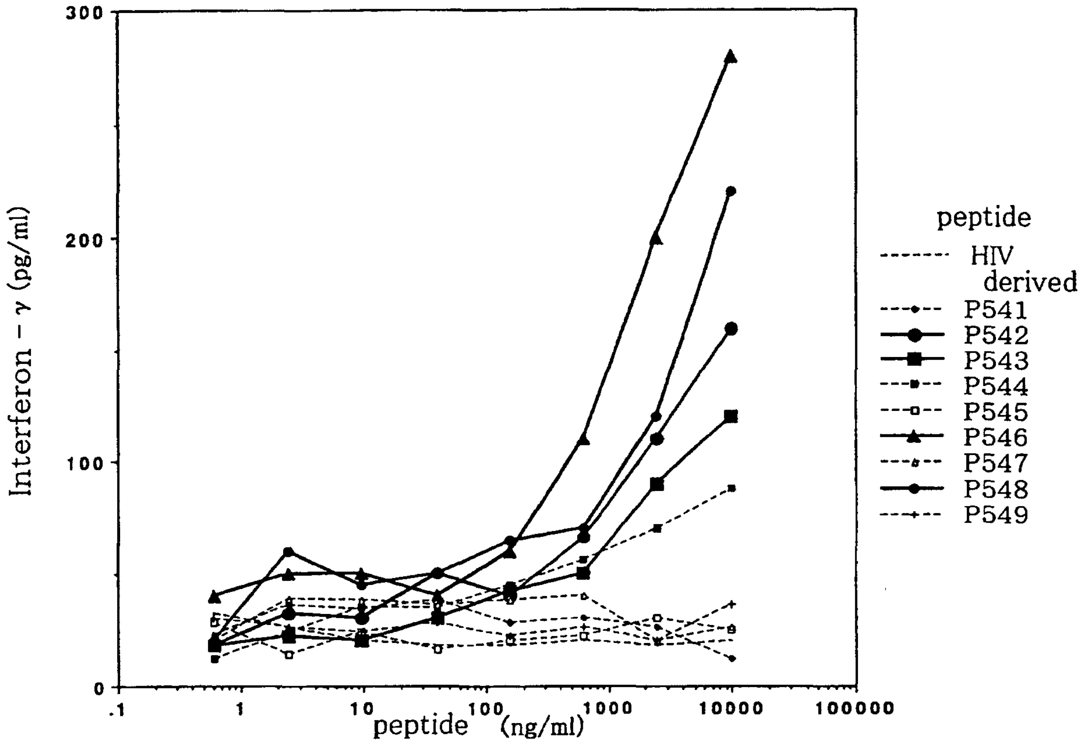


Figure 6

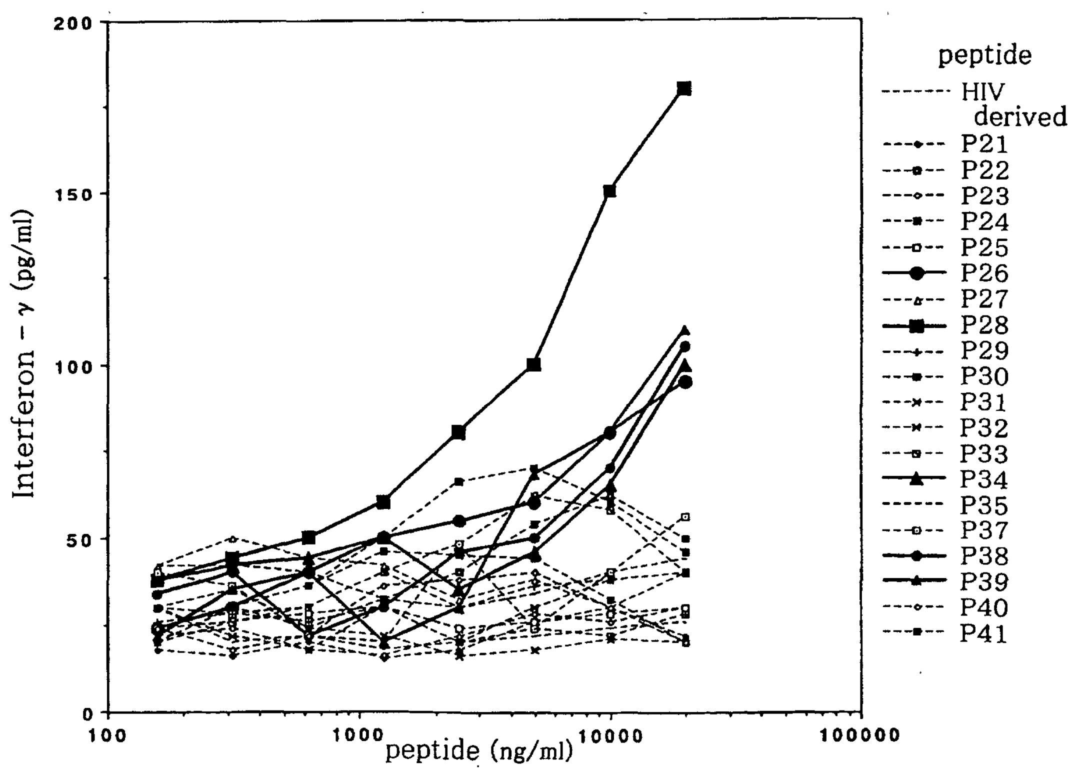

Figure 7a

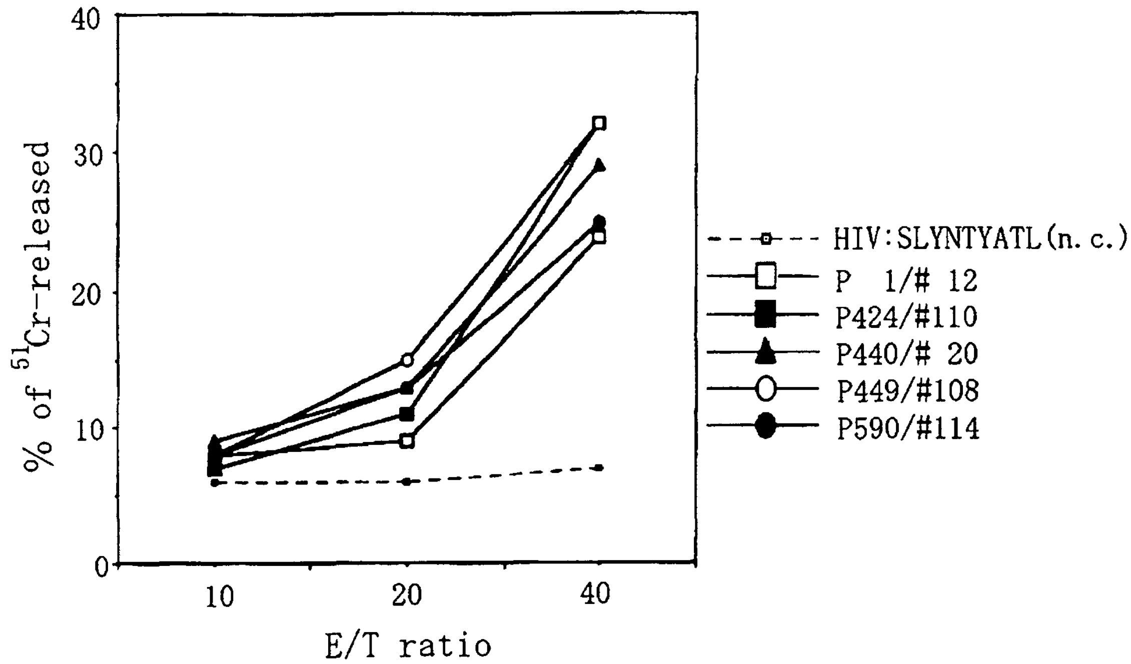


Figure 7b

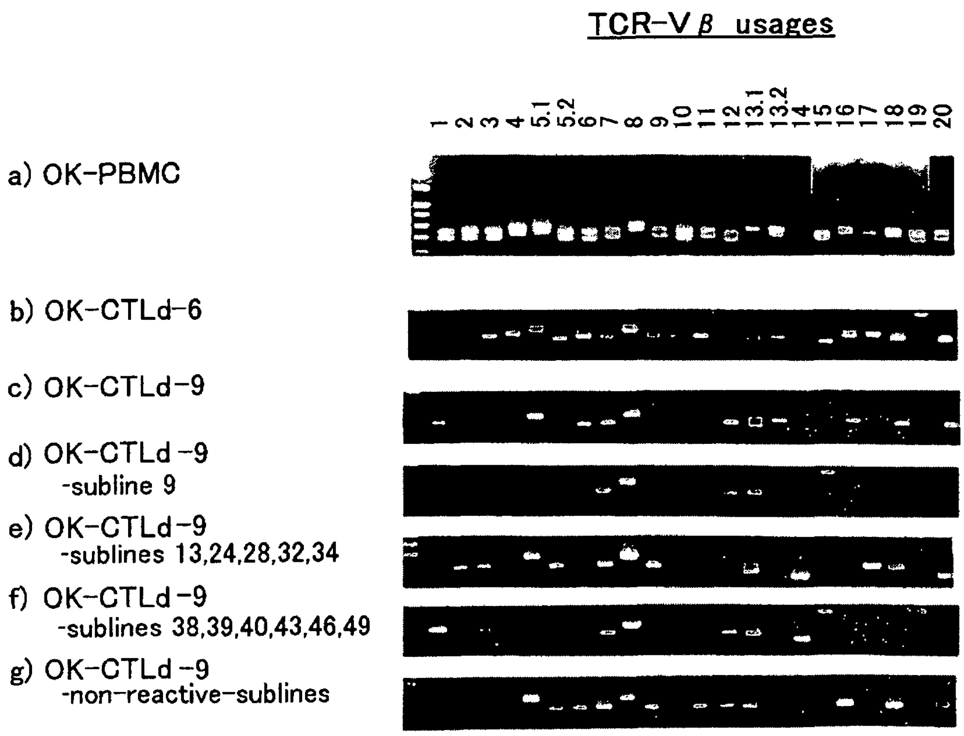

Figure 8a

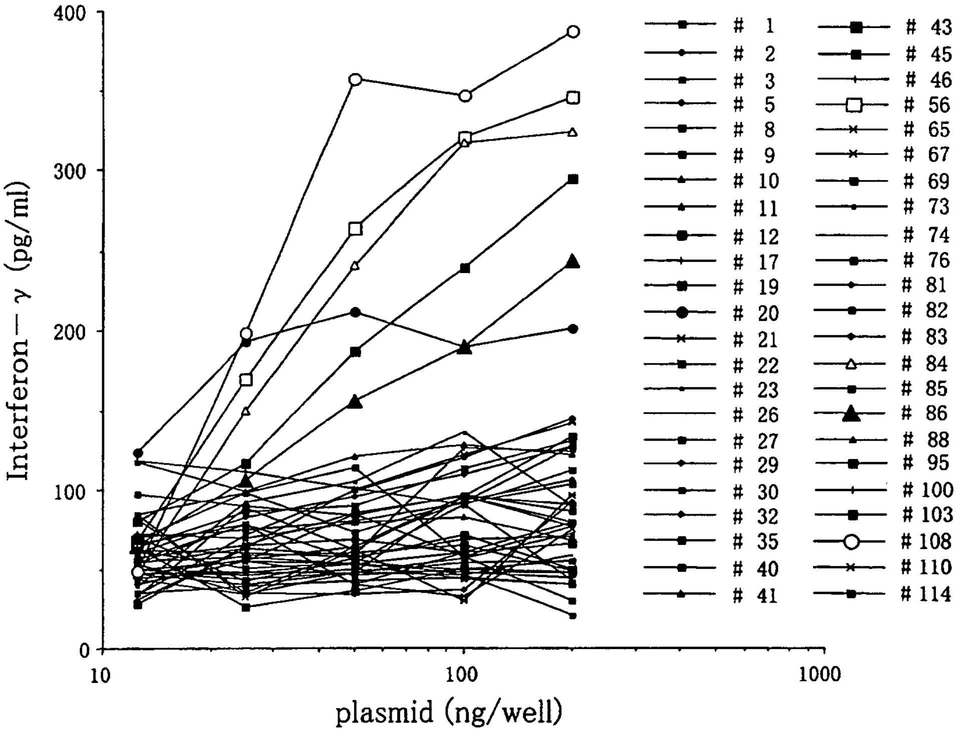


Figure 8b

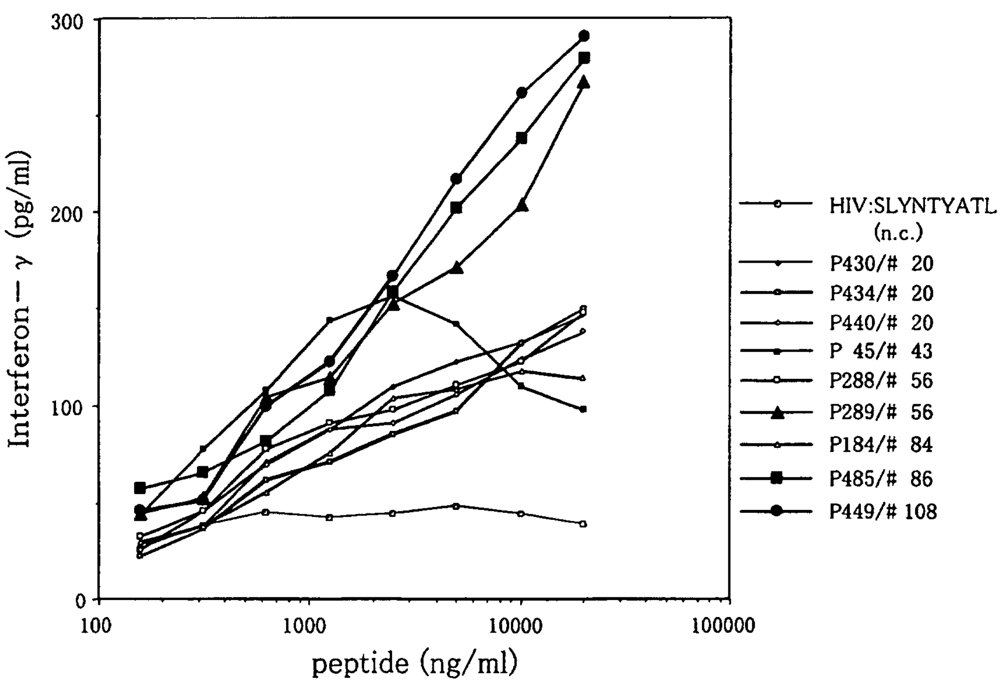

Figure 9

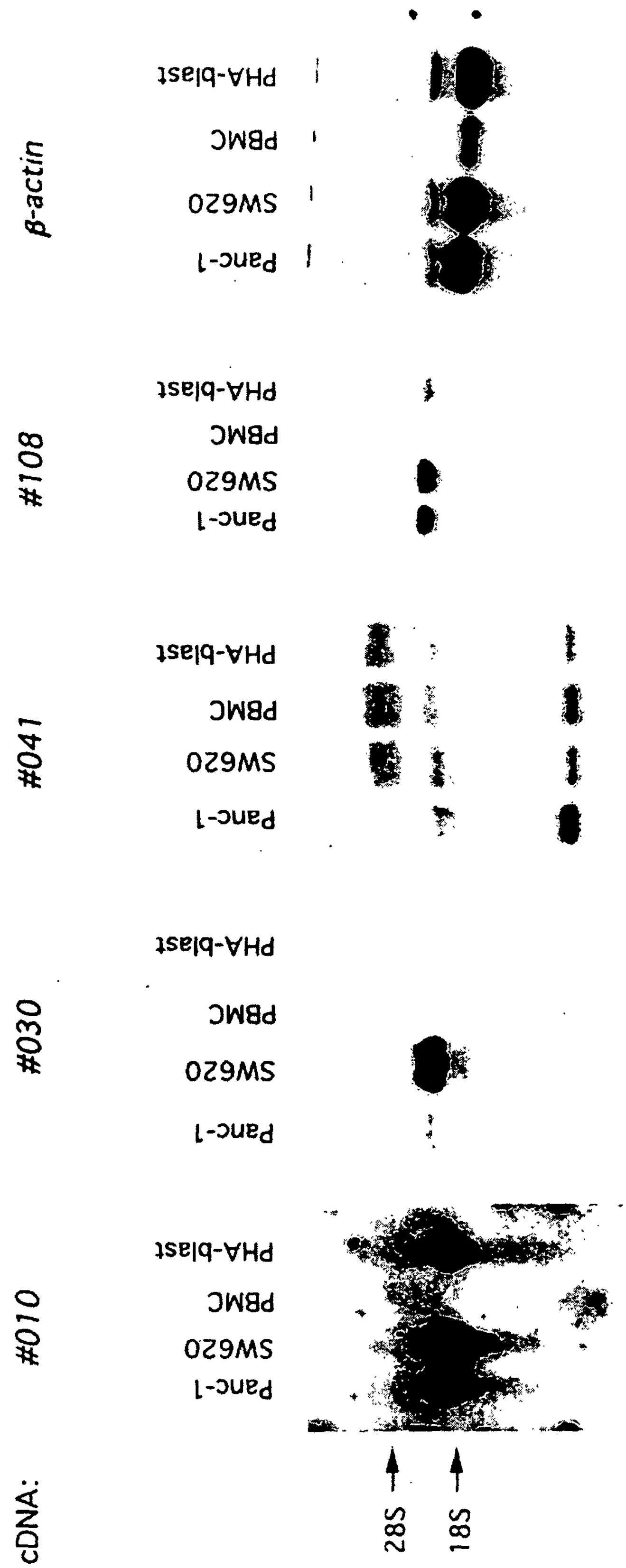
β-actin
PHA-blast
PBMC
SW620
Panc-1
#108
PHA-blast
PBMC
SW620
Panc-1
#041
PHA-blast
PBMC
SW620
Panc-1
#030
PHA-blast
PBMC
SW620
Panc-1
#010
PHA-blast
PBMC
SW620
Panc-1
cDNA:
28S
18S

Figure 10a

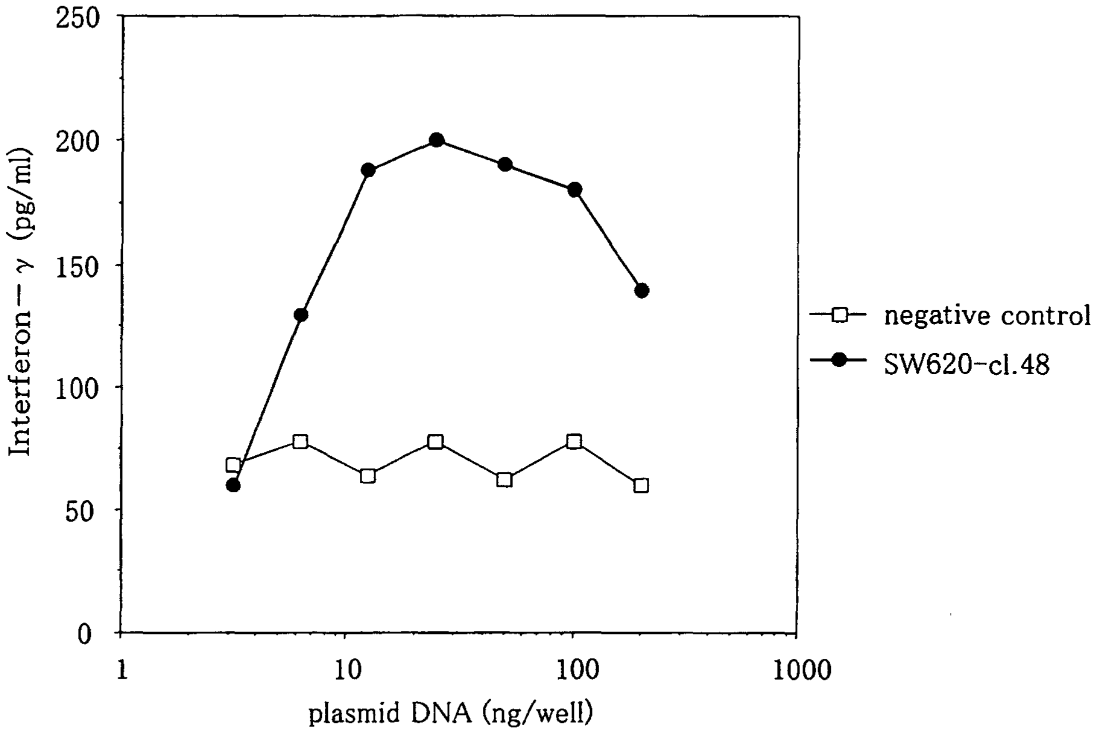


Figure 10b

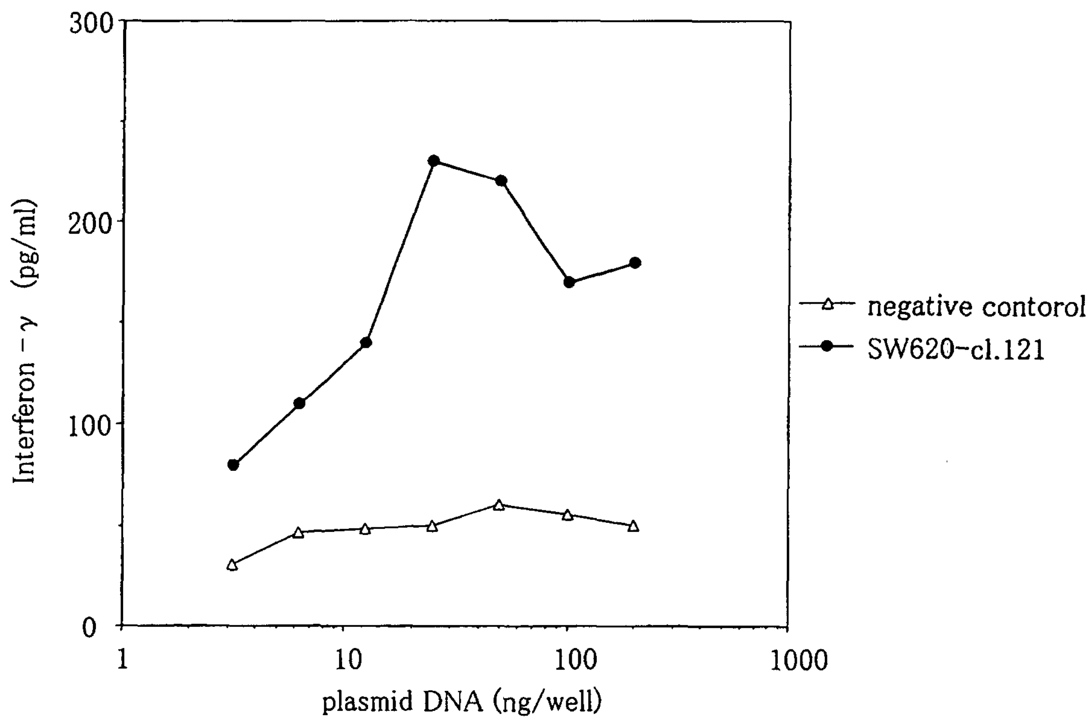

Figure 11

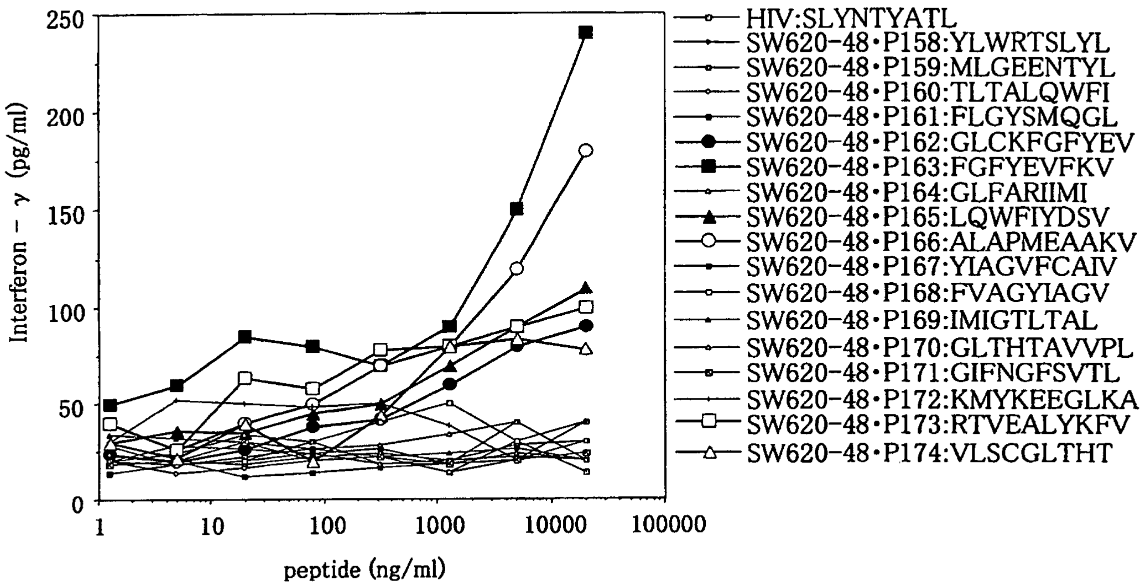


Figure 12

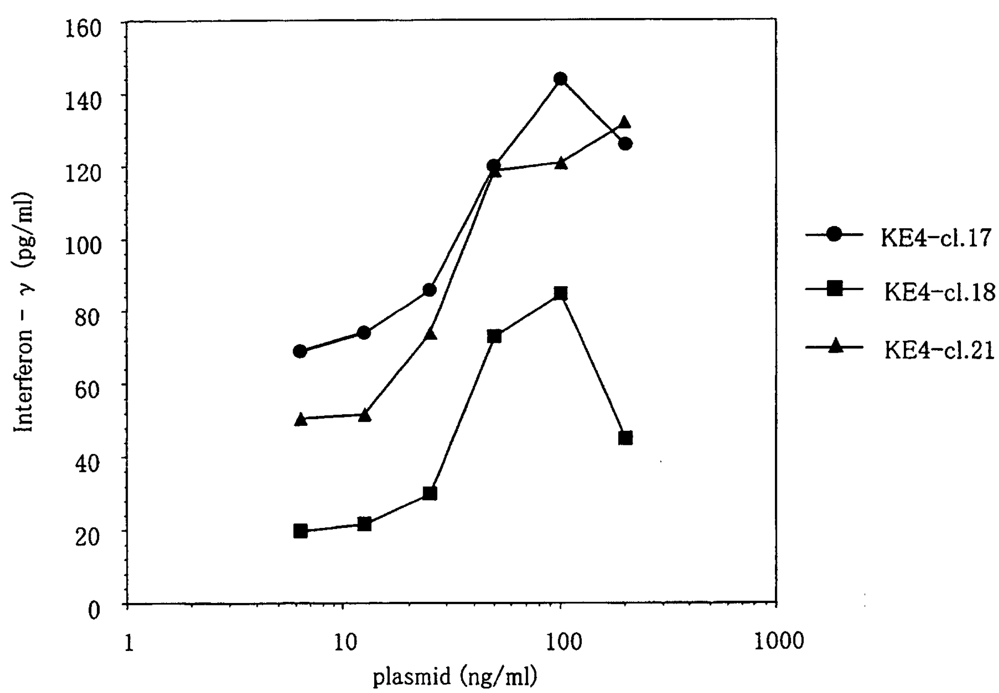

Figure 13a

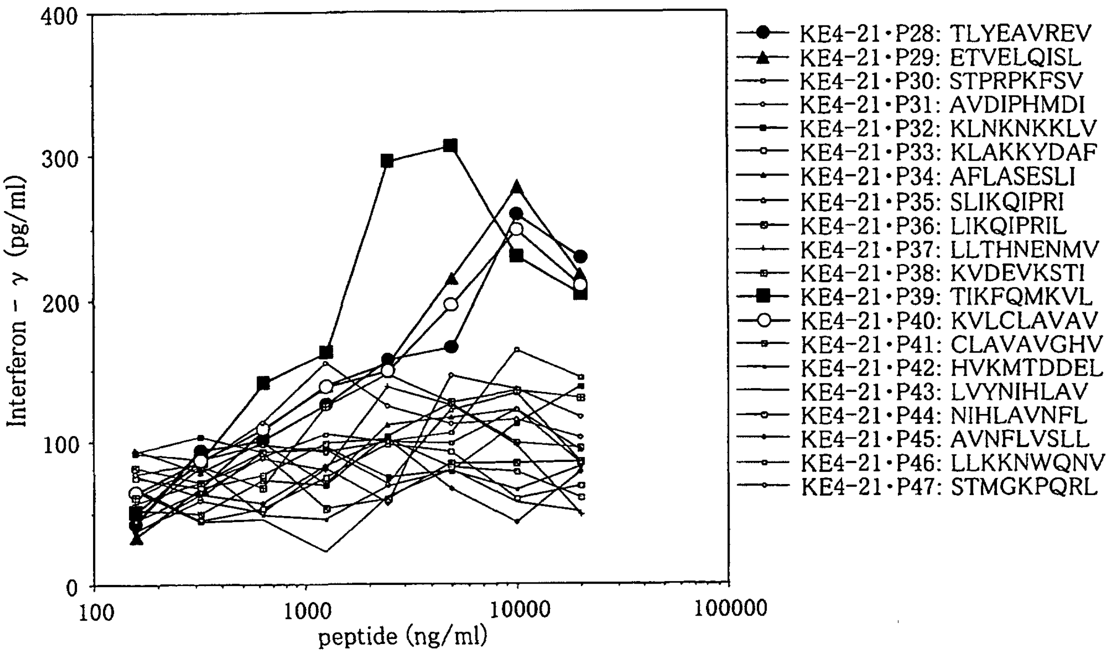


Figure 13b

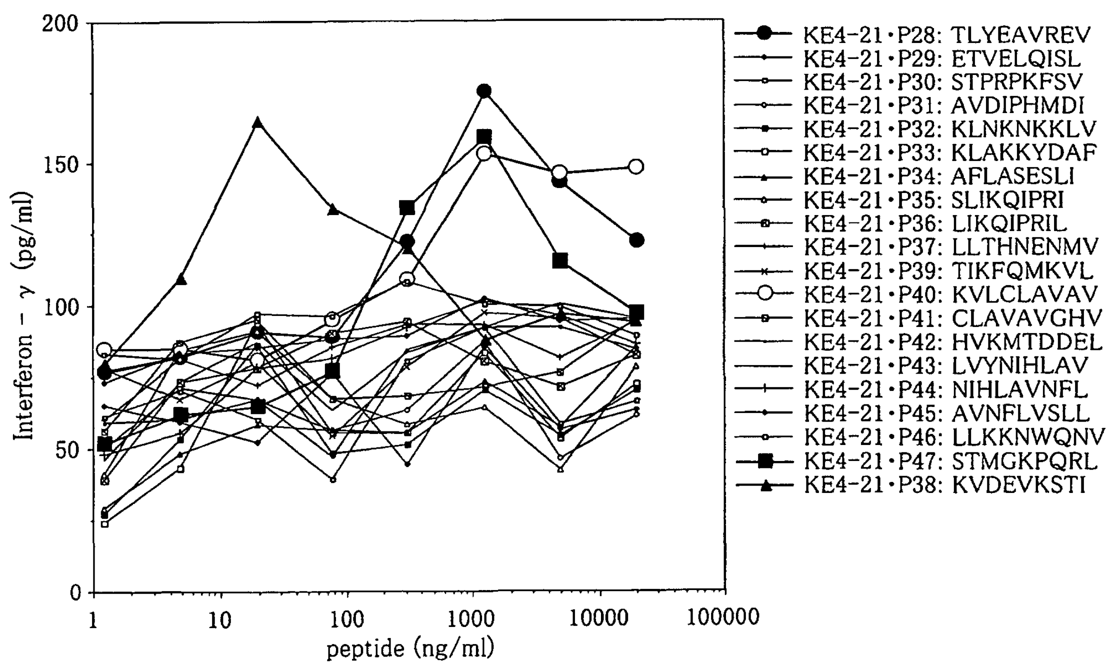

Figure 14a

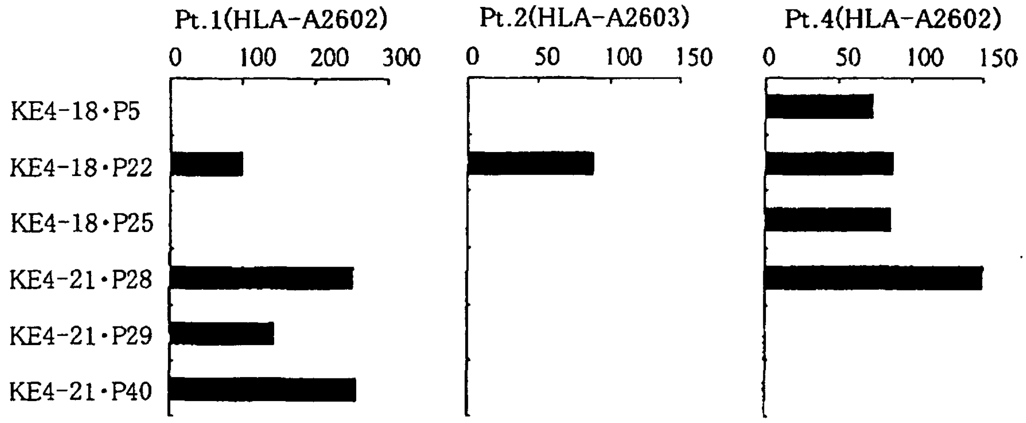


Figure 14b

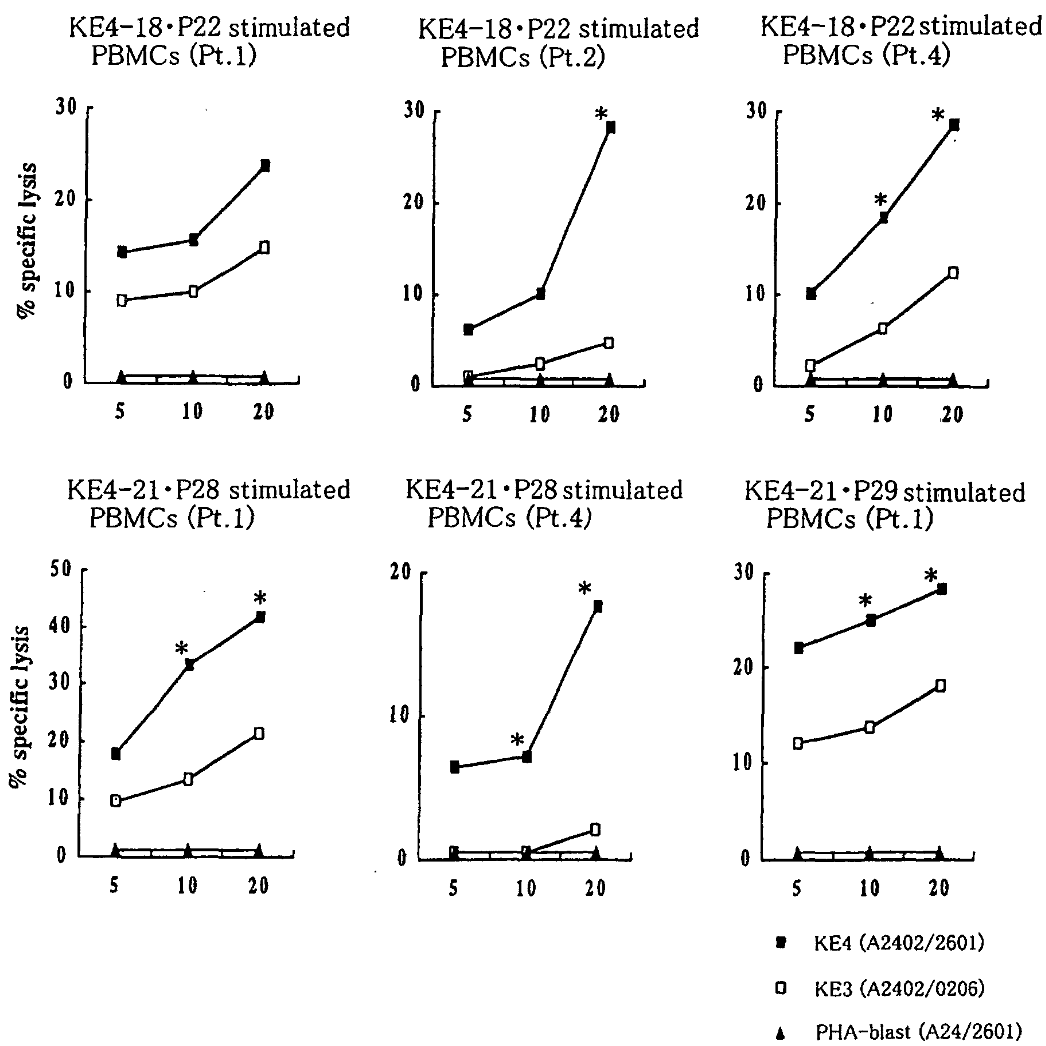

**INTERNATIONAL SEARCH REPORT**

International application No.
PCT/JP02/05799

A. CLASSIFICATION OF SUBJECT MATTER
Int.Cl[7] C07K14/705, A61K38/08, C12N15/12, A61K38/17, A61P35/00, C12P21/02, C07K16/30, G01N33/50, G01N33/15, C12Q1/68

According to International Patent Classification (IPC) or to both national classification and IPC

B. FIELDS SEARCHED

Minimum documentation searched (classification system followed by classification symbols)
Int.Cl[7] C07K14/705, A61K38/08, C12N15/12, A61K38/17, A61P35/00, C12P21/02, C07K16/30, G01N33/50, G01N33/15, C12Q1/68

Documentation searched other than minimum documentation to the extent that such documents are included in the fields searched

Electronic data base consulted during the international search (name of data base and, where practicable, search terms used)
SwissProt/PIR/GeneSeq, WPI(DIALOG), BIOSIS(DIALOG)

C. DOCUMENTS CONSIDERED TO BE RELEVANT

| Category* | Citation of document, with indication, where appropriate, of the relevant passages | Relevant to claim No. |
|---|---|---|
| A | Shinya GOMI et al., A Cycloplilin B Gene Encodes Antigenic Epitopes Recognized by HLA-A24-Restricted and Tumor-Specific CTLs[1]., The Journal of Immunology (1999), Vol.163, pages 4994 to 5004 | 1,3,4,7-12, 16-22,25-29 |
| A | Megumi KIKUCHI et al., Identification of a Sart-1-Derived Peptide Capablé of Inducing HLA-A24-Restricted and Tumor-Specific Cytotoxic T Lymphocytes., Int. J.Cancer (1999), Vol.81, No.3, pages 459 to 466 | 1,3,4,7-12, 16-22,25-29 |
| P,A | WO 01/75067 A2 (Hyseq, Inc.), 11 October, 2001 (11.10.01), (Family: none) | 1,3,4,7-12, 16-22,25-29 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 September, 2002 (18.09.02) | 08 October, 2002 (08.10.02) |
| Name and mailing address of the ISA/ Japanese Patent Office | Authorized officer |
| Facsimile No. | Telephone No. |

Form PCT/ISA/210 (second sheet) (July 1998)

INTERNATIONAL SEARCH REPORT

International application No.
PCT/JP02/05799

Continuation of Box No.I-2 of continuation of first sheet(1)

at the point of the application is taken into consideration, it is completely unknown what specific compounds are involved in the scope thereof and what are not. Thus, these claims are described in an extremely unclear manner. The same applies to the part of claim 25 depending on claims 23 and 24.

Continuation of Box No.II of continuation of first sheet(1)

It is therefore recognized that there are groups of inventions respectively consisting of 338 (poly)peptides represented by SEQ ID NOS:1 to 213, 358 to 381, 388 to 408, 214 to 283, 356, 357 and 385 to 387 and inventions (drugs, vaccines, polynucleotides, etc.) relating to respective (poly)peptides.

Such being the case, the claims have 338 inventions.

Form PCT/ISA/210 (extra sheet) (July 1998)

## INTERNATIONAL SEARCH REPORT

International application No.
PCT/JP02/05799

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 23, 24 and part of 25
because they relate to parts of the international application that do not comply with the prescribed requirements to such an extent that no meaningful international search can be carried out, specifically:
Concerning a compound specified by the screening method as set forth in claim 23 and the compound specified exclusively by the function as set forth in claim 24, no specific compound is presented in the description. Even though the common technical knowledge (continued to extra sheet)

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:
As the applicant approves in the description, a peptide recognized by cytotoxic T lymphocytes had been publicly known. Also, a peptide recognized by HLA-A26 or HLA-A2 restricted cytotoxic T lymphocytes had been also known. See, if necessary, J. Immunol., Vol. 163, No. 9, P. 4994-5004 (1999), J. Exp. Med., Vol. 187, P. 277-288 (1998), Cancer Res., Vol. 55, No. 19, P. 4248-4252 (1995), and Int. J. Cancer, Vol. 81, No.3, P. 459-466 (1999).
Thus, "a peptide recognized by HLA-A2 or HLA-A26 restricted cytotoxic T lymphocytes" cannot be considered as "a special technical feature". Moreover, it cannot be considered that there is a significant homology among the (poly)peptides as set forth in claims 1 to 6. (Continued to extra sheet.)

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
The peptide of SEQ ID NO:1 and inventions relating thereto.

**Remark on Protest** ☐ The additional search fees were accompanied by the applicant's protest.
☐ No protest accompanied the payment of additional search fees.

Form PCT/ISA/210 (continuation of first sheet (1)) (July 1998)